# EUROPEAN PATENT APPLICATION

(11) **EP 1 767 632 A2**
(43) Date of publication of application: **28.03.2007**
(21) Application number: 06021209.9
(22) Date of filing: 29.05.2002
(51) Int. Cl.: C12N 15/11, A61K 31/713, C07H 21/04

(54) **A method for local administration of synthetic double-stranded oligonucleotides targeting a VEGF receptor**

(30) Priority: 29.05.2001 US 870161; 30.11.2001 US 334461 P; 03.05.2002 US 138674
(62) Divisional of application: 02752028.7
(71) Applicant: SIRNA THERAPEUTICS, INC., Boulder, CO 80301 (US); Novartis Vaccines and Diagnostics, Inc., Emeryville, CA 94608 (US)
(72) Inventor: Escobedo, Jaime, Alamo, CA 94507 (US); Pavco, Pamela, Lafayette, CO 80026 (US); Sandburg, Jennifer, Westminister, CO 80021 (US); McSwiggen, James, Boulder, CO 80301 (US); Stinchcomb, Dan, Ft. Collins, CO 80528 (US); Gordon, Gilad, Boulder, CO 50305 (US)
(74) Representative: Maschio, Antonio

(57) **Abstract**

The present invention relates to nucleic acid molecules, including dsRNA, siRNA, antisense, 2,5-A chimeras, aptamers, and enzymatic nucleic acid molecules, such as hammerhead ribozymes, DNAzymes, and allozymes, which modulate the expression of vascular endothelial growth factor receptor (VEGF) and/or vascular endothelial growth factor receptor (VEGFr) genes for the treatment and/or diagnosis of diseases and conditions associated with angiogenesis, such as cancer, tumor angiogenesis, or ocular indications such as diabetic retinopathy, or age related macular degeneration, proliferative diabetic retinopathy, hypoxia-induced angiogenesis, rheumatoid arthritis, psoriasis, wound healing, and female reproductive disorders and conditions, including but not limited to endometriosis, endometrial carcinoma, gynecologic bleeding disorders, irregular menstrual cycles, ovulation, premenstrual syndrome (PMS), and menopausal dysfunction.

## Description

This patent application claims priority from Sandberg et al., USSN 60/334,461, filed November 30, 2001, entitled "Method and Reagent for the Modulation of Female Reproductive Diseases and Conditions" and Pavco et al., USSN 10/138,674, filed May 3, 2002, which is a continuation in part of Pavco et al., USSN 09/870,161, which is a continuation-in-part of Pavco et al., USSN 09/708,690, filed November 7, 2000, which is a continuation-in-part of Pavco et al., USSN 09/371,722, filed August 10, 1999, which is a continuation-in-part of Pavco et al., USSN 08/584,040, filed January 11, 1996, which claims the benefit of Pavco et al., USSN 60/005,974, filed on October 26, 1995; these earlier applications are entitled "Method and Reagent for Treatment of Diseases or Conditions Related to Levels of Vascular Endothelial Growth Factor Receptor". Each of these applications is hereby incorporated by reference herein in it's entirety including the drawings and tables.

### Technical Field Of The Invention

This invention relates to methods and reagents for the treatment of diseases or conditions relating to the levels of expression of vascular endothelial growth factor (VEGF) and vascular endothelial growth factor receptor(s). Specifically, the instant invention features nucleic-acid based molecules and methods that modulate the expression of vascular endothelial growth factor and/or vascular endothelial growth factor receptors, such as VEGFR1 and/or VEGFR2, that are useful in preventing, treating, controlling and/or diagnosing disorders and conditions related to angiogenesis, including but not limited to cancer, tumor angiogenesis, or ocular indications such as diabetic retinopathy, or age related macular degeneration, proliferative diabetic retinopathy, hypoxia-induced angiogenesis, rheumatoid arthritis, psoriasis, wound healing, endometriosis, endometrial carcinoma, gynecologic bleeding disorders, irregular menstrual cycles, ovulation, premenstrual syndrome (PMS), and menopausal dysfunction.

### Background Of The Invention

The following is a discussion of relevant art, none of which is admitted to be prior art to the present invention.

VEGF, also referred to as vascular permeability factor (VPF) and vasculotropin, is a potent and highly specific mitogen of vascular endothelial cells (for a review see Ferrara, 1993 Trends Cardiovas. Med 3, 244; Neufeld et al., 1994, Prog. Growth Factor Res. 5, 89). VEGF-induced neovascularization is implicated in various pathological conditions such as tumor angiogenesis, or ocular indications such as diabetic retinopathy, or age related macular degeneration, proliferative diabetic retinopathy, hypoxia-induced angiogenesis, rheumatoid arthritis, psoriasis, wound healing and others.

VEGF, an endothelial cell-specific mitogen, is a 34-45 kDa glycoprotein with a wide range of activities that include promotion of angiogenesis, enhancement of vascular-permeability and others. VEGF belongs to the platelet-derived growth factor (PPGF) family of growth factors with approximately 18% homology with the A and B chain of PDGF at the amino acid level. Additionally, VEGF contains the eight conserved cysteine residues common to all growth factors belonging to the PDGF family (Neufeld *et al., supra).* VEGF protein is believed to exist predominantly as disulfide-linked homodimers; monomers of VEGF have been shown to be inactive (Plouet et al., 1989 EMBO J. 8, 3801).

VEGF exerts its influence on vascular endothelial cells by binding to specific high-affinity cell surface receptors. Covalent cross-linking experiments with ¹²⁵I-labeled VEGF protein have led to the identification of three high molecular weight complexes of 225, 195 and 175 kDa presumed to be VEGF and VEGF receptor complexes (Vaisman et al., 1990 J. Biol. Chem. 265, 19461). Based on these studies VEGF-specific receptors of 180, 150 and 130 kDa molecular mass were predicted. In endothelial cells, receptors of 150 and 130 kDa have been identified. The VEGF receptors belong to the superfamily of receptor tyrosine kinases (RTKs) characterized by a conserved cytoplasmic catalytic kinase domain and a hydrophilic kinase sequence. The extracellular domains of the VEGF receptors consist of seven immunoglobulin-Iike domains that are thought to be involved in VEGF binding functions.

The two most abundant and high-affnity receptors of VEGF are flt-1 (VEGFR1) (*fms-*like tyrosine kinase) cloned by Shibuya et al., 1990 Oncogene 5, 519 and KDR (VEGFR2) (kinase-insert-domain-containing receptor) cloned by Terman et al., 1991 Oncogene 6, 1677. The murine homolog of KDR, cloned by Mathews et al., 1991, Proc. Natl. Acad. Sci., USA, 88, 9026, shares 85% amino acid homology with KDR and is termed as flk-1 (fetal liver kinase-1). The high-affinity binding of VEGF to its receptors is modulated by cell surface-associated heparin and heparin-like molecules (Gitay-Goren et al., 1992 J. Biol. Chem. 267, 6093).

VEGF expression has been associated with several pathological states such as tumor angiogenesis, several forms of blindness, rheumatoid arthritis, psoriasis and others. In addition, a number of studies have demonstrated that VEGF is both necessary and sufficient for neovascularization. Takashita et al., 1995 J. Clin. Invest. 93, 662, demonstrated that a single injection of VEGF augmented collateral vessel development in a rabbit model of ischemia. VEGF also can induce neovascularization when injected into the cornea. Expression of the VEGF gene in CHO cells is sufficient to confer tumorigenic potential to the cells. Kim *et al., supra* and Millauer *et al., supra* used monoclonal antibodies against VEGF or a dominant negative form of VEGFR2 receptor to inhibit tumor-induced neovascularization.

During development, VEGF and its receptors are associated with regions of new vascular growth (Millauer et al., 1993 Cell 72, 835; Shalaby et al., 1993 J. Clin. Invest 91, 2235). Furthermore, transgenic mice lacking either of the VEGF receptors are defective in blood vessel formation and these mice do not survive; VEGFR2 appears to be required for differentiation of endothelial cells, while VEGFR1 appears to be required at later stages of vessel formation (Shalaby et al., 1995 Nature 376, 62; Fung et aL, 1995 Nature 376, 66). Thus, these receptors apparently need to be present to properly signal endothelial cells or their precursors to respond to vascularization-promoting stimuli.

Increasing evidence suggests that the VEGF family may also be involved with both the etiology and maintenance of peritoneal endometriosis. Peritoneal endometriosis is a significant debilitating gynecological problem of widespread prevalence. It is now generally accepted that the pathogenesis of peritoneal endometriosis involves the implantation of exfoliated endometrium. Maintenance of exfoliated endometrial tissue is dependent upon the generation and maintenance of an extensive blood supply both within and surrounding the ectopic tissue.

Endometriosis is a disease affecting an estimated 77 million women and teenagers worldwide. Endometriosis is a leading cause of infertility, chronic pelvic pain and hysterectomy. Endometriosis can be characterized when endometrial tissue (the tissue inside the uterus which builds up and is shed each month during menses) is found outside the uterus, in other areas of the body. The endometrial tissue can respond to hormonal commands each month and break down and bleed. However, unlike the endometrium, these tissue deposits have no way of leaving the body. The result is internal bleeding, degeneration of blood and tissue shed from the growths, inflammation of the surrounding areas, expression of irritating enzymes and formation of scar tissue. In addition, depending on the location of the growths, interference with the bowel, bladder, intestines and other areas of the pelvic cavity can occur. Endometrial tissue has even been found lodged in the skin and at other extrapelvic locations like the arm, leg and even brain.

Currently, the presence of Endometriosis can only be confirmed through surgery such as laparoscopy, but can be suspected based on symptoms, physical findings and diagnostic tests. Endometriosis can be treated in many different ways; both surgically and medically. Most commonly, surgery will be performed during which the disease will be excised, ablated, fulgarated, cauterized or otherwise removed, and adhesions will also be freed. Surgeries include but are not limited to laparoscopy; laparotomy; presacral and uterosacral and various levels of hysterectomies, where some or all of the reproductive organs are removed. Often, this method will only relieve the symptoms associated with growths on the reproductive organs, not the bowels or kidneys and related areas where Endometriosis can be present.

There are several drugs used to treat Endometriosis that are utilized either alone or in combination with surgery. These include contraceptives, GnRH agonists, and/or synthetic hormones. GnRH agonists are commonly used on women in all stages of the disease and may sometimes have serious side affects. GnRH (gonadotropin releasing hormone) analogues are classified into 2 groups: agonists and antagonists. Agonists are commonly used in the treatment of Endometriosis by suppressing the manufacture of follicle stimulating hormone (FSH) and luteinizing hormone (LH), common hormones required in ovulation. When they are not secreted, the body will go into "pseudo-menopause," stalling the growth of more implants. However, these are again only stop-gap measures that can be utilized only for short term intervals. Once the body returns to it's normal state, the Endometriosis will again begin to implant itself.

Angiogenesis is likely to be involved in the pathogenesis of endometriosis. According to the transplantation theory, when the exfoliated endometrium is attached to the peritoneal layer, the establishment of a new blood supplyis essential for the survival of the endometrial implant and development of endometriosis (Donnez et al., 1998, Hum. Reprod., 13, 1686-1690). Endometrial growth and repair after menstruation are associated with profound angiogenesis. Abnormalities in these processes result in excessive or unpredictable bleeding patterns and are common in many women. It is therefore important to understand which factors regulate normal endometrial angiogenesis. Vascular endothelial growth factor (VEGF) is an endothelial cell-specific mitogen that plays an important role in normal and pathological angiogenesis (Fasciani et al., 2000, Mol. Hum. Reprod., 6, 50-54; Sharkey et al., 2000, J. Clin. Endocrinol. Metab., 85, 402-409). Sources of this factor include the eutopic endometrium, ectopic endometriotic tissue and peritoneal fluid macrophages. Important to its etiology is the correct peritoneal environment in which the exfoliated endometrium is seeded and implants. Established ectopic tissue is then dependent on the peritoneal environment for its survival, an environment that supports angiogenesis. The increasing knowledge of the involvement of the VEGF family in endometriotic angiogenesis raises the possibility=of novel approaches to its medical management, with particular focus on the anti-angiogenic control of the action of VEGF (McLaren, 2001, Hum. Reprod Update, 6, 45-55).

Pavco et al., International PCT Publication No. WO 97/15662, describes methods and reagents for treating diseases or conditions related to levels of vascular endothelial growth factor receptor.

Robinson, International PCT Publication No. WO 95/04142, describes the use of certain antisense oligonucleotides targeted against VEGF RNA to inhibit VEGF expression.

Jellinek et al., 1994 Biochemistry 33, 10450 describe the use of specific VEGF-specific high-affinity RNA aptamers to inhibit the binding of VEGF to its receptors.

Rockwell and Goldstein, International PCT Publication No. WO 95/21868, describe the use of certain anti-VEGF receptor monoclonal antibodies to neutralize the effect of VEGF on endothelial cells.

Pappa, International PCT Publication No. WO 01/32920, describes inhibitors, including certain ribozyme and antisense nucleic acid molecules, of specific genes, including cathepsin D, AEBP-1, stromelysin-3, cystatin B, protease inhibitor 1, sFRP4, gelsolin, IGFBP-3, dual specificity phosphatase 1, PABP, Ig gamma chain, ferritin, complement component 3, pro-alpha-1 type III collagen, proline 4-hydroxylase, alpha-2 type I collagen, claudin-4, melanoma adhesion protein, procollagen C-endopeptidase enhancer, nascent-polypeptide-associated complex alpha polypeptide, elongation factor 1 alpha (EF-1-alpha), vitamin D3 25 hydroxylase, CSRP-1, steroidogenic acute regulatory protein, apolipoprotein E, transcobalamin II, prosaposin, early growth response 1 (EGR1), ribosomal protein S6, adenosine deaminase RNA-specific protein, RAD21, guanine nucleotide binding protein beta polypeptide 2-like 1 (RACK1) and podocalyxin genes which are all differentially expressed in tissues within individual patients with endometriosis.

Labarbera et al., International PCT Publication No. WO 00/73416, describes specific antisense nucleic acid molecules targeting follicle-stimulating hormone receptor.

Storella et al., International PCT Publication No. WO 99/63116, describes modulators of Prothymosin gene products for treating endometriosis, including certain ribozymes and antisense nucleic acid molecules.

### Summary Of The Invention

This invention features nucleic acid-based molecules, for example, enzymatic nucleic acid molecules, allozymes, antisense nucleic acids, 2-5A antisense chimeras, triplex forming oligonucleotides, decoy RNA, dsRNA, siRNA, aptamers, and antisense nucleic acids containing nucleic acid cleaving chemical groups, and methods to modulate vascular endothelial growth factor (VEGF) and/or vascular endothelial growth factor receptor (VEGFr) gene expression. Non-limiting examples of genes that encode vascular endothelial growth factor receptors of the invention include VEGFR1, VEGFR2 or combinations thereof. In particular, the instant invention features nucleic acid-based molecules and methods that modulate the expression of vascular endothelial growth factor and/or vascular endothelial growth factor receptors, such as VEGFR1 and/or VEGFR2, that are useful in preventing, treating, controlling, and/or diagnosing angiogenesis related diseases and conditions, including but not limited to tumor angiogenesis, cancers such as breast cancer, lung cancer, colorectal cancer, renal cancer, pancreatic cancer, or melanoma, or ocular indications such as diabetic retinopathy, or age related macular degeneration, and female reproductive disorders and conditions, including but not limited to endometriosis, endometrial carcinoma, gynecologic bleeding disorders, irregular menstrual cycles, ovulation, premenstrual syndrome (PMS), and menopausal dysfunction.

In one embodiment, the invention features one or more nucleic acid-based molecules and methods that independently or in combination modulate the expression of gene(s) encoding vascular endothelial growth factor receptors. Specifically, the present invention features nucleic acid molecules that modulate the expression of VEGF (for example Genbank Accession No. NM_003376), VEGFR1 receptor (for example Genbank Accession No. NM_002019), and VEGFR2 receptor (for example Genbank Accession No. NM_002253) that are useful in preventing, treating, controlling, and/or diagnosing tumor angiogenesis, cancers such as breast cancer, lung cancer, colorectal cancer, renal cancer, pancreatic cancer, or melanoma, or ocular indications such as diabetic retinopathy, or age related macular degeneration, and female reproductive disorders and conditions, including but not limited to endometriosis, endometrial carcinoma, gynecologic bleeding disorders, irregular menstrual cycles, ovulation, premenstrual syndrome (PMS), and menopausal dysfunction.

In one embodiment, the present invention features a compound having Formula I: (SEQ ID NO: 5977)

**5' gₛaₛgₛuₛugcUGAuGagg ccgaaa ggccGaaAgucugB 3'**

wherein each a is 2'-O-methyl adenosine nucleotide, each g is a 2'-O-methyl guanosine nucleotide, each c is a 2'-O-methyl cytidine nucleotide, each u is a 2'-O-methyl uridine nucleotide, each A is adenosine, each G is guanosine, each s individually represents a phosphorothioate internucleotide linkage, U is 2'-deoxy-2'-C-allyl uridine, and B is an inverted deoxyabasic moiety. This compound is also referred to as ANGIOZYME^{™} ribozyme.

In another embodiment, the present invention features a compound having **Formula II:** (SEQ ID NO: 5978).

**5'-uₛaₛcₛ aₛaᵤ ucU GAu Gag gcg aaa gcc Gaa Aag aca aB-3'**

wherein each **a** is 2'-O-methyl adenosine nucleotide, each **g** is a 2'-O-methyl guanosine nucleotide, each **c** is a 2'-O-methyl cytidine nucleotide, each **u** is a 2'-O-methyl uridine nucleotide, each **A** is adenosine, each **G** is guanosine, each **s** individually represents a phosphorothioate internucleotide linkage, **U** is 2'-deoxy-2'-C-allyl uridine, and **B** is an inverted deoxyabasic moiety.

In one embodiment, the invention features a composition comprising a nucleic acid molecule of the invention in a pharmaceutically acceptable carrier. In another embodiment, the invention features a composition comprising a compound of Formula I and/or Formula II in a pharmaceutically acceptable carrier or diluent.

In one embodiment, the invention features a method of administering to a cell, for example a mammalian cell, including a human cell, a nucleic acid molecule of the invention comprising contacting the cell with the nucleic acid molecule under conditions suitable for administration, for example in the presence of a delivery reagent such as a lipid, cationic lipid, phospholipid, or liposome. In another embodiment, the invention features a method of administering to a cell, for example a mammalian cell , including a human cell, a compound of Formula I and/or Formula IIcomprising contacting the cell with the compound under conditions suitable for administration, for example in the presence of a delivery reagent such as a lipid, cationic lipid, phospholipid, or liposome.

In one embodiment, the present invention features a mammalian cell comprising a nucleic acid molecule of the invention, wherein the mammalian cell is, for example, a human cell. In another embodiment, the present invention also features a mammalian cell comprising the compound of Formula I and/or Formula II, wherein the mammalian cell is, for example, a human cell.

In one embodiment, the invention features a method of inhibiting angiogenesis, for example tumor angiogenesis, or ocular indications such as diabetic retinopathy, or age related macular degeneration, or endometrial neovascularization, in a subject comprising contacting the subject with a nucleic acid molecule of the invention, under conditions suitable for the inhibition. In another embodiment, the invention features a method of inhibiting angiogenesis, for example tumor angiogenesis, or ocular indications such as diabetic retinopathy, or age related macular degeneration, or endometrial neovascularization, in a subject, comprising contacting the subject with a compound of Formula I and/or Formula II, under conditions suitable for the inhibition.

In another embodiment, the invention features a method of treatment of a subjecthaving an ocular condition associated with the increased level of a VEGF receptor, for example diabetic retinopathy, or age related macular degeneration, comprising contacting cells of the subject with a nucleic acid molecule, such as an enzymatic nucleic acid molecule targeted against a VEGF receptor RNA, e.g., molecule according to Formula I and/or II, under conditions suitable for the treatment.

In another embodiment, the invention features a method of treatment of a subjecthaving a condition associated with an increased level of VEGR and/or a VEGF receptor, for example tumor angiogenesis, cancers such as breast cancer, lung cancer, colorectal cancer, renal cancer, pancreatic cancer, or melanoma, ocular diseases or ocular indications such as diabetic retinopathy, or age related macular degeneration, rhuematoid arthritis, psoriasis endometriosis, endometrial carcinoma, gynecologic bleeding disorders, irregular menstrual cycles, ovulation, premenstrual syndrome (PMS), or menopausal dysfunction, comprising contacting cells of the subject with a nucleic acid molecule of the invention, such as a compound of Formula I and/or Formula II, under conditions suitable for the treatment.

In yet another embodiment, the inventive method of treatment further comprises the use of one or more drug therapies under conditions suitable for the treatment. Non-limiting examples of other drug therapies that can be used in combination with nucleic acid molecules of the invention include to 5-fluoro uridine, Leucovorin, Irinotecan (CAMPTOSAR® or CPT-11 or Camptothecin-11 or Campto), Paclitaxel, or Carboplatin, GnRH (gonadotropin releasing hormone) agonists, Lupron Depot (Leuprolide Acetate), Synarel (naferalin acetate), Zolodex (goserelin acetate), Suprefact (buserelin acetate), Danazol, or oral contraceptives including but not limited to Depo-Provera or Provera (medroxyprogesterone acetate), or any other estrogen/progesterone contraceptive.

In one embodiment, the invention features a method of administering to a mammal, for example a human, a nucleic acid molecule of the invention comprising contacting the mammal with the nucleic acid molecule under conditions suitable for the administration, for example, in the presence of a delivery reagent such as a lipid, cationic lipid, phospholipid, or liposome. In another embodiment, the invention features a method of administering to a mammal, for example a human, a compound of Formula I and/or Formula II comprising contacting the mammal with the compound under conditions suitable for the administration, for example, in the presence of a delivery reagent such as a lipid, cationic lipid, phospholipid, or liposome.

In one embodiment, the invention features a nucleic acid molecule which down regulates expression of a vascular endothelial growth factor (VEGF) and/or vascular endothelial growth factor receptor (VEGFr) gene, for example, wherein the VEGFr gene comprises VEGFR1 or VEGFR2 and any combination thereof.

In one embodiment, a nucleic acid molecule of the invention, such as an enzymatic nucleic acid molecule, antisense nucleic acid molecule, 2-5A antisense chimera, triplex forming oligonucleotide, decoy RNA, dsRNA, siRNA, aptamer, or antisense nucleic acid containing nucleic acid cleaving chemical groups, is adapted to treat, control and/or diagnose tumor angiogenesis, cancers such as breast cancer, lung cancer, colorectal cancer, renal cancer, pancreatic cancer, or melanoma, ocular diseases or ocular indications, such as diabetic retinopathy, or age related macular degeneration, rhuematoid arthritis, psoriasis endometriosis, endometrial carcinoma, gynecologic bleeding disorders, irregular menstrual cycles, ovulation, premenstrual syndrome (PMS), or menopausal dysfunction.

Such nucleic acid molecules are also useful for the prevention of the diseases and conditions including diabetic retinopathy, macular degeneration, neovascular glaucoma, myopic degeneration, verruca vulgaris, angiofibroma of tuberous sclerosis, port-wine stains, Sturge Weber syndrome, Kippel-Trenaunay-Weber syndrome, Osler-Weber-Rendu syndrome and other diseases or conditions that are related to the levels of VEGFR1 or VEGFR2 in a cell or tissue.

In another embodiment, the invention features a composition in a pharmaceutically acceptable carrier or diluent, comprising the nucleic acid molecule of the instant invention.

In another embodiment, an enzymatic nucleic acid molecule, antisense nucleic acid molecule, 2-5A antisense chimera, triplex forming oligonucleotide, decoy RNA, dsRNA, siRNA, aptamer, or antisense nucleic acid containing nucleic acid cleaving chemical groups of the invention is adapted for birth control.

In one embodiment, an enzymatic nucleic acid molecule of the invention is in a hammerhead, Inozyme, Zinzyme, DNAzyme, Amberzyme, or G-cleaver configuration.

In one embodiment, an enzymatic nucleic acid molecule of the invention comprises between 8 and 100 bases complementary to RNA of VEGFR1 and/or VEGFR2 gene. In another embodiment, an enzymatic nucleic acid molecule of the invention comprises between 14 and 24 bases complementary to RNA of VEGFR1 and/or VEGFR2 gene.

In one embodiment, a siRNA molecule of the invention comprises a double stranded RNA wherein one strand of the RNA is complementary to RNA of a VEGFR1 and/or VEGFR2 gene. In another embodiment, a siRNA molecule of the invention comprises a double stranded RNA wherein one strand of the RNA comprises a portion of a sequence of RNA having a VEGFR1 and/or VEGFR2 sequence. In yet another embodiment, a siRNA molecule of the invention comprises a double stranded RNA wherein both strands of RNA are connected by a non-nucleotide linker. Alternately, a siRNA molecule of the invention comprises a double stranded RNA wherein both strands of RNA are connected by a nucleotide linker, such as a loop or stem loop structure.

In one embodiment, a single strand component of a siRNA molecule of the invention is from about 14 to about 50 nucleotides in length. In another embodiment, a single strand component of a siRNA molecule of the invention is about 14, 15, 16, 17, 18, 19, 20, 21, 22, 23, 24, 25, 26, 27, or 28 nucleotides in length. In yet another embodiment, a single strand component of a siRNA molecule of the invention is about 23 nucleotides in length. In one embodiment, a siRNA molecule of the invention is from about 28 to about 56 nucleotides in length. In another embodiment, a siRNA molecule of the invention is about 40, 41, 42, 43, 44, 45, 46, 47, 48, 49, 50, 51, or 52 nucleotides in length. In yet another embodiment, a siRNA molecule of the invention is about 46 nucleotides in length.

In one embodiment, an enzymatic nucleic acid molecule, antisense nucleic acid molecule, 2-5A antisense chimera, triplex forming oligonucleotide, decoy RNA, dsRNA, siRNA, aptamer, or antisense nucleic acid containing nucleic acid cleaving chemical groups of the invention is chemically synthesized.

In another embodiment, an enzymatic nucleic acid molecule, antisense nucleic acid molecule, 2-5A antisense chimera, triplex forming oligonucleotide, decoy RNA, dsRNA, siRNA, aptamer, or antisense nucleic acid containing nucleic acid cleaving chemical groups of the invention comprises at least one 2'-sugar modification.

In another embodiment, an enzymatic nucleic acid molecule, antisense nucleic acid molecule, 2-5A antisense chimera, triplex forming oligonucleotide, decoy RNA, dsRNA, siRNA, aptamer, or antisense nucleic acids containing nucleic acid cleaving chemical groups of the invention comprises at least one nucleic acid base modification.

In another embodiment, an enzymatic nucleic acid molecule, antisense nucleic acid molecule, 2-5A antisense chimera, triplex forming oligonucleotide, decoy RNA, dsRNA, siRNA, aptamer, or antisense nucleic acid containing nucleic acid cleaving chemical groups of the invention comprises at least one phosphate backbone modification.

In one embodiment, the invention features a mammalian cell, for example a human cell, comprising a nucleic acid molecule of the invention.

In another embodiment, the invention features a method of reducing VEGF and/or VEGFr, such as VEGFR1 and/or VEGFR2 expression or activity in a cell comprising contacting the cell with a nucleic acid molecule of the invention that modulates the expression and/or activity of VEGF and/or VEGFr, under conditions suitable for the reduction.

In another embodiment, a method of treatment of a subject having a condition associated with the level of VEGF and/or VEGFr, such as VEGFR1 and/or VEGFR2 is featured, wherein the method further comprises the use of one or more drug therapies under conditions suitable for the treatment.

In one embodiment, the invention features a method for treatment of a subject having tumor angiogenesis, tumor angiogenesis, cancers including but not limited to tumor and cancer types shown under Diagnosis in **Table III,** ocular diseases or ocular indications such as diabetic retinopathy, or age related macular degeneration, rhuematoid arthritis, psoriasis and/or endometriosis, endometrial carcinoma, gynecologic bleeding disorders, irregular menstrual cycles, ovulation, premenstrual syndrome (PMS), or menopausal dysfunction, comprising administering to the subject a nucleic acid molecule of the invention that modulates the expression and/or activity of VEGF and/or VEGFr under conditions suitable for the treatment.

In another embodiment, the invention features a method for birth control in a subject comprising administering to the subject a nucleic acid molecule of the invention that modulates the expression and/or activity of VEGF and/or VEGFr under conditions suitable for the treatment.

In another embodiment, the invention features a method of cleaving RNA encoded by a VEGF, VEGFR1 and/or VEGFR2 gene comprising contacting an enzymatic nucleic acid molecule of the invention having endonuclease activity with RNA encoded by a VEGFR1 and/or VEGFR2 gene under conditions suitable for the cleavage, for example, wherein the cleavage is carried out in the presence of a divalent cation, such as Mg²⁺.

In one embodiment, a nucleic acid molecule of the invention comprises a cap structure, for example a 3',3'-linked or 5',5'-linked deoxyabasic ribose derivative, wherein the cap structure is at the 5'-end, or 3'-end, or both the 5'-end and the 3'-end of the enzymatic nucleic acid molecule.

In another embodiment, a nucleic acid molecule of the invention comprises a cap structure, for example a 3',3'-linked or 5',5'-linked deoxyabasic ribose derivative, wherein the cap structure is at the 5'-end, or 3'-end, or both the 5'-end and the 3'-end of the antisense nucleic acid molecule.

In one embodiment, the invention features an expression vector comprising a nucleic acid sequence encoding at least one nucleic acid molecule of the invention such that the vector allows expression of the nucleic acid molecule.

In another embodiment, the invention features a mammalian cell, for example, a human cellcomprising an expression vector of the invention.

In yet another embodiment, an expression vector of the invention further comprises a sequence for a nucleic acid molecule complementary to RNA encoded by a VEGF and/or VEGFr, such as VEGFR1 and/or VEGFR2 gene.

In one embodiment, an expression vector of the invention comprises a nucleic acid sequence encoding two or more nucleic acid molecules of the invention, which can be the same or different.

In another embodiment, the invention features a method for treatment or control of tumor angiogenesis, cancers such as breast cancer, lung cancer, colorectal cancer, renal cancer, pancreatic cancer, or melanoma, or ocular indications such as diabetic retinopathy, or age related macular degeneration, and/or endometriosis, endometrial carcinoma, gynecologic bleeding disorders, irregular menstrual cycles, ovulation, premenstrual syndrome (PMS), or menopausal dysfunction, comprising administering to a subject a nucleic acid molecule of the invention that modulates the expression and/or activity of VEGF and/or VEGFr, such as an enzymatic nucleic acid molecule, antisense nucleic acid molecule, 2-5A antisense chimera, triplex forming oligonucleotide, decoy RNA, dsRNA, siRNA, aptamer, or antisense nucleic acid containing nucleic acid cleaving chemical groups of the invention, under conditions suitable for the treatment, including administering to the subject one or more other therapies, for example, 5-fluoro uridine, Leucovorin, Irinotecan (CAMPTOSAR® or CPT-11 or Camptothecin-11 or Campto), Paclitaxel, or Carboplatin.GnRH (gonadotropin releasing hormone) agonists, Lupron Depot (Leuprolide Acetate), Synarel (naferalin acetate), Zolodex (goserelin acetate), Suprefact (buserelin acetate), Danazol, or oral contraceptives including but not limited to Depo-Provera or Provera (medroxyprogesterone acetate), or any other estrogen/progesterone contraceptive.

In one embodiment, the method of treatment features a nucleic acid molecule of the invention, such as an enzymatic nucleic acid or antisense nucleic acid molecule, that comprises at least five ribose residues, at least ten 2'-*O*-methyl modifications, and a 3'-end modification, such as a 3'-3' inverted abasic moiety. In another embodiment, a nucleic acid molecule of the invention further comprises phosphorothioate linkages on at least three of the 5' terminal nucleotides.

In another embodiment, the invention features a method of administering to a mammal, for example a human, an enzymatic nucleic acid molecule, antisense nucleic acid molecule, 2-5A antisense chimera, triplex forming oligonucleotide, decoy RNA, dsRNA, siRNA, aptamer, or antisense nucleic acid containing nucleic acid cleaving chemical groups of the invention, comprising contacting the mammal with the nucleic acid molecule under conditions suitable for the administration, for example, in the presence of a delivery reagent such as a lipid, cationic lipid, phospholipid, or liposome.

In yet another embodiment, the invention features a method of administering to a mammal an enzymatic nucleic acid molecule, antisense nucleic acid molecule, 2-5A antisense chimera, triplex forming oligonucleotide, decoy RNA, dsRNA, siRNA, aptamer, or antisense nucleic acid containing nucleic acid cleaving chemical groups of the invention in conjunction with other therapies, comprising contacting the mammal, for example a human, with the nucleic acid molecule and the other therapy under conditions suitable for the administration.

In another embodiment, other therapies contemplated by the instant invention that can be used in conjunction with the nucleic acid molecules of the instant invention include, but are not limited to, 5-fluoro uridine, Leucovorin, Irinotecan (CAMPTOSAR® or CPT-11 or Camptothecin-11 or Campto), Paclitaxel, or Carboplatin, GnRH (gonadotropin releasing hormone) agonists, Lupron Depot (Leuprolide Acetate), Synarel (naferalin acetate), Zolodex (goserelin acetate), Suprefact (buserelin acetate), Danazol, or oral contraceptives including but not limited to Depo-Provera or Provera (medroxyprogesterone acetate), or other estrogen/progesterone contraceptive.

In one embodiment, the invention features the use of an enzymatic nucleic acid molecule, to down-regulate the expression of VEGFR1 and/or VEGFR2 genes in the treatment or control of tumor angiogenesis, cancers such as breast cancer, lung cancer, colorectal cancer, renal cancer, pancreatic cancer, or melanoma, or ocular indications such as diabetic retinopathy, or age related macular degeneration, and/or endometriosis, endometrial carcinoma; gynecologic bleeding disorders, irregular menstrual cycles, ovulation, premenstrual syndrome (PMS), or menopausal dysfunction. Such enzymatic nucleic acid molecule can be in the hammerhead, NCH, G-cleaver, Amberzyme, Zinzyme, and/or DNAzyme motif.

In another embodiment, the invention features the use of an enzymatic nucleic acid moleculeto down-regulate the expression of VEGF and/or VEGFr, such as VEGFR1 and/or VEGFR2 genes, as a method of birth control. Such enzymatic nucleic acid molecule can be in the hammerhead, NCH, G-cleaver, Amberzyme, Zinzyme, and/or DNAzyme motif. In one embodiment, the nucleic acid molecules of the invention have complementarity to the substrate sequences in **Tables V and VI.** Examples of enzymatic nucleic acid molecules of the invention are shown in **Tables V and VI**. Examples of such enzymatic nucleic acid molecules consist essentially of sequences defined in these Tables.

By "inhibit", "down-regulate", or "reduce", it is meant that the expression of the gene, or level of nucleic acids or equivalent nucleic acids encoding one or more proteins or protein subunits, or activity of one or more proteins or protein subunits, such as VEGFR1, VEGFR2 and/or flk-1, is reduced below that observed in the absence of the nucleic acid molecules of the invention. In one embodiment, inhibition, down-regulation or reduction with enzymatic nucleic acid molecule preferably is below that level observed in the presence of an enzymatically inactive or attenuated molecule that is able to bind to the same site on the target nucleic acid, but is unable to cleave that nucleic acid. In another embodiment, inhibition, down-regulation; or reduction with antisense oligonucleotides is preferably below that level observed in the presence of, for example, an oligonucleotide with scrambled sequence or with mismatches. In another embodiment, inhibition, down regulation, or reduction of VEGF and/or VEGFr, such as VEGFR1 and/or VEGFR2 with the nucleic acid molecule of the instant invention is greater in the presence of the nucleic acid molecule than in its absence.

By "up-regulate" is meant that the expression of a gene, or level of nucleic acids or equivalent nucleic acids encoding one or more proteins or protein subunits, or activity of one or more proteins or protein subunits, such as VEGFR1 and/or VBGFR2, is greater than that observed in the absence of the nucleic acid molecules of the invention. For example, the expression of a gene, such as VEGF and/or VEGFr, such as VEGFR1 and/or VEGFR2 gene, can be increased in order to treat, prevent, ameliorate, or modulate a pathological condition caused or exacerbated by an absence or low level of gene expression.

By "modulate" is meant that the expression of a gene, or level of nucleic acids or equivalent nucleic acids encoding one or more proteins or protein subunits, or activity of one or more proteins protein subunit(s) is up-regulated or down-regulated, such that the expression, level, or activity is greater than or less than that observed in the absence of the nucleic acid molecules of the invention.

By "enzymatic nucleic acid molecule" it is meant a nucleic acid molecule which has complementarity in a substrate binding region to a specified gene target, and also has an enzymatic activity which is active to specifically cleave a target nucleic acid. That is, the enzymatic nucleic acid molecule is able to intermolecularly cleave a nucleic acid and thereby inactivate a target nucleic acid molecule. These complementary regions allow sufficient hybridization of the enzymatic nucleic acid molecule to the target nucleic acid and thus permit cleavage. One hundred percent complementarity is preferred, but complementarity as low as 50-75% can also be useful in this invention (see for example Werner and Uhlenbeck, 1995, Nucleic Acids Research, 23, 2092-2096; Hammann et al., 1999, Antisense and Nucleic Acid Drug Dev., 9, 25-31). The nucleic acids can be modified at the base, sugar, and/or phosphate groups. The term enzymatic nucleic acid is used interchangeably with phrases such as ribozymes, catalytic RNA, enzymatic RNA, catalytic DNA, aptazyme or aptamer-binding ribozyme, regulatable ribozyme, catalytic oligonucleotides, nucleozyme, DNAzyme, RNA enzyme, endoribonuclease, endonuclease, minizyme, leadzyme, oligozyme or DNA enzyme. All of these terminologies describe nucleic acid molecules with enzymatic activity. The specific enzymatic nucleic acid molecules described in the instant application are not4imiting in the invention and those skilled in the art will recognize that all that is important in an enzymatic nucleic acid molecule of this invention is that it has a specific substrate binding site which is complementary to one or more of the target nucleic acid regions, and that it have nucleotide sequences within or surrounding that substrate binding site which impart a nucleic acid cleaving and/or ligation activity to the molecule (Cech et al., U.S. Patent No. 4,987,071; Cech et al., 1988, 260 JAMA 3030).

Several varieties of naturally-occurring enzymatic nucleic acids are known presently. Each can catalyze the hydrolysis of nucleic acid phosphodiester bonds in *trans* (and thus can cleave other nucleic acid molecules) under physiological conditions. **Table I** summarizes some of the characteristics of these ribozymes. In general, enzymatic nucleic acids act by first binding to a target nucleic acid. Such binding occurs through the target binding portion of a enzymatic nucleic acid which is held in close proximity to an enzymatic portion of the molecule that acts to cleave the target nucleic acid. Thus, the enzymatic nucleic acid first recognizes and then binds a target nucleic acid through complementary base-pairing, and once bound to the correct site, acts enzymatically to cut the target nucleic acid. Strategic cleavage of such a target nucleic acid will destroy its ability to direct synthesis of an encoded protein. After an enzymatic nucleic acid has bound and cleaved its nucleic acid target, it is released from that nucleic acid to search for another target and can repeatedly bind and cleave new targets. Thus, a single ribozyme molecule is able to cleave many molecules of target nucleic acid. In addition, the ribozyme is a highly specific inhibitor of gene expression, with the specificity of inhibition depending not only on the base-pairing mechanism of binding to the target nucleic acid, but also on the mechanism of target nucleic acid cleavage. Single mismatches, or base-substitutions, near the site of cleavage can completely eliminate catalytic activity of a ribozyme.

In one embodiment of the inventions described herein, an enzymatic nucleic acid molecule of the invention is formed in a hammerhead or hairpin motif, but can also be formed in the motif of a hepatitis delta virus, group I intron, group II intron or RNase P RNA (in association with an RNA guide sequence), *Neurospora* VS RNA, DNAzymes, NCH cleaving motifs, or G-cleavers. Examples of such hammerhead motifs are described by Dreyfus, *supra,* Rossi et al., 1992, AIDS Research and Human Retroviruses 8, 183; of hairpin motifs by Hampel et al., EP0360257, Hampel and Tritz, 1989 Biochemistry 28, 4929, Feldstein et al., 1989, Gene 82, 53, Haseloff and Gerlach, 1989, Gene, 82, 43, and Hampel et al., 1990 Nucleic Acids Res. 18, 299; Chowrira & McSwiggen, US. Patent No. 5,631,359; an examples of a hepatitis delta virus motif is described by Perrotta and Been, 1992 Biochemistry 31, 16; examples of RNase P motifs are described by Guerrier-Takada et al., 1983 Cell 35, 849; Forster and Altman, 1990, Science 249, 783; Li and Altman, 1996, Nucleic Acids Res. 24, 835; examples of *Neurospora* VS RNA ribozyme motifs are described by Collins (Saville and Collins, 1990 Cell 61, 685-696; Saville and Collins, 1991 Proc. Natl. Acad. Sci. USA 88, 8826-8830; Collins and Olive, 1993 Biochemistry 32, 2795-2799; Guo and Collins, 1995, EMBO. J. 14, 363); examples of Group II introns are described by Griffin et al., 1995, Chem. Biol. 2, 761; Michels and Pyle, 1995, Biochemistry 34, 2965; Pyle et al., International PCT Publication No. WO 96/22689*;* an example of a Group I intron is described by Cech et al., U.S. Patent 4,987,071; and examples of DNAzymes are described by Usman et al., International PCT Publication No. WO 95/11304; Chartrand et al., 1995, NAR 23, 4092; Breaker et al., 1995, Chem. Bio. 2, 655; Santoro et al., 1997, PNAS 94, 4262, and Beigelman et al., International PCT publication No. WO 99/55857. NCH cleaving motifs are described in Ludwig & Sproat, International PCT Publication No. WO 98/58058; and G-cleavers are described in Kore et al., 1998, Nucleic Acids Research 26, 4116-4120 and Eckstein et *al.,* International PCT Publication No. WO 99/16871. Additional motifs such as the Aptazyme (Breaker *et al.,* WO 98/43993), Amberzyme (Beigelman et al., U.S. Serial No. 09/301,511) and Zinzyme **(Figure 7)** (Beigelman et al., U.S. Serial No. 09/918,728), all included by reference herein including drawings, can also be used in the present invention. These specific motifs or configurations are not limiting in the invention and those skilled in the art will recognize that all hat is important in an enzymatic nucleic acid molecule of this invention is that it have a specific substrate binding site which is complementary to one or more of the target gene RNA regions, and that it have nucleotide sequences within or surrounding that substrate binding site which impart a RNA cleaving activity to the molecule (Cech et.al., U.S. Patent No. 4,987,071).

By "nucleic acid molecule" as used herein is meant a molecule having nucleotides. The nucleic acid can be single, double, or multiple stranded and can comprise modified or unmodified nucleotides or non-nucleotides or various mixtures and combinations thereof.

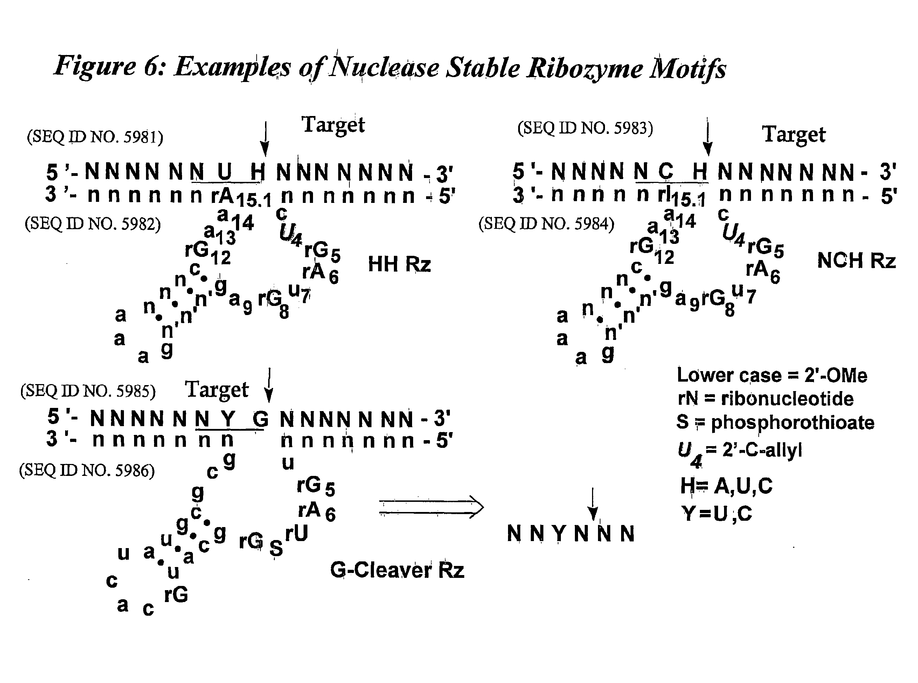

By "enzymatic portion" or "catalytic domain" is meant that portion/region of a enzymatic nucleic acid molecule essential for cleavage of a nucleic acid substrate (for example see **Figure 6**).

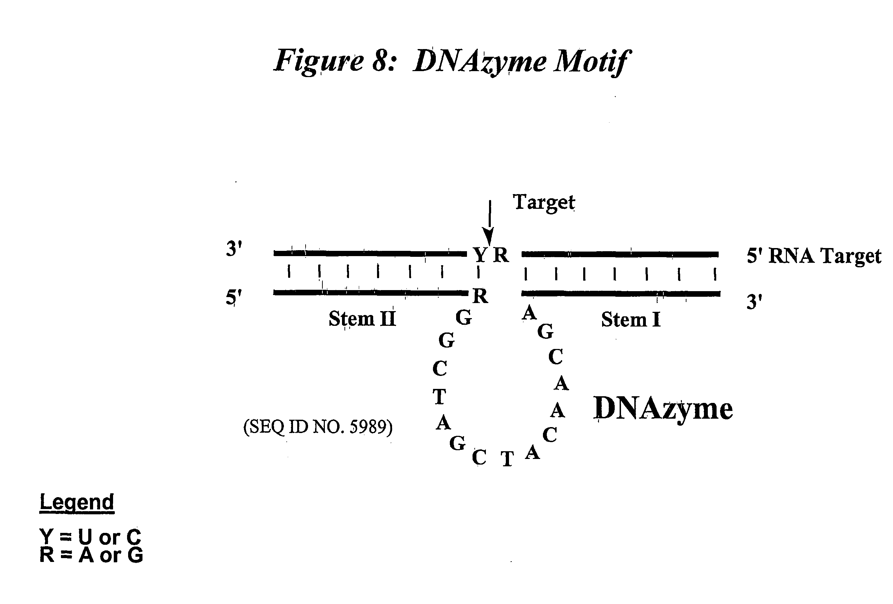

By "substrate binding arm" or "substrate binding domain" is meant that portion/region of a enzymatic nucleic acid which is able to interact, for example via complementarity (*i.e*., able to base-pair with), with a portion of its substrate. Preferably, such complementarity is 100%, but can be less if desired. For example, as few as 10 bases out of 14 can be base-paired (see for example Werner and Uhlenbeck, 1995, Nucleic Acids Research, 23, 2092-2096; Hammann et al., 1999, Antisense and Nucleic Acid Drug Dev., 9, 25-31). Examples of such arms are shown generally in **Figures 6**-**8**. That is, these arms contain sequences within a enzymatic nucleic acid which are intended to bring enzymatic nucleic acid and target nucleic acid together through complementary base-pairing interactions. An enzymatic nucleic acid of the invention can have binding arms that are contiguous or non-contiguous and can be of varying lengths. The length of the binding arm(s) are preferably greater than or equal to four nucleotides and of sufficient length to stably interact with the target nucleic acid; preferably 12-100 nucleotides; more preferably 14-24 nucleotides long (see for example Werner and Uhlenbeck, *supra;* Hamman *et al., supra;* Hampel et al., EP0360257; Berzal-Herranz et al., 1993, EMBO J., 12, 2567-73) or between 8 and 14 nucleotides long. If two binding arms are chosen, the design is such that the length of the binding arms are symmetrical (i.e., each of the binding arms is of the same length; e.g., four and four, five and five nucleotides, or six and six nucleotides, or seven and seven nucleotides long) or asymmetrical (*i.e.,* the binding arms are of different length; *e.g.,* three and five, six and three nucleotides; three and six nucleotides long; four and five nucleotides long; four and six nucleotides long; four and seven nucleotides long; and the like).

By "Inozyme" or "NCH" motif or configuration is meant, an enzymatic nucleic acid molecule comprising a motif as is generally described as NCH Rz in **Figure 6** and in Ludwig et al., International PCT Publication No. WO 98/58058 and US Patent Application Serial No. 08/878,640. Inozymes possess endonuclease activity to cleave nucleic acid substrates having a cleavage triplet NCH/, where N is a nucleotide, C is cytidine and H is adenosine, uridine or cytidine, and "/'' represents the cleavage site. H is used interchangeably with X. Inozymes can also possess endonuclease activity to cleave nucleic acid substrates having a cleavage triplet NCN/, where N is a nucleotide, C is cytidine, and "/" represents the cleavage site. "I" in **Figure 6** represents an Inosine nucleotide, preferably a ribo-Inosine or xylo-Inosine nucleoside.

By "G-cleaver" motif or configuration is meant, an enzymatic nucleic acid molecule comprising a motif as is generally described as G-cleaver Rz in **Figure 6** and in Eckstein et al., US 6,127,173. G-cleavers possess endonuclease activity to cleave nucleic acid substrates having a cleavage triplet NYN/, where N is a nucleotide, Y is uridine or cytidine and "/" represents the cleavage site. G-cleavers can be chemically modified as is generally shown in **Figure 6.**

By "amberzyme" motif or configuration is meant, an enzymatic nucleic acid molecule comprising a motif as is generally described in Beigelman et al., International PCT publication No. WO 99/55857 and US Patent Application Serial No. 09/476,387. Amberzymes possess endonuclease activity to cleave nucleic acid substrates having a cleavage triplet NG/N, where N is a nucleotide, G is guanosine, and "/" represents the cleavage site. Amberzymes can be chemically modified to increase nuclease stability through substitutions using modified nucleotides. In addition, differing nucleoside and/or non-nucleoside linkers can be used to substitute the 5'-gaaa-3' loops shown in the figure. Amberzymes represent a non-limiting example of an enzymatic nucleic acid molecule that does not require a ribonucleotide (2'-OH) group within its own nucleic acid sequence for activity.

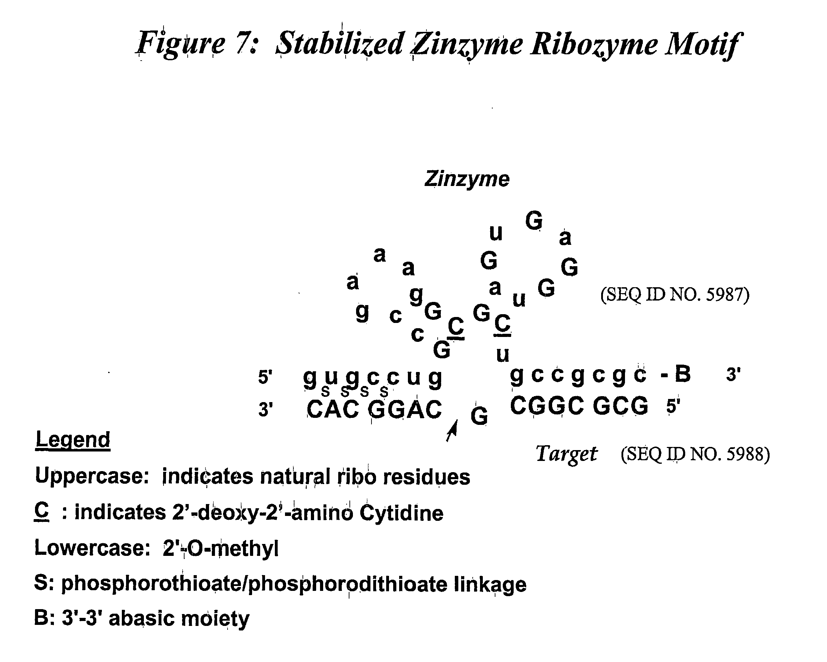

By "zinzyme" motif or configuration is meant, an enzymatic nucleic acid molecule comprising a motif as is generally described in Figure 7 and in Beigelman et al., International PCT publication No. WO 99/55857 and US Patent Application Serial No. 09/918,728. Zinzymes possess endonuclease activity to cleave nucleic acid substrates having a cleavage triplet including but not limited to YG/Y, where Y is uridine or cytidine, and G is guanosine and "/" represents the cleavage site. Zinzymes can be chemically modified to increase nuclease stability through substitutions as are generally shown in **Figure 7**, including substituting 2'-O-methyl guanosine nucleotides for guanosine nucleotides. In addition, differing nucleotide and/or non nucleotide linkers can be used to substitute the 5'-gaaa-2' loop shown in the figure. Zinzymes represent a non-limiting example of an enzymatic nucleic acid molecule that does not require a ribonucleotide (2'-OH) group within its own nucleic acid sequence for activity.

By 'DNAzyme' is meant, an enzymatic nucleic acid molecule that does not require the presence of a 2'-OH group within its own nucleic acid sequence for activity. In particular embodiments the enzymatic nucleic acid molecule can have an attached linker or linkers or other attached or associated groups, moieties, or chains containing one or more nucleotides with 2'-OH groups. DNAzymes can be synthesized chemically or expressed endogenously in *vivo,* by means of a single stranded DNA vector or equivalent thereof. An example of a DNAzyme is shown in **Figure 8** and is generally reviewed in Usman et al., US patent No., 6,159,714; Chartrand et al., 1995, NAR 23, 4092; Breaker et al., 1995, Chem. Bio. 2, 655; Santoro et al., 1997, PNAS 94, 4262; Breaker, 1999, Nature Biotechnology, 17, 422-423; and Santoro et. al., 2000, J. Am. Chem. Soc., 122, 2433-39. The "10-23" DNAzyme motif is one particular type of DNAzyme that was evolved using *in vitro* selection, see Santoro *et al., supra* and as generally described in Joyce et al., US 5,807,718. Additional DNAzyme motifs can be selected for using techniques similar to those described in these references, and hence, are within the scope of the present invention.

By "sufficient length" is meant a nucleic acid molecule of the invention is long enough to provide the intended function under the expected condition. For example, a nucleic acid molecule of the invention needs to be of "sufficient length" to provide stable interaction with a target nucleic acid molecule under the expected binding conditions and environment. In another non-limiting example, for the binding arms of an enzymatic nucleic acid, "sufficient length" means that the binding arm sequence is long enough to provide stable binding to a target site under the expected reaction conditions and environment. The binding arms are not so long as to prevent useful turnover of the nucleic acid molecule.

By "stably interact" is meant interaction of an oligonucleotides with target nucleic acid (*e.g.,* by forming hydrogen bonds with complementary nucleotides in the target under physiological conditions) that is sufficient to the intended purpose (e.g., cleavage of target nucleic acid by an enzyme).

By "equivalent" RNA to VEGF, VEGFR1 and/or VEGFR2 is meant to include nucleic acid molecules having homology (partial or complete) to a nucleic acid encoding VEGF, VEGFR1 and/or VEGFR2 proteins or encoding proteins with similar function as VEGF, VEGFR1 and/or VEGFR2 proteins in various organisms, including human, rodent, primate, rabbit, pig, protozoans, fungi, plants, and other microorganisms and parasites. The equivalent nucleic acid sequence also includes, in addition to the coding region, regions such as 5'-untranslated region, 3'-untranslated region, introns, intron-exon junction and the like.

By "homology" is meant the nucleotide sequence of two or more nucleic acid molecules is partially or completely identical.

By "antisense nucleic acid", it is meant a non-enzymatic nucleic acid molecule that binds to target nucleic acid by means of RNA-RNA or RNA-DNA or RNA-PNA (protein nucleic acid; Egholm et al., 1993 Nature 365, 566) interactions and alters the activity of the target nucleic acid (for a review, see Stein and Cheng, 1993 Science 261, 1004 and Woolf et al., US patent No. 5,849,902). Typically, antisense molecules are complementary to a target sequence along a single contiguous sequence of the antisense molecule. However, in certain embodiments, an antisense molecule can bind to substrate such that the substrate molecule forms a loop, and/or an antisense molecule can bind such that the antisense molecule forms a loop. Thus, an antisense molecule can be complementary to two (or even more) non-contiguous substrate sequences or two (or even more) non-contiguous sequence portions of an antisense molecule can be complementary to a target sequence or both. For a review of current antisense strategies, see Schmajuk et al., 1999, J. Biol. Chem., 274, 21783-21789, Delihas et al., 1997, Nature, 15, 751-753, Stein et al., 1997, Antisense N. A. Drug Dev., 7, 151, Crooke, 2000, Methods Enzymol., 313, 3-45; Crooke, 1998, Biotech. Genet. Eng. Rev., 15, 121-157, Crooke, 1997, Ad. Pharmacol., 40, 1-49. In addition, antisense DNA can be used to target nucleic acid by means of DNA-RNA interactions, thereby activating RNase H, which digests the target nucleic acid in the duplex. The antisense oligonucleotides can comprise one or more RNAse H activating region, which is capable of activating RNAse H cleavage of a target nucleic acid. Antisense DNA can be synthesized chemically or expressed via the use of a single stranded DNA expression vector or equivalent thereof

By "RNase H activating region" is meant a region (generally greater than or equal to 4-25 nucleotides in length, preferably from 5-11 nucleotides in length) of a nucleic acid molecule capable of binding to a target nucleic acid to form a non-covalent complex that is recognized by cellular RNase H enzyme (see for example Arrow et al., US 5,849,902; Arrow et al., US 5,989,912). The RNase H enzyme binds to a nucleic acid molecule-target nucleic acid complex and cleaves the target nucleic acid sequence. The RNase H activating region comprises, for example, phosphodiester, phosphorothioate (preferably at least four of the nucleotides are phosphorothiote substitutions; more specifically, 4-11 of the nucleotides are phosphorothiote substitutions); phosphorodithioate, 5'-thiophosphate, or methylphosphonate backbone chemistry or a combination thereof. In addition to one or more backbone chemistries described above, the RNase H activating region can also comprise a variety of sugar chemistries. For example, the RNase H activating region can comprise deoxyribose, arabino, fluoroarabino or a combination thereof, nucleotide sugar chemistry. Those skilled in the art will recognize that the foregoing are non-limiting examples and that any combination of phosphate, sugar and base chemistry=of a nucleic acid that supports the activity of RNase H enzyme is within the scope of the definition of the RNase H activating region and the instant invention.

By "2-5A antisense chimera" is meant an antisense oligonucleotide containing a 5'-phosphorylated 2'-5'-linked adenylate residue. These chimeras bind to target nucleic acid in a sequence-specific manner and activate a cellular 2-5A-dependent ribonuclease which, in turn, cleaves the target nucleic acid (Torrence et al., 1993 Proc. Natl. Acad. Sci. USA 90, 1300; Silverman et al., 2000, Methods Enzymol., 313, 522-533; Player and Torrence, 1998, Pharmacol. Ther., 78, 55-113).

By "triplex forming oligonucleotides" is meant an oligonucleotide that can bind to a double-stranded polynucleotide, such as DNA, in a sequence-specific manner to form a triple-strand helix. Formation of such triple helix structure has been shown to inhibit transcription of the targeted gene (Duval-Valentin et al., 1992 Proc. Natl. Acad. Sci. USA 89, 504; Fox, 2000, Curr. Med. Chem., 7, 17-37; Praseuth et. al., 2000, Biochim. Biophys. Acta, 1489, 181-206).

By "gene" it is meant a nucleic acid that encodes an RNA, for example, nucleic acid sequences including but not limited to structural genes encoding a polypeptide.

The term "complementarity" as used herein refers to the ability of a nucleic acid to form hydrogen bond(s) with another nucleic acid sequence by either traditional Watson-Crick or other non-traditional types. In reference to nucleic molecules of the present invention, the binding free energy for a nucleic acid molecule with its target or complementary sequence is sufficient to allow the relevant function of the nucleic acid to proceed, e.g., enzymatic nucleic acid cleavage, antisense or triple helix inhibition. Determination of binding free energies for nucleic acid molecules is well known in the art (see, e.g., Turner et al., 1987, CSH Symp. Quant. Biol. LII pp.123-133; Frier et al., 1986, Proc. Nat. Acad. Sci. USA 83:9373-9377; Turner et al., 1987, J. Am. Chem. Soc. 109:3783-3785). A percent complementarity indicates the percentage of contiguous residues in a nucleic acid molecule which can form hydrogen bonds (e.g., Watson-Crick base pairing) with a second nucleic acid sequence (e.g., 5, 6, 7, 8, 9; 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence.

By "RNA" is meant a molecule comprising at least one ribonucleotide residue. By "ribonucleotide" or "2'-OH" is meant a nucleotide with a hydroxyl group at the 2' position of a β-D-ribo-furanose moiety.

By "nucleic acid decoy molecule", or "decoy" as used herein is meant a nucleic acid molecule that mimics the natural binding domain for a ligand. The decoy therefore competes with the natural binding target for the binding of a specific ligand. For example, it has been shown that over-expression of HIV trans-activation response (TAR) RNA can act as a "decoy" and efficiently binds HIV tat protein, thereby preventing it from binding to TAR sequences encoded in the HIV RNA (Sullenger et al., 1990, Cell, 63, 601-608).

By "aptamer" or "nucleic acid aptamer" as used herein is meant a nucleic acid molecule that binds specifically to a target molecule wherein the nucleic acid molecule has sequence that is distinct from sequence recognized by the target molecule in its natural setting. Alternately, an aptamer can be a nucleic acid molecule that binds to a target molecule where the target molecule does not naturally bind to a nucleic acid. The target molecule can be any molecule of interest. For example, the aptamer can be used to bind to a ligand binding domain of a protein, thereby preventing interaction of the naturally occurring ligand with the protein. Similarly, the nucleic acid molecules of the instant invention can bind to VEGFR1 or VEGFR2 receptors to block activity of the receptor. This is a non-limiting example and those in the art will recognize that other embodiments can be readily generated using techniques generally known in the art, see for example Gold et al., US 5,475,096 and 5,2701,163; Gold et al., 1995, Annu. Rev. Biochem., 64, 763; Brody and Gold, 2000, J. Biotechnol., 74, 5; Sun, 2000, Curr. Opin. Mol. Ther., 2, 100; Kusser, 2000, J. Biotechnol., 74, 27; Hermann and Patel, 2000, Science, 287, 820; and Jayasena, 1999, Clinical Chemistry, 45, 1628.

The term "double stranded RNA" or "dsRNA" as used herein refers to a double stranded RNA molecule capable of RNA interference "RNAi", including short interfering RNA "siRNA" see for example Bass, 2001, Nature, 411, 428-429; Elbashir et al., 2001, Nature, 411, 494-498; and Kreutzer et al., International PCT Publication No. WO. 00/44895; Zernicka-Goetz et al., International PCT Publication No. WO 01/36646; Fire, International PCT Publication No. WO 99/32619; Plaetinck et al., International PCT Publication No. WO 00/01846; Mello and Fire, International PCT Publication No: WO 01/29058; Deschamps-Depaillette, International PCT Publication No. WO 99/07409; and Li et al., International PCT Publication No. WO 00/44914.

By "nucleic acid sensor molecule" or "allozyme" as used herein is meant a nucleic acid molecule comprising an enzymatic domain and a sensor domain, where the enzymatic nucleic acid domain's ability to catalyze a chemical reaction is dependent on the interaction with a target signaling molecule, such as a nucleic acid, polynucleotide, oligonucleotide, peptide, polypeptide, or protein, for example VEGF, VEGFR1 and/or VEGFR2. The introduction of chemical modifications, additional functional groups, and/or linkers, to the nucleic acid sensor molecule can provide enhanced catalytic activity of the nucleic acid sensor molecule, increased binding affinity of the sensor domain to a target nucleic acid, and/or improved nuclease/chemical stability of the nucleic acid sensor molecule, and are hence within the scope of the present invention (see for example Usman et al., US Patent Application No. 09/877,526, George et al., US Patent Nos. 5,834,186 and 5,741,679, Shih et al., US Patent No. 5,589,332, Nathan et al., US Patent No 5,871,914, Nathan and Ellington, International PCT publication No. WO 00/24931, Breaker et al., International PCT Publication Nos. WO 00/26226. and 98/27104, and Sullenger et al., US Patent Application Serial No. 09/205,520).

By "sensor component" or "sensor domain" of the nucleic acid sensor molecule as used herein is meant, a nucleic acid sequence (e.g., RNA or DNA or analogs thereof) which interacts with a target signaling molecule, for example a nucleic acid sequence in one or more regions of a target nucleic acid molecule or more than one target nucleic acid molecule, and which interaction causes the enzymatic nucleic acid component of the nucleic acid sensor molecule to either catalyze a reaction or stop catalyzing a reaction. In the presence of target signaling molecule of the invention, such as VEGF, VEGFR1 and/or VEGFR2, the ability of the sensor component, for example, to modulate the catalytic activity of the nucleic acid sensor molecule, is inhibited or diminished. The sensor component can comprise recognition properties relating to chemical or physical signals capable of modulating the nucleic acid sensor molecule via chemical or physical changes to the structure of the nucleic acid sensor molecule. The sensor component can be derived from a naturally occurring nucleic acid binding sequence, for example, RNAs that bind to other nucleic acid sequences in vivo. Alternately, the sensor component can be derived from a nucleic acid molecule (aptamer) which is evolved to bind to a nucleic acid sequence within a target nucleic acid molecule (see for example Gold et al., US 5,475,096 and 5,270,163). The sensor component can be covalently linked to the nucleic acid sensor molecule, or can be non-covalently associated. A person skilled in the art will recognize that all that is required is that the sensor component is able to selectively inhibit the activity of the nucleic acid sensor molecule to catalyze a reaction:

By "target molecule" or "target signaling molecule" is meant a molecule capable of interacting with a nucleic acid sensor molecule, specifically a sensor domain of a nucleic acid sensor molecule, in a manner that causes the nucleic acid sensor molecule to be active or inactive. The interaction of the signaling agent with a nucleic acid sensor molecule can result in modification of the enzymatic nucleic acid component of the nucleic acid sensor molecule via chemical, physical, topological, or conformational changes to the structure of the molecule, such that the activity of the enzymatic nucleic acid component of the nucleic acid sensor molecule is modulated, for example is activated or deactivated. Signaling agents can comprise target signaling molecules such as macromolecules, ligands, small molecules, metals and ions, nucleic acid molecules including but not limited to RNA and DNA or analogs thereof, proteins, peptides, antibodies, polysaccharides, lipids, sugars, microbial or cellular metabolites, pharmaceuticals, and organic and inorganic molecules in a purified or unpurified form, for example VEGF, VEGFR1 and/or VEGFR2.

The term "triplex forming oligonucleotides" as used herein refers to an oligonucleotide that can bind to a double-stranded DNA in a sequence-specific manner to form a triple-strand helix. Formation of such a triple helix structure has been shown to inhibit transcription of a targeted gene (Duval-Valentin et al., 1992 Proc. Natl. Acad. Sci. USA 89, 504; Fox, 2000, Curr. Med. Chem., 7, 17-37; Praseuth et. al., 2000, Biochim. Biophys. Acta, 1489, 181-206).

The nucleic acid molecules that modulate the expression of VEGF and/or VEGFr, such as VEGFR1 and/or VEGFR2 specific nucleic acids, represent a novel therapeutic approach to treat or control a variety of angiogenesis related disorders and conditions, including but not limited to tumor angiogenesis, cancers such as breast cancer, lung cancer, colorectal cancer, renal cancer, pancreatic cancer, or melanoma, or ocular indications such as diabetic retinopathy, or age related macular degeneration, and/or endometriosis, endometrial carcinoma, gynecologic bleeding disorders, irregular menstrual cycles, ovulation, premenstrual syndrome (PMS), and/or menopausal dysfunction. The nucleic acid molecules that modulate the expression of VEGF and/or VEGFr, such as VEGFR1 and/or VEGFR2 specific nucleic acids also represent a novel approach to control ovulation or embryonic implantation and therefore provide a novel means of birth control.

In one embodiment of the present invention, a nucleic acid molecule of the instant invention can be between 12 and 100 nucleotides in length. An exemplary enzymatic nucleic acid molecule of the invention is shown as Formula I and/or Formula II. For example, enzymatic nucleic acid molecules of the invention are preferably between 15 and 50 nucleotides in length, more preferably between 25 and 40 nucleotides in length, *e.g.,* 34, 36, or 38 nucleotides in length (for example see Jarvis et al., 1996, J. Biol. Chem., 271, 29107-29112). Exemplary DNAzymes of the invention are preferably between 15 and 40 nucleotides in length, more preferably between 25 and 35 nucleotides in length, *e.g.,* 29, 30, 31, or 32 nucleotides in length (see for example Santoro et al., 1998, Biochemistry, 37, 13330-13342; Chartrand et al., 1995, Nucleic Acids Research, 23, 4092-4096). Exemplary antisense molecules of the invention are preferably between 15 and 75 nucleotides in length, more preferably between 20 and 35 nucleotides in length, *e.g.,* 25, 26, 27, or 28 nucleotides in length (see for example Woolf et al., 1992, PNAS., 89, 7305-7309; Milner et al., 1997, Nature Biotechnology, 15, 537-541). Exemplary triplex forming oligonucleotide molecules of the invention are preferably between 10 and 40 nucleotides in length, more preferably between 12 and 25 nucleotides in length, *e.g.,* 18, 19, 20, or 21 nucleotides in length (see for example Maher et al., 1990, Biochemistry, 29, 8820-8826; Strobel and Dervan, 1990, Science, 249, 73-75). Those skilled in the art will recognize that all that is required is that the nucleic acid molecule be of length and conformation sufficient and suitable for the nucleic acid molecule to catalyze a reaction contemplated herein. The length of the nucleic acid molecules of the instant invention are not limiting within the general limits stated.

In a preferred embodiment, a nucleic acid molecule that modulates, for example, down-regulates, VEGF and/or VEGFr, such as VEGFR1 and/or VEGFR2 replication or expression comprises between 8 and 100 bases complementary to a nucleic acid molecule of VEGFR1 and/or VEGFR2. More preferably, a nucleic acid molecule that modulates VEGF and/or VEGFr, such as VEGFR1 and/or VEGFR2 replication or expression comprises between 14 and 24 bases complementary to a nucleic acid molecule of VEGFR1 and/or VEGFR2.

The invention provides a method for producing a class of nucleic acid-based gene modulating agents which exhibit a high degree of specificity for the nucleic acid of a desired target. For example, a nucleic acid molecule of the invention is preferably targeted to a highly conserved sequence region of target nucleic acids encoding VEGF and/or VEGFr, such as VEGFR1 and/or VEGFR2 (specifically VEGF, VEGFR1 and/or VEGFR2 genes) such that specific treatment of a disease or condition can be provided with either one or several nucleic acid molecules of the invention. Such nucleic acid molecules can be delivered exogenously to specific tissue or cellular targets as required. Alternatively, the nucleic acid molecules can be expressed from DNA and/or RNA vectors that are delivered to specific cells.

As used in herein "cell" is used in its usual biological sense, and does not refer to an entire multicellular organism. The cell can, for example, be *in vitro,* .e.g., in cell culture, or present in a multicellular organism, including,, e.g., birds, plants and mammals such as humans, cows, sheep, apes, monkeys, swine, dogs, and cats. The cell may be prokaryotic (e.g., bacterial cell) or eukaryotic (e.g., mammalian or plant cell).

By "VEGFR1 and/or VEGFR2 proteins" is meant, protein receptor or a mutant protein derivative thereof, having vascular endothelial growth factor receptor activity, for example, having the ability to bind vascular endothelial growth factor and/or having tyrosine kinase activity.

By "highly conserved sequence region" is meant, a nucleotide sequence of one or more regions in a target gene does not vary significantly from one generation to the other or from one biological system to the other.

"Angiogenesis" refers to formation of new blood vessels which is an essential process in reproduction, development and wound repair. "Tumor angiogenesis" refers to the induction of the growth of blood vessels from surrounding tissue into a solid tumor. Tumor growth and tumor metastasis are dependent on angiogenesis (for a review see Folkman, 1985 *supra;* Folkman 1990 J. Natl. Cancer Inst., 82, 4; Folkman and Shing, 1992 J. Biol. Chem. 267, 10931).

Angiogenesis plays an important role in other diseases such as arthritis wherein new blood vessels have been shown to invade the joints and degrade cartilage (Folkman and Shing, *supra).*

"Retinopathy" refers to inflammation of the retina and/or degenerative condition of the retina which may lead to occlusion of the retina and eventual blindness. In "diabetic retinopathy" angiogenesis causes the capillaries in the retina to invade the vitreous resulting in bleeding and blindness which is also seen in neonatal retinopathy (for a review see Folkman, 1985 *supra;* Folkman 1990 *supra;* Folkman and Shing, 1992 *supra)*.

Nucleic acid-based inhibitors of VEGF and/or VEGFr, such as VEGFR1 and/or VEGFR2; expression are useful for the prevention, treatment, and/or control of angiogenesis related disorders and conditions, including but not limited to, tumor angiogenesis, cancers such as breast cancer, lung cancer, colorectal cancer, renal cancer, pancreatic cancer, or melanoma, or ocular indications such as diabetic retinopathy, or age related macular degeneration, and/or endometriosis, endometrial carcinoma, gynecologic bleeding disorders, irregular menstrual cycles, ovulation, premenstrual syndrome (PMS), menopausal dysfunction, and other diseases or conditions that are related to or will respond to the levels of VEGF, VEGFR1 and/or VEGFR2 in a cell or tissue, alone or in combination with other therapies. The reduction of VEGF and/or VEGFr, such as VEGFR1 and/or VEGFR2 expression (specifically VEGF, VEGFR1 and/or VEGFR2 gene RNA levels) and thus reduction in the level of the respective protein relieves, to some degree, the symptoms of the disease or condition. Nucleic acid-based inhibitors of VEGF and/or VEGFr, such as VEGFR1 and/or VEGFR2 expression are also useful as birth control agents, for example by inhibition of ovulation or embryonic uterine implantation.

The nucleic acid molecules of the invention can be added directly, or can be complexed with cationic lipids, packaged within liposomes, or otherwise delivered to target cells or tissues. The nucleic acid complexes can be locally administered to relevant tissues ex vivo, or in vivo through injection or infusion pump, with or without their incorporation in biopolymers. In preferred embodiments, the nucleic acid inhibitors comprise sequences, which are complementary to polynucleotides, for example DNA and RNA, having VEGF and/or VEGFr, such as VEGFR1 and/or VEGFR2 sequence.

Triplex molecules of the invention can be provided targeted to DNA target regions, and containing the DNA equivalent of a target sequence or a sequence complementary to the specified target (substrate) sequence. Antisense molecules typically are complementary to a target sequence along a single contiguous sequence of the antisense molecule. However, in certain embodiments, an antisense molecule can bind to substrate such that the substrate molecule forms a loop, and/or an antisense molecule can bind such that the antisense molecule forms a loop. Thus, the antisense molecule can be complementary to two (or even more) non-contiguous substrate sequences or two (or even more) non-contiguous sequence portions of an antisense molecule can be complementary to a target sequence or both.

By "consists essentially of" is meant that the active nucleic acid molecule of the invention, for example, an enzymatic nucleic acid molecule, contains an enzymatic center or core equivalent to those in the examples, and binding arms able to bind nucleic acid such that cleavage at the target site occurs. Other sequences can be present which do not interfere with such cleavage. Thus, a core region can, for example, include one or more loop, stem-loop structure, or linker which does not prevent enzymatic activity. Thus, a particular region of a nucleic acid molecule of the invention can be such a loop, stem-loop, nucleotide linker, and/or non-nucleotide linker and can be represented generally as sequence "X". Thus, a core region may, for example, include one or more loop or stem-loop-structures which=do not prevent enzymatic activity. For example, a core sequence for a hammerhead enzymatic nucleic acid can comprise a conserved sequence, such as 5'-CUGAUGAG-3' and 5'-CGAA-3' connected by "X", where X is 5'-GCCGUUAGGC-3' (SEQ ID NO 5979), or any other Stem II region known in the art, or a nucleotide and/or non-nucleotide linker. Similarly, for other nucleic acid molecules of the instant invention, such as Inozyme, G-cleaver, amberzyme, zinzyme, DNAzyme, antisense, 2-5A antisense, triplex forming nucleic acid, aptamers, decoy nucleic acids, dsRNA or siRNA, other sequences or non-nucleotide linkers can be present that do not interfere with the function of the nucleic acid molecule.

Sequence X can be a linker of ≥ 2 nucleotides in length, preferably 3, 4, 5, 6, 7, 8, 9, 10, 15, 20, 26, 30, where the nucleotides can preferably be internally base-paired to form a stem of preferably ≥ 2 base pairs. Alternatively or in addition, sequence X can be a non-nucleotide linker. In yet another embodiment, the nucleotide linker X can be a nucleic acid aptamer, such as an ATP aptamer, HIV Rev aptamer (RRE), HIV Tat aptamer (TAR) and others (for a review see Gold et al., 1995, Annu. Rev. Biochem., 64, 763; and Szostak & Ellington, 1993, in The RNA World, ed. Gesteland and Atkins, pp. 511, CSH Laboratory Press). A nucleic acid aptamer includes a nucleic acid sequence capable of interacting with a ligand. The ligand can be any natural or a synthetic molecule, including but not limited to a resin, metabolites, nucleosides, nucleotides, drugs, toxins, transition state analogs, peptides, lipids, proteins, amino acids, nucleic acid molecules, hormones, carbohydrates, receptors, cells, viruses, bacteria and others.

In yet another embodiment, the non-nucleotide linker X is as defined herein. The term "non-nucleotide" as used herein include either abasic nucleotide, polyether, polyamine, polyamide, peptide, carbohydrate, lipid, or polyhydrocarbon compounds. Specific examples include those described by Seela and Kaiser, Nucleic Acids Res. 1990, 18:6353 and Nucleic Acids Res. 1987, 15:3113; Cload and Schepartz, J. Am. Chem. Soc. 1991, 113:6324; Richardson and Schepartz, J. Am. Chem. Soc. 1991, 113:5109; Ma et al., Nucleic Acids Res. 1993, 21:2585 and Biochemistry 1993, 32:1751; Durand et al., Nucleic Acids Res. 1990, 18:6353; McCurdy et al., Nucleosides & Nucleotides 1991, 10:287; Jschke et al., Tetrahedron Lett. 1993, 34:301; Ono et al., Biochemistry 1991, 30:9914; Arnold et al., International Publication No. WO 89/02439; Usman et al., International Publication No. WO 95/06731; Dudycz et al., International Publication No. WO 95/11910 and Ferentz and Verdine, J. Am. Chem. Soc. 1991, 113:4000, all hereby incorporated by reference herein.

A "non-nucleotide" further means any group or compound which can be incorporated into a nucleic acid chain in the place of one or more nucleotide units, including either sugar and/or phosphate substitutions, and allows the remaining bases to exhibit their enzymatic activity. The group or compound can be abasic in that it does not contain a commonly recognized-nucleotide base, such as adenosine, guanine, cytosine, uracil or thymine. Thus, in one embodiment, the invention features an enzymatic nucleic acid molecule having one or more non-nucleotide moieties, and having enzymatic activity to cleave an RNA or DNA molecule.

In another aspect of the invention, nucleic acid molecules that interact with target nucleic acid molecules and down-regulate VEGF and/or VEGFr, such as VBGFR1 and/or VEGFR2 (specifically VEGF, VEGFR1 and/or VEGFR2 gene) activity are expressed from transcription units inserted into DNA or RNA vectors. The recombinant vectors are preferably DNA plasmids or viral vectors. Enzymatic nucleic acid molecule or antisense expressing viral vectors can be constructed based on, but not limited to, adeno-associated virus, retrovirus, adenovirus, or alphavirus. The recombinant vectors capable of expressing the enzymatic nucleic acid molecules or antisense are delivered as described above, and persist in target cells. Alternatively, viral vectors can be used that provide for transient expression of enzymatic nucleic acid molecules or antisense. Such vectors can be repeatedly administered as necessary. Once expressed, the enzymatic nucleic acid molecules or antisense bind to the target nucleic acid and down-regulate its function or expression. Delivery of enzymatic nucleic acid molecule or antisense expressing vectors can be systemic, such as by intravenous or intramuscular administration, by administration to target cells explanted from the patient followed by reintroduction into the patient, or by any other means that would allow for introduction into the desired target cell. Antisense DNA can be expressed via the use of a single stranded DNA intracellular expression vector.

By "vectors" is meant any nucleic acid- and/or viral-based technique used to deliver a desired nucleic acid.

By "subject" or "patient" is meant an organism, which is a donor or recipient of explanted cells, or the cells themselves. "Subject" or "Patient" also refers to an organism to which the nucleic acid molecules of the invention can be administered. Preferably, a subject or patient is a mammal or mammalian cells. More preferably, a subject or patient is a human or human cells.

By "enhanced enzymatic activity" is meant to include activity measured in cells and/or in vivo where the activity is a reflection of both the catalytic activity and the stability of the nucleic acid molecules of the invention. In this invention, the product of these properties can be increased *in vivo* compared to an all RNA enzymatic nucleic acid or all DNA enzyme. In some cases, the activity or stability of the nucleic acid molecule can be decreased (i.e., less than ten-fold), but the overall activity of the nucleic acid molecule is enhanced, *in vivo.*

The nucleic acid molecules of the instant invention, individually, or in combination or in conjunction with other drugs, can be used to treat diseases or conditions discussed above. For example, to treat a disease or condition associated with the levels of VEGFR1 and/or VEGFR2, the patient can be treated, or other appropriate cells can be treated, as is evident to those skilled in the art, individually or in combination with one or more drugs under conditions suitable for the treatment.

In a further embodiment, the described molecules of the invention can be used in combination with other known treatments to treat conditions or diseases discussed above. For example, the described molecules can be used in combination with one or more known therapeutic agents to treat angiogenesis related disorders and conditions, including but not limited to tumor angiogenesis, cancers such as breast cancer, lung cancer, colorectal cancer, renal cancer, pancreatic cancer, or melanoma, or ocular indications such as diabetic retinopathy, or age related macular degeneration, and/or endometriosis, birth control, endometrial tumors, gynecologic bleeding disorders, irregular menstrual cycles, ovulation, premenstrual syndrome (PMS), menopausal dysfunction, endometrial carcinoma, and/or other diseases or conditions which respond to the modulation of VEGF and/or VEGFr, such as VEGFR1 and/or VEGFR2 expression.

Other features and advantages of the invention will be apparent from the following description of the preferred embodiments thereof, and from the claims.

### Brief Description of the Drawings

**Figure 1** shows a secondary structure model of ANGIOZYME^{™} ribozyme bound to its RNA target
**Figure 2** shows a time course of inhibition of primary tumor growth following systemic administration of ANGIOZYME^{™} in the LLC mouse model.
**Figure 3** shows inhibition of primary tumor growth following systemic administration of ANGIOZYME^{™} according to a certain dosing regimen in the LLC mouse model.
**Figure 4** shows a dose-dependent inhibition of tumor metastases following systemic administration of ANGIOZYME^{™} in a mouse colorectal model.
**Figure 5** is a graph showing the plasma concentration profile of ANGIOZYME^{™} after a single subcutaneous (SC) dose of 10, 30, 100 or 300 mg/m².
**Figure 6** shows examples of chemically stabilized ribozyme motifs. **HH Rz**, represents hammerhead ribozyme motif (Usman et al., 1996, Curr. Op. Struct. Bio., 1, 527); **NCH Rz** represents the NCH ribozyme motif (Ludwig et al., International PCT Publication No. WO 98/58058 and US Patent Application Serial No. 08/878,640); **G-Cleaver**, represents G-cleaver ribozyme motif (Kore et al., 1998, Nucleic Acids Research 26, 4116-4120, Eckstein et al., US 6,127,173). N or n, represent independently a nucleotide which can be same or different and have complementarity to each other; rI, represents ribo-Inosine nucleotide; arrow indicates the site of cleavage within the target. Position 4 of the HH Rz and the NCH Rz is shown as having 2'-*C*-allyl modification, but those skilled in the art will recognize that this position can be modified with other modifications well known in the art, so long as such modifications do not significantly inhibit the activity of the ribozyme.
**Figure 7** shows an example of a Zinzyme A ribozyme motif that is chemically stabilized (see for example Beigelman et al., International PCT publication No. WO 99/55857 and US Patent Application Serial No. 09/918,728).
**Figure 8** shows an example of a DNAzyme motif described by Santoro et al., 1997, PNAS, 94,4262 and Joyce et al., US 5,807,718.
**Figure 9** shows data demonstrating the inhibition of soluble VEGFR1 in a clinical study using ANGIOZYME (SEQ ID NO:5977).
**Figure 10** shows an generalized outline for the mouse model of proliferative retinopathy showing the points of ribozyme administration.
**Figure 11** shows a graph demonstrating the efficacy of a VEGF-receptor-targeted enzymatic nucleic acid molecule in a mouse model of proliferative retinopathy.

### Detailed Description of the Invention

### Nucleic Acid Molecules and Mechanism of Action

Enzymatic Nucleic Acid: Several varieties of naturally-occurring enzymatic nucleic acids are presently known. In addition, several *in vitro* selection (evolution) strategies (Orgel, 1979, Proc. R. Soc. London, B 205, 435) have been used to evolve new nucleic acid catalysts capable of catalyzing cleavage and ligation of phosphodiester linkages (Joyce, 1989, Gene, 82, 83-87; Beaudry et al., 1992, Science 257, 635-641; Joyce, 1992, Scientific American 267, 90-97; Breaker et al., 1994, TTBTECH 12, 268; Bartel et al., 1993, Science 261:1411-1418; Szostak, 1993, TIBS 17, 89-93; Kumar et al., 1995, FASEB J., 9, 1183; Breaker, 1996, Curr. Op. Biotech., 7, 442; Santoro et al., 1997, Proc. Natl. Acad. Sci., 94, 4262; Tang et al., 1997, RNA 3, 914; Nakamaye & Eckstein, 1994, *supra;* Long & Uhlenbeck, 1994, supra; Ishizaka *et al.,* 1995, *supra;* Vaish et al., 1997, Biochemistry 36, 6495; all of these are incorporated by reference herein). Each can catalyze a series of reactions including the hydrolysis of phosphodiester bonds in *trans* (and- thus can cleave other nucleic acid molecules) under physiological conditions.

The enzymatic nature of an enzymatic nucleic acid molecule has significant advantages, one advantage being that the concentration of enzymatic nucleic acid molecule necessary to affect a therapeutic treatment is lower. This advantage reflects the ability of the enzymatic nucleic acid molecule to act enzymatically. Thus, a single enzymatic nucleic acid molecule is able to cleave many molecules of target nucleic acid. In addition, the enzymatic nucleic acid molecule is a highly specific inhibitor, with the specificity of inhibition depending not only on the base-pairing mechanism of binding to the target nucleic acid , but also on the mechanism of target nucleic acid cleavage. Single mismatches, or base-substitutions, near the site of cleavage can be chosen to completely eliminate catalytic activity of a enzymatic nucleic acid molecule.

Nucleic acid molecules having an endonuclease enzymatic activity are able to repeatedly cleave other separate nucleic acid molecules in a nucleotide base sequence-specific manner. With the proper design, such enzymatic nucleic acid molecules can be targeted to RNA transcripts, and achieve efficient cleavage *in vitro* (Zaug et al., 324, Nature 429 1986; Uhlenbeck, 1987 Nature 328, 596; Kim et al., 84 Proc. Natl. Acad. Sci. USA 8788, 1987; Dreyfus, 1988, Einstein Quart. J. Bio. Med., 6, 92; Haseloff and Gerlach, 334 Nature 585, 1988; Cech; 260 JAMA 3030, 1988; and Jefferies et al., 17 Nucleic Acids Research 1371, 1989; Santoro *et al.,* 1997 *supra).*

Because of their sequence specificity, trans-cleaving enzymatic nucleic acid molecules can be used as therapeutic agents for human disease (Usman & McSwiggen, 1995 Ann. Rep. Med. Chem. 30, 285-294; Christoffersen and Marr, 1995 J. Med. Chem. 38, 2023-2037). Enzymatic nucleic acid molecules can be designed to cleave specific nucleic acid targets within the background of cellular nucleic acid. Such a cleavage event renders the nucleic acid non-functional and abrogates protein expression from that nucleic acid. In this manner, synthesis of a protein associated with a disease state can be selectively inhibited (Warashina et al., 1999, Chemistry and Biology, 6, 237-250).

Enzymatic nucleic acid molecules of the invention that are allosterically regulated ("allozymes") can be used to down-regulate VEGF and/or VEGFr, such as VEGFR1 and/or VEGFR2, expression. These allosteric enzymatic nucleic acids or allozymes (see for example Usman et al., US Patent Application No. 09/877,526, George et al., US Patent Nos. 5,834,186 and 5,741,679, Shih et al., US Patent No. 5,589,332, Nathan et al., US Patent No 5,871,914, Nathan and Ellington, International PCT publication No. WO 00/24931, Breaker et al., International PCT Publication Nos. WO 00/26226 and 98/27104, and Sullenger et al., US Patent Application Serial No. 09/205,520) are designed to respond to a signaling agent, for example, mutant VEGFR1 and/or VEGFR2 protein, wild-type VEGFR1 and/or VEGFR2 protein, mutant VEGFR1 and/or VEGFR2 RNA, wild-type VEGFR1 and/or VEGFR2 RNA, other proteins and/or RNAs involved in VEGF signal transduction, compounds, metals, polymers, molecules and/or drugs that are targeted to VEGFR1 and/or VEGFR2 expression, which in turn modulates the activity of the enzymatic nucleic acid molecule. In response to interaction with a predetermined signaling agent, the activity of the allosteric enzymatic nucleic acid is activated or inhibited such that the expression of a particular target is selectively down-regulated. The target can comprise wild-type VEGFR1 and/or VEGFR2, mutant VEGFR1 and/or VEGFR2, and/or a predetermined component of the VEGF signal transduction pathway. In a specific example, allosteric enzymatic nucleic acid molecules that are activated by interaction with a RNA encoding VEGF protein are used as therapeutic agents in *vivo.* The presence of RNA encoding the VEGF protein activates the allosteric enzymatic nucleic acid molecule that subsequently cleaves the RNA encoding a VEGFR1 and/or VEGFR2 protein resulting in the inhibition of VEGFR1 and/or VEGFR2 protein expression.

In another non-limiting example, an allozyme can be activated by a VEGF and/or VEGFr, such as VEGFR1 and/or VEGFR2 protein, peptide, or mutant polypeptide that causes the allozyme to inhibit the expression of VEGF and/or VEGFr, such as VEGFR1 and/or VEGFR2 genes, by, for example, cleaving RNA encoded by VEGF, VEGFR1 and/or VEGFR2 gene. In this non-limiting example, the allozyme acts as a decoy to inhibit the function of VEGF, VEGFR1 and/or VEGER2 and also inhibit the expression of VEGF, VEGFR1 and/or VEGFR2 once activated by the VEGF, VEGFR1 and/or VEGFR2 protein.

Antisense: Antisense molecules can be modified or unmodified RNA, DNA, or mixed polymer oligonucleotides and primarily function by specifically binding to matching sequences resulting in inhibition of peptide synthesis (Wu-Pong, Nov 1994, BioPharm, 20-33). The antisense oligonucleotide binds to target RNA by Watson Crick base-pairing and blocks gene expression by preventing ribosomal translation of the bound sequences either by steric blocking or by activating RNase H enzyme. Antisense molecules can also alter protein synthesis by interfering with RNA processing or transport from the nucleus into the cytoplasm (Mukhopadhyay & Roth, 1996, Crit. Rev. in Oncogenesis 7, 151-190).

In addition, binding of single stranded DNA to RNA can result in nuclease degradation of the heteroduplex (Wu-Pong, *supra;* Crooke, *supra).* To date, the only backbone modified DNA chemistry which act as substrates for RNase H are phosphorothioates, phosphorodithioates, and borontrifluoridates. Recently it has been reported that 2'-arabino and 2'-fluoro arabino- containing oligos can also activate RNase H activity.

A number of antisense molecules have been described that utilize novel configurations of chemically modified nucleotides, secondary structure, and/or RNase H substrate domains (Woolf et al., International PCT Publication No. WO 98/13526; Thompson et al., International PCT Publication No. WO 99/54459; Hartmann et al., USSN 60/101,174 which was filed on September 21, 1998) all of these are incorporated by reference herein in their entirety.

In addition, antisense deoxyoligoribonucleotides can be used to target RNA by means of DNA-RNA interactions, thereby activating RNase H, which digests the target RNA in the duplex. Antisense DNA can be expressed via the use of a single stranded DNA intracellular expression vector or equivalents and variations thereof.

Triplex Forming Oligonucleotides (TFO): Single stranded DNA can be designed to bind to genomic DNA in a sequence specific manner. TFOs are comprised of pyrimidine-rich oligonucleotides which bind DNA helices through Hoogsteen Base-pairing (Wu-Pong, *supra).* The resulting triple helix composed of the DNA sense, DNA antisense, and TFO disrupts RNA synthesis by RNA polymerase. The TFO mechanism can result in gene expression or cell death since binding can be irreversible (Mukhopadhyay & Roth, *supra*).

2-5A Antisense Chimera: The 2-5A system is an interferon mediated mechanism for RNA degradation found in higher vertebrates (Mitra et al., 1996, Proc Nat Acad Sci USA 93, 6780-6785). Two types of enzymes, 2-5A synthetase and RNase L, are required for RNA cleavage. The 2-5A synthetases require double stranded RNA to form 2'-5' oligoadenylates (2-5A). 2-5A then acts as an allosteric effector for utilizing RNase L which has the ability to cleave single stranded RNA. The ability to form 2-5A structures with double stranded RNA makes this system particularly useful for inhibition of viral replication.

(2'-5') oligoadenylate structures can be covalently linked to antisense molecules to form chimeric oligonucleotides capable of RNA cleavage (Torrence, *supra).* These molecules putatively bind and activate a 2-5A dependent RNase, the oligonucleotide/enzyme complex then binds to a target RNA molecule which can then be cleaved by the RNase enzyme.

RNAi: Double-stranded RNAs can suppress expression of homologous genes through an evolutionarily conserved process named RNA interference (RNAi) or post-transcriptional gene silencing (PTGS). One mechanism underlying silencing is the degradation of target mRNAs by an RNP complex, which contains short interfering RNAs (siRNAs) as guides to substrate selection. Short interfering RNAs are typically 21 to 23 nucleotides in length. A bidentate nuclease called Dicer has been implicated as the protein responsible for siRNA production. For example, a double-stranded RNA (dsRNA) matching a gene sequence is synthesized *in vitro* and introduced into a cell. The dsRNA feeds into a biological pathway and is broken into short pieces of short interfering (si) RNAs. With the help of cellular enzymes such as Dicer, the siRNA triggers the degradation of the messenger RNA that matches its sequence (see for example Tuschl et al., International PCT Publication No. WO 01/75164; Bass, 2001, Nature, 411,428-429; Elbashir et al., 2001, Nature, 411, 494-498; and Kreutzer et al., International PCT Publication No. WO 00/44895).

### Target sites

Targets for useful nucleic acid molecules of the invention, such as enzymatic nucleic acid molecules, dsRNA, and antisense nucleic acids can be determined as disclosed in Draper et al., WO 93/23569; Sullivan et al.; WO 93/23057; Thompson et al., WO 94/02595; Draper et al., WO 95/04818; McSwiggen et al., US Patent No. 5,525,468, and hereby incorporated by reference herein in totality. Other examples include the following PCT applications, which concern inactivation of expression of disease-related genes: WO 95/23225, WO 95/13380, WO 94/02595, incorporated by reference herein. Ramer than repeat the guidance provided in those documents here, below are provided specific examples of such methods, not limiting to those in the art. Enzymatic nucleic acid molecules and antisense to such targets are designed as described in those applications and synthesized to be tested *in vitro* and *in vivo,* as also described. The sequences of human VEGF, VEGFR1 and/or VEGFR2 RNAs are screened for optimal nucleic acid target sites using a computer-folding algorithm: Potential nucleic acid binding/cleavage sites are identified. While human sequences can be screened and nucleic acid molecules thereafter designed, as discussed in Stinchcomb et al., WO 95/23225, mouse targeted enzymatic nucleic acid molecules can be useful to test efficacy of action of the nucleic acid molecule prior to testing in humans.

Nucleic acid molecule binding/cleavage sites are identified, for example enzymatic nucleic acid, antisense, and dsRNA mediated binding sites are chosen. For enzymatic nucleic acid molecules of the invention, the nucleic acid molecules are individually analyzed by computer folding (Jaeger et al., 1989 Proc. Natl. Acad. Sci. USA, 86, 7706) to assess whether the sequences fold into the appropriate secondary structure. Those nucleic acid molecules with unfavorable intramolecular interactions such as between the binding arms and the catalytic core can be eliminated from consideration. Varying binding arm lengths can be chosen to optimize activity.

Nucleic acids, such as antisense, RNAi, and/or enzymatic nucleic acid molecule binding/cleavage sites are identified and are designed to anneal to various sites in the nucleic acid target. The binding arms of enzymatic nucleic acid molecules of the invention are complementary to the target site sequences described above. Antisense and RNAi sequences are designed to have partial or complete complementarity to the nucleic acid target. The nucleic acid molecules can be chemically synthesized. The method of synthesis used follows the procedure for normal DNA/RNA synthesis as described below and in Usman et al., 1987 J. Am Chem. Soc., 109, 7845; Scaringe et al., 1990 Nucleic Acids Res., 18, 5433; and Wincott et al., 1995 Nucleic Acids Res. 23, 2677-2684; Caruthers et al., 1992, Methods in Enzymology 211, 3-19.

### Synthesis of Nucleic acid Molecules

Synthesis of nucleic acids greate than 100 nucleotides in length is difficult using automated methods, and the therapeutic cost of such molecules is prohibitive. In this invention; small nucleic acid motifs ("small refers to nucleic acid motifs less than about 100 nucleotides in length, preferably less than about 80 nucleotides in length, and more preferably less than about 50 nucleotides in length; e.g., antisense oligonucleotides, enzymatic nucleic acids, aptamers, allozymes, decoys, siRNA etc.) are preferably used for exogenous delivery. The simple structure of these molecules increases the ability of the nucleic acid to invade targeted regions of RNA structure. Exemplary molecules of the instant invention are chemically synthesized, and others can similarly be synthesized.

DNA Oligonucleotides are synthesized using protocols known in the art as described in Caruthers et al., 1992, Methods in Enzymology 211, 3-19, Thompson et al., International PCT Publication No. WO 99/54459, Wincott et al., 1995, Nucleic Acids Res. 23, 2677-2684, Wincott et al., 1997, Methods Mol. Bio., 74, 59, Brennan et al., 1998, Biotechnol Bioeng., 61, 33-45, and Brennan, US patent No. 6,001,311. All of these references are incorporated herein by reference. The synthesis of oligonucleotides makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 µmol scale protocol with a 2.5 min coupling step for 2'-O-methylated nucleotides and a 45 sec coupling step for 2'-deoxy nucleotides. **Table II** outlines the amounts and the contact times of the reagents used in the synthesis cycle. Alternatively, syntheses at the 0.2 µmol scale can be performed on a 96-well plate synthesizer, such as the instrument produced by Protogene (Palo Alto, CA) with minimal modification to the cycle. A 33-fold excess (60 µL of 0.11 M = 6.6 µmol) of 2'-O-methyl phosphoramidite and a 105-fold excess-of S-ethyl tetrazole (60 µL of 0.25 M =15 µmol) can be used in each coupling cycle of 2'-O-methyl residues relative to polymer-bound 5'-hydroxyl. A 22-fold excess (40 µL of 0:11 M = 4.4 µmol) of deoxy phosphoramidite and a 70-fold excess of S-ethyl tetrazole (40 µL of 0.25 M = 10 µmol) can be used in each coupling cycle of deoxy residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer include; detritylation solution is 3% TCA in methylene chloride (ABI); capping is performed with 16% *N*-methyl imidazole in THF (ABI) and 10% acetic anhydride/10% 2,6-lutidine in THF (ABI); and oxidation solution is 16.9 mM I₂, 49 mM pyridine, 9% water in THF (PERSEPTIVE^{™}). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzodithiol-3-one 1,1-dioxide, 0.05 M in acetonitrile) is used.

Deprotection of the DNA polynucleotides is performed as follows: the polymer-bound trityl-on oligoribonucleotide is transferred to a 4 mL glass screw top vial and suspended in a solution of 40% aq. methylamine (1 mL) at 65 °C for 10 min. After cooling to -20°C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder.

The method of synthesis used for RNA oligonucleotides including certain nucleic acid molecules of the invention follows the procedure as described in Usman et al., 1987, J. Am. Chem. Soc., 109, 7845; Scaringe et al., 1990, Nucleic Acids Res., 18, 5433; and Wincott et al., 1995, Nucleic Acids Res. 23, 2677-2684 Wincott et al., 1997, Methods Mol. Bio., 74, 59, and makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 µmol scale protocol with a 7.5 min coupling step for alkylsilyl protected nucleotides and a 2.5 min coupling step for 2'-O-methylated nucleotides. **Table II** outlines the amounts and the contact times of the reagents used in the synthesis cycle. Alternatively, syntheses at the 0.2 µmol scale can be done on a 96-well plate synthesizer, such as the instrument produced by Protogene (Palo Alto, CA) with minimal modification to the cycle. A 33-fold excess (60 µL of 0.11 M = 6.6 µmol) of 2'-O-methyl phosphoramidite and a 75-fold excess of S-ethyl tetrazole (60 µL of 0.25 M = 15 µmol) can be used in each coupling cycle of 2'-O-methyl residues relative to polymer-bound 5'-hydroxyl. A 66-fold excess (120 µL of 0.11 M = 13.2 µmol) of alkylsilyl (ribo) protected phosphoramidite and a 150 fold excess of S-ethyl tetrazole (120 µL of 0.25 M = 30 µmol) can be used in each coupling cycle of ribo residues relative to polymer-bound 5'-hydroxyl. Average coupling yields on the 394 Applied Biosystems, Inc. synthesizer; determined by colorimetric quantitation of the trityl fractions, are typically 97.5-99%. Other oligonucleotide synthesis reagents for the 394 Applied Biosystems; Inc. synthesizer include; detritylation solution is 3% TCA in methylene chloride (ABI); capping is performed with 16% *N*-methyl imidazole in THF (ABI) and 10% acetic anhydride/10% 2,6-lutidine in THF (ABI); oxidation solution is 16.9 mM I₂, 49 mM pyridine, 9% water in THF (PERSEPTIVE^{™}). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzodithiol-3-one 1,1-dioxide0.05 M in acetonitrile) is used.

Deprotection of the RNA is performed using either a two-pot or one-pot protocol. For the two-pot protocol, the polymer-bound trityl-on oligoribonucleotide is transferred to a 4 mL glass screw top vial and suspended in a solution of 40% aq. methylamine (1 mL) at 65 °C for 10 min. After cooling to -20 °C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder. The base deprotected oligoribonucleotide is resuspended in anhydrous TEA/HF/NMP solution (300 µL of a solution of 1.5 mL N-methylpyrrolidinone, 750 µL TEA and 1 mL TEA•3HF to provide a 1.4 M HF concentration) and heated to 65 °C. After 1.5 h, the oligomer is quenched with 1.5 M NH₄HCO₃.

Alternatively, for the one-pot protocol, the polymer-bound trityl-on oligoribonucleotide is transferred to a 4 mL glass screw top vial and suspended in a solution of 33% ethanolic methylamine/DMSO: 1/1 (0.8 mL) at 65 °C for 15 min. The vial is brought to r.t. TEA•3HF (0.1 mL) is added and the vial is heated at 65 °C for 15 min. The sample is cooled at -20 °C and then quenched with 1.5 M NH₄CO₃.

For purification of the trityl-on oligomers, the quenched NH₄HCO₃ solution is loaded onto a C-18 containing cartridge that bad been prewashed with acetonitrile followed by 50 mM TEAA. After washing the loaded cartridge with water, the RNA is detritylated with 0.5% TFA for 13 min. The cartridge is then washed again with water, salt exchanged with 1 M NaCl and washed with water again. The oligonucleotide is then eluted with 30% acetonitrile.

Inactive hammerhead ribozymes or binding attenuated control (BAC) oligonucleotides) are synthesized by substituting a U for G₅ and a U for A₁₄ (numbering from Hertel, K.J., et al., 1992, Nucleic Acids Res., 20, 3252). Similarly, one or more nucleotide substitutions can be introduced in other enzymatic nucleic acid molecules to inactivate the molecule and such molecules can serve as a negative control.

The average stepwise coupling yields are typically >98% (Wincott et al., 1995 Nucleic Acids Res. 23, 2677-2684). Those of ordinary skill in the art will recognize that the scale of synthesis can be adapted to be larger or smaller than the example described above including but not limited to 96 well format, all that is important is the ratio of chemicals used in the reaction.

Alternatively, the nucleic acid molecules of the present invention can be synthesized separately and joined together post-synthetically, for example by ligation (Moore et al., 1992, Science 256, 9923; Draper et al., International PCT publication No. WO 93/23569; Shabarova et al., 1991, Nucleic Acids Research 19; 4247; Bellon et al., 1997, Nucleosides & Nucleotides, 16, 951; Bellon et al., 1997, Bioconjugate Chem. 8, 204).

Preferably, the nucleic acid molecules of the present invention are modified extensively to enhance stability by modification with nuclease resistant groups, for example, 2'-amino, 2'-Gallyl, 2'-flouro, 2'-*O*-methyl, 2'-H (for a review see Usman and Cedergren, 1992, TIBS 17, 34; Usman et aL, 1994, Nucleic Acids Symp. Ser. 31, 163). Ribozymes are purified by gel electrophoresis using general methods or are purified by high pressure liquid chromatography (HPLC; See Wincott *et al.,* Supra, the totality of which is hereby incorporated herein by reference) and are re-suspended in water.

### Optimizing Activity of the nucleic acid molecule of the invention.

Chemically synthesizing nucleic acid molecules with modifications (base, sugar and/or phosphate) that prevent their degradation by serum ribonucleases can increase their potency (see *e.g.,* Eckstein et al., International Publication No. WO 92/07065; Perrault et al., 1990 Nature 344, 565; Pieken et al., 1991, Science 253, 314; Usman and Cedergren, 1992, Trends in Biochem. Sci. 17, 334; Usman et al., International Publication No. WO 93/15187; and Rossi et al., International Publication No. WO 91/03162; Sproat, US Patent No. 5,334;711; Gold et al., US 6,300,074; and *Burgin et al., supra;* all of which are incorporated by reference herein). Modifications which enhance their efficacy in cells, and removal of bases from nucleic acid molecules to shorten oligonucleotide synthesis times and reduce chemical requirements are desired. (All these publications are hereby incorporated by reference herein).

There are several examples in the art describing sugar, base and phosphate modifications that can be introduced into nucleic acid molecules with significant enhancement in their nuclease stability and efficacy. For example, oligonucleotides are modified to enhance stability and/or enhance biological activity by modification with nuclease resistant groups, for example, 2'-amino, 2'-*C*-allyl, 2'-flouro, 2'-*O*-methyl, 2'-H, nucleotide base modifications (for a review see Usman and Cedergren, 1992, TIBS. 17, 34; Usman et al., 1994, Nucleic Acids Symp. Ser. 31, 163; Burgin et al., 1996, Biochemistry, 35, 14090). Sugar modification of nucleic acid molecules have been extensively described in the art (see Eckstein et al., International Publication PCT No. WO 92/07065; Perrault et al. Nature, 1990, 344, 565-568; Pieken et al. Science, 1991, 253, 314-317; Usman and Cedergren, Trends in Biochem. Sci., 1992, 17, 334-339; Usman *et al.* International Publication PCT No. WO 93/15187; Sproat, US Patent No. 5,334,711 and Beigelman et al., 1995, J. Biol. Chem., 270, 25702; Beigelman et al., International PCT publication No. WO 97/26270; Beigelman et al., US Patent No. 5,716,824; Usman et al., US patent No. 5,627,053; Woolf et al., International PCT Publication No. WO 98/13526; Thompson et al., USSN 60/082,404 which was filed on April 20, 1998; Karpeisky et al., 1998, Tetrahedron Lett., 39, 1131; Earnshaw and Gait, 1998, Biopolymers (Nucleic acid Sciences), 48, 39-55; Verma and Eckstein, 1998, Annu. Rev. Biochem., 67, 99-134; and Burlina et al., 1997, Bioorg. Med. Chem., 5, 1999-2010; all of the references are hereby incorporated in their totality by reference herein). Such publications describe general methods and strategies to determine the location of incorporation of sugar, base and/or phosphate modifications and the like into ribozymes without inhibiting catalysis, and are incorporated by reference herein. In view of such teachings, similar modifications can be used as described herein to modify the nucleic acid molecules of the instant invention.

While chemical modification of oligonucleotide internucleotide linkages with phosphorothioate, phosphorothioate, and/or 5'-methylphosphonate linkages improves stability, too many of these modifications can cause some toxicity. Therefore when designing nucleic acid molecules the amount of these internucleotide linkages should be minimized. The reduction in the concentration of these linkages should lower toxicity resulting in increased efficacy and higher specificity of these molecules.

Nucleic acid molecules having chemical modifications that maintain or enhance activity are provided. Such nucleic acid is also generally more resistant to nucleases than unmodified nucleic acid. Thus, in a cell and/or *in vivo* the activity may not be significantly lowered. Therapeutic nucleic acid molecules delivered exogenously are optimally stable within cells until translation of the target RNA has been inhibited long enough to reduce the levels of the undesirable protein. This period of time varies between hours to days depending upon the disease state. Clearly, nucleic acid molecules must be resistant to nucleases in order to function as effective intracellular therapeutic agents. Improvements in the chemical synthesis of RNA and DNA (Wincott et al., 1995 Nucleic Acids Res. 23, 2677; Caruthers et al., 1992, Methods in Enzymology 211,3-19 (incorporated by reference herein) have expanded the ability to modify nucleic acid molecules by introducing nucleotide modifications to enhance their nuclease stability as described above.

In one embodiment, nucleic acid molecules of the invention include one or more G-clamp nucleotides. A G-clamp nucleotide is a modified cytosine analog wherein the modifications confer the ability to hydrogen bond both Watson-Crick and Hoogsteen faces of a complementary guanine within a duplex, see for example Lin and Matteucci, 1998, J. Am. Chem. Soc., 120, 8531-8532. A single G-clamp analog substitution within an oligonucleotide can result in substantially enhanced helical thermal stability and mismatch discrimination when hybridized to complementary oligonucleotides. The inclusion of such nucleotides in nucleic acid molecules of the invention results in both enhanced affinity and specificity to nucleic acid targets. In another embodiment, nucleic acid molecules of the invention include one or more LNA "locked nucleic acid" nucleotides such as a 2', 4'-C mythylene bicyclo nucleotide (see for example Wengel et al., International PCT Publication No. WO 00/66604 and WO 99/14226).

In another embodiment, the invention features conjugates and/or complexes of nucleic acid molecules targeting VEGF receptors such as VEGFR1 and/or VEGFR2. Such conjugates and/or complexes can be used to facilitate delivery of molecules into a biological system, such as cells. The conjugates and complexes provided by the instant invention can impart therapeutic activity by transferring therapeutic compounds across cellular membranes, altering the pharmacokinetics, and/or modulating the localization of nucleic acid molecules of the invention. The present invention encompasses the design and synthesis of novel conjugates and complexes for the delivery of molecules, including but not limited to small molecules, lipids, phospholipids, nucleosides, nucleotides, nucleic acids, antibodies, toxins, negatively charged polymers and other polymers, for example proteins, peptides, hormones, carbohydrates, polyethylene glycols, or polyamines, across cellular membranes. In general, the transporters described are designed to be used either individually or as part of a multi-component system, with or without degradable linkers. These compounds are expected to improve delivery and/or localization of nucleic acid molecules of the invention into a number of cell types originating from different tissues, in the presence or absence of serum (see Sullenger and Cech, US 5,854;038). Conjugates of the molecules described herein can be attached to biologically active molecules via linkers that are biodegradable, such as biodegradable nucleic acid linker molecules.

The term "biodegradable nucleic acid linker molecule" as used herein, refers to a nucleic acid molecule that is designed as a biodegradable linker to connect one molecule to another molecule, for example, a biologically active molecule. The stability of the biodegradable nucleic acid linker molecule can be modulated by using various combinations of ribonucleotides, deoxyribonucleotides, and chemically modified nucleotides, for example, 2'-O-methyl, 2'-fluoro, 2'-amino, 2'-O-amino, 2'-C-allyl, 2'-O-allyl, and other 2'-modified or base modified nucleotides. The biodegradable nucleic acid linker molecule can be a dimer, trimer, tetramer or longer nucleic acid molecule, for example, an oligonucleotide of about 2, 3, 4, 5, 6, 7, 8, 9, 10, 11, 12, 13, 14, 15, 16, 17, 18, 19, or 20 nucleotides in length, or can comprise a single nucleotide with a phosphorus based linkage, for example, a phosphoramidate or phosphodiester linkage. The biodegradable nucleic acid linker molecule can also comprise nucleic acid backbone, nucleic acid sugar, or nucleic acid base modifications.

The term "biodegradable" as used herein, refers to degradation in a biological system, for example enzymatic degradation or chemical degradation.

The term "biologically active molecule" as used herein, refers to compounds or molecules that are capable of eliciting or modifying a biological response in a system. Non-limiting examples of biologically active molecules contemplated by the instant invention include therapeutically active molecules such as antibodies, hormones, antivirals, peptides, proteins, chemotherapeutics, small molecules, vitamins, co-factors, nucleosides, nucleotides, oligonucleotides, enzymatic nucleic acids, antisense nucleic acids, triplex forming oligonucleotides, 2,5-A chimeras, siRNA, dsRNA, allozymes, aptamers, decoys and analogs thereof. Biologically active molecules of the invention also include molecules capable of modulating the pharmacokinetics and/or pharmacodynamics of other biologically active molecules, for example, lipids and polymers such as polyamines, polyamides, polyethylene glycol and other polyethers.

The term "phospholipid" as used herein, refers to a hydrophobic molecule comprising at least one phosphorus group. For example, a phospholipid can comprise a phosphorus containing group and saturated or unsaturated alkyl group, optionally substituted with OH, COOH, oxo, amine, or substituted or unsubstituted aryl groups.

Therapeutic nucleic acid molecules (e.g., enzymatic nucleic acid molecules and antisense nucleic acid molecules) delivered exogenously are optimally stable within cells until translation of the target RNA has been inhibited long enough to reduce the levels of the undesirable protein. This period of time varies between hours to days depending upon the disease state. These nucleic acid molecules should be resistant to nucleases in order to function as effective intracellular therapeutic agents. Improvements in the chemical synthesis of nucleic acid molecules described in the instant invention and in the art have expanded the ability to modify nucleic acid molecules by introducing nucleotide modifications to enhance their nuclease stability as described above.

In another embodiment, nucleic acid catalysts having chemical modifications that maintain or enhance enzymatic activity are provided. Such nucleic acids are also generally more resistant to nucleases than unmodified nucleic acid. Thus, in a cell and/or in *vivo* the activity of the nucleic acid may not be significantly lowered. As exemplified herein such enzymatic nucleic acids are useful in a cell and/or *in vivo* even if activity over all is reduced 10 fold (Burgin et al., 1996, Biochemistry, 35, 14090). Such enzymatic nucleic acids herein are said to "maintain" the enzymatic activity of an all RNA ribozyme or all DNA DNAzyme.

In another aspect the nucleic acid molecules comprise a 5' and/or a 3'-cap structure.

By "cap structure" is meant chemical modifications, which have been incorporated at either terminus of the oligonucleotide (see for example Wincott et al., WO 97/26270, incorporated by reference herein). These terminal modifications protect the nucleic acid molecule from exonuclease degradation, and can help in delivery and/or localization within a cell. The cap can be present at the 5'-terminus (5'-cap) or at the 3'-terminus (3'-cap) or can be present on both terminus. In non-limiting examples, the 5'-cap includes inverted abasic residue (moiety), 4',5'-methylene nucleotide; 1-(beta-D-erythrofuranosyl) nucleotide, 4'-thio nucleotide, carbocyclic nucleotide; 1,5-anhydrohexitol nucleotide; L-nucleotides; alpha-nucleotides; modified base nucleotide; phosphorodithioate linkage; *threo*-pentofuranosyl nucleotide; acyclic 3',4'-seco nucleotide; acyclic 3,4-dihydroxybutyl nucleotide; acyclic 3,5-dihydroxypentyl nucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety (for more details see Wincott et al., International PCT publication No. WO 97/26270, incorporated by reference herein).

In another embodiment the 3'-cap includes, for example 4',5'-methylene nucleotide; 1-(beta-D-erythrofuranosyl) nucleotide; 4'-thio nucleotide, carbocyclic nucleotide; 5'-aminoalkyl phosphate; 1,3-diamino-2-propyl phosphate, 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; *threo*-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inverted abasic moiety; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moieties (for more details see Beaucage and Iyer, 1993, Tetrahedron 49, 1925; incorporated by reference herein).

By the term "non-nucleotide" is meant any group or compound which can be incorporated into a nucleic acid chain in the place of one or more nucleotide units, including either sugar and/or phosphate substitutions, and allows the remaining bases to exhibit their enzymatic activity. The group or compound is abasic in that it does not contain a commonly recognized nucleotide base, such as adenosine, guanine, cytosine, uracil or thymine.

An "alkyl" group refers to a saturated aliphatic hydrocarbon, including straight-chain, branched-chain, and cyclic alkyl groups. Preferably, the alkyl group has 1 to 12 carbons. More preferably it is a lower alkyl of from 1 to 7 carbons, more preferably 1 to 4 carbons. The alkyl group can be substituted or unsubstituted. When substituted the substituted group(s) is preferably, hydroxyl, cyano, alkoxy, =O, =S, NO₂ or N(CH3)₂, amino, or SH. The term also includes alkenyl groups which are unsaturated hydrocarbon groups containing at least one carbon-carbon double bond, including straight-chain, branched-chain, and cyclic groups. Preferably, the alkenyl group has 1 to 12 carbons. More preferably it is a lower alkenyl of from 1 to 7 carbons, more preferably 1 to 4 carbons. The alkenyl group can be substituted or unsubstituted. When substituted the substituted group(s) is preferably, hydroxyl, cyano, alkoxy, =O, =S, NO₂, halogen, N(CH₃)₂, amino, or SH. The term "alkyl" also includes alkynyl groups which have an unsaturated hydrocarbon group containing at least one carbon-carbon triple bond, including straight-chain, branched-chain, and cyclic groups. Preferably, the alkynyl group has 1 to 12 carbons. More preferably it is a lower alkynyl of from 1 to 7 carbons, more preferably 1 to 4 carbons. The alkynyl group can be substituted or unsubstituted. When substituted the substituted group(s) is preferably, hydroxyl, cyano, alkoxy, =O, =S, NO₂ or N(CH₃)₂, amino or SH.

Such alkyl groups can also include aryl, alkylaryl, carbocyclic aryl, heterocyclic aryl, amide and ester groups. An "aryl" group refers to an aromatic group which has at least one ring having a conjugated p electron system and includes carbocyclic aryl, heterocyclic aryl and biaryl groups, all of which can be optionally substituted. The preferred substituent(s) of aryl groups are halogen, trihalomethyl, hydroxyl, SH, OH, cyano, alkoxy, alkyl, alkenyl, alkynyl, and amino groups. An "alkylaryl" group refers to an alkyl group (as described above) covalently joined to an aryl group (as described above). Carbocyclic aryl groups are groups wherein the ring atoms on the aromatic ring are all carbon atoms. The carbon atoms are optionally substituted. Heterocyclic aryl groups are groups having from 1 to 3 heteroatoms as ring atoms in the aromatic ring and the remainder of the ring atoms are carbon atoms. Suitable heteroatoms include oxygen, sulfur, and nitrogen, and include furanyl, thienyl, pyridyl, pyrrolyl, N-lower alkyl pyrrolo, pyrimidyl, pyrazinyl, imidazolyl and the like, all optionally substituted. An "amide" refers to an -C(O)-NH-R, where R is either alkyl, aryl, alkylaryl or hydrogen. An "ester" refers to an -C(O)-OR', where R is either alkyl, aryl, alkylaryl or hydrogen.

By "nucleotide" is meant a heterocyclic nitrogenous base in N-glycosidic linkage with a phosphorylated sugar. Nucleotides are recognized in the art to include natural bases (standard), and modified bases well known in the art Such bases are generally located at the 1' position of a nucleotide sugar moiety. Nucleotides generally comprise a base, sugar and a phosphate group. The nucleotides can be unmodified or modified at the sugar, phosphate and/or base moiety, (also referred to interchangeably as nucleotide analogs, modified nucleotides, non-natural nucleotides, non-standard nucleotides and other, see for example, Usman and McSwiggen, supra; Eckstein et al., International PCT Publication No. WO 92/07065; Usman et al., International PCT Publication No. WO 93/15187; Uhlman & Peyman, supra all are hereby incorporated by reference herein). There are several examples of modified nucleic acid bases known in the art as summarized by Limbach et al., 1994, Nucleic Acids Res. 22, 2183. Some of the non-limiting examples of chemically modified and other natural nucleic acid bases that can be introduced into nucleic acids include, for example, inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (e.g., 5-methylcytidine), 5-alkyluridines (e.g., ribothymidine), 5-halouridine (e.g., 5-bromouridine) or 6-azapyrimidines or 6-alkylpyrimidines (e.g. 6-methyluridine), propyne, quesosine, 2-thiouridine, 4-thiouridine, wybutosine, wybutoxosine, 4-acetylcytidine, 5-(carboxyhydroxymethyl)uridine, 5'-carboxymethylaminomethyl-2-thiouridine, 5-carboxymethylaminomethyluridine; beta-D-galactosylqueosine, 1-methyladenosine, 1-methylinosine, 2,2-dimethylguanosine; 3-methylcytidine, 2₋methyladenosine, 2-methylguanosine, N6-methyladenosine, 7-methylguanosine, 5-methoxyaminomethyl-2-thiouridine, 5-methylaminomethyluridine, 5-methylcarbonylmethyluridine, 5-methyloxyuridine, 5-methyl-2-thiouridine, 2-methylthio-N6-isopentenyladenosine, beta-D-mannosylqueosine, uridine-5-oxyacetic acid; 2-thiocytidine, threonine derivatives and others (Burgin et al., 1996, Biochemistry, 35, 14090; Uhlman & Peyman, supra). By "modified bases" in this aspect is meant nucleotide bases other than adenine, guanine, cytosine and uracil at 1' position or their equivalents; such bases can be used at any position, for example, within the catalytic core of an enzymatic nucleic acid molecule and/or in the substrate binding regions of the nucleic acid molecule.

By "nucleoside" is meant a heterocyclic nitrogenous base in N-glycosidic linkage with a sugar. Nucleosides are recognized in the art to include natural bases (standard), and modified bases well known in the art. Such bases are generally located at the 1' position of a nucleoside sugar moiety. Nucleosides generally comprise a base and sugar group. The nucleosides can be unmodified or modified at the sugar, and/or base moiety, (also referred to interchangeably as nucleoside analogs, modified nucleosides, non-natural nucleosides, non-standard nucleosides and other, see for example, Usman and McSwiggen, supra; Eckstein et al., International PCT Publication No. WO 92/07065; Usman et al., International PCT Publication No. WO 93/15187; Uhlman & Peyman, supra all are hereby incorporated by reference herein). There are several examples of modified nucleic acid bases known in the art as summarized by Limbach et al., 1994, Nucleic Acids Res. 22, 2183. Some of the non-limiting examples of chemically modified and other natural nucleic acid bases that can be introduced into nucleic acids include, inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (e.g., 5-methylcytidine), 5-alkyluridines (e.g., ribothymidine), 5-halouridine (e.g., 5-bromouridine) or 6-azapyrimidines or 6-alkylpyrimidines (e.g. 6-methyluridine), propyne, quesosine, 2-thiouridine, 4-thiouridine, wybutosine, wybutoxosine, 4-acetylcytidine, 5-(carboxyhydroxymethyl)uridine, 5'-carboxymethylaminomethyl-2-thiouridine, 5-carboxymethylaminomethyluridine, beta-D-galactosylqueosine, 1-methyladenosine, 1-methylinosine, 2,2-dimethylguanosine, 3-methylcytidine, 2-methyladenosine, 2-methylguanosine, N6-methyladenosine, 7-methylguanosine, 5-methoxyaminomethyl-2-thiouridine, 5-methylaminomethyluridine, 5-methylcarbonylmethyluridine, 5-methyloxyuridine, 5-methyl-2-thiouridine, 2-methylthio-N6-isopentenyladenosine, beta-D-mannosylqueosine, uridine-5-oxyacetic acid, 2-thiocytidine, threonine derivatives and others (Burgin et al., 1996, Biochemistry, 35, 14090; Uhlman & Peyman, supra). By "modified bases" in this aspect is meant nucleoside bases other than adenine, guanine, cytosine and uracil at 1' position or their equivalents; such bases can be used at any position, for example, within the catalytic core of an enzymatic nucleic acid molecule and/or in the substrate-binding regions of the nucleic acid molecule.

In one embodiment, the invention features modified enzymatic nucleic acid molecules with phosphate backbone modifications comprising one or more phosphorothioate, phosphorodithioate, methylphosphonate, morpholino, amidate carbamate, carboxymethyl, acetamidate, polyamide, sulfonate, sulfonamide, sulfamate, formacetal, thioformacetal, and/or alkylsilyl, substitutions. For a review of oligonucleotide backbone modifications see Hunziker and Leumann, 1995, Nucleic Acid Analogues: Synthesis and Properties, in Modern Synthetic Methods, VCH, 331-417, and Mesmaeker et al., 1994, Novel Backbone Replacements for Oligonucleotides, in Carbohydrate Modifacations in Antisense Research, ACS, 24-39. These references are hereby incorporated by reference herein.

By "abasic" is meant sugar moieties lacking a base or having other chemical groups in place of a base at the 1' position, for example a 3',3'-linked or 5',5'-linked deoxyabasic ribose derivative (for more details see Wincott et al., International PCT publication No. WO 97/26270).

By "unmodified nucleoside" is meant one of the bases adenine, cytosine, guanine, thymine, uracil joined to the 1' carbon of β-D-ribo-furanose.

By "modified nucleoside" is meant any nucleotide base which contains a modification in the chemical structure of an unmodified nucleotide base, sugar and/or phosphate.

In connection with 2'-modified nucleotides as described for the present invention, by "amino" is meant 2'-NH₂ or 2'-*O*- NH₂, which can be modified or unmodified. Such modified groups are described, for example, in Eckstein et al., U.S. Patent 5,672,695 and Matulic-Adamic et al., WO 98/28317, respectively, which are both incorporated by reference in their entireties.

Various modifications to nucleic acid (e.g., antisense and ribozyme) structure can be made to enhance the utility of these molecules. For example, such modifications can enhance shelf-life, half-life *in vitro,* stability, and ease of introduction of such oligonucleotides to the target site, including, e.g., enhancing penetration of cellular membranes and conferring the ability to recognize and bind to targeted cells.

Use of the nucleic acid-based molecules of the invention can lead to better treatment of the disease progression by affording the possibility of combination therapies (e.g., multiple enzymatic nucleic acid molecules targeted to different genes, enzymatic nucleic acid molecules coupled with known small molecule inhibitors; or intermittent treatment with combinations of enzymatic nucleic acid molecules (including different enzymatic nucleic acid molecule motifs) and/or other chemical or biological molecules). The treatment of patients with nucleic acid molecules can also include combinations of different types of nucleic acid molecules. Therapies can be devised which include a mixture of enzymatic nucleic acid molecules (including different enzymatic nucleic acid molecule motifs), allozymes, antisense, dsRNA, aptamers, and/or 2-5A chimera molecules to one or more targets to alleviate symptoms of a disease.

### Administration of Nucleic Acid Molecules

Methods for the delivery of nucleic acid molecules are described in Akhtar et al., 1992, Trends Cell Bio., 2, 139; and Delivery Strategies for Antisense Oligonucteotide Therapeutics, ed. Akhtar, 1995 which are both incorporated herein by reference. Sullivan et al., PCT WO 94/02595, further describes the general methods for delivery of enzymatic RNA molecules. These protocols can be utilized for the delivery of virtually any nucleic acid molecule. Nucleic acid molecules can be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. Alternatively, the nucleic acid/vehicle combination is locally delivered by direct injection or by use of an infusion pump. Other routes of delivery include, but are not limited to oral (tablet or pill form) and/or intrathecal delivery (Gold, 1997, Neuroscience, 76, 1153-1158). Other approaches include the use of various transport and carrier systems, for example though the use of conjugates and biodegradable polymers. For a comprehensive review on drug delivery strategies including CNS delivery, see Ho et al., 1999, Curr. Opin. Mol. Ther., 1, 336-343 and Jain, Drug Delivery Systems: Technologies and Commercial Opportunities, Decision Resources, 1998 and Groothuis et al., 1997, J. NeuroVirol., 3, 387-400. More detailed descriptions of nucleic acid delivery and administration are provided in *Sullivan et al.,* supra, Draper et al., PCT WO93/23569, Beigelman et al., PCT WO99/05094, and Klimuk et al., PCT WO99/04819 all of which have been incorporated by reference herein.

The molecules of the instant invention can be used as pharmaceutical agents. Pharmaceutical agents prevent, inhibit the occurrence, or treat (alleviate a symptom to some extent, preferably all of the symptoms) of a disease state in a patient.

The polynucleotides of the invention can be administered (e.g., RNA, DNA or protein) and introduced into a patient by any standard means, with or without stabilizers, buffers, and the like, to form a pharmaceutical composition. When it is desired to use a liposome delivery mechanism, standard protocols for formation of liposomes can be followed. The compositions of the present invention can also be formulated and used as tablets, capsules or elixirs for oral administration; suppositories for rectal administration; sterile solutions; suspensions for injectable administration; and the other compositions known in the art.

The present invention also includes pharmaceutically acceptable formulations of the compounds described. These formulations include salts of the above compounds, *e.g.,* acid addition salts, for example, salts of hydrochloric, hydrobromic, acetic acid, and benzene sulfonic acid.

A pharmacological composition or formulation refers to a composition or formulation in a form suitable for administration, *e.g.,* systemic administration, into a cell or patient, preferably a human. Suitable forms, in part, depend upon the use or the route of entry, for example oral, transdermal, or by injection. Such forms should not prevent the composition or formulation from reaching a target cell (*i.e.,* a cell to which the negatively charged polymer is desired to be delivered to). For example, pharmacological compositions injected into the blood stream should be soluble. Other factors are known in the art, and include considerations such as toxicity and forms which prevent the composition or formulation from exerting its effect.

By "systemic administration" is meant *in vivo* systemic absorption or accumulation of drugs in the blood stream followed by distribution throughout the entire body. Administration routes which lead to systemic absorption include, without limitations: intravenous, subcutaneous, intraperitoneal, inhalation, oral, intrapulmonary and intramuscular. Each of these administration routes expose the desired negatively charged polymers, e.g., nucleic acids, to an accessible diseased tissue. The rate of entry of a drug into the circulation has been shown to be a function of molecular weight or size. The use of a liposome or other drug carrier comprising the compounds of the instant invention can potentially localize the drug, for example, in certain tissue types, such as the tissues of the reticular endothelial system (RES). A liposome formulation which can facilitate the association of drug with the surface of cells, such as, lymphocytes and macrophages is also useful. This approach can provide enhanced delivery of the drug to target cells by taking advantage of the specificity of macrophage and lymphocyte immune recognition of abnormal cells, such as cells implicated in endometriosis, birth control, endometrial tumors, gynecologic bleeding disorders, irregular menstrual cycles, ovulation, premenstrual syndrome (PMS), menopausal dysfunction; and endometrial carcinoma.

By pharmaceutically acceptable formulation is meant, a composition or formulation that allows for the effective distribution of the nucleic acid molecules of the instant invention in the physical location most suitable for their desired activity. Non-limiting examples of agents suitable for formulation with the nucleic acid molecules of the instant invention include: PEG conjugated nucleic acids, phospholipid conjugated nucleic acids, nucleic acids containing lipophilic moieties, phosphorothioates, P-glycoprotein inhibitors (such as Pluronic P85) which can enhance entry of drugs into various tissues, for example the CNS (Jolliet-Riant and Tillement, 1999, Fundam. Clin. Pharmacol., 13, 16-26); biodegradable polymers, such as poly (DL-lactide-coglycolide) microspheres for sustained release delivery after implantation (Emerich, DF et al, 1999, Cell Transplant, 8, 47-58) Alkermes, Inc. Cambridge, MA; and loaded nanoparticles, such as those made of polybutylcyanoacrylate, which can deliver drugs across the blood brain barrier and can alter neuronal uptake mechanisms *(*Prog Neuropsychopharmacol Biol Psychiatry, 23, 941-949, 1999). Other non-limiting examples of delivery strategies, including CNS delivery of the nucleic acid molecules of the instant invention include material described in Boado et al., 1998, J. Pharm. Sci., 87, 1308-1315; Tyler et al., 1999, FEBS Lett., 421, 280-284; Pardridge et al., 1995, PNAS USA., 92, 5592-5596; Boado, 1995, Adv. Drug Delivery Rev., 15, 73-107; Aldrian-Herrada et al., 1998, Nucleic Acids Res., 26, 4910-4916; and Tyler et al., 1999, PNAS USA., 96, 7053-7058. All these references are hereby incorporated herein by reference.

The invention also features the use of the composition comprising surface-modified liposomes containing poly (ethylene glycol) lipids (PEG-modified, or long-circulating liposomes or stealth liposomes). Nucleic acid molecules of the invention can also comprise covalently attached PEG molecules of various molecular weights. These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al. Chem. Rev. 1995, 95, 2601-2627; Ishiwata et al., Chem. Pharm. Bull. 1995, 43, 1005-1011). Such liposomes have been shown to accumulate selectively in tumors, presumably by extravasation and capture in the neovascularized target tissues (Lasic et al., Science 1995, 267, 1275-1276; Oku et al., 1995, Biochim. Biophys. Acta, 1238, 86-90). The long-circulating liposomes enhance the pharmacokinetics and pharmacodynamics of DNA and RNA, particularly compared to conventional cationic liposomes which are known to accumulate in tissues of the MPS (Liu et al., J. Biol. Chem. 1995, 42, 24864-24870; Choi et al., International PCT Publication No. WO 96/10391; Ansell et al., International PCT Publication No. WO 96/10390; Holland et al., International PCT Publication No. WO 96/10392; all of which are incorporated by reference herein). Long-circulating liposomes are also likely to protect drugs from nuclease degradation to a greater extent compared to cationic liposomes, based on their ability to avoid accumulation in metabolically aggressive MPS tissues such as the liver and spleen. All of these references are incorporated by reference herein.

The present invention also includes compositions prepared for storage or administration which include a pharmaceutically effective amount of the desired compounds in a pharmaceutically acceptable carrier or diluent. Acceptable carriers or diluents for therapeutic use are well known in the pharmaceutical art, and are described, for example, in *Remington's Pharmaceutical Sciences,* Mack Publishing Co. (A.R. Gennaro edit. 1985) hereby incorporated by reference herein. For example, preservatives, stabilizers, dyes and flavoring agents can be provided. These include sodium benzoate, sorbic acid and esters of *p-*hydroxybenzoic acid. In addition, antioxidants and suspending agents can be used.

A pharmaceutically effective dose is that dose required to prevent, inhibit the occurrence, or treat (alleviate a symptom to some extent, preferably all of the symptoms) of a disease state. The pharmaceutically effective dose depends on the type of disease, the composition used, the route of administration, the type of mammal being treated, the physical characteristics of the specific mammal under consideration, concurrent medication, and other factors which those skilled in the medical arts will recognize. Generally, an amount between 0.1 mg/kg and 100 mg/kg body weight/day of active ingredients is administered dependent upon potency of the negatively charged polymer.

The nucleic acid molecules of the invention and formulations thereof can be administered orally, topically, parenterally, by inhalation or spray or rectally in dosage unit formulations containing conventional non-toxic pharmaceutically acceptable carriers, adjuvants and vehicles. The term parenteral as used herein includes percutaneous, subcutaneous, intravascular (e.g., intravenous), intramuscular, or intrathecal injection or infusion techniques and the like. In addition, there is provided a pharmaceutical formulation comprising a nucleic acid molecule of the invention and a pharmaceutically acceptable carrier. One or more nucleic acid molecules of the invention can be present in association with one or more non-toxic pharmaceutically acceptable carriers and/or diluents and/or adjuvants, and if desired other active ingredients. The pharmaceutical compositions containing nucleic acid molecules of the invention can be in a form suitable for oral use, for example, as tablets, troches, lozenges, aqueous or oily suspensions, dispersible powders or granules, emulsion, hard or soft capsules, or syrups or elixirs.

Compositions intended for oral use can be prepared according to any method known to the art for the manufacture of pharmaceutical compositions and such compositions can contain one or more such sweetening agents, flavoring agents, coloring agents or preservative agents in order to provide pharmaceutically elegant and palatable preparations. Tablets contain the active ingredient in admixture with non-toxic pharmaceutically acceptable excipients that are suitable for the manufacture of tablets. These excipients can be for example, inert diluents, such as calcium carbonate, sodium carbonate, lactose, calcium phosphate or sodium phosphate; granulating and disintegrating agents, for example, com starch, or alginic acid; binding agents, for example starch, gelatin or acacia, and lubricating agents, for example magnesium stearate, stearic acid or talc. The tablets can be uncoated or they can be coated by known techniques. In some cases such coatings can be prepared by known techniques to delay disintegration and absorption in the gastrointestinal tract and thereby provide a sustained action over a longer period. For example, a time delay material such as glyceryl monosterate or glyceryl distearate can be employed.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents and flavoring agents can be added to provide palatable oral preparations. These compositions can be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, can also be present.

Pharmaceutical compositions of the invention can also be in the form of oil-in-water emulsions. The oily phase can be a vegetable oil or a mineral oil or mixtures of these. Suitable emulsifying agents can be naturally occurring gums, for example gum acacia or gum tragacanth, naturally occurring phosphatides, for example soy bean, lecithin, and esters or partial esters derived from fatty acids and hexitol, anhydrides, for example sorbitan monooleate; and condensation products of the said partial esters with ethylene oxide, for example polyoxyethylene sorbitan monooleate. The emulsions can also contain sweetening and flavoring agents.

Syrups and elixirs can be formulated with sweetening agents, for example glycerol, propylene glycol, sorbitol, glucose or sucrose. Such formulations can also contain a demulcent, a preservative and flavoring and coloring agents. The pharmaceutical compositions can be in the form of a sterile injectable aqueous or oleaginous suspension. This suspension can be formulated according to the known art using those suitable dispersing or wetting agents and suspending agents that have been mentioned above. The sterile injectable preparation can also be a sterile injectable solution or suspension in a non-toxic parentally acceptable diluent or solvent, for example as a solution in 1,3-butanediol. Among the acceptable vehicles and solvents that can be employed are water, Ringer's solution and isotonic sodium chloride solution. In addition, sterile, fixed oils are conventionally employed as a solvent or suspending medium. For this purpose any bland fixed oil can be employed including synthetic mono-or diglycerides. In addition, fatty acids such as oleic acid find use in the preparation of injectables,

The nucleic acid molecules of the invention can also be administered in the form of suppositories, e.g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Nucleic acid molecules of the invention can be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

Dosage levels of the order of from about 0.1 mg to about 140 mg per kilogram of body weight per day are useful in the treatment of the above-indicated conditions (about 0.5 mg to about 7 g per patient per day). The amount of active ingredient that can be combined with the carrier materials to produce a single dosage form varies depending upon the host treated and the particular mode of administration. Dosage unit forms generally contain between from about 1 mg to about 500 mg of an active ingredient.

It is understood that the specific dose level for any particular patient depends upon a variety of factors including the activity of the specific compound employed, the age, body weight, general health, sex, diet, time of administration, route of administration, and rate of excretion, drug combination and the severity of the particular disease undergoing therapy.

For administration to non-human animals, the composition can also be added to the animal feed or drinking water. It can be convenient to formulate the animal feed and drinking water compositions so that the animal takes in a therapeutically appropriate quantity of the composition along with its diet. It can also be convenient to present the composition as a premix for addition to the feed or drinking water.

The nucleic acid molecules of the present invention can also be administered to a patient in combination with other therapeutic compounds to increase the overall therapeutic effect. The use of multiple compounds to treat an indication can increase the beneficial effects while reducing the presence of side effects.

Alternatively, certain of the nucleic acid molecules of the instant invention can be expressed within cells from eukaryotic promoters (*e.g.,* Izant and Weintraub, 1985, Science, 229, 345; McGarry and Lindquist, 1986, Proc. Natl. Acad. Sci., USA 83, 399; Scanlon et al., 1991, Proc. Natl. Acad. Sci. USA, 88, 10591-5; Kashani-Sabet et al., 1992, Antisense Res. Dev., 2, 3-15; Dropulic et al., 1992, J. Virol., 66, 1432-41; Weerasinghe et al., 1991, J. Virol., 65, 5531-4; Ojwang et al., 1992, Proc. Natl. Acad. Sci. USA, 89, 10802-6; Chen et al., 1992, Nucleic Acids Res., 20, 4581-9; Sarver et al., 1990 Science, 247, 1222-1225; Thompson et al., 1995, Nucleic Acids Res., 23, 2259; Good et al.; 1997, Gene Therapy, 4,45; all of these references are hereby incorporated in their totalities by reference herein). Those skilled in the art realize that any nucleic acid can be expressed in eukaryotic cells from the appropriate DNA/RNA vector. The activity of such nucleic acids can be augmented by their release from the primary transcript by a enzymatic nucleic acid (Draper et al., PCT WO 93/23569, and Sullivan et al., PCT WO 94/02595; Ohkawa et al., 1992, Nucleic Acids Symp. Ser., 27, 15-6; Taira et al., 1991, Nucleic Acids Res., 19, 5125-30; Ventura et al, 1993, Nucleic Acids Res., 21, 3249-55; Chowrira et al., 1994, J. Biol. Chem., 269, 25856; all of these references are hereby incorporated in their totalities by reference herein). Gene therapy approaches specific to the CNS are described by Blesch et al., 2000, Drug News Perspect., 13, 269-280; Peterson et al., 2000, Cent. Nerv. Syst Dis., 485-508; Peel and Klein, 2000; J. Neurosci. Methods, 98, 95-104; Hagihara et al., 2000, Gene Ther., 7, 759-763; and Herrlinger et al., 2000, Methods Mol. Med., 35, 287-312. AAV-mediated delivery of nucleic acid to cells of the nervous system is further described by Kaplitt et al., US 6,180,613.

In another aspect of the invention, RNA molecules of the present invention are preferably expressed from transcription units (see for example Couture et al., 1996, TIG., 12, 510) inserted into DNA or RNA vectors. The recombinant vectors are preferably DNA plasmids or viral-vectors. Ribozyme expressing viral vectors can be constructed based on, but not limited to, adeno-associated virus, retrovirus, adenovirus, or alphavirus. Preferably, the recombinant vectors capable of expressing the nucleic acid molecules are delivered as described above, and persist in target cells. Alternatively, viral vectors can be used that provide for transient expression of nucleic acid molecules. Such vectors can be repeatedly administered as necessary. Once expressed, the nucleic acid molecule binds to the target mRNA. Delivery of nucleic acid molecule expressing vectors can be systemic, such as by intravenous or intra-muscular administration, by administration to target cells ex-planted from the patient followed by reintroduction into the patient, or by any other means that would allow for introduction into the desired target cell (for a review see Couture et al., 1996, TIG., 12, 510).

In one aspect the invention features an expression vector comprising a nucleic acid sequence encoding at least one of the nucleic acid molecules of the instant invention. The nucleic acid sequence encoding the nucleic acid molecule of the instant invention is operably linked in a manner which allows expression of that nucleic acid molecule.

In another aspect the invention features an expression vector comprising: a) a transcription initiation region (e.g., eukaryotic pol I, II or III initiation region); b) a transcription termination region (e.g., eukaryotic pol I, II or III termination region); c) a nucleic acid sequence encoding at least one of the nucleic acid catalyst of the instant invention; and wherein said sequence is operably linked to said initiation region and said termination region, in a manner which allows expression and/or delivery of said nucleic acid molecule. The vector can optionally include an open reading frame (ORF) for a protein operably linked on the 5' side or the 3'-side of the sequence encoding the nucleic acid catalyst of the invention; and/or an intron (intervening sequences).

Transcription of the nucleic acid molecule sequences are driven from a promoter for eukaryotic RNA polymerase I (pol I), RNA polymerase II (pol II), or RNA polymerase III (pol III). Transcripts from pol II or pol III promoters are expressed at high levels in all cells; the levels of a given pol II promoter in a given cell type depends on the nature of the gene regulatory sequences (enhancers, silencers, etc.) present nearby. Prokaryotic RNA polymerase promoters are also used, providing that the prokaryotic RNA polymerase enzyme is expressed in the appropriate cells (Elroy-Stein and Moss, 1990, Proc. Natl. Acad. Sci. U S A, 87, 6743-7; Gao and Huang 1993, Nucleic Acids Res.., 21, 2867-72; Lieber et al., 1993, Methods Enzymol., 217, 47-66; Zhou et al., 1990, Mol. Cell. Biol., 10, 4529-37). All of these references are incorporated by reference herein. Several investigators have demonstrated that nucleic acid molecules, such as ribozymes expressed from such promoters can function in mammalian cells (e.g. Kashani-Sabet et al., 1992, Antisense Res. Dev., 2,3-15; Ojwang et al., 1992, Proc. Natl. Acad. Sci. USA, 89, 10802-6; Chen et al., 1992, Nucleic Acids Res., 20, 4581-9; Yu et al., 1993, Proc. Natl. Acad. Sci. USA, 90, 6340-4; L'Huillier et al., 1992, EMBO J., 11, 4411-8; Lisziewicz et al., 1993, Proc. Natl. Acad. Sci. U.S.A, 90, 8000-4; Thompson et al., 1995, Nucleic Acids Res., 23, 2259; Sullenger & Cech, 1993, Science, 262, 1566). More specifically, transcription units such as the ones derived from genes encoding U6 small nuclear (snRNA), transfer RNA (tRNA) and adenovirus VA RNA are useful in generating high concentrations of desired RNA molecules such as ribozymes in cells (Thompson *et al., supra;* Couture and Stinchcomb, *1996, supra;* Noonberg et al., 1994, Nucleic Acid Res., 22, 2830; Noonberg et al., US Patent No. 5,624,803; Good et al., 1997, Gene Ther., 4, 45; Beigelman *et al.,* International PCT Publication No. *WO 96*/*18736;* all of these publications are incorporated by reference herein. The above ribozyme transcription units can be incorporated into a variety of vectors for introduction into mammalian cells, including but not restricted to, plasmid DNA vectors, viral DNA vectors (such as adenovirus or adeno-associated virus vectors), or viral RNA vectors (such as retroviral or alphavirus vectors) (for a review see Couture and Stinchcomb, *1996, supra*).

In another aspect the invention features an expression vector comprising nucleic acid sequence encoding at least one of the nucleic acid molecules of the invention, in a manner which allows expression of that nucleic acid molecule. The expression vector comprises in one embodiment; a) a transcription initiation region; b) a transcription termination region; c) a nucleic acid sequence encoding at least one said nucleic acid molecule; and wherein said sequence is operably linked to said initiation region and said termination region, in a manner which allows expression and/or delivery of said nucleic acid molecule.

In another embodiment the expression vector comprises: a) a transcription initiation region; b) a transcription termination region; c) an open reading frame; d) a nucleic acid sequence encoding at least one said nucleic acid molecule, wherein said sequence is operably linked to the 3'-end of said open reading frame; and wherein said sequence is operably linked to said initiation region, said open reading frame and said termination region, in a manner which allows expression and/or delivery of said nucleic acid molecule. In yet another embodiment the expression vector comprises: a) a transcription initiation region; b) a transcription termination region; c) an intron; d) a nucleic acid sequence encoding at least one said nucleic acid molecule; and wherein said sequence is operably linked to said initiation region, said intron and said termination region, in a manner which allows expression and/or delivery of said nucleic acid molecule.

In another embodiment, the expression vector comprises: a) a transcription initiation region; b) a transcription termination region; c) an intron; d) an open reading frame; e) a nucleic acid sequence encoding at least one said nucleic acid molecule, wherein said sequence is operably linked to the 3'-end of said open reading frame; and wherein said sequence is operably linked to said initiation region, said intron, said open reading frame and said termination region, in a manner which allows expression and/or delivery of said nucleic acid molecule.

Flt-1 (VEGFR1), KDR (VEGFR2) and/or flk-1 are attractive nucleic acid-based therapeutic targets by several criteria. The interaction between VEGF and VEGF-R is well-established. Efficacy can be tested in well-defined and predictive animal models. Finally, the disease conditions are serious and current therapies are inadequate. Whereas protein-based therapies are designed to affect VEGF activity, nucleic acid-based therapy based on the molecules and methods described herein provides a direct and elegant approach to directly modulate flt-1, KDR and/or flk-1 expression.

Because VEGFR1 and VEGFR2 mRNAs are highly homologous in certain regions, some nucleic acid target sites are also homologous. In this case, a single nucleic acid molecule of the invention can target both VEGFR1 and VEGFR2 mRNAs. At partially homologous sites, a single nucleic acid molecule can sometimes be designed to accommodate a site on both mRNAs by including G/U base pairing. For example, if there is a G present in a enzymatic nucleic acid target site in VEGFR1 mRNA at the same position there is an A in the VEGFR2 enzymatic nucleic acid target site, the enzymatic nucleic acid can be synthesized with a U at the complementary position and it will bind both to sites. The advantage of one enzymatic nucleic acid that targets both VEGFR1 and VEGFR2 mRNAs is clear, especially in cases where both VEGF receptors may contribute to the progression of angiogenesis in the disease state.

### Examples

The following are non-limiting examples showing the selection, isolation, synthesis and activity of exemplary nucleic acids of the instant invention.

The following examples demonstrate the selection and design of antisense; aptamer, dsRNA, allozyme, hammerhead, DNAzyme, NCH, Amberzyme, Zinzyme, or G-Cleaver ribozyme molecules and binding/cleavage sites within VEGF, VEGFR1 and/or VEGFR2 RNA.

### Example 1: Enzymatic nucleic acid-mediated inhibition of angiogenesis in vivo

The study described below was performed to assess the anti-angiogenic activity of hammerhead ribozymes targeted against flt-1 4229 site (SED ID-NO: 5977) in the rat cornea model of VEGF induced angiogenesis (see above). These ribozymes have either active or inactive catalytic core and either bind and cleave or just bind to VEGF-R mRNA of the flt-1 subtype. The active ribozymes, that are able to bind and cleave the target RNA, have been shown to inhibit (¹²⁵I-labeled) VEGF binding in cultured endothelial cells and produce a dose-dependent decrease in VEGF induced endothelial cell proliferation in these cells. The catalytically inactive forms of these ribozymes, which can only bind to the RNA but cannot catalyze RNA cleavage, failed to inhibit VEGF binding and failed to decrease VEGF induced endothelial cell proliferation. The ribozymes and VEGF were co-delivered using the filter disk method: Nitrocellulose filter disks (Millipore^{®}) of 0.057 diameter were immersed in appropriate solutions and were surgically implanted in rat cornea as described by Pandey *et al., supra.* This delivery method has been shown to deliver rhodamine-labeled free ribozyme to scleral cells and, in all likelihood cells of the pericorneal vascular plexus. Since the active ribozymes show cell culture efficacy and can be delivered to the target site using the disk method, it is essential that these ribozymes be assessed for *in vivo* anti-angiogenic activity.

The stimulus for angiogenesis in this study was the treatment of the filter disk with 30 µM VEGF which is implanted within the cornea's stroma. This dose yields reproducible neovascularization stemming from the pericorneal vascular plexus growing toward the disk in a dose-response study 5 days following implant. Filter disks treated only with the vehicle for VEGF show no angiogenic response. The ribozymes were co-adminstered with VEGF on a disk in two different ribozyme concentrations. One concern with the simultaneous administration is that the ribozymes will not be able to inhibit angiogenesis since VEGF receptors can be stimulated. However, we have observed that in low VEGF doses, the neovascular response reverts to normal suggesting that the VEGF stimulus is essential for maintaining the angiogenic response. Blocking the production of VEGF receptors using simultaneous administration of anti-VEGF-R mRNA ribozymes could attenuate the normal neovascularization induced by the filter disk treated with VEGF.

### Materials and Methods:

1. Stock hammerhead ribozyme_solutions:
   a. flt-1 4229 (786 µM)-Active
   b. flt-1 4229 (736 µM)-Inactive
2. Experimantal solutions/groups:
   Group 1 Solution 1 Control VEGF solution: 30 µM in 82mM Tris base
   Group 2 Solution 2 flt-1 4229 (1 µg/µL) in 30 µM VEGF/82 mM Tris base
   Group 3 Solution 3 flt-1 4229 (10 µg/µL) in 30 µM VEGF/82 mM Tris base
   Group 4 Solution 4 No VEGF, flt-1 4229 (10 µg/µL) in 82 mM Tris base
   Group 5 Solution 5 No VEGF, No ribozyme in 82 mM Tris base
   10 eyes per group, 5 animals (Since they have similar molecular weights, the molar concentrations should be essentially similar).
      Each solution (VEGF and RIBOZYMES) were prepared as a 2X solution for 1:1 mixing for final concentrations above, with the exception of solution 1 in which VEGF was 2X and diluted with ribozyme diluent (sterile water).
3. VEGF Solutions
   The 2X VEGF solution (60 µM) was prepared from a stock of 0.82 µg/µL in 50 mM Tris base. 200 µL of VEGF stock was concentrated by speed vac to a final volume of 60.8 µL, for a final concentration of 2.7 µg/µL or 60 µM. Six 10 µL aliquots was prepared for daily mixing. 2X solutions for VEGF and Ribozyme was stored at 4°C until the day of the surgery. Solutions were mixed for each day of surgery. Original 2X solutions was prepared on the day before the first day of the surgery.
4. Surgical Solutions:
   Anesthesia**:**
      stock ketamine hydrochloride 100 mg/mL
      stock xylazine hydrochloride 20 mg/mL
      stock acepromazine 10 mg/mL
   Final anesthesia solution: 50 mg/mL ketamine, 10 mg/mL xylazine, and 0.5 mg/mL acepromazine
      5% povidone iodine for opthalmic surgical wash
      2% lidocaine (sterile) for opthalmic administration (2 drops per eye)
      sterile 0.9% NaCl for opthalmic irrigation
5. Surgical Methods:
   Standard surgical procedure as described in Pandey *et al., supra.* Filter disks were incubated in 1 µL of each solution for approximately 30 minutes prior to implantation.
6. Experimental Protocol:
   The animal cornea were treated with the treatment groups as described above. Animals were allowed to recover for 5 days after treatment with daily observation (scoring 0 - 3). On the fifth day animals were euthanized and digital images of each eye was obtained for quantitaion using Image Pro Plus. Quantitated neovascular surface area were analyzed by ANOVA followed by two post-hoc tests including Dunnets and Tukey-Kramer tests for significance at the 95% confidence level. Dunnets provide information on the significance between the differences within the means of treatments vs. controls while Tukey-Kramer provide information on the significance of differences within the means of each group.
   The flt-1 4229 (SEQ ID NO: 5977) active hammerhead ribozyme at both concentrations was effective at inhibiting angiogenesis while the inactive ribozyme did not show any significant reduction in angiogenesis. A statistically signifiant reduction in neovascular surface area was observed only with active ribozymes. This result clearly shows that the ribozymes are capable of significantly inhibiting angiogenesis *in vivo.* Specifically, given ribozyme mechanism of action, the observed inhibition is by the binding and cleavage of target RNA by ribozymes.

### Example 2: Bioactivity of anti-angiogenesis ribozymes targeting flt-1 and kdr RNA

### MATERIALS AND METHODS

**Ribozymes:** Hammerhead ribozymes and controls designed to have attenuated activity (attenuated controls) were synthesized and purified as previously described above. The attenuated ribozyme controls maintain the binding arm sequence of the parent ribozyme and thus are still capable of binding to the mRNA target. However, they have two nucleotide changes in the core sequence that substantially reduce their ability to carry out the cleavage reaction. Ribozymes were designed to target *Flt-1* or *KDR* mRNA sites conserved in human, mouse, and rat. In general, ribozymes with binding arms of seven nucleotides were designed and tested. If, however, only six nucleotides surrounding the cleavage site were conserved in all three species, six nucleotide binding arms were used. Data are presented herein for 2'-NH₂ uridine modified ribozymes in cell proliferation studies and for 2'-*C-*allyl uridine modified ribozymes in RNAse protection, *in vitro* cleavage and corneal studies.

***In vitro* ribozyme cleavage assays:** *In vitro* RNA cleavage rates on a 15 nucleotide synthetic RNA substrate were measured as previously described above.

**Cell culture:** Human dermal microvascular endothelial cells (HMVEC-d, Clonetics Corp.) were maintained at 37°C in flasks or plates coated with 1.5% porcine skin gelatin (300 bloom, Sigma) in Growth medium (Clonetics Corp.) supplemented with 10-20% fetal bovine serum (FBS, Hyclone). Cells were grown to confluency and used up to the seventh passage. Stimulation medium consisted of 50% Sigma 99 media and 50% RPMI 1640 with L-glutamine and additional supplementation with 10 µg/mL Insulin-Transferrin-Selenium (Gibco BRL) and 10% FBS. Cell growth was stimulated by incubation in Stimulation medium supplemented with 20 ng/mL of either VEGF₁₆₅ or bFGF. VEGF₁₆₅ (165 amino acids) was selected for cell culture and animal studies because it is the predominant form of the four native forms of VEGF generated by alternative mRNA splicing. Cell culture assays were carried out in triplicate.

### Ribozyme and ribozyme/LIPOFECTAMINE^{™} formulations:

*Cell culture:* Ribozymes or attenuated controls (50-200 nM) were formulated for cell culture studies and used immediately. Formulations were carried out with LIPOFECTAMINE^{™} (Gibco BRL) at a 3:1 lipid to phosphate charge ratio in serum-free medium (OPTI-MEM^{™}, Gibco BRL) by mixing for 20 minutes at room temperature. For example, a 3:1 lipid to phosphate charge ratio was established by complexing 200 nM ribozyme with 10.8 µg/µL LIPOFECTAMINE^{™} (13.5 µM DOSPA).

*In vivo:* For corneal studies, lyophilized ribozyme or attenuated controls were resuspended in sterile water at a final stock concentration of 170 µg/µL (highest dose). Lower doses (1.7-50 µg/µL) were prepared by serial dilution in sterile water.

**Proliferation assay:** HMVEC-d were seeded (5 x 10³ cells/well) in 48-well plates (Costar) and incubated 24-30 hours in Growth medium at 37°C. After removal of the Growth medium, cells were treated with 50-200 nM LIPOFECTAMINE^{™} complexes of ribozyme or attenuated controls for 2 hours in OPTI-MEM^{™}. The ribozyme/control-containing medium was removed and the cells were washed extensively in 1X PBS. The medium was then replaced with Stimulation medium or Stimulation medium supplemented with 20 ng/mL VEGF₁₆₅ or bFGF. After 48 hours, the cell number was determined using a Coulter^{™} cell counter. Data are presented as cell number per well following 48 hours of VEGF stimulation.

**RNAse protection assay:** HMVEC-d were seeded (2 x 10⁵ cells/well) in 6-well plates (Costar) and allowed to grow 32-36 hours in Growth medium at 37°C. Cells were treated with LIPOFECTAMINE^{™} complexes containing 200 nM ribozyme or attenuated control for 2 h as described under "Proliferation Assay" and then incubated in Growth medium containing 20 ng/mL VEGF₁₆₅ for 24 hours. Cells were harvested and an RNAse protection assay was carried out using the Ambion Direct Protect kit and protocol with the exception that 50 mM EDTA was added to the lysis buffer to eliminate the possibility of ribozyme cleavage during sample preparation. Antisense RNA probes targeting portions of *Flt-1* and KDR were prepared by transcription in the presence of [³²P]-UTP. Samples were analyzed on polyacrylamide gels and the level of protected RNA fragments was quantified using a Molecular Dynamics PhosphorImager. The levels of *Flt*-1 and KDR were normalized to the level of cyclophilin (human cyclophilin probe template, Ambion) in each sample. The coefficient of variation for cyclophilin levels was 11% [265940 cpm ± 29386 (SD)] for all conditions tested here (*i.e.* in the presence of either active ribozymes or attenuated controls). Thus, cyclophilin is useful as an internal standard in these studies.

### Rat corneal pocket assay of VEGF-induced angiogenesis:

*Animal guidelines and anesthesia.* Animal housing and experimentation adhered to standards outlined in the 1996 Guide for the Care and Use of Laboratory Animals (National Research Council). Male Sprague Dawley rats (250-300 g) were anesthetized with ketamine (50 mg/kg), xylazine (10 mg/kg), and acepromazine (0.5 mg/kg) administered intramuscularly (im). The level of anesthesia was monitored every 2-3 min by applying hind limb paw pressure and examining for limb withdrawal. Atropine (0.4 mg/kg, im) was also administered to prevent potential corneal reflex-induced bradycardia.

*Preparation of VEGF soaked disk.* For corneal implantation, 0.57 mm diameter nitrocellulose disks, prepared from 0.45 µm pore diameter nitrocellulose filter membranes (Millipore Corporation), were soaked for 30 min in 1 µL of 30 µM VEGF₁₆₅ in 82 mM Tris HCl (pH 6.9) in covered petri dishes on ice.

*Corneal surgery.* The rat corneal model used in this study was a modified from Koch *et al. Supra* and Pandey *et al., supra.* Briefly, corneas were irrigated with 0.5% povidone iodine solution followed by normal saline and two drops of 2% lidocaine. Under a dissecting microscope (Leica MZ-6), a stromal pocket was created and a presoaked filter disk (see above) was inserted into the pocket such that its edge was 1 mm from the corneal limbus.

*Intraconjunctival injection of test solutions.* Immediately after disk insertion, the tip of a 40-50 µm OD injector (constructed in our laboratory) was inserted within the conjunctival tissue 1 mm away from the edge of the corneal limbus that was directly adjacent to the VEGF-soaked filter disk. Six hundred nanoliters of test solution (ribozyme, attenuated control or sterile water vehicle) were dispensed at a rate of 1.2 µL/min using a syringe pump (Kd Scientific). The injector was then removed, serially rinsed in 70% ethanol and sterile water and immersed in sterile water between each injection. Once the test solution was injected, closure of the eyelid was maintained using microaneurism clips until the animal began to recover gross motor activity. Following treatment, animals were warmed on a heating pad at 37°C.

*Animal treatment groups*/*experimental protocol.* Ribozymes targeting *Flt-1* site 4229 (SEQ ID NO: 5977) and *KDR* mRNA site 726 (SEQ ID NO: 5978) were tested in the corneal model along with their attenuated controls. Five treatment groups were assigned to examine the effects of five doses of each test substance over a dose range of 1-100 µg on VEGF-stimulated angiogenesis. Negative (30 µM VEGF soaked filter disk and intraconjunctival injection of 600 nL sterile water) and no stimulus (Tris-soaked filter disk and intraconjunctival injection of sterile water) control groups were also included. Each group consisted of five animals (10 eyes) receiving the same treatment.

*Quantitation of angiogenic response.* Five days after disk implantation, animals were euthanized following im administration of 0.4 mg/kg atropine and corneas were digitally imaged. The neovascular surface area (NSA, expressed in pixels) was measured *postmortem* from blood-filled corneal vessels using computerized morphometry (Image Pro Plus, Media Cybernetics, v2.0). The individual mean NSA was determined in triplicate from three regions of identical size in the area of maximal neovascularization between the filter disk and the limbus. The number of pixels corresponding to the blood-filled corneal vessels in these regions was summated to produce an index of NSA. A group mean NSA was then calculated. Data from each treatment group were normalized to VEGF/ribozyme vehicle-treated control NSA and finally expressed as percent inhibition of VEGF-induced angiogenesis.

*Statistics.* After determining the normality of treatment group means, group mean percent inhibition of VEGF-induced angiogenesis was subjected to a one-way analysis of variance. This was followed by two post-hoc tests for significance including Dunnett's (comparison to VEGF control) and Tukey-Kramer (all other group mean comparisons) at alpha = 0.05. Statistical analyses were performed using JMP v.3.1.6 (SAS Institute).

### RESULTS

**Ribozyme-mediated reduction of VEGF-induced cell proliferation:** Ribozyme cleavage of *Flt-1 or* KDR mRNA should result in a decrease in the density of cell surface VEGF receptors. This decrease should limit VEGF binding and consequently interfere with the mitogenic signaling induced by VEGF. To determine if cell proliferation was impacted by *anti-Flt-1* and/or anti-*KDR* ribozyme treatment, proliferation assays using cultured human microvascular cells were carried out. Ribozymes included in the proliferation assays were initially chosen by their ability to decrease the level of VEGF binding to treated cells. In these initial studies, ribozymes targeting 20 sites in the coding region of each mRNA were screened. The most effective ribozymes against two sites in each target, *Flt-1* sites 1358 and 4229 and *KDR* sites 726 and 3950, were included in the proliferation assays reported here. In addition, attenuated analogs of each ribozyme were used as controls. These attenuated controls are still capable of binding to the mRNA target since the binding arm sequence is maintained. However, these controls have two nucleotide changes in the core sequence that substantially reduce their ability to carry out the cleavage reaction.

The active ribozymes tested decreased the relative proliferation of HMVEC-d after VEGF stimulation, an effect that increased with ribozyme concentration. This concentration dependency was not observed following treatment with the attenuated controls designed for these sites. In fact, little or no change in cell growth was noted following treatment with the attenuated controls, even though these controls can still bind to the specific target sequences. At 200 nM, there was a distinct "window" between the anti-proliferative effects of each ribozyme and its attenuated control; a trend also observed at lower doses. This window of inhibition of proliferation (56-77% based on total cells/well) reflects the contribution of ribozyme-mediated activity. In comparison, no effect of *anti-Flt-1* or anti-*KDR* ribozymes was noted on bFGF-stimulated cell proliferation. Moreover, an irrelevant, but active, ribozyme whose binding sequence is not found in either *Flt-1* or KDR mRNA had no effect in this assay. These data are consistent with the basic ribozyme mechanism in which binding and cleavage are necessary components. Although the relative surface distribution of *Flt*-1 and KDR receptors in this cell type is not known, the antiproliferative effects of these ribozymes indicate that, at least in cell culture, both receptors are functionally coupled to proliferation.

**Specific reduction of *Flt-1 or KDR* mRNA by ribozyme treatment**: To confirm that anti*-Flt-1* and anti-*KDR* ribozymes reduce their respective mRNA targets, cellular levels of *Flt-1* or *KDR* were quantified using an RNAse protection assay with specific *Flt-1 or KDR* probes. For each target, one ribozyme/attenuated control pair was chosen for continued study. Exposure of HMVEC-d to active ribozyme targeting *Flt-1* site 4229 decreased *Flt-1* mRNA, but not *KDR* mRNA. Likewise, treatment with the active ribozyme targeting KDR site 726 decreased KDR, but not *Flt-1* mRNA. Both ribozymes decreased the level of their respective target RNA by greater than 50%. The degree of reduction associated with the corresponding attenuated controls was not greater than 13%.

### In vitro activity of anti-Flt and anti-KDR ribozymes.

To confirm further the necessity of an active ribozyme core, *in vitro* cleavage activities were determined for the *Flt-1* site 4229 ribozyme and the KDR site 726 ribozyme as well as their paired attenuated controls. The first order rate constants calculated from the time-course of short substrate cleavage for the anti-*Flt-1* ribozyme and its attenuated control were 0.081 ± 0.0007 min⁻¹ and 0.001 ± 6 x 10⁻⁵ min⁻¹, respectively. For the anti-*KDR* ribozyme and its paired control, the first order rate constants were 0.434 ± 0.024 min⁻¹ and 0.002 ± 1 x 10⁻⁴ min⁻¹, respectively. Although the attenuated controls retain a very slight level of cleavage activity under these optimized conditions, the decrease in *in-vitro* cleavage activity between each active ribozyme and its paired attenuated control is about two orders of magnitude. Thus, an active core is essential for cleavage activity *in vitro* and is also necessary for ribozyme activity in cell culture.

**Ribozyme-mediated reduction of VEGF-induced angiogenesis *in vivo.*** To assess whether ribozymes targeting VEGF receptor mRNA could impact the complex process of angiogenesis, prototypic anti-*Flt*-*1* and *KDR* ribozymes that were identified in cell culture studies were screened in a rat corneal pocket assay of VEGF-induced angiogenesis. In this assay, corneas implanted with VEGF-containing filter disks exhibited a robust neovascular response in the corneal region between the disk and the corneal limbus (from which the new vessels emerge). Disks containing a vehicle solution elicited no angiogenic response. In separate studies, intraconjunctival injections of sterile water vehicle did not affect the magnitude of the VEGF-induced angiogenic response. In addition, ribozyme injections alone did not induce angiogenesis.

The dose-related effects of anti-*Flt*-*1* or *KDR* ribozymes on the VEGF induced angiogenic response were then examined. The antiangiogenic effect of the anti-*Flt-1* (site 4229) and *KDR* (site 726) ribozymes and their attenuated controls over a dose range from 1 to 100 µg, respectively was determined. For both ribozymes, the maximal antiangiogenic response (48 and 36% for anti-*Flt-1* and KDR ribozymes, respectively) was observed at a dose of 10 µg.

The anti-*Flt-1* ribozyme produced a significantly greater antiangiogenic response than its attenuated control at 3 and 10 µg (p<0.05). Its attenuated control exhibited a small but significant antiangiogenic response at doses above 10 µg compared to vehicle treated VEGF controls (p<0.05). At its maximum, this response was not significantly greater than that observed with the lowest dose of active anti-*Flt*-1 ribozyme. The anti-*KDR* ribozyme significantly inhibited angiogenesis from 3 to 30 µg (p<0.05). The anti-*KDR* attenuated control had no significant effect at any dose tested.

### Example 3. In vivo inhibition of tumor growth and metastases by VEGF-R ribozymes.

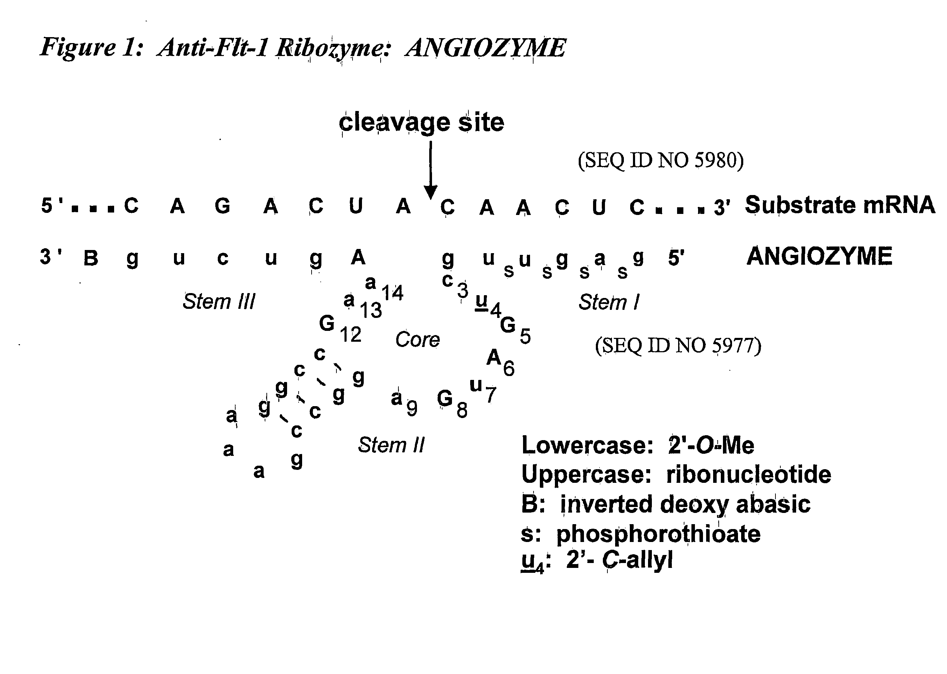

### A. Lewis Lung Carcinoma Mouse Model: Ribozymes were chemically synthesized as described above. The sequence of ANGIOZYME^{™} bound to its target RNA is shown in Figure 1.

The tumors in this study were derived from a cell line (LLC-HM) which gives rise to reproducible numbers of spontaneous lung metastases when propagated *in vivo.* The LLC-HM line was obtained from Dr. Michael O'Reilly, Harvard University. Tumor neovascularization in Lewis lung carcinoma has been shown to be VEGF-dependent. Tumors from mice bearing LLC-HM (selected for the highly metastatic phenotype by serial propagation) were harvested 20 days post-inoculation. A tumor brei suspension was prepared from these tumors according to standard protocols. On day 0 of the study, 0.5 x 10⁶ viable LLC-HM tumor cells were injected subcutaneously (sc) into the dorsum or flank of previously untreated mice (100 µL injectate). Tumors were allowed to grow for a period of 3 days prior to initiating continuous intravenous administration of saline or 30 mg/kg/d ANGIOZYME^{™} *via* Alzet mini-pumps. One set of animals was dosed from days 3 to 17, inclusive. Tumor length and width measurements and volumes were calculated according to the formula: Volume = 0:5(length)(width)². At post-inoculation day 25, animals were euthanized and lungs harvested. The number of lung macrometastatic nodules was counted. It should be noted that metastatic foci were quantified 8 days after the cessation of dosing. Ribozyme solutions were prepared to deliver to another set of animals 100, 10, 3, or 1 mg/kg/day of ANGIOZYME^{™} via Alzet mini-pumps. A total of 10 animals per dose or saline control group were surgically implanted on the left flank with osmotic mini-pumps prefilled with the respective test solution three days following tumor inoculation. Pumps were attached to indwelling jugular vein catheters.

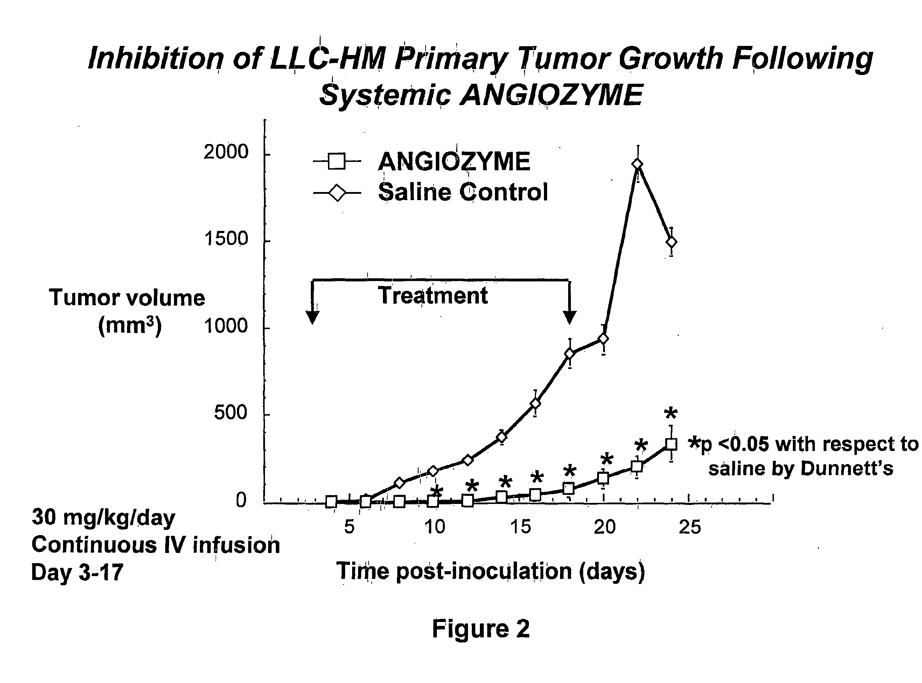

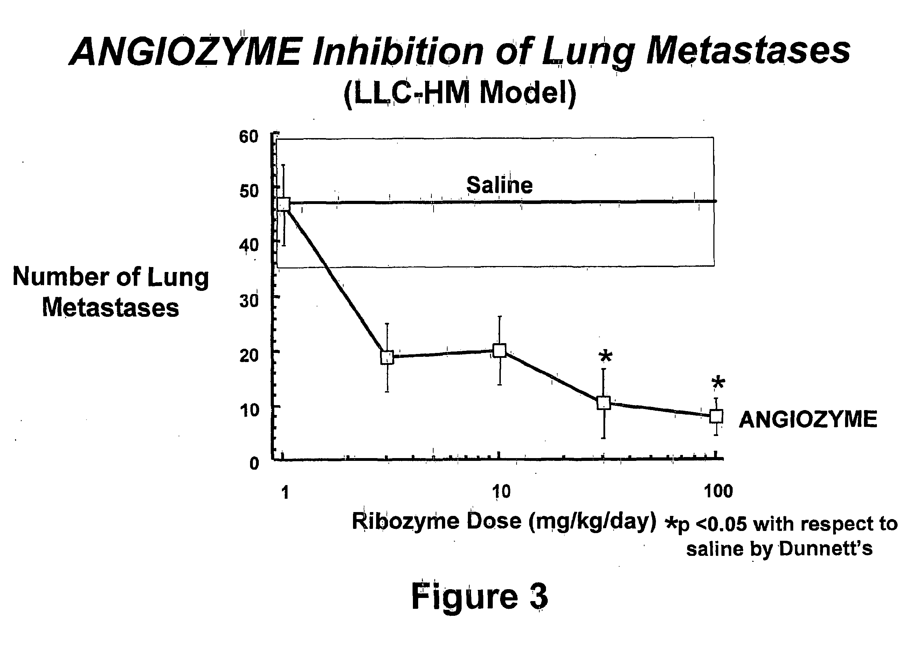

**Figure 2** shows the antitumor effects of ANGIOZYME^{™}. There is a statistically significant inhibition (p < 0.05) of primary LLC-HM tumor growth in tumors grown in the flank regions compared to saline control. ANGIOZYME^{™} significantly reduced (p < 0.05) the number of lung metastatic foci in animals inoculated either in the flank regions. **Figure 3** illustrates the dose-dependent anti-metastatic effect of ANGIOZYME^{™} compared to saline control.

### B. Mouse Colorectal Cancer Model. KM12L4a-16 is a human colorectal cancer cell line. On day 0 of the study, 0.5 x 10⁶ KM12L4a-16 cells were implanted into the spleen of nude mice. Three days after tumor inoculation, Alzet minipumps were implanted and continuous subcutaneous delivery of either saline or 12, 36 or 100 mg/kg/ day of ANGIOZYME^{™} was initiated. On day 5, the spleens containing the primary tumors were removed. On day 18, the Alzet minipumps were replaced with fresh pumps so that delivery of saline or ANGIOZYME^{™} was continuous over a 28 day period from day 3 to day 32. Animals were euthanized on day 41 and the liver tumor burden was evaluated.

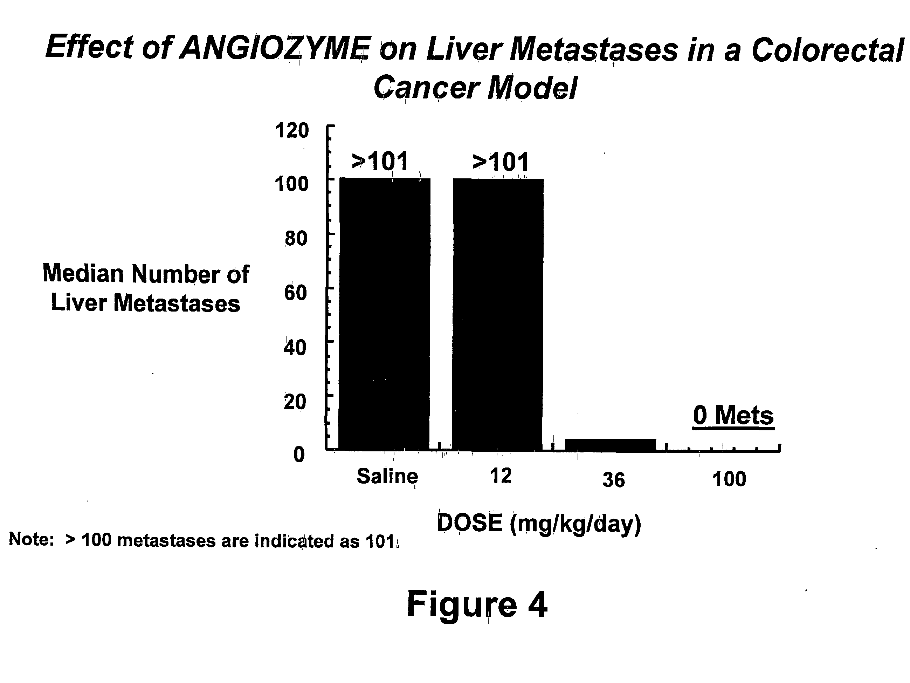

Following treatment with 100 mg/kg/day of ANGIOZYME^{™}, there was a significant reduction in the incidence and median number of liver metastasis (**Figure 4**). In saline-treated animals, the median number of metastases was 101. However, at the high dose of ANGIOZYME^{™} (100 mg/kg/day), the median number of metastases was zero.

### Example 4: Effect of ANGIOZYME^{™} alone or in combination with chemotherapeutic agents in the mouse Lewis Lung Carcinoma Model.

### Methods

**Tumor inoculations.** Male C57/BL6 mice, age 6 to 8 weeks, were inoculated subcutaneously in the flank with 5 x 10⁵ LLC-HM cells from brei preparations made from tumors grown in mice.

**Ribozymes and controls.** RPL4610, also known as ANGIOZYME^{™} (SEQ ID NO: 5977), is an anti-*Flt*-*1* ribozyme that targets site 4229 in the human *Flt-1* receptor mRNA (EMBL accession no. X51602). The controls tested include RPI. 13141, an attenuated version of RPI. 4610 in which four nucleotides in the catalytic core are changed so that the cleavage activity is dramatically decreased. RPI. 13141, however, maintains the base composition and binding arms of RPI.4610 and so is still capable of binding to the target site. The second control (RPI.13030) also has changes to the catalytic core (three) to inhibit cleavage activity, but in addition the sequence of the binding arms has been scrambled so that it can no longer bind to the target sequence. One nucleotide in the arm of RPI.13030 is also changed to maintain the same base composition as RPI.4610.

**Ribozyme administrations.** Ribozymes and controls were resuspended in normal saline. Administration was initiated seven days following tumor inoculation. Animals either received a daily subcutaneous injection (30 mg/kg test substance) from day 7 to day 20 or were instrumented with an Alzet osmotic minipump (12 µL/day flow rate) containing a solution of ribozyme or control. Subcutaneous infusion pumps delivered the test substances (30 mg/kglday) from day 7 to 20 (14-day pumps, 420 mg/kg total test substance) or days 7-34 (28-day pumps, 840 mg/kg total test substance). Where indicated, chemotherapeutic agents were given in combination with ribozyme treatment. Cyclophosphamide was given by intraperitoneal administration on days 7, 9 and 11 (125 mg/kg). Gemcitabine was given by intraperitoneal administration on days 8, 11 and 14 (125 mg/kg). Untreated, uninstrumented animals were used as comparison. Five animals were included in each group.

### Results

The antiangiogenic ribozyme, ANGIOZYME^{™}, was tested in a model of Lewis lung carcinoma alone and in combination with two chemotherapeutic agents. Previously (see above), 30 mg/kg/day ANGIOZYME^{™} alone was determined to inhibit both primary tumor growth and lung metastases in a highly metastatic variant of Lewis lung (continuous 14-day iv delivery *via* Alzet minipump, manuscript in preparation).

In this study, 30 mg/kg/day ANGIOZYME^{™} delivered either as a daily subcutaneous bolus injection or as a continuous infusion from an Alzet minipump resulted in a delay in tumor growth. On average, tumor growth to 500 mm³ was delayed by ~7 days in animals being treated with ANGIOZYME^{™} compared to an untreated group. Growth of tumors in animals being treated with either of two attenuated controls was delayed by only ~ 2 days.
ANGIOZYME^{™} delivered by subcutaneous bolus was also tested in combination with either Gemcytabine or cyclophosphamide. Tumor growth delay increased by about 3 days in the presence of combination therapy with ANGIOZYME^{™} and Gemcytabine over the effects of either treatment alone. The combination of ANGIOZYME^{™} and cyclophosphamide did not increase tumor growth delay over that of cyclophosphamide alone, however, suboptimal doses of cyclophosphamide were not included in this study. Neither of the attenuated controls increased the effect of the chemotherapeutic agents.

The effect of ANGIOZYME^{™} on metastases to the lung was also determined in the presence and absence of additional chemotherapeutic treatment. Macrometastases to the lungs were counted in two animals in each treatment group on day 20. In the presence of ANGIOZYME^{™}, with or without a chemotherapeutic agent, the lung metastases were reduced to zero. Treatment with either Gemcytabine or cyclophosphamide alone (mean number of metastases 4.5 and 4, respectively) were not as effective as ANGIOZYME^{™} alone or when used in combination with ANGIOZYME^{™}. Neither of the attenuated controls increased the effect of the chemotherapeutic agents.

The effect on metastases to the lung was also determined following continuous treatment with ANGIOZYME^{™}. At day 20, an average of ~8 macrometastases were noted in the treatment groups which had been instrumented with Alzet minipumps (either 14- or 28-day pumps). This is a decrease in metastases of ~50% from the untreated group. Since ANGIOZYME^{™} delivered by a daily subcutaneous bolus resulted in zero metastases (Fig.4) in the two animals counted, it is possible that the additional burden of being instrumented with the minipump contributes to a slightly decreased response to ANGIOZYME^{™}.

### Example 5: Identification of Potential Target Sites in Human VEGFR1 and/or VEGFR2 RNA

The sequence of human VEGFR1 and/or VEGFR2 genes are screened for accessible sites using a computer-folding algorithm. Regions of the RNA that do not form secondary folding structures and contain potential enzymatic nucleic acid molecule and/or antisense binding/cleavage sites are identified. An exemplary sequence of an enzymatic nucleic acid molecule of the invention is shown in Formula I and/or Formula II (SEQ ID Nos: 5977 and 5978, respectively). Other nucleic acid molecules and targets contemplated by the invention are described in Pavco et al., US Patent Application No. 09/870,161, incorporated by reference herein in its entirety. Similarly, other nucleic acid molecules of the invention, including antisense, aptamers, dsRNA, siRNA, and/or 2,5-A chimeras, can be designed to modulate the expression of the nucleic acid targets described in Pavco et al., US Patent Application No. 09/870,161.

### Example 6: Selection of Enzymatic Nucleic Acid Cleavage Sites in Human VEGFR1 and/or VEGFR2 RNA

Enzymatic nucleic acid molecule target sites are chosen by analyzing sequences of human VEGFR1 receptor (for example Genbank Accession No. NM_002019), and VEGFR2 receptor (for example Genbank Accession No. NM_002253) genes and prioritizing the sites on the basis of folding. Enzymatic nucleic acid molecules are designed that can bind each target and are individually analyzed by computer folding (Christoffersen et al., 1994 J. Mol. Struc. Theochem, 311, 273; Jaeger et al., 1989, Proc. Natl. Acad. Sci. USA, 86, 7706) to assess whether the enzymatic nucleic acid molecule sequences fold into the appropriate secondary structure. Those enzymatic nucleic acid molecules with unfavorable intramolecular interactions between the binding arms and the catalytic core can be eliminated from consideration. As discussed herein, varying binding arm lengths can be chosen to optimize activity. Generally, at least 4 bases on each arm are able to bind to, or otherwise interact with, the target RNA.

### Example 7: Chemical Synthesis and Purification of Ribozymes and Antisense for Efficient Cleavage and/or blocking of VEGFR1 and/or VEGFR2 RNA

Enzymatic nucleic acid molecules and antisense constructs are designed to anneal to various sites in the RNA message. The binding arms of the enzymatic nucleic acid molecules are complementary to the target site sequences described above, while the antisense constructs are fully complementary to the target site sequences described above. RNAi molecules (dsRNA) likewise have one strand of RNA or a portion of RNA complementarity to the target site sequence or a portion of the target site sequence. For example, complementarity within the double-strand RNAi structure is formed from two separate individual RNA strands or from self-complementary areas of a topologically closed, individual RNA strand which can be optionally circular. The nucleic acid molecules were chemically synthesized. The method of synthesis used followed the procedure for normal RNA synthesis as described above and in Usman et al., (1987 J. Am. Chem. Soc., 109, 7845), Scaringe et al., (1990 Nucleic Acids Res., 18, 5433) and Wincott *et al.,* supra, and made use of common nucleic acid protecting and coupling groups, such as dimemoxytrityl at the 5'-end, and phosphoramidites at the 3'-end. The average stepwise coupling yields were typically>98%.

Nucleic acid molecules are also synthesized from DNA templates using bacteriophage T7 RNA polymerase (Milligan and Uhlenbeck, 1989, Methods Enzymol. 180, 51). Nucleic acid molecules of the invention are purified by gel electrophoresis using general methods or are purified by high pressure liquid chromatography (HPLC; See Wincott *et al.,* supra, the totality of which is hereby incorporated herein by reference) and are resuspended in water. Examples of sequences of chemically synthesized enzymatic nucleic acid molecules are shown in Formula I (SEQ ID NO: 5977), Formula II (SEQ ID NO: 5978) and in Pavco et al., US Patent Application No. 09/870,161.

### Example 8: Enzymatic Nucleic Acid Molecule Cleavage of VEGFR1 and/or VEGFR2 RNA Target in vitro

Enzymatic nucleic acid molecules targeted to the human VEGFR1 and/or VEGFR2 RNA are designed and synthesized as described above. These enzymatic nucleic acid molecules can be tested for cleavage activity in *vitro,* for example, using the following procedure. The target sequences and the nucleotide location within the VEGFR1 and/or VEGFR2 RNA are described in Pavco et al., US Patent Application No. 09/870,161.

*Cleavage Reactions:* Full-length or partially full-length, internally-labeled target RNA for enzymatic nucleic acid molecule cleavage assay is prepared by in *vitro* transcription in the presence of [a-³²P] CTP, passed over a G 50 Sephadex column by spin chromatography and used as substrate RNA without further purification. Alternately, substrates are 5'-³²P-end labeled using T4 polynucleotide kinase enzyme. Assays are performed by pre-warming a 2X concentration of purified enzymatic nucleic acid molecule in enzymatic nucleic acid molecule cleavage buffer (50 mM Tris-HCl, pH 7.5 at 37°C, 10 mM MgCl₂) and the cleavage reaction was initiated by adding the 2X enzymatic nucleic acid molecule mix to an equal volume of substrate RNA (maximum of 1-5 nM) that was also pre-warmed in cleavage buffer. As an initial screen, assays are carried-out for 1 hour at 37°C using a final concentration of either 40 nM or 1 mM enzymatic nucleic acid molecule, *i.e.,* enzymatic nucleic acid molecule excess. The reaction is quenched by the addition of an equal volume of 95% formamide, 20 mM EDTA, 0.05% bromophenol blue and 0.05% xylene cyanol after which the sample is heated to 95°C for 2 minutes, quick chilled and loaded onto a denaturing polyacrylamide gel. Substrate RNA and the specific RNA cleavage products generated by enzymatic nucleic acid molecule cleavage are visualized on an autoradiograph of the gel. The percentage of cleavage is determined by Phosphor Imager^{®} quantitation of bands representing the intact substrate and the cleavage products.

### Example 9: Phase I/II Study of Repetitive Dosing of ANGIOZYME^{™} Targeting the VEGFR1 (FLT-1) Receptor of VEGF

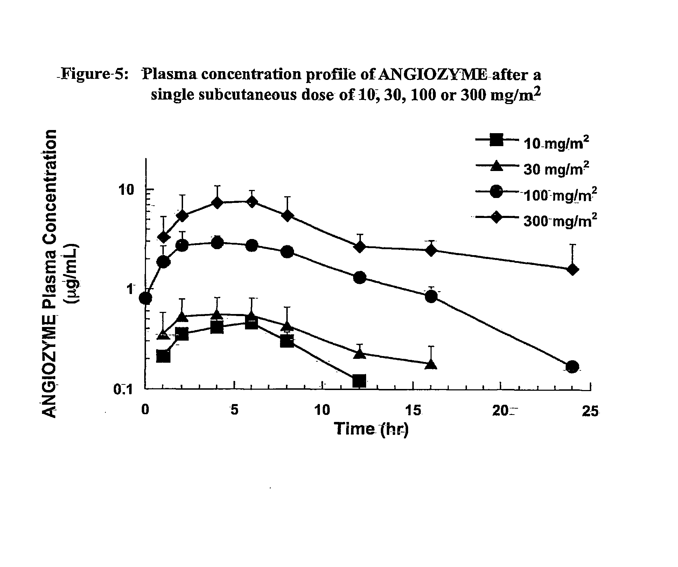

A ribozyme therapeutic agent ANGIOZYME^{™} (SEQ ID NO: 5977), was assessed by daily subcutaneous administration in a phase I/II trial for 31 patients with refractory solid tumors. Demographic information relating to patients enrolled in the study are shown in **Table III**. The primary study endpoint was to determine the safety and maximum tolerated dose of ANGIOZYME^{™}. Secondary endpoints assessed ANGIOZYME^{™} pharmacokinetics and clinical response. Patients were treated at the following doses: 3 patients received doses of 10 mg/m²/day, 4 patients received 30 mg/m²/day, 20 patients received 100 mg/m²/day, and 4 patients received 300 mg/m²/day. All but one patient were dosed for a minimum of 29 consecutive days with 24-hour pharmacokinetic analyses on Day 1 and 29. Clinical response was assessed monthly. Results The data from 20 patients indicated that ANGIOZYME^{™} was well tolerated, with no systemic adverse events. Figure 5 shows the plasma concentration profile of ANGIOZYME^{™} after a single subcutaneous dose of 10, 30, 100, or 300 mg/m². The pharmacokinetic parameters of ANGIOZYME^{™} after subcutaneous bolus administration are outlined in **Table IV**. An MTD (maximum tolerated dose) could not be established. One patient in the 300 mg/m²/d group experienced a grade 3 injection site reaction. Patients in the other groups experienced intermittent grade 1 and grade 2 injection site reactions with erythema and induration. No systemic or laboratory toxicities were observed. Pharmacokinetic analyses demonstrated dose-dependent plasma concentrations with good bioavailability (70-90%), tl/2 = 209-384 min, and no accumulation after repeated doses. To date, 17/28 (61%) of evaluable patients have had stable disease for periods of one to six months and two patients (nasopharyngeal squamous cell carcinoma and melanoma) had minor clinical responses. The patient with nasopharyngeal carcinoma demonstrated central tumor necrosis as indicated by MRI. The longest period of treatment thus far has been 8 months for two patients at 100 mg/m²/d (breast, peritoneal mesothelioma).

### Example 10: Down-regulation of VEGFR1 gene expression to treat gynecologic neovascularization dependent conditions

One patient in the Phase I/II trial described in Example 19 was menstruating prior to enrollment in the ANGIOZYME^{™} monotherapy trial. After 1-2 months on trial, the patient's menstrual cycles ceased. The patient remained on trial for approximately 11 months and did not menstruate. The patient then went off the trial for about 4 months and the menstrual cycles resumed. Re-enrollment in the ANGIOZYME^{™} trial resulted in the patient's menstrual cycle stopping again. This clinical observation suggests that ANGIOZYME^{™} is interfering with the patient's menstrual cycle, perhaps by inhibiting neovascularization of uterine tissue. This data also suggests that ANGIOZYME^{™} has a direct effect on the endometrial tissue or an effect on LH/FSH stimulation. These results suggest the treatment or control, using ANGIOZYME^{™} (SEQ ID NO: 5977) and/or other nucleic acid molecules of the instant invention, of various clinical targets and/or processes associated with female reproduction and gynecologic neovascularization, such as endometriosis, birth control, gynecologic bleeding disorders, irregular menstrual cycles, ovulation, premenstrual syndrome (PMS), menopausal dysfunction, endometrial carcinoma or other condition associated with the expression of VEGFR1 and/or VEGFR2 VEGF receptors.

### Example 11: Down-regulation of VEGFR1 in clinical setting

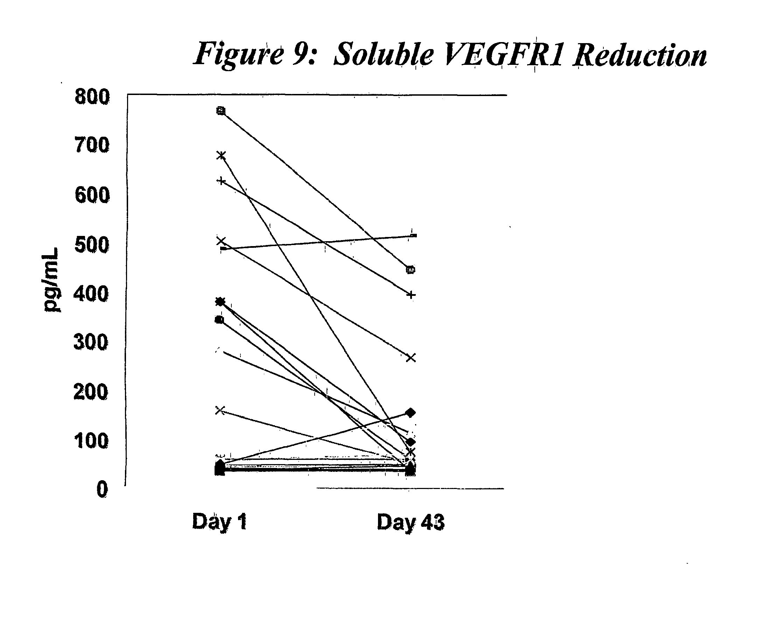

Twenty-seven of the patients enrolled in the Phase 1/D trial described in Example 19 had day 1 (baseline) and day 43 (six-week) serum samples assayed for VEGFR1 biomarker. VEGERI levels were statistically different after six weeks of ANGIOZYME treatment (Figure 9). Although statistical testing involving all 27 patients showed statistical support for effects, not all patients presented with elevated levels of VEGF-R1. Since the effects of ANGIOZYME on VEGF-R1 may only be demonstrated when sufficient levels are present at baseline, a cutoff of 100 pg/mL was chosen and changes in this VEGF-R1 were re-analyzed. Ten of the 27 patients presented with baseline VEGF-R1 levels in excess of 100 pg/mL. For this subgroup VEGF-R1 levels were lower by 3-fold, p<.001. After six weeks of treatment the average (geometric mean) of VEGF-R1 decreased for this subgroup from 419 pg/ml to 132pg/ml, p<.001. These results show that treatment with ANGIOZYME results in a statistically significant reduction in VEGFR1 expression.

### Example 22: In vivo inhibition of neovascularization in an ocular animal model by VEGF-R ribozymes.

**Summary of the Mouse Model**: A mouse model of proliferative retinopathy (Aiello et al., 1995, Proc. Natl. Acad. Sci. USA 92: 10457-10461; Robinson et al., 1996, Proc. Natl. Acad. Sci. USA 93: 4851-4856; Pierce et al., 1996, Archives of Ophthalmology 114: 1219-1228) in which neovascularization of the mouse retina is induced by exposure of 7-day old mice to 75% oxygen followed by a return to normal room air. The initial period in high oxygen causes an obliteration of developing blood vessels in the retina. Exposure to room air five days later is perceived as hypoxia by the now underperfused retina. The result is an immediate upregulation of VEGF mRNA and VEGF protein (between 6-12 hours) followed by an extensive retinal neovascularization that peaks in ~5 days. Although this model is more representative of retinopathy of prematurity than diabetic retinopathy, it is an accepted small animal model in which to study neovascular pathophysiology of the retina. In fact, intravitreal injection of certain antisense DNA constructs targeting VEGF mRNA have been found to be antiangiogenic in this model, as were soluble VEGF receptor chimeric proteins designed to bind VEGF in the vitreous humor (Aiello et al., 1995, Proc. Natl. Acad. Sci. USA 92: 10457-10461; Robinson et al., 1996, Proc. Natl. Acad. Sci. USA 93: 4851-4856; Pierce et al., 1996, Archives of Ophthalmology 114: 1219-1228).

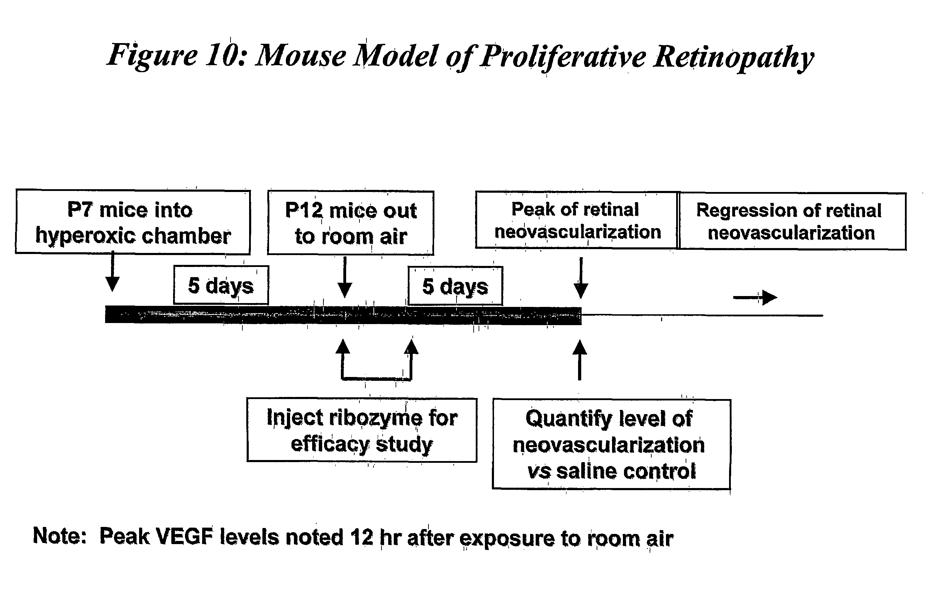

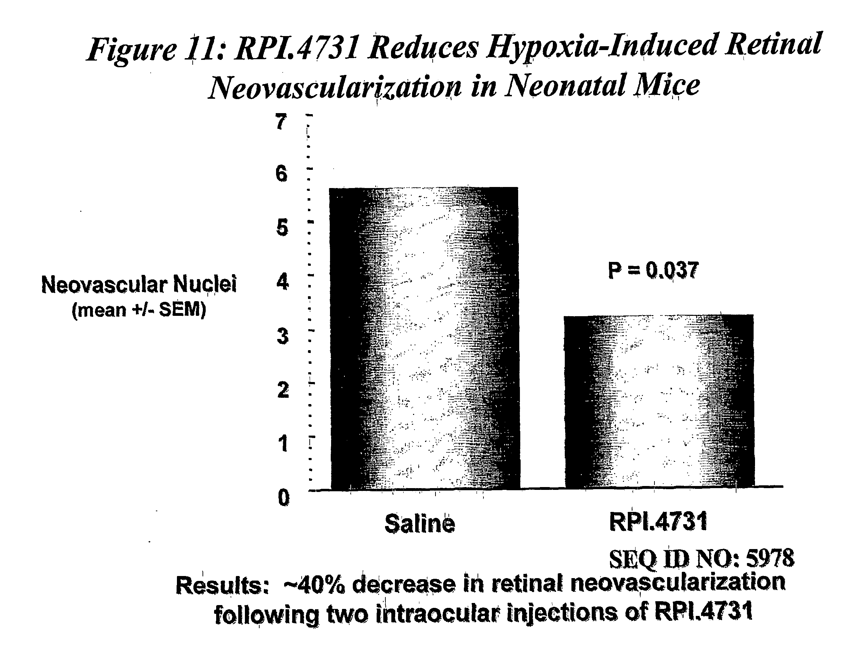

**Summary of experiment:** The effect of an *anti-KDR*/*Flk-1* ribozyme on the peak level of neovascularization was tested in the mouse model described above. As shown in Figure 10, P7 mice were removed from the hyperoxic chamber and the mice received two intraocular injections (P12 and P13) in the right eye of 10 µg RPI.4731, the anti- *KDR*/*Flk-1* ribozyme. The left eye of each mouse was treated as a control and received intraocular injections of saline. Five days after being exposed to room air, neovascular nuclei in the retina of both eyes were counted. Data are presented in **Figure 11**. There was a significant decrease in retinal neovascularization (~40%) compared to the control, saline-injected eyes.
RPL4731 sequence and chemical composition:
5'-uₛaₛcₛ aₛau uc**U** GAu Gag gcg aaa gcc Gaa Aag aca a**B**-3' (SEQ ID NO: 5978)
where:
uppercase G, A = ribonucleotides
lowercase = 2'-*O*Me
U = 2'-*C*-allyl uridine
B = inverted abasic nucleotide
S = phosphorothioate internucleotide linkage

### Indications

1) Tumor angiogenesis: Angiogenesis has been shown to be necessary for tumors to grow into pathological size (Folkman. 1971, PNAS 76, 5217-5221; Wellstein & Czubayko, 1996, Breast Cancer Res and Treatment 38, 109-119). In addition, it allows tumor cells to travel through the circulatory system during metastasis. Increased levels of gene expression of a number of angiogenic factors such as vascular endothelial growth factor (VEGF) have been reported in vascularized and edema-associated brain tumors (Berkman et al., 1993 J. Clini. Invest. 91, 153). A more direct demostration of the role of VEGF in tumor angiogenesis was demonstrated by Jim Kim et al., 1993 Nature 362,841 wherein, monoclonal antibodies against VEGF were successfully used to inhibit the growth of rhabdomyosarcoma, glioblastoma multiforme cells in nude mice. Similarly, expression of a dominant negative mutated form of the flt-1 VEGF receptor inhibits vascularization induced by human glioblastoma cells in nude mice (Millauer et al., 1994, Nature 367, 576). Specific tumor/cancer types that can be targeted using the nucleic acid molecules of the invention include but are not limited to the tumor/cancer types described under Diagnosis in **Table III:**
2) Ocular diseases: Neovascularization has been shown to cause or exacerbate ocular diseases including but not limited to, macular degeneration, neovascular glaucoma, diabetic retinopathy, myopic degeneration, and trachoma (Norrby, 1997, APMIS 105, 417-437). Aiello et al., 1994 New Engl. J. Med. 331, 1480, showed that the ocular fluid, of a majority of patients suffering from diabetic retinopathy and other retinal disorders, contains a high concentration of VEGF. Miller et al., 1994 Am. J. Pathol. 145, 574, reported elevated levels of VEGF mRNA in patients suffering from retinal ischemia. These observations support a direct role for VEGF in ocular diseases. Other factors including those that stimulate VEGF synthesis may also contribute to these indications.
3) Dermatological Disorders: Many indications have been identified which may by angiogenesis dependent including but not limited to psoriasis, verruca vulgaris, angiofibroma of tuberous sclerosis, pot-wine stains, Sturge Weber syndrome, Kippel-Trenaunay-Weber syndrome, and Osler-Weber-Rendu syndrome (Norrby, *supra).* Intradermal injection of the angiogenic factor b-FGF demonstrated angiogenesis in nude mice (Weckbecker et al., 1992, Angiogenesis: Key principles-Science-Technology-Medicine, ed R. Steiner) Detmar et al., 1994 J. Exp. Med. 180, 1141 reported that VEGF and its receptors were over-expressed in psoriatic skin and psoriatic dermal microvessels, suggesting that VEGF plays a significant role in psoriasis.
4) Rheumatoid arthritis: Immunohistochemistry and *in situ* hybridization studies on tissues from the joints of patients suffering from rheumatoid arthritis show an increased level of VEGF and its receptors (Fava et al., 1994 J. Exp. Med. 180, 341). Additionally, Koch et al., 1994 J. Immunol. 152, 4149, found that VEGF-specific antibodies were able to significantly reduce the mitogenic activity of synovial tissues from patients suffering from rheumatoid arthritis. These observations support a direct role for VEGF in rheumatoid arthritis. Other angiogenic factors including those of the present invention may also be involved in arthritis.
5) Endometriosis: Various studies indicate that VEGF is directly implicated in endometriosis. In one study, VEGF concentrations measured by ELISA in peritoneal fluid were found to be significantly higher in women with endometriosis than in women without endometriosis (24.1 ± 15 ng/ml vs 13.3 ± 7.2 ng/ml in normals). In patients with endometriosis, higher concentrations of VEGF were detected in the proliferative phase of the menstrual cycle (33 ± 13 ng/ml) compared to the secretory phase (10.7 ± 5 ng/ml). The cyclic variation was not noted in fluid from normal patients (McLaren et al., 1996, Human Reprod 11, 220-223). In another study, women with moderate to severe endometriosis had significantly higher concentrations of peritoneal fluid VEGF than women without endometriosis. There was a positive correlation between the severity of endometriosis and the concentration of VEGF in peritoneal fluid. In human endometrial biopsies, VEGF expression increased relative to the early proliferative phase approximately 1.6-, 2-, and 3.6-fold in midproliferative, late proliferative, and secretory endometrium (Shifren et al., 1996, J. Clin. Endocrinol. Metab. 81, 3112-3118).

In a third study, VEGF-positive staining of human ectopic endometrium was shown to be localized to macrophages (double immunofluorescent staining with CD14 marker). Peritoneal fluid macrophages demonstrated VEGF staining in women with and without endometriosis. However, increased activation of macrophages (acid phosphatatse activity) was demonstrated in fluid from women with endometriosis compared with controls. Peritoneal fluid macrophage conditioned media from patients with endometriosis resulted in significantly increased cell proliferation ([³H] thymidine incorporation) in HUVEC cells compared to controls. The percentage of peritoneal fluid macrophages with VEGFR2 mRNA was higher during the secretory phase, and significantly higher in fluid from women with endometriosis (80 ± 15%) compared with controls (32 ± 20%). Flt-mRNA was detected in peritoneal fluid macrophages from women with and without endometriosis, but there was no difference between the groups or any evidence of cyclic dependence (MeLaren et al., 1996, J. Clin. Invest. 98, 482-489).

In the early proliferative phase of the menstrual cycle, VEGF has been found to be expressed in secretory columnar epithelium (estrogen-responsive) lining both the oviducts and the uterus in female mice. During the secretory phase, VEGF expression was shown to have shifted to the underlying stroma composing the functional endometrium. In addition to examining the endometrium, neovascularization of ovarian follicles and the corpus luteum, as well as angiogenesis in embryonic implantation sites have been analyzed. For these processes, VEGF was expressed in spatial and temporal proximity to forming vasculature (Shweild et al., 1993, J. Clin. Invest. 91, 2235-2243).

The present body of knowledge in VEGFR1 and/or VEGFR2 research indicates the need for methods to assay VBGFR1 and/or VEGFR2 activity and for compounds that can regulate VEGFR1 and/or VEGFR2 expression for research, diagnostic, and therapeutic use. As described herein, the nucleic acid molecules of the present invention can be used in assays to diagnose disease state related of VEGF, VEGFR1 and/or VEGFR2 levels. In addition, the nucleic acid molecules can be used to treat disease state related to VEGF and/or VEGFr, such as VEGFR1 and/or VEGFR2 levels.

Particular processes, diseases, or conditions that can be associated with VEGFR1 and/or VEGFR2 levels include, but are not limited to, gynecologic neovascularization, such as endometriosis, endometrial carcinoma, gynecologic bleeding disorders, irregular menstrual cycles, ovulation, premenstrual syndrome (PMS), menopausal dysfunction, other diseases and conditions discussed herein, and other diseases or conditions that are related to or respond to the levels of VEGF and/or VEGFr, such as VEGFR1 and/or VEGFR2, in a cell or tissue, alone or in combination with other therapies

The use of GnRH (gonadotropin releasing hormone) agonists, Lupron Depot (Leuprolide Acetate), Synarel (naferalin acetate), Zolodex (goserelin acetate), Suprefact (buserelin acetate), Danazol, or oral contraceptives including, but not limited to, Depo-Provera or Provera (medroxyprogesterone acetate), or any other estrogen/progesterone contraceptive, are all non-limiting examples of compounds and methods that can be combined with or used in conjunction with the nucleic acid molecules of the instant invention. Various chemotherapies can be readily combined with nucleic acid molecules of the invention for the treatment of endometrial carcinoma. Common chemotherapies that can be combined with nucleic acid molecules of the instant invention include various combinations of cytotoxic drugs to kill the cancer cells. These drugs include but are not limited to paclitaxel (Taxol), docetaxel, cisplatin, methotrexate, cyclophosphamide, doxorubin, fluorouracil carboplatin, edatrexate, gemcitabine, vinorelbine etc. Those skilled in the art will recognize that other drug compounds and therapies can be readily combined with the nucleic acid molecules of the instant invention and are hence within the scope of the instant invention.

### Animal Models

There are several animal models in which the anti-angiogenesis effect of nucleic acids of the present invention, such as ribozymes, directed against VEGF-R mRNAs can be tested: Typically; a corneal model has been used to study angiogenesis in rat and rabbit since recruitment of vessels can easily be followed in this normally avascular tissue (Pandey et al., 1995 Science 268: 567-569). In these models, a small Teflon or Hydron disk pretreated with an angiogenesis factor (e.g. bFGF or VEGF) is inserted into a pocket surgically created in the cornea. Angiogenesis is monitored 3 to 5 days later. Ribozymes directed against VEGF-R mRNAs would be delivered in the disk as well; or dropwise to the eye over the time course of the experiment. In another eye model, hypoxia has been shown to cause both increased expression of VEGF and neovascularization in the retina (Pierce et al., 1995 Proc. Natl. Acad. Sci. USA. 92: 905-909; Shweiki et al., 1992 J Clin. Invest: 91: 2235-2243).

In human glioblastomas, it has been shown that VEGF is at least partially responsible for tumor angiogenesis (Plate et al., 1992 Nature 359, 845). Animal models have been developed in which glioblastoma cells are implanted subcutaneously into nude mice and the progress of tumor growth and angiogenesism is studied (Kim *et al.,* 1993 *supra;* Millauer *et al.,* 1994 *supra).*

Another animal model that addresses neovascularization involves Matrigel, an extract of basement membrane that becomes a solid gel when injected subcutaneously (Passaniti et al., 1992 Lab. Invest. 67: 519-528). When the Matrigel is supplemented with angiogenesis factors such as VEGF, vessels grow into the Matrigel over a period of 3 to 5 days and angiogenesis can be assessed. Ribozymes directed against VEGF-R mRNAs can be delivered in the Matrigel to assess anti-angiogesis effect.

Several animal models exist for screening of anti-angiogenic agents. These include corneal vessel formation following corneal injury (Burger et al., 1985 Cornea 4: 35-41; Lepri, et al., 1994 J. Ocular Pharmacol. 10: 273-280; Ormerod et al., 1990 Am. J. Pathol. 137: 1243-1252) or intracorneal growth factor implant (Grant et al., 1993 Diabetologia 36: 282-291; Pandey *et al.* 1995 *supra;* Zieche et al., 1992 Lab. Invest. 67: 711-715), vessel growth into Matrigel matrix containing growth factors (Passaniti *et al.,* 1992 *supra*), female reproductive organ neovascularization following hormonal manipulation (Shweiki et al., 1993 Clin. Invest. 91: 2235-2243), several models involving inhibition of tumor growth in highly vascularized solid tumors (O'Reilly et al., 1994 Cell 79: 315-328; Senger et al., 1993 Cancer and Metas. Rev. 12: 303-324; Takahasi et al., 1994 Cancer Res. 54: 4233-4237; Kim *et al.,* 1993 *supra*), and transient hypoxia-induced neovascularization in the mouse retina (Pierce et al., 1995 Proc. Natl. Acad. Sci. USA. 92: 905-909).

The cornea model; described in Pandey et al. *supra,* is the most common and well characterized anti-angiogenic agent efficacy screening model. This model involves an avascular tissue into which vessels are recruited by a stimulating agent (growth factor, thermal or alkalai burn, endotoxin). The corneal model utilizes the intrastromal corneal implantation of a Teflon pellet soaked in a VEGF-Hydron solution to recruit blood vessels toward the pellet which can be quantitated using standard microscopic and image analysis techniques. To evaluate their anti-angiogenic efficacy, ribozymes are applied topically to the eye or bound within Hydron on the Teflon pellet itself. This avascular cornea as well as the Matrigel (see below) provide for low background assays. While the corneal model has been performed extensively in the rabbit, studies in the rat have also been conducted.

The mouse model (Passaniti et al., *supra)* is a non-tissue model which utilizes Matrigel, an extract of basement membrane (Kleinman et al., 1986) or Millipore^{®} filter disk, which can be impregnated with growth factors and anti-angiogenic agents in a liquid form prior to injection. Upon subcutaneous administration at body temperature, the Matrigel or Millipore^{®} filter disk forms a solid implant. VEGF embedded in the Matrigel or Millipore^{®} filter disk would be used to recruit vessels within the matrix of the Matrigel or Millipore^{®} filter disk which can be processed histologically for endothelial cell specific vWF (factor VIII antigen) immunohistochemistry, Trichrome-Masson stain, or hemoglobin content. Like the cornea, the Matrigel or Millipore^{®} filter disk are avascular; however, it is not tissue. In the Matrigel or Millipore^{®} filter disk model, ribozymes are administered within the matrix of the Matrigel or Millipore^{®} filter disk to test their anti-angiogenic efficacy. Thus, delivery issues in this model, as with delivery of ribozymes by Hydron- coated Teflon pellets in the rat cornea model, are minimized due to the homogeneous presence of the ribozyme within the respective matrix.

These models offer a distinct advantage over several other angiogenic models listed previously. The ability to use VEGF as a pro-angiogenic stimulus in both models is highly desirable since ribozymes target only VEGFr mRNA. In other words, the involvement of other non-specific types of stimuli in the cornea and Matrigel models is not advantageous from the standpoint of understanding the pharmacologic mechanism by which the anti-VEGFr mRNA ribozymes produce their effects. In addition, the models allow for testing the specificity of the anti-VEGFr mRNA ribozymes by using either aFGF or bFGF as a pro-angiogenic factor. Vessel recruitment using FGF should not be affected in either model by anti-VEGFr mRNA ribozymes. Other models of angiogenesis, including vessel formation in the female reproductive system using hormonal manipulation (Shweiki *et al.,* 1993 *supra*)*;* a variety of vascular solid tumor models which involve indirect correlations with angiogenesis (O'Reilly *et al.,* 1994 *supra*; Senger *et al.,* 1993 *supra;* Takahasi *et al.,* 1994 *supra*; Kim *et al.,* 1993 *supra*); and retinal neovascularization following transient hypoxia (Pierce *et al.,* 1995 *supra),* were not selected for efficacy screening due to their non-specific nature, although they can be useful models due to a demonstrated correlation between VEGF and angiogenesis.

Other model systems to study tumor angiogenesis is reviewed by Folkman, 1985 Adv. Cancer. Res.. 43, 175.

### Use of murine models

For a typical systemic study involving 10 mice (20 g each) per dose group, 5 doses (1, 3, 10, 30 and 100 mg/kg daily over 14 days continuous administration), approximately 400 mg of ribozyme, formulated in saline would be used. A similar study in young adult rats (200 g) would require over 4 g. Parallel pharmacokinetic studies involve the use of similar quantities of ribozymes further justifying the use of murine models.

### Ribozymes and Lewis lung carcinoma and B-16 melanoma murine models

Identifying a common animal model for systemic efficacy testing of ribozymes is an efficient way of screening ribozymes for systemic efficacy.

The Lewis lung carcinoma and B-16 murine melanoma models are well accepted models of primary and metastatic cancer and are used for initial screening of anti-cancer agents. These murine models are not dependent upon the use of immunodeficient mice, are relatively inexpensive, and minimize housing concerns. Both the Lewis lung and B-16 melanoma models involve subcutaneous implantation of approximately 10⁶ tumor cells from metastatically aggressive tumor cell lines (Lewis lung lines 3LL or D122, LLc-LN7; B-16-BL6 melanoma) in C57BL/6J mice. Alternatively, the Lewis lung model can be produced by the surgical implantation of tumor spheres (approximately 0.8 mm in diameter). Metastasis also can be modeled by injecting the tumor cells directly intraveneously. In the Lewis lung model, microscopic metastases can be observed approximately 14 days following implantation with quantifiable macroscopic metastatic tumors developing within 21-25 days. The B-16 melanoma exhibits a similar time course with tumor neovascularization beginning 4 days following implantation. Since both primary and metastatic tumors exist in these models after 21-25 days in the same animal, multiple measurements can be taken as indices of efficacy. Primary tumor volume and growth latency as well as the number of micro- and macroscopic metastatic lung foci or number of animals exhibiting metastases can be quantitated. The percent increase in lifespan can also be measured. Thus, these models provide suitable primary efficacy assays for screening systemically administered ribozymes/ribozyme formulations.

In the Lewis lung and B-16 melanoma models, systemic pharmacotherapy with a wide variety of agents usually begins 1-7 days following tumor implantation/inoculation with either continuous or multiple administration regimens. Concurrent pharmacokinetic studies can be performed to determine whether sufficient tissue levels of ribozymes can be achieved for pharmacodynamic effect to be expected. Furthermore, primary tumors and secondary lung metastases can be removed and subjected to a variety of *in vitro* studies (i.e. target RNA reduction). Flt-1, KDR and/or flk-1 protein levels can be measured clinically or experimentally by FACS analysis. Flt-1, KDR and/or flk-1 encoded mRNA levels can be assessed by Northern analysis, RNase-protection, primer extension analysis and/or quantitative RT-PCR. Ribozymes that block fit-1, KDR and/or fk-1 protein encoding mRNAs and therefore result in decreased levels of flt-1, KDR and/or flk-1 activity by more than 20% in *vitro* can be identified.

Ribozymes and/or genes encoding them are delivered by either free delivery, liposome delivery, cationic lipid delivery, adeno-associated virus vector delivery, adenovirus vector delivery, retrovirus vector delivery or plasmid vector delivery in these animal model experiments (see above).

Subjects can be treated by locally administering nucleic acids targeted against VEGF-R by direct injection. Routes of administration include, but are not limited to, intravascular, intramuscular, subcutaneous, intraarticular, aerosol inhalation, oral (tablet, capsule or pill form), topical, systemic, ocular, intraperitoneal and/or intrathecal delivery.

Surgically induced models of endometriosis have been developed in rats, mice, and rabbits. Non-human primates demonstrate spontaneous endometriosis, but surgical induction can also be used. In addition to the surgical technique, cycle monitoring can be performed by daily vaginal cytology in primates. For all of the surgically induced models of endometriosis, the following general procedure is used. An initial laparotomy is performed to implant tissue from a donor animal. A portion of one uterine horn (or one complete horn in the case of mice) is removed. The endometrium of this piece of uterus is separated from the myometrium and cut into small segments (4-10 mm2). Segments (approximately 3) are sutured to various locations within the abdominal cavity (peritoneum, intestinal mesentery vessels, uterus, broad ligament). Cummings and Metcalf (1996) attached whole segments of mouse uterus without separating the endometrium from the myometrium. Implants are allowed to grow for 3-6 weeks. A second laparotomy is sometimes performed to verify development of endometriosis-like foci (vascularization and cysts filled with clear fluid). This second laparotomy was done in the studies by Quereda *et al.,* (1996) and Stoeckemann *et al.,* (1995). After 3-6 weeks post-surgery and/or following visualization of endometriosis, drug treatment is initiated and continued for a prescribed period of time. At the termination of these studies, animals are euthanized. Endpoints include, but are not limited to, changes in the surface area of the implants and tissue mass of the ectopic endometrial implants (see for example Brogniez et al., 1995, Human Reprod. 10, 927-931; Cummings et al., 1996, Tox. Appl. Pharm. 138, 131-139; Cummings and Metcalf, 1996, Proc. Soc. Exp. Biol. Med. 212, 332-337; D'Hooghe et al., 1996, Fertility and Sterility. 66, 809-813; Quereda et al., 1996, Eur. J. Obstet. Gynecol. Rep. Biol. 67, 35-40; and Stoeckemann et al., 1995, Human Reprod. 10, 3264-3271).

### Combination therapies

Gemcytabine and cyclophosphamide are non-limiting examples of chemotherapeutic agents that can be combined with or used in conjunction with the nucleic acid molecules (*e.g*. ribozymes and antisense molecules) of the instant invention. Those skilled in the art will recognize that other anti-angiogenic and/or anti-cancer compounds and therapies can be similarly be readily combined with the nucleic acid molecules of the instant invention (e.g. ribozymes and antisense molecules) and are hence within the scope of the instant invention. Such compounds and therapies are well known in the art (see for example Cancer: Principles and Pranctice of Oncology, Volumes 1 and 2, eds Devita, V.T., Hellman, S., and Rosenberg, S.A., J.B. Lippincott Company, Philadelphia, USA; incorporated herein by reference) and include, without limitations, folates, antifolates, pyrimidine analogs, fluoropyrimidines, purine analogs, adenosine analogs, topoisomerase I inhibitors, anthrapyrazoles, retinoids, antibiotics, anthacyclins, platinum analogs, alkylating agents, nitrosoureas; plant derived compounds such as vinca alkaloids, epipodophyllotoxins, tyrosine kinase inhibitors, taxols, radiation therapy, surgery, nutritional supplements, gene therapy, radiotherapy, for example 3D-CRT, immunotoxin therapy, for example ricin, and monoclonal antibodies. Specific examples of chemotherapeutic compounds than can be combined with or used in conjuction with the nucleic acid molecules of the invention include but are not limited to Paclitaxel; Docetaxel; Methotrexate; Doxorubin; Edatrexate; Vinorelbine; Tomaxifen; Leucovorin; 5-fluoro uridine (5-FU); Irinotecan (CAMPTOSAR® or CPT-11 or Camptothecin-11 or Campto); Cisplatin; Carboplatin; Amsacrine; Cytarabine; Bleomycin; Mitomycin C; Dactinomycin; Mithramycin; Hexamethylmelamine; Dacarbazine; L-asperginase; Nitrogen mustard; Melphalan, Chlorambucil; Busulfan; Ifosfamide; 4-hydroperoxycyclophosphamide, Thiotepa; Tamoxifen, Herceptin; IMC C225; ABX-EGF: and combinations thereof.

### Diagnostic uses

The nucleic acid molecules of this invention (e.g., enzymatic nucleic acid molecules) can be used as diagnostic tools to examine genetic drift and mutations within diseased cells or to detect the presence of VEGF and/or VEGFr, such as VEGFR1 and/or VEGFR2 RNA in a cell. The close relationship between enzymatic nucleic acid molecule activity and the structure of the target RNA allows the detection of mutations in any region of the molecule which alters the base-pairing and three-dimensional structure of the target RNA. By using multiple enzymatic nucleic acid molecules described in this invention, one can map nucleotide changes which are important to RNA structure and function in *vitro,* as well as in cells and tissues. Cleavage of target RNAs with enzymatic nucleic acid molecules can be used to inhibit gene expression and define the role (essentially) of specified gene products in the progression of disease. In this manner, other genetic targets can be defined as important mediators of the disease. These experiments can lead to better treatment of the disease progression by affording the possibility of combinational therapies (e.g., multiple enzymatic nucleic acid molecules targeted to different genes, enzymatic nucleic acid molecules coupled with known small molecule inhibitors, or intermittent treatment with combinations of enzymatic nucleic acid molecules and/or other chemical or biological molecules). Other in *vitro* uses of enzymatic nucleic acid molecules of this invention are well known in the art, and include detection of the presence of mRNAs associated with VEGF, VEGFR1 and/or VEGFR2-related condition. Such RNA is detected by determining the presence of a cleavage product after treatment with an enzymatic nucleic acid molecule using standard methodology.

In a specific example, enzymatic nucleic acid molecules which cleave only wild-type or mutant forms of the target RNA are used for the assay. The first enzymatic nucleic acid molecule is used to identify wild-type RNA present in the sample and the second enzymatic nucleic acid molecule is used to identify mutant RNA in the sample. As reaction controls, synthetic substrates of both wild-type and mutant RNA are cleaved by both enzymatic nucleic acid molecules to demonstrate the relative enzymatic nucleic acid molecule efficiencies in the reactions and the absence of cleavage of the "non-targeted" RNA species. The cleavage products from the synthetic substrates also serve to generate size markers for the analysis of wild-type and mutant RNAs in the sample population. Thus each analysis requires two enzymatic nucleic acid molecules, two substrates and one unknown sample which is combined into six reactions. The presence of cleavage products is determined using an RNAse protection assay so that full-length and cleavage fragments of each RNA can be analyzed in one lane of a polyacrylamide gel. It is not absolutely required to quantify the results to gain insight into the expression of mutant RNAs and putative risk of the desired phenotypic changes in target cells. The expression of mRNA whose protein product is implicated in the development of the phenotype (*i.e.*, VEGFR1 and/or VEGFR2) is adequate to establish risk. If probes of comparable specific activity are used for both transcripts, then a qualitative comparison of RNA levels will be adequate and will decrease the cost of the initial diagnosis: Higher mutant form to wild-type ratios are correlated with higher risk whether RNA levels are compared qualitatively or quantitatively. The use of enzymatic nucleic acid molecules in diagnostic applications contemplated by the instant invention is described, for example, in Usman et al., US Patent Application No. 09/877,526, George et al., US Patent Nos. 5,834,186 and 5,741,679, Shih et al., US Patent No. 5,589,332, Nathan et al., US Patent No 5,871,914, Nathan and Ellington, International PCT publication No. WO 00/24931, Breaker et al., International PCT Publication Nos. WO 00/26226 and 98/27104, and Sullenger et al., US Patent Application Serial No. 09/205,520.

### Additional Uses

Uses of sequence-specific enzymatic nucleic acid molecules of the instant invention can have many of the same applications for the study of RNA that DNA restriction endonucleases have for the study of DNA (Nathans et al., 1975 Ann. Rev. Biochem. 44:273). For example, the pattern of restriction fragments can be used to establish sequence relationships between two related RNAs, and large RNAs can be specifically cleaved to fragments of a size more useful for study. The ability to engineer sequence specificity of the enzymatic nucleic acid molecule is ideal for cleavage of RNAs of unknown sequence. Applicant has described the use of nucleic acid molecules to down-regulate gene expression of target genes in bacterial, microbial, fungal, viral, and eukaryotic systems including plant, or mammalian cells.

All patents and publications mentioned in the specification are indicative of the levels of skill of those skilled in the art to which the invention pertains. All references cited in this disclosure are incorporated by reference to the same extent as if each reference had been incorporated by reference in its entirety individually.

One skilled in the art would readily appreciate that the present invention is well adapted to carry out the objects and obtain the ends and advantages mentioned, as well as those inherent therein. The methods and compositions described herein as presently representative of preferred embodiments are exemplary and are not intended as limitations on the scope of the invention. Changes therein and other uses will occur to those skilled in the art, which are encompassed within the spirit of the invention, are defined by the scope of the claims.

It will be readily apparent to one skilled in the art that varying substitutions and modifications may be made to the invention disclosed herein without departing from the scope and spirit of the invention. Thus, such additional embodiments are within the scope of the present invention and the following claims.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments, optional features, modification and variation of the concepts herein disclosed may be resorted to by those skilled in the art, and that such modifications and variations are considered to be within the scope of this invention as defined by the description and the appended claims.

In addition, where features or aspects of the invention are described in terms of Markush groups or other grouping of alternatives, those skilled in the art will recognize that the invention is also thereby described in terms of any individual member or subgroup of members of the Markush group or other group.

Other embodiments are within the following claims.

**TABLE I**

| Characteristics of Ribozvmes |
|---|
| **Group I Introns** |
| Size: ~200 to >1000 nucleotides. |
| Requires a U in the target sequence immediately 5' of the cleavage site. |
| Binds 4-6 nucleotides at 5' side of cleavage site. |
| Over 75 known members of this class. Found in *Tetrahymena thermophila* rRNA, fungal mitochondria, chloroplasts, phage T4, blue-green algae, and others. |

| **RNAseP RNA (M1 RNA)** |
|---|
| Size: ~290 to 400 nucleotides. |
| RNA portion of a ribonucleoprotein enzyme. Cleaves tRNA precursors to form mature tRNA. |
| Roughly 10 known members of this group all are bacterial in origin. |

| **Hammerhead Ribozyme** |
|---|
| Size: ~13 to 40 nucleotides. |
| Requires the target sequence UH immediately 5' of the cleavage site. |
| Binds a variable number of nucleotides on both sides of the cleavage site. |
| 14 known members of this class. Found in a number of plant pathogens (virusoids) that use RNA as the infectious agent (Figure 1 and 2) |

| **Hairpin Ribozyme** |
|---|
| Size: ~50 nucleotides. |
| Requires the target sequence GUC immediately 3' of the cleavage site. |
| Binds 4-6 nucleotides at 5' side of the cleavage site and a variable number to the 3' side of the cleavage site. |
| Only 3 known member of this class. Found in three plant pathogen (satellite RNAs of the tobacco ringspot virus, arabis mosaic virus and chicory yellow mottle virus) which uses RNA as the infectious agent (Figure 3). |

| **Hepatitis Delta Virus (HDV) Ribozyme** |
|---|
| Size: 50 - 60 nucleotides (at present). |
| Sequence requirements not fully determined. |
| Binding sites and structural requirements not fully determined, |
| although no sequences 5' of cleavage site are required. |
| Only 1 known member of this class. Found in human HDV (Figure 4). |

| ***Neurospora* VS RNA Ribozyme** |
|---|
| Size: ~144 nucleotides (at present) |
| Cleavage of target RNAs recently demonstrated. |
| Sequence requirements not fully determined. |
| Binding sites and structural requirements not fully determined. Only 1 known member of this class. Found in *Neurospora* VS RNA (Figure 5). |

**Table II:**

| **A. 2.5 µmol Synthesis Cycle ABI 394 Instrument** | | | | | |
|---|---|---|---|---|---|
| **Reagent** | **Equivalents** | **Amount** | **Wait Time* DNA** | **Wait Time* 2'-O-methyl** | **Wait Time* RNA** |
| Phosphoramidites | 6.5 | 163 µL | 45 sec | 2.5min | 7.5 min |
| *S*-Ethyl Tetrazole | 23.8 | 238 µL | 45 sec | 2.5 min | 7.5 min |
| Acetic Anhydride | 100 | 233 µL | 5 sec | 5 sec | 5 sec |
| *N*-Methyl Imidazole | 186 | 233 µL | 5 sec | 5 sec | 5 sec |
| TCA | 176 | 2.,3 mL | 21 sec | 21 sec | 21 sec |
| iodine | 11.2 | 1.7 mL | 45 sec | 45 sec | 45 sec |
| Beaucage | 12.9 | 645 µL | 100 sec | 300 ,sec | 300 sec |
| Acetonitrile | NA | 6.67 mL | NA | NA | NA |

| **B. 0.2 µmol Synthesis Cycle ABI 394 Instrument** | | | | | |
|---|---|---|---|---|---|
| **Reagent** | **Equivalents** | **Amount** | **Wait Time* DNA** | **Wait Time* 2'-O-methyl** | **Wait Time* RNA** |
| Phosphoramidites | 15 | 31 µL | 45 sec | 233 sec | 465 sec |
| *S*-Ethyl Tetrazole | 38.7 | 31 µL | 45 sec | 233 min | 465 sec |
| Acetic Anhydride | 655 | 124 µL | 5 sec | 5 sec | 5 sec |
| *N*-Methyl Imidazole | 1245 | 124 µL | 5 sec | 5 sec | 5 sec |
| TCA | 700 | 732 µL | 10 sec | 10 sec | 10 sec |
| Iodine | 20.6 | 244 µL | 15 sec | 15 sec | 15 sec |
| Beaucage | 7.7 | 232 µL | 100 sec | 300 sec | 300 sec |
| Acetonitrile | NA | 2.64 mL | NA | NA | NA |

| **C. 0.2 µmol Synthesis Cycle 96 well Instrument** | | | | | |
|---|---|---|---|---|---|
| **Reagent** | **Equivalents DNA/2'-O-methyl/Ribo** | **Amount DNA/2'-O-methyl/Ribo** | **Wait Time* DNA** | **Wait Time* 2'-O-methyl** | **Walt Time* Ribo** |
| Phosphoramidites | 22/33/66 | 40/60/120 µL | 60 sec | 180 sec | 360sec |
| *S*-Ethyl Tetrazole | 70/105/210 | 40/60/120 µL | 60 sec | 180 min | 360 sec |
| Acetic Anhydride | 265/265/265 | 60/50/50 µL | 10 sec | 10 sec | 10 sec |
| *N*-Methyl Imidazole | 502/502/502 | 50/50/50 µL | 10 sec | 10 sec | 10 sec |
| TCA | 238/475/475 | 250/500/500 µL | 15 sec | 15 sec | 15 sec |
| Iodine | 6.8/6.8/6.8 | 80/80/80 µL | 30 sec | 30 sec | 30 sec |
| Beaucage | 34/51/51 | 80/120/120 | 100 sec | 200 sec | 200 sec |
| Acetonitrile | NA | 1150/1150/1150 µL | NA | NA | NA |

| | | | | | |
|---|---|---|---|---|---|
| * Wait time does not Include contact time during delivery. | | | | | |

**Table III: Patient Demographics**

| Dose cohort (mg/m²) | Pt# | Age | Sex | Diagnosis | Doses |
|---|---|---|---|---|---|
| 10 | 1001 | 49 | F | NSC Lung | 29 |
| 10 | 1002 | 65 | F | liposarcoma | 120 |
| 10 | 1003 | 49 | M | nasopharyngeal CA | 109 |
| 30 | 1004 | 35 | M | non-small cell lung | 1 |
| 30 | 1005 | 45 | F | melanoma (ocular) | 113 |
| 30 | 1006 | 57 | M | colon | 199 |
| 30 | 1007 | 39 | F | epitheliod hemangioendothelioma | 198 |
| 100 | 1008 | 52 | M | adrenal CA | 57 |
| 100 | 1009 | 44 | F | breast | 35 |
| 100 | 1010 | 62 | F | renal | 134 |
| 300 | 1011 | 24 | F | melanoma | 31 |
| 300 | 1012 | 57 | M | renal cell | 178 |
| 300 | 1013 | 53 | M | nasopharyngeal SCCA | 29 |
| 300 | 1014 | 64 | F | peritoneal mesothelioma | 324 |
| 100 | 1015 | 65 | M | melanoma | 140 |
| 100 | 1016 | 77 | F | breast | 265 |
| 100 | 1017 | | F | melanoma | 35 |
| 100 | 1018 | 26 | F | melanoma | 7 |
| 100 | 1019 | 69 | F | endometrial sarcoma | 500 |
| 100 | 1020 | 65 | M | carcinoid | 124 |
| 100 | 1021 | 59 | M | gallbladder adeno carcinoma | 34 |
| 100 | 1022 | 43 | M | colorectal | 8 |
| 100. | 1023 | 78 | F | breast | 50 |
| 100 | 1024 | 40 | F | parotid adenocarcinoma | 285 |
| 100 | 1025 | 52 | F | breast | 71 |
| 100 | 1026 | 39 | F | breast | 34 |
| 100 | 1027 | 55 | F | breast | 36 |
| 100 | 1028 | 52 | M | melanoma | 29 |
| 100 | 1029 | 38 | M | pancreatic | 36 |
| 100 | 1030 | 83 | M | melanoma | 41 |
| 100 | 1031 | 50 | M | medullary thyroid | 108 |

| | | | | | |
|---|---|---|---|---|---|
| **One patient taken off study due to progressive disease. Allowed to resume ANGIOZYIME on a compassionate basis**. **As of September 1, 2001, all patients were off study. (Although one patient resumed treatment per above note)** | | | | | |

**Table IV Pharmacokinetic parameters of ANGIOZYME after bolus subcutaneous administration.**

| | | **10 mg/m²** | | **30 mg/m²** | | **100 mg/m²** | | **300 mg/m²** | |
|---|---|---|---|---|---|---|---|---|---|
| | | **Mean** | **SD** | **Mean** | **SD** | **Mean** | **SD** | **Mean** | **SD** |
| ***Day 1*** | **Cmax (ug/mL)** | 0.43 | 0.07 | 0.62 | 0.28 | 3.17 | 0.69 | 8.91 | 2.93 |
| | **AUCt (ug*hr/mL)** | 2.60 | 1.43 | 6.04 | 2.70 | 34.14 | 2.28 | 89.87 | 21.68 |
| | **AUCinf (ug*hr/mL)** | 4.40 | 0.06 | 7.99 | 1.66 | 37.51 | 1.91 | 101.57 | 13.47 |
| | **t(1/2) (hr)** | 3.62 | 0.79 | 7.32 | 6.94 | 4.58 | 0.02 | 9.26 | 6.20 |
| | **CL/F (L/hr/m²)** | 2.24 | 0.08 | 3.73 | 0.92 | 2.96 | 0.61 | 2.99 | 0.43 |
| ***Day 29*** | **Cmax (ug/mL)** | 0.35 | 0.19 | 1.17 | 0.53 | 3.23 | 0.35 | 8.93 | 6.71 |
| | **AUCT (ug*hr/mL)** | 2.11 | 1.31 | 7.29 | 1.16 | 31.87 | 1.91 | 119.42 | 65.84 |
| | **AUCinf(ug*hr/mL)** | 3.38 | 1.31 | 8.54 | 2.46 | 33.61 | 2.16 | 132.73 | 67.82 |
| | **t(1/2) (hr)** | 4.49 | 1.60 | 3.26 | 1.01 | 4.66 | 0.35 | 7.24 | 0.70 |
| | **CL/F (L/hr/m²)** | 2.49 | 1.48 | 3.69 | 0.94 | 3.21 | 0.56 | 2.72 | 1.40 |

**Table V: Human FLT DNAzyme and Substrate Sequence**

| **Pos** | **Substrate** | **Seq ID No** | **DNAzyme** | **Seq ID No** |
|---|---|---|---|---|
| 17 | UCCUCUCG G CUCCUCCC | 1 | GGGAGGAG GGCTAGCTACAACGA CGAGAGGA | 1703 |
| 28 | CCUCCCCG G CAGCGGCG | 2 | CGCCGCTG GGCTAGCTACAACGA CGGGGAGG | 1704 |
| 31 | CCCCGGCA G CGGCGGCG | 3 | CGCCGCCG GGCTAGCTACAACGA TGCCGGGG | 1705 |
| 34 | CGGCAGCG G CGGCGGCU | 4 | . AGCCGCCG GGCTAGCTACAACGA CGCTGCCG | 1706 |
| 37 | CAGCGGCG G CGGCUCGG | 5 | CCGAGCCG GGCTAGCTACAACGA CGCCGCTG | 1707 |
| 40 | CGGCGGCG G CUCGGAGC | 6 | GCTCCGAG GGCTAGCTACAACGA CGCCGCCG | 1708 |
| 47 | GGCUCGGA G CGGGCUCC | 7 | GGAGCCCG GGCTAGCTACAACGA TCCGAGCC | 1709 |
| 51 | CGGAGCGG G CUCCGGGG | 8 | GCCCGGAG GGCTAGCTACAACGA CCGCTCCG | 1710 |
| 59 | GCUCCGGG G CUCGGGUG | 9 | CACCCGAG GGCTAGCTACAACGA CCCGGAGC | 1711 |
| 65 | GGGCUCGG G UGCAGCGG | 10 | CCGCTGCA GGCTAGCTACAACGA CCGAGCCC | 1712 |
| 67 | GCUCGGGU G CAGCGGCC | 11 | GGCCGCTG GGCTAGCTACAACGA ACCCGAGC | 1713 |
| 70 | CGGGUGCA G CGGCCAGC | 12 | GCTGGCCG GGCTAGCTACAACGA TGCACCCG | 1714 |
| 73 | GUGCAGCG G CCAGCGGG | 13 | CCCGCTGG GGCTAGCTACAACGA CGCTGCAC | 1715 |
| 77 | AGCGGCCA G CGGGCCUG | 14 | CAGGCCCG GGCTAGCTACAACGA TGGCCGCT | 1716 |
| 81. | GCCAGCGG G CCUGGCGG | 15 | CCGCCAGG GGCTAGCTACAACGA CCGCTGGC | 1717 |
| 86 | CGGGCCUG G CGGCGAGG | 16 | CCTCGCCG GGCTAGCTACAACGA CAGGCCCG | 1718 |
| 89 | GCCUGGCG G CGAGGAUU | 17 | AATCCTCG GGCTAGCTACAACGA CGCCAGGC | 1719 |
| 95 | CGGCGAGG A UUACCCGG | 18 | CCGGGTAAGGCTAGCTACAACGA CCTCGCCG | 1720 |
| 98 | CGAGGAUU A CCCGGGGA | 19 | TCCCCGGG GGCTAGCTACAACGA AATCCTCG | 1721 |
| 108 | CCGGGGAA G UGGUUGUC | 20 | GACAACCA GGCTAGCTACAACGA TTCCCCGG | 1722 |
| 111 | GGGAAGUG G UUGUCUCC | 21 | GGAGACAA GGCTAGCTACAACGA CACTTCCC | 1723 |
| 114 | AAGUGGUU G UCUCCUGG | 22 | CCAGGAGA GGCTAGCTACAACGA AACCACTT | 1724 |
| 122 | GUCUCCUG G CUGGAGCC | 23 | GGCTCCAG GGCTAGCTACAACGA CAGGAGAC | 1725 |
| 128 | UGGCUGGA G CCGCGAGA | 24 | TCTCGCGG GGCTAGCTACAACGA TCCAGCCA | 1726 |
| 131 | CUGGAGCC G CGAGACGG | 25 | CCGTCTCG GGCTAGCTACAACGA GGCTCCAG | 1727 |
| 136 | GCCGCGAG A CGGGCGCU | 26 | AGCGCCCG GGCTAGCTACAACGA CTCGCGGC | 1728 |
| 140 | CGAGACGG G CGCUCAGG | 27 | CCTGAGCG GGCTAGCTACAACGA CCGTCTCG | 1729 |
| 142 | AGACGGGC G CUCAGGGC | 28 | GCCCTGAG GGCTAGCTACAACGA GCCCGTCT | 1730 |
| 149 | CGCUCAGG G CGCGGGGC | 29 | GCCCCGCG GGCTAGCTACAACGA CCTGAGCG | 1731 |
| 151 | CUCAGGGC G CGGGGCCG | 30 | CGGCCCCG GGCTAGCTACAACGA GCCCTGAG | 1732 |
| 156 | GGCGCGGG G CCGGCGGC | 31 | GCCGCCGG GGCTAGCTACAACGA CCCGCGCC | 1733 |
| 160 | CGGGGCCG G CGGCGGCG | 32 | CGCCGCCG GGCTAGCTACAACGA CGGCCCCG | 1734 |
| 163 | GGCCCGCG G CGGCGAAC | 33 | GTTCGCCG GGCTAGCTACAACGA CGCCGGCC | 1735 |
| 166 | CGGCGGCG G CGAACGAG | 34 | CTCGTTCG GGCTAGCTACAACGA CGCCGCCG | 1736 |
| 170 | GGCGGCGA A CGAGAGGA | 35 | TCCTCTCG GGCTAGCTACAACGA TCGCCGCC | 1737 |
| 178 | ACGAGAGG A CGGACUCU | 36 | AGAGTCCG GGCTAGCTACAACGA CCTCTCGT | 1738 |
| 182 | GAGGACGG A CUCUGGCG | 37 | CGCCAGAG GGCTAGCTACAACGA CCGTCCTC | 1739 |
| 188 | GGACUCUG G CGGCCGGG | 38 | CCCGGCCG GGCTAGCTACAACGA CAGAGTCC | 1740 |
| 191 | CUCUGGCG G CCGGGUCG | 39 | CGACCCGG GGCTAGCTACAACGA CGCCAGAG | 1741 |
| 196 | GCGGCCGG G UCGUUGGC | 40 | GCCAACGA GGCTAGCTACAACGA CCGGCCGC | 1742 |
| 199 | GCCGGGUC G UUGGCCGG | 41 | CCGGCCAA GGCTAGCTACAACGA GACCCGGC | 1743 |
| 203 | GGUCGUUG G CCGGGGGA | 42 | TCCCCCGG GGCTAGCTACAACGA CAACGACC | 1744 |
| 212 | CCGGGGGA G CGCGGGCA | 43 | TGCCCGCG GGCTAGCTACAACGA TCCCCCGG | 1745 |
| 214 | GGGGGAGC G CGGGCACC | 44 | GGTGCCCG GGCTAGCTACAACGA GCTCCCCC | 1746 |
| 218 | GAGCGCGG G CACCGGGC | 45 | GCCCGGTG GGCTAGCTACAACGA CCGCGCTC | 1747 |
| 220 | GCGCGGGC A CCGGGCGA | 46 | TCGCCCGG GGCTAGCTACAACGA GCCCGCGC | 1748 |
| 225 | GGCACCGG G CGAGCAGG | 47 | CCTGCTCG GGCTAGCTACAACGA CCGGTGCC | 1749 |
| 229 | CCGGGCGA G CAGGCCGC | 48 | GCGGCCTG GGCTAGCTACAACGA TCGCCCGG | 1750 |
| 233 | GCGAGCAG G CCGCGUCG | 49 | CGACGCGG GGCTAGCTACAACGA CTGCTCGC | 1751 |
| 236 | AGCAGGCC G CGUCGCGC | 50 | GCGCGACG GGCTAGCTACAACGA GGCCTGCT | 1752 |
| 238 | CAGGCCGC G UCGCGCUC | 51 | GAGCGCGA GGCTAGCTACAACGA GCGGCCTG | 1753 |
| 241 | GCCGCGUC G CGCUCACC | 52 | GGTGAGCG GGCTAGCTACAACGA GACGCGGC | 1754 |
| 243 | CGCGUCGC G CUCACCAU | 53 | ATGGTGAG GGCTAGCTACAACGA GCGACGCG | 1755 |
| 247 | UCGCGCUC A CCAUGGUC | 54 | GACCATGG GGCTAGCTACAACGA GAGCGCGA | 1756 |
| 250 | CGCUCACC A UGGUCAGC | 55 | GCTGACCA GGCTAGCTACAACGA GGTGAGCG | 1757 |
| 253 | UCACCAUG G UCAGCUAC | 56 | GTAGCTGA GGCTAGCTACAACGA CATGGTGA | 1758 |
| 257 | CAUGGUCA G CUACUGGG | 57 | CCCAGTAG GGCTAGCTACAACGA TGACCATG | 1759 |
| 260 | GGUCAGCU A CUGGGACA | 58 | TGTCCCAG GGCTAGCTACAACGA AGCTGACC | 1760 |
| 266 | CUACUGGG A CACCGGGG | 59 | CCCCGGTG GGCTAGCTACAACGA CCCAGTAG | 1761 |
| 268 | ACUGGGAC A CCGGGGUC | 60 | GACCCCGG GGCTAGCTACAACGA GTCCCAGT | 1762 |
| 274 | ACACCGGG G UCCUGCUG | 61 | CAGCAGGA GGCTAGCTACAACGA CCCGGTGT | 1763 |
| 279 | GGGGUCCU G CUGUGCGC | 62 | GCGCACAG GGCTAGCTACAACGA AGGACCCC | 1764 |
| 282 | GUCCUGCU G UGCGCGCU | 63 | AGCGCGCA GGCTAGCTACAACGA AGCAGAC | 1765 |
| 284 | CCUGCUGU G CGCGCUGC | 64 | GCAGCGCG GGCTAGCTACAACGA ACAGCAGG | 1766 |
| 286 | UGCUGUGC G CGCUGCUC | 65 | GAGCAGCG GGCTAGCTACAACGA GCACAGCA | 1767 |
| 288 | CUGUGCGC G CUGCUCAC | 66 | CTGAGCAG GGCTAGCTACAACGA GCGCACAG | 1768 |
| 291 | UGCGCGCU G CUCAGCUG | 67 | CAGCTGAG GGCTAGCTACAACGA AGCGCGCA | 1769 |
| 296 | GCUGCUCA G CUGUCUGC | 68 | GCAGACAG GGCTAGCTACAACGA TGAGCAGC | 1770: |
| 299 | GCUCAGCU G UCUGCUUC | 69 | GAAGCAGA GGCTAGCTACAACGA AGCTGAGC | 1771 |
| 303 | AGCUGUCU G CUUCUCAC | 70 | GTGAGAAG GGCTAGCTACAACGA AGACAGCT | 1772 |
| 310 | UGCUUCUC A CAGGAUCU | 71 | AGATCCTG GGCTAGCTACAACGA GAGAAGCA | 1773 |
| 315 | CUCACAGG A UCUAGUUC | 72 | GAACTAGA GGCTAGCTACAACGA CCTGTGAG | 1774 |
| 320 | AGGAUCUA G UUCAGGUU | 73 | AACCTGAA GGCTAGCTACAACGA TAGATCCT | 1775 |
| 326 | UAGUUCAG G UUCAAAAU | 74 | ATTTTGAA GGCTAGCTACAACGA CTGAACTA | 1776 |
| 333 | GGUUCAAA A UUAAAAGA | 75 | TCTTTTAA GGCTAGCTACAACGA TTTGAACC | 1777 |
| 341 | AUUAAAAG A UCCUGAAC | 76 | GTTCAGGA GGCTAGCTACAACGA CTTTTAAT | 1778 |
| 348 | GAUCCUGA A CUGAGUUU | 77 | AAACTCAG GGCTAGCTACAACGA TCAGGATC | 1779 |
| 353 | UGAACUGA G UUUAAAAG | 78 | CTTTTAAA GGCTAGCTACAACGA TCAGTTCA | 1780 |
| 362 | UUUAAAAG G CACCCAGC | 79 | GCTGGGTG GGCTAGCTACAACGA CTTTTAAA | 1781 |
| 364 | UAAAAGGC A CCCAGCAC | 80 | GTGCTGGG GGCTAGCTACAACGA GCCTTTTA | 1782 |
| 369 | GGCACCCA G CACAUCAU | 81 | ATGATGTG GGCTAGCTAGAACGA TGGGTGCC | 1783 |
| 371 | CACCCAGC A CAUCAUGC | 82 | GCATGATG GGCTAGCTACAACGA GCTGGGTG | 1784 |
| 373 | CCCAGCAC A UCAUGCAA | 83 | TTGCATGA GGCTAGCTACAACGA GTGCTGGG | 1785 |
| 376 | AGCACAUC A UGCAAGGA | 84 | TGCTTGCA GGCTAGCTACAACGA GATGTGCT | 1786 |
| 378 | CACAUCAU G CAAGCAGG | 85 | CCTGCTTG GGCTAGCTACAACGA ATGATGTG | 1787 |
| 382 | UCAUGCAA G CAGGCCAG | 86 | CTGGCCTG GGCTAGCTACAACGA TTGCATGA | 1788 |
| 386 | GCAAGCAG G CCAGACAC | 87 | GTGTCTGG GGCTAGCTACAACGA CTGCTTGC | 1789 |
| 391 | CAGGCCAG A CACUGCAU | 88 | ATGCAGTG GGCTAGCTACAACGA CTGGCCTG | 1790 |
| 393 | GGCCAGAC A CUGCAUCU | 89 | AGATGCAG GGCTAGCTACAACGA GTCTGGCC | 1791 |
| 396 | CAGACACU G CAUCUCCA | 90 | TGGAGATG GGCTAGCTACAACGA AGTGTCTG | 1792 |
| 398 | GACACUGC A UCUCCAAU | 91 | ATTGGAGA GGCTAGCTACAACGA GCAGTGTC | 1793 |
| 405 | CAUCUCCA A UGCAGGGG | 92 | CCCCTGCA GGCTAGCTACAACGA TGGAGATG | 1794 |
| 407 | UCUCCAAU G CAGGGGGG | 93 | CCCCCCTG GGCTAGCTACAACGA ATTGGAGA | 1795 |
| 418 | GGGGGGAA G CAGCCCAU | 94 | ATGGGCTG GGCTAGCTACAACGA TTCCCCCC | 1796 |
| 421 | GGGAAGCA G CCCAUAAA | 95 | TTTATGGG GGCTAGCTACAACGA TGCTTCCC | 1797 |
| 425 | AGCAGCCC A UAAAUGGU | 96 | ACCATTTA GGCTAGCTACAACGA GGGCTGCT | 1798 |
| 429 | GCCCAUAA A UGGUCUUU | 97 | AAAGACCA GGCTAGCTACAACGA TTATGGGC | 1799 |
| 432 | CAUAAAUG G UCUUUGCC | 98 | GGCAAAGA GGCTAGCTACAACGA CATTTATG | 1800 |
| 438 | UGGUCUUU G CCUGAAAU | 99 | ATTTCAGG GGCTAGCTACAACGA AAAGACCA | 1801 |
| 445 | UGCCUGAA A UGGUGAGU | 100 | ACTCACCA GGCTAGCTACAACGA TTCAGGCA | 1802 |
| 448 | CUGAAAUG G UGAGUAAG | 101 | CTTACTCA GGCTAGCTACAACGA CATTTCAG | 1803 |
| 452 | AAUGGUGA G UAAGGAAA | 102 | TTTCCTTA GGCTAGCTACAACGA TCACCATT | 1804 |
| 461 | UAAGGAAA G CGAAAGGC | 103 | GCCTTTCG GGCTAGCTACAACGA TTTCCTTA | 1805 |
| 468 | AGCGAAAG G CUGAGCAU | 104 | ATGCTCAG GGCTAGCTACAACGA CTTTCGCT | 1806 |
| 473 | AAGGCUGA G CAUAACUA | 105 | TAGTTATG GGCTAGCTACAACGA TCAGCCTT | 1807 |
| 475 | GGCUGAGC A UAACUAAA | 106 | TTTAGTTA GGCTAGCTACAACGA GCTCAGCC | 1808 |
| 478 | UGAGCAUA A CUAAAUCU | 107 | AGATTTAG GGCTAGCTACAACGA TATGCTCA | 1809 |
| 483 | AUAACUAA A UCUGCCUG | 108 | CAGGCAGA GGCTAGCTACAACGA TTAGTTAT | 1810 |
| 487 | CUAAAUCU G CCUGUGGA | 109 | TCCACAGG GGCTAGCTACAACGA AGATTTAG | 1811 |
| 491 | AUCUGCCU G UGGAAGAA | 110 | TTCTTCCA GGCTAGCTACAACGA AGGCAGAT | 1812 |
| 500 | UGGAAGAA A UGGCAAAC | 111 | GTTTGCCA GGCTAGCTACAACGA TTCTTCCA | 1813 |
| 503 | AAGAAAUG G CAAACAAU | 112 | ATTGTTTG GGCTAGCTACAACGA CATTTCTT | 1814 |
| 507 | AAUGGCAA A CAAUUCUG | 113 | CAGAATTG GGCTAGCTACAACGA TTGCCATT | 1815 |
| 510 | GGCAAACA A UUCUGCAG | 114 | CTGCAGAA GGCTAGCTACAACGA TGTTTGCC | 1816 |
| 515 | ACAAUUCU G CAGUACUU | 115 | AAGTACTG GGCTAGCTACAACGA AGAATTGT | 1817 |
| 518 | AUUCUGCA G UACUUUAA | 116 | TTAAAGTA GGCTAGCTACAACGA TGCAGAAT | 1818 |
| 520 | UCUGCAGU A CUUUAACC | 117 | GGTTAAAG GGCTAGCTACAACGA ACTGCAGA | 1819 |
| 526 | GUACUUUA A CCUUGAAC | 118 | GTTCAAGG GGCTAGCTACAACGA TAAAGTAC | 1820 |
| 533 | AACCUUGA A CACAGCUC | 119 | GAGCTGTG GGCTAGCTACAACGA TCAAGGTT | 1821 |
| 535 | CCUUGAAC A CAGCUCAA | 120 | TTGAGCTG GGCTAGCTACAACGA GTTCAAGG | 1822 |
| 538 | UGAACACA G CUCAAGCA | 121 | TGCTTGAG GGCTAGCTACAACGA TGTGTTCA | 1823 |
| 544 | CAGCUCAA G CAAACCAC | 122 | GTGGTTTG GGCTAGCTACAACGA TTGAGCTG | 1824 |
| 548 | UCAAGCAA A CCACACUG | 123 | CAGTGTGG GGCTAGCTACAACGA TTGCTTGA | 1825 |
| 551 | AGCAAACC A CACUGGCU | 124 | AGCCAGTG GGCTAGCTACAACGA GGTTTGCT | 1826 |
| 553 | CAAACCAC A CUGGCUUC | 125 | GAAGCCAG GGCTAGCTACAACGA GTGGTTTG | 1827 |
| 557 | CCACACUG G CUUCUACA | 126 | TGTAGAAG GGCTAGCTACAACGA CAGTGTGG | 1828 |
| 563 | UGGCUUCU A CAGCUGCA | 127 | TGCAGCTG GGCTAGCTACAACGA AGAAGCCA | 1829 |
| 566 | CUUCUACA G CUGCAAAU | 128 | ATTTGCAG GGCTAGCTACAACGA TGTAGAAG | 1830 |
| 569 | CUACAGCU G CAAAUAUC | 129 | GATATTTG GGCTAGCTACAACGA AGCTGTAG | 1831 |
| 573 | AGCUGCAA A UAUCUAGC | 130 | GCTAGATA GGCTAGCTACAACGA TTGCAGCT | 1832 |
| 575 | CUGCAAAU A UCUAGCUG | 131 | CAGCTAGA GGCTAGCTACAACGA ATTTGCAG | 1833 |
| 580 | AAUAUCUA G CUGUACCU | 132 | AGGTACAG GGCTAGCTACAACGA TAGATATT | 1834 |
| 583 | AUCUAGCU G UACCUACU | 133 | AGTAGGTA GGCTAGCTACAACGA AGCTAGAT | 1835 |
| 585 | CUAGCUGU A CCUACUUC | 134 | GAAGTAGG GGCTAGCTACAACGA ACAGCTAG | 1836 |
| 589 | CUGUACCU A CUUCAAAG | 135 | CTTTGAAG GGCTAGCTACAACGA AGGTACAG | 1837 |
| 607 | AGAAGGAA A CAGAAUCU | 136 | AGATTCTG GGCTAGCTACAACGA TTCCTTCT | 1838 |
| 612 | GAAACAGA A UCUCCAAU | 137 | ATTGCAGA GGCTAGCTACAACGA TCTGTTTC | 1839 |
| 616 | CAGAAUCU G CAAUCUAU | 138 | ATAGATTG GGCTAGCTACAACGA AGATTCTG | 1840 |
| 619 | AAUCUGCA A UCUAUAUA | 139 | TATATAGA GGCTAGCTACAACGA TGCAGATT | 1841 |
| 623 | UGCAAUCU A UAUAUUUA | 140 | TAAATATA GGCTAGCTACAACGA AGATTGCA | 1842 |
| 625 | CAAUCUAU A UAUUUAUU | 141 | AATAAATA GGCTAGCTACAACGA ATAGATTG | 1843 |
| 627 | AUCUAUAU A UUUAUUAG | 142 | CTAATAAA GGCTAGCTACAACGA ATATAGAT | 1844 |
| 631 | AUAUAUUU A UUAGUGAU | 143 | ATCACTAA GGCTAGCTACAACGA AAATATAT | 1845 |
| 635 | AUUUAUUA G UGAUACAG | 144 | CTGTATCA GGCTAGCTACAACGA TAATAAAT | 1846 |
| 638 | UAUUAGUG A UACAGGUA | 145 | TACCTGTA GGCTAGCTACAACGA CACTAATA | 1847 |
| 640 | UUAGUGAU A CAGGUAGA | 146 | TCTACCTG GGCTAGCTACAACGA ATCACTAA | 1848 |
| 644 | UGAUACAG G UAGACCUU | 147 | AAGGTCTA GGCTAGCTACAACGA CTGTATCA | 1849 |
| 648 | ACAGGUAG A CCUUUCGU | 148 | ACGAAAGG GGCTAGCTACAACGA CTACCTGT | 1850 |
| 655 | GACCUUUC G UAGAGAUG | 149 | CATCTCTA GGCTAGCTACAACGA GAAAGGTC | 1851 |
| 661 | UCGUAGAG A UGUACAGU | 150 | ACTGTACA GGCTAGCTACAACGA CTCTACGA | 1852 |
| 663 | GUAGAGAU G UACAGUGA | 151 | TCACTGTA GGCTAGCTACAACGA ATCTCTAC | 1853 |
| 665 | AGAGAUGU A CAGUGAAA | 152 | TTTCACTG GGCTAGCTACAACGA ACATCTCT | 1854 |
| 668 | GAUGUACA G UGAAAUCC | 153 | GGATTTCA GGCTAGCTACAACGA TGTACATC | 1855 |
| 673 | ACAGUGAA A UCCCCGAA | 154 | TTCGGGGA GGCTAGCTACAACGA TTCACTGT | 1856 |
| 682 | UCCCCGAA A UUAUACAC | 155 | GTGTATAA GGCTAGCTACAACGA TTCGGGGA | 1857 |
| 685 | CCGAAAUU A UACACAUG | 156 | CATGTGTA GGCTAGCTACAACGA AATTTCGG | 1858 |
| 687 | GAAAUUAU A CACAUGAC | 157 | GTCATGTG GGCTAGCTACAACGA ATAATTTC | 1859 |
| 689 | AAUUAUAC A CAUGACUG | 158 | CAGTCATG GGCTAGCTACAACGA GTATAATT | 1860 |
| 691 | UUAUACAC A UGACUGAA | 159 | TTCAGTCA GGCTAGCTACAACGA GTGTATAA | 1861 |
| 694 | UACACAUG A CUGAAGGA | 160 | TCCTTCAG GGCTAGCTACAACGA CATGTGTA | 1862 |
| 708 | GGAAGGGA G CUCGUCAU | 161 | ATGACGAG GGCTAGCTACAACGA TCCCTTCC | 1863 |
| 712 | GGGAGCUC G UCAUUCCC | 162 | GGGAATGA GGCTAGCTACAACGA GAGCTCCC | 1864 |
| 715 | AGCUCGUC A UUCCCUGC | 163 | GCAGGGAA GGCTAGCTACAACGA GACGAGCT | 1865 |
| 722 | CAUUCCCU G CCGGGUUA | 164 | TAACCCGG GGCTAGCTACAACGA AGGGAATG | 1866 |
| 727 | CCUGCCGG G UUACGUCA | 165 | TGACGTAA GGCTAGCTACAACGA CCGGCAGG | 1867 |
| 730 | GCCGGGUU A CGUCACCU | 166 | AGGTGACG GGCTAGCTACAACGA AACCCGGC | 1868 |
| 732 | CGGGUUAC G UCACCUAA | 167 | TTAGGTGA GGCTAGCTACAACGA GTAACCCG | 1869 |
| 735 | GUUACGUC A CCUAACAU | 168 | ATGTTAGG GGCTAGCTACAACGA GACGTAAC | 1870 |
| 740 | GUCACCUA A CAUCACUG | 169 | CAGTGATG GGCTAGCTACAACGA TAGGTGAC | 1871 |
| 742 | CACCUAAC A UCACUGUU | 170 | AACAGTGA GGCTAGCTACAACGA GTTAGGTG | 1872 |
| 745 | CUAACAUC A CUGUUACU | 171 | AGTAACAG GGCTAGCTACAACGA GATGTTAG | 1873 |
| 748 | ACAUCACU G UUACUUUA | 172 | TAAAGTAA GGCTAGCTACAACGA AGTGATGT | 1874 |
| 751 | UCACUGUU A CUUUAAAA | 173 | TTTTAAAG GGCTAGCTACAACGA AACAGTGA | 1875 |
| 762 | UUAAAAAA G UUUCCACU | 174 | AGTGGAAA GGCTAGCTACAACGA TTTTTTAA | 1876 |
| 768 | AAGUUUCC A CUUGACAC | 175 | GTGTCAAG GGCTAGCTACAACGA GGAAACTT | 1877 |
| 773 | UCCACUUG A CACUUUGA | 176 | TCAAAGTG GGCTAGCTACAACGA CAAGTGGA | 1878 |
| 775 | CACLTUGAC A CUUUGAUC | 177 | GATCAAAG GGCTAGCTACAACGA GTCAAGTG | 1879 |
| 781 | ACACUUUG A UCCCUGAU | 178 | ATCAGGGA GGCTAGCTACAACGA CAAAGTGT | 1880 |
| 788 | GAUCCCUG A UGGAAAAC | 179 | GTTTTCCA GGCTAGCTACAACGA CAGGGATC | 1881 |
| 795 | GAUGGAAA A CGCAUAAU | 180 | ATTATGCG GGCTAGCTACAACGA TTTCCATC | 1882 |
| 797 | UGGAAAAC G CAUAAUCU | 181 | AGATTATG GGCTAGCTACAACGA GTTTTCCA | 1883 |
| 799 | GAAAACGC A UAAUCUGG | 182 | CCAGATTA GGCTAGCTACAACGA GCGTTTTC | 1884 |
| 802 | AACGCAUA A UCUGGGAC | 183 | GTCCCAGA GGCTAGCTACAACGA TATGCGTT | 1885 |
| 809 | AAUCUGGG A CAGUAGAA | 184 | TTCTACTG GGCTAGCTACAACGA CCCAGATT | 1886 |
| 812 | CUGGGACA G UAGAAAGG | 185 | CCTTTCTA GGCTAGCTACAACGA TGTCCCAG | 1887 |
| 821 | UAGAAAGG G CUUCAUCA | 186 | TGATGAAG GGCTAGCTACAACGA CCTTTCTA | 1888 |
| 826 | AGGGCUUC A UCAUAUCA | 187 | TGATATGA GGCTAGCTACAACGA GAAGCCCT | 1889 |
| 829 | GCUUCAUC A UAUCAAAU | 188 | ATTTGATA GGCTAGCTACAACGA GATGAAGC | 1890 |
| 831 | UUCAUCAU A UCAAAUGC | 189 | GCATTTGA GGCTAGCTACAACGA ATGATGAA | 1891 |
| 836 | CAUAUCAA A UGCAACGU | 190 | ACGTTGCA GGCTAGCTACAACGA TTGATATG | 1892 |
| 838 | UAUCAAAU G CAACGUAC | 191 | GTACGTTG GGCTAGCTACAAGGA ATTTGATA | 1893 |
| 841 | CAAAUGCA A CGUACAAA | 192 | TTTGTACG GGCTAGCTACAACGA TGCATTTG | 1894 |
| 843 | AAUGCAAC G UACAAAGA | 193 | TCTTTGTA GGCTAGCTACAACGA GTTGCATT | 1895 |
| 845 | UGCAACGU A CAAAGAAA | 194 | TTTCTTTG GGCTAGCTACAACGA ACGTTGCA | 1896 |
| 853 | ACAAAGAA A UAGGGCUU | 195 | AAGCCCTA GGCTAGCTACAACGA TTCTTTGT | 1897 |
| 858 | GAAAUAGG G CUUCUGAC | 196 | GTCAGAAG GGCTAGCTACAACGA CCTATTTC | 1898 |
| 865 | GGCUUCUG A CCUGUGAA | 197 | TTCACAGG GGCTAGCTACAACGA CAGAAGCC | 1899 |
| 869 | UCUGACCU G UGAAGCAA | 198 | TTGCTTCA GGCTAGCTACAACGA AGGTCAGA | 1900 |
| 874 | CCUGUGAA G CAACAGUC | 199 | GACTGTTG GGCTAGCTACAACGA TTCACAGG | 1901 |
| 877 | GUGAAGCA A CAGUCAAU | 200 | ATTGACTG GGCTAGCTACAACGA TGCTTCAC | 1902 |
| 880 | AAGCAACA G UCAAUGGG | 201 | CCCATTGA GGCTAGCTACAACGA TGTTGCTT | 1903 |
| 884 | AACAGUCA A UGGGCAUU | 202 | AATGCCCA GGCTAGCTACAACGA TGACTGTT | 1904 |
| 888 | GUCAAUGG G CAUUUGUA | 203 | TACAAATG GGCTAGCTACAACGA CCATTGAC | 1905 |
| 890 | CAAUGGGC A UUUGUAUA | 204 | TATACAAA GGCTAGCTACAACGA GCCCATTG | 1906 |
| 894 | GGGCAUUU G UAUAAGAC | 205 | GTCTTATA GGCTAGCTACAACGA AAATGCCC | 1907 |
| 896 | GCAUUUGU A UAAGACAA | 206 | TTGTCTTA GGCTAGCTACAACGA ACAAATGC | 1908 |
| 901 | UGUAUAAG A CAAACUAU | 207 | ATAGTTTG GGCTAGCTACAACGA CTTATACA | 1909 |
| 905 | UAAGACAA A CUAUCUCA | 208 | TGAGATAG GGCTAGCTACAACGA TTGTCTTA | 1910 |
| 908 | GACAAACU A UCUCACAC | 209 | GTGTGAGA GGCTAGCTACAACGA AGTTTGTC | 1911 |
| 913 | ACUAUCUC A CACAUCGA | 210 | TCGATGTG GGCTAGCTACAACGA GAGATAGT | 1912 |
| 915 | UAUCUCAC A CAUCGACA | 211 | TGTCGATG GGCTAGCTACAACGA GTGAGATA | 1913 |
| 917 | UCUCACAC A UCGACAAA | 212 | TTTGTCGA GGCTAGCTACAACGA GTGTGAGA | 1914 |
| 921 | ACACAUCG ACAAACCAA | 213 | TTGGTTTG GGCTAGCTACAACGA CGATGTGT | 1915 |
| 925 | AUCGACAA A CCAAOACA | 214 | TGTATTGG GGCTAGCTACAACGA TTGTCGAT | 1916 |
| 929 | ACAAACCA A UACAAUCA | 215 | TGATTGTA GGCTAGCTACAACGA TGGTTTGT | 1917 |
| 931 | AAACCAAU A CAAUCAUA | 216 | TATGATTG GGCTAGCTACAACGA ATTGGTTT | 1918 |
| 934 | CCAAUACA A UCAUAGAU | 217 | ATCTATGA GGCTAGCTACAACGA TGTATTGG | 1919 |
| 937 | AUACAAUC A UAGAUGUC | 218 | GACATCTA GGCTAGCTACAACGA GATTGTAT | 1920 |
| 941 | AAUCAUAG A.UGUCCAAA | 219 | TTTGGACA GGCTAGCTACAACGA CTATGATT | 1921 |
| 943 | UCAUAGAU G UCCAAAUA | 220 | TATTTGGA GGCTAGCTACAACGA ATCTATGA | 1922 |
| 949 | AUGUCCAA A UAAGCACA | 221 | 3'GTGCTTA GGCTAGCTACAACGA TTGGACAT | 1923 |
| 953 | CCAAAUAA G CACACCAC | 222 | GTGGTGTG GGCTAGCTACAACGA TTATTTGG | 1924 |
| 955 | AAAUAAGC A CACCACGC | 223 | GCGTGGTG GGCTAGCTACAACGA GCTTATTT | 1925 |
| 957 | AUAAGCAC A CCACGCCC | 224 | GGGCGTGG GGCTAGCTACAACGA GTGCTTAT | 1926 |
| 960 | AGCACACC A CGCCCAGU | 225 | ACTGGGCG GGCTAGCTACAACGA GGTGTGCT | 1927 |
| 962 | CACACCAC G CCCAGUCA | 226 | TGACTGGG GGCTAGCTACAACGA GTGGTGTG | 1928 |
| 967 | CACGCCCA G UCAAAUUA | 227 | TAATTTGA GGCTAGCTACAACGA TGGGCGTG | 1929 |
| 972 | CCAGUCAA A UUACUUAG | 228 | CTAAGTAA GGCTAGCTACAACGA TTGACTGG | 1930 |
| 975 | GUCAAAUU A CUUAGAGG | 229 | CCTCTAAG GGCTAGCTACAACGA AATTTGAC | 1931 |
| 983 | ACUUAGAG G CCAUACUC | 230 | GAGTATGG GGCTAGCTACAACGA CTCTAAGT | 1932 |
| 986 | UAGAGGCC A UACUCUUG | 231 | CAAGAGTA GGCTAGCTACAACGA GGCCTCTA | 1933 |
| 988 | GAGGCCAU A CUCUUGUC | 232 | GACAAGAG GGCTAGCTACAACGA ATGGCCTC | 1934 |
| 994 | AUACUCUU G UCCUCAAU | 233 | ATTGAGGA GGCTAGCTACAACGA AAGAGTAT | 1935 |
| 1001 | UGUCCUCA A UUGUACUG | 234 | CAGTACAA GGCTAGCTACAACGA TGAGGACA | 1936 |
| 1004 | CCUCAAUU G UACUGCUA | 235 | TAGCAGTA GGCTAGCTACAACGA AATTGAGG | 1937 |
| 1006 | UTCAAUUGU A CUGCUACC | 236 | GGTAGCAG GGCTAGCTACAACGA ACAATTGA | 1938 |
| 1009 | AUUGUACU G CUACCACU | 237 | AGTGGTAG GGCTAGCTACAACGA AGTACAAT | 1939 |
| 1012 | GUACUGCU A CCACUCCC | 238 | GGGAGTGG GGCTAGCTACAACGA AGCAGTAC | 1940 |
| 1015 | CUGCUACC A CUCCCUUG | 239 | CAAGGGAG GGCTAGCTACAACGA GGTAGCAG | 1941 |
| 1025 | UCCCUUGA A CACGAGAG | 240 | CTCTCGTG GGCTAGCTACAACGA TCAAGGGA | 1942 |
| 1027 | CCUUGAAC A CGAGAGUU | 241 | AACTCTCG GGCTAGCTACAACGA GTTCAAGG | 1943 |
| 1033 | ACACGAGA G UUCAAAUG | 242 | CATTTGAA GGCTAGCTACAACGA TCTCGTGT | 1944 |
| 1039 | GAGUUCAA A UGACCUGG | 243 | CCAGGTCA GGCTAGCTACAACGA TTGAACTC | 1945 |
| 1042 | UUCAAAUG A CCUGGAGU | 244 | ACTCCAGG GGCTAGCTACAACGA CATTTGAA | 1946 |
| 1049 | GACCUGGA G UUACCCUG | 245 | CAGGGTAA GGCTAGCTACAACGA TCCAGGTC | 1947 |
| 1052 | CUGGAGUU A CCCUGAUG | 246 | CATCAGGG GGCTAGCTACAACGA AACTCCAG | 1948 |
| 1058 | UUACCCUG A UGAAAAAA | 247 | TTTTTTCA GGCTAGCTACAACGA CAGGGTAA | 1949 |
| 1067 | UGAAAAAA A UAAGAGAG | 248 | CTCTCTTA GGCTAGCTACAACGA TTTTTTCA | 1950 |
| 1075 | AUAAGAGA G CUUCCGUA | 249 | TACGGAAG GGCTAGCTACAACGA TCTCTTAT | 1951 |
| 1081 | GAGCUUCC G UAAGGCGA | 250 | TCGCCTTA GGCTAGCTACAACGA GGAAGCTC | 1952 |
| 1086 | UCCGUAAG G CGACGAAU | 251 | ATTCGTCG GGCTAGCTACAACGA CTTACGGA | 1953 |
| 1089 | GUAAGGCG A CGAAUUGA | 252 | TCAATTCG GGCTAGCTACAACGA CGCCTTAC | 1954 |
| 1093 | GGCGACGA A UUGACCAA | 253 | TTGGTCAA GGCTAGCTACAACGA TCGTCGCC | 1955 |
| 1097 | ACGAAUUG A CCAAAGCA | 254 | TGCTTTGG GGCTAGCTACAACGA CAATTCGT | 1956 |
| 1103 | UGACCAAA G CAAUUCCC | 255 | GGGAATTG GGCTAGCTACAACGA TTTGGTCA | 1957 |
| 1106 | CCAAAGCA A UUCCCAUG | 256 | CATGGGAA GGCTAGCTACAACGA TGCTTTGG | 1958 |
| 1112 | CAAUUCCC A UGCCAACA | 257 | TGTTGGCA GGCTAGCTACAACGA GGGAATTG | 1959 |
| 1114 | AUUCCCAU G CCAACAUA | 258 | TATGTTGG GGCTAGCTACAACGA ATGGGAAT | 1960 |
| 1118 | CCAUGCCA A CAUAUUCU | 239 | AGAATATG GGCTAGCTACAACGA TGGCATGG | 1961 |
| 1120 | AUGCCAAC A UAUUCUAC | 260 | GTAGAATA GGCTAGCTACAACGA GTTGGCAT | 1962 |
| 1122 | GCCAACAU A UUCUAGAG | 261 | CTGTAGAA GGCTAGCTACAACGA ATGTTGGC | 1963 |
| 1127 | CAUAUUCU A CAGUGUUC | 262 | GAACACTG GGCTAGCTACAACGA AGAATATG | 1964 |
| 1130 | AUUCUACA G UGUUCUUA | 263 | TAAGAACA GGCTAGCTACAACGA TGTAGAAT | 1965 |
| 1132 | UCUACAGU G UUCUUACU | 264 | AGTAAGAA GGCTAGCTAGAACGA ACTGTAGA | 1966 |
| 1138 | GUGUUCUU A CUAUUGAC | 265 | GTCAATAG GGCTAGCTACAACGA AAGAACAC | 1967 |
| 1141 | UUCUUACU A UUGACAAA | 266 | TTTGTCAA GGCTAGCTACAACGA AGTAAGAA | 1968 |
| 1145 | UACUAUUG A CAAAAUGC | 26.7 | GCATTTTG GGCTAGCTACAACGA CAATAGTA | 1969 |
| 1150 | UUGACAAA A UGCAGAAC | 268 | GTTCTGGA GGCTAGCTACAACGA TTTGTCAA | 1970 |
| 1152 | GACAAAAU G CAGAACAA | 269 | TTGTTCTG GGCTAGCTACAACGA ATTTTGTC | 1971 |
| 1157 | AAUGCAGA A CAAAGACA | 270 | TGTCTTTG GGCTAGCTACAACGA TCTGCATT | 1972 |
| 1163 | GAACAAAG A CAAAGGAC | 271 | GTCCTTTG GGCTAGCTACAACGA CTTTGTTC | 1973 |
| 1170 | GACAAAGG A CUUUAUAC | 272 | GTATAAAG GGCTAGCTACAACGA CCTTTGTC | 1974 |
| 1175 | AGGACUUU A UACUUGUC | 273 | GACAAGTA GGCTAGCTACAACGA AAAGTCCT | 1975 |
| 1177 | GACUUUAU A CUUGUCGU | 274 | ACCACAAG GGCTAGCTACAACGA ATAAAGTC | 1976 |
| 1181 | UUAUACUU G UCGUGUAA | 275 | TTACACGA GGCTAGCTACAACGA AAGTATAA | 1977 |
| 1184 | UACUUGUC G UGUAAGGA | 276 | TCCTTACA GGCTAGCTACAACGA GACAAGTA | 1978 |
| 1186 | CUUGUCGU G UAAGGAGU | 277 | ACTCCTTA GGCTAGCTACAACGA ACGACAAG | 1979 |
| 1193 | UGUAAGGA G UGGACCAU | 278 | ATGGTCCA GGCTAGCTACAACGA TCCTTACA | 1980 |
| 1197 | AGGAGUGG A CCAUCAUU | 279 | AATGATGG GGCTAGCTACAACGA CCACTCCT | 1981 |
| 1200 | AGUGGACC A UCAUUCAA | 280 | TTGAATGA GGCTAGCTACAACGA GGTCCACT | 1982 |
| 1203 | GGACCAUC A UUCAAAUC | 281 | GATTTGAA GGCTAGCTACAACGA GATGGTCC | 1983 |
| 1209 | UCAUUCAA A UCUGUUAA | 282 | TTAACAGA GGCTAGCTACAACGA TTGAATGA | 1984 |
| 1213 | UCAAAUCU G UUAACACC | 283 | GGTGTTAA GGCTAGCTACAACGA AGATTTGA | 1985 |
| 1217 | AUCUGUUA A CACCUCAG | 284 | CTGAGGTG GGCTAGCTACAACGA TAACAGAT | 1986 |
| 1219 | CUGUUAAC A CCUCAGUG | 285 | CACTGAGG GGCTAGCTACAACGA GTTAACAG | 1987 |
| 1225 | ACACCUCA G UGCAUAUA | 286 | TATATGCA GGCTAGCTACAACGA TGAGGTGT | 1988 |
| 1227 | ACCUCAGU G CAUAUAUA | 287 | TATATATG GGCTAGCTACAACGA ACTGAGGT | 1989 |
| 1229 | CUCAGUGC A UAUAUAUG | 288 | CATATATA GGCTAGCTACAACGA GCACTGAG | 1990 |
| 1231 | CAGUGCAU A UAUAUGAU | 289 | ATCATATA GGCTAGCTACAACGA ATGCACTG | 1991 |
| 1233 | GUGCAUAU A UAUGAUAA | 290 | TTATCATA GGCTAGCTACAACGA ATATGCAC | 1992 |
| 1235 | GCAUAUAU A UGAUAAAG | 291 | CTTTATCA GGCTAGCTACAACGA ATATATGC | 1993 |
| 1238 | UAUAUAUG A UAAAGCAU | 292 | ATGCTTTA GGCTAGCTACAACGA CATATATA | 1994 |
| 1243 | AUGAUAAA G CALTUCAUC | 293 | GATGAATG GGCTAGCTACAACGA TTTATCAT | 1995 |
| 1245 | GAUAAAGC A UUCAUCAC | 294 | GTGATGAA GGCTAGCTACAACGA GCTTTATC | 1996 |
| 1249 | AAGCAUUC A UCACUGUG | 295 | CACAGTGA GGCTAGCTACAACGA GAATGCTT | 1997 |
| 1252 | CAUUCAUC A CUGUGAAA | 296 | TTTCACAG GGCTAGCTACAACGA GATGAATS | 1998 |
| 1255 | UCAUCACU G UGAAACAU | 297 | ATGTTTCA GGCTAGCTACAACGA AGTGATGA | 1999 |
| 1260 | ACUGUGAA A CAUCGAAA | 298 | TTTCGATG GGCTAGCTACAACGA TTCACAGT | 2000 |
| 1262 | UGUGAAAC A UCGAAAAC | 299 | GTTTTCGA GGCTAGCTACAACGA GTTTCACA | 2001 |
| 1269 | CAUCGAAA A CAGCAGGU | 300 | ACCTGCTG GGCTAGCTACAACGA TTTCGATG | 2002 |
| 1272 | CGAAAACA G CAGGUGCU | 301 | AGCACCTG GGCTAGCTACAACGA TGTTTTCG | 2003 |
| 1276 | AACAGCAG G UGCUUGAA | 302 | TTCAAGCA GGCTAGCTACAACGA CTGCTGTT | 2004 |
| 1278 | CAGCAGGU G CUUGAAAC | 303 | GTTTCAAG GGCTAGCTACAACGA ACCTGCTG | 2005 |
| 1285 | UGCUUGAA A CCGUAGCU | 304 | AGCTACGG GGCTAGCTACAACGA TTCAAGCA | 2006 |
| 1288 | UUGAAACC G UAGCUGGC | 305 | GCCAGCTA GGCTAGCTACAACGA GGTTTCAA | 2007 |
| 1291 | AAACCGUA G CUGGCAAG | 306 | CTTGCCAG GGCTAGCTACAACGA TACGGTTT | 2008 |
| 1295 | CGUAGCUG G CAAGCGGU | 307 | ACCGCTTG GGCTAGCTACAACGA CAGCTACG | 2009 |
| 1299 | GCUGGCAA G CGGUCUUA | 308 | TAAGACCG GGCTAGCTACAACGA TTGCCAGC | 2010 |
| 1302 | GGCAAGCG G UCUUACCG | 309 | CGGTAAGA GGCTAGCTACAACGA CGCTTGCC | 2011 |
| 1307 | GCGGUCUU A CCGGCUCU | 310 | AGAGCCGG GGCTAGCTACAACGA AAGACCGC | 2012 |
| 1311 | UCUUACCG G CUCUCUAU | 311 | ATAGAGAG GGCTAGCTACAACGA CGGTAAGA | 2013 |
| 1318 | GGCUCUCU A UGAAAGUG | 312 | CACTTTCA GGCTAGCTACAACGA AGAGAGCC | 2014 |
| 1324 | CUAUGAAA G UGAAGGCA | 313 | TGCCTTCA GGCTAGCTACAACGA TTTCATAG | 2015 |
| 1330 | AAGUGAAG G CAUUUCCC | 314 | GGGAAATG GGCTAGCTACAACGA CTTCACTT | 2016 |
| 1332 | GUGAAGGC A UUUCCCUC | 315 | GAGGGAAA GGCTAGCTACAACGA GCCTTCAC | 2017 |
| 1341 | UUUCCCUC G CCGGAAGU | 316 | ACTTCCGG GGCTAGCTACAACGA GAGGGAAA | 2018 |
| 1348 | CGCCGGAA G UUGUAUGG | 317 | CCATACAA GGCTAGCTACAACGA TTCCGGCG | 2019 |
| 1351 | CGGAAGUU G UAUGGUUA | 318 | TAACCATA GGCTAGCTACAACGA AACTTCCG | 2020 |
| 1353 | GAAGUUGU A UGGUUAAA | 319 | TTTAACCA GGCTAGCTACAACGA ACAACTTC | 2021 |
| 1356 | GUUGUAUG G UUAAAAGA | 320 | TCTTTTAA GGCTAGCTACAACGA CATACAAC | 2022 |
| 1364 | GUUAAAAG A UGGGUUAC | 321 | GTAACCCA GGCTAGCTACAACGA CTTTTAAC | 2023 |
| 1368 | AAAGAUGG G UUACCUGC | 322 | GCAGGTAA GGCTAGCTACAACGA CCATCTTT | 2024 |
| 1371 | GAUGGGUU A CCUGCGAC | 323 | GTCGCAGG GGCTAGCTACAACGA AACCCATC | 2025 |
| 1375 | GGUUACCU G CGACUGAG | 324 | CTCAGTCG GGCTAGCTACAACGA AGGTAACC | 2026 |
| 1378 | UACCUGCG A CUGAGAAA | 325 | TTTCTCAG GGCTAGCTACAACGA CGCAGGTA | 2027 |
| 1386 | ACUGAGAA A UCUGCUCG | 326 | CGAGCAGA GGCTAGCTACAACGA TTCTCAGT | 2028 |
| 1390 | AGAAAUCU G CUCGCUAU | 327 | ATAGCGAG GGCTAGCTACAACGA AGATTTCT | 2029 |
| 1394 | AUCUGCUC G CUAUUUGA | 328 | TCAAATAG GGCTAGCTACAACGA GAGCAGAT | 2030 |
| 1397 | UGCUCGCU A UUUGACUC | 329 | GAGTCAAA GGCTAGCTACAACGA AGCGAGCA | 2031 |
| 1402 | GCUAUUUG A CUCGUGGC | 330 | GCCACGAG GGCTAGCTACAACGA CAAATAGC | 2032 |
| 1406 | UUUGACUC G UGGCUACU | 331 | AGTAGCCA GGCTAGCTACAACGA GAGTCAAA | 2033 |
| 1409 | GACUCGUG G CUACUCGU | 332 | ACGAGTAG GGCTAGCTACAACGA CACGAGTC | 2034 |
| 1412 | UCGUGGCU A CUCGUUAA | 333 | TTAACGAG GGCTAGCTACAACGA AGCCACGA | 2035 |
| 1416 | GGCUACUC G UUAAUUAU | 334 | ATAATTAA GGCTAGCTACAACGA GAGTAGCC | 2036 |
| 1420 | ACUCGUUA A UUAUCAAG | 335 | CTTGATAA GGCTAGCTACAACGA TAACGAGT | 2037 |
| 1423 | CGUUAAUU A UCAAGGAC | 336 | GTCCTTGA GGCTAGCTACAACGA AATTAACG | 2038 |
| 1430 | UAUCAAGG A CGUAACUG | 337 | CAGTTACG GGCTAGCTACAACGA CCTTGATA | 2039 |
| 1432 | UCAAGGAC G UAACUGAA | 338 | TTCAGTTA GGCTAGCTACAACGA GTCCTTGA | 2040 |
| 1435 | AGGACGUA A CUGAAGAG | 339 | CTCTTCAG GGCTAGCTACAACGA TACGTCCT | 2041 |
| 1445 | UGAAGAGG A UGGAGGGA | 340 | TCCCTGCA GGCTAGCTACAACGA CCTCTTCA | 2042 |
| 1447 | AAGAGGAU G CAGGGAAU | 341 | ATTCCCTG GGCTAGCTACAACGA ATCCTCTT | 2043 |
| 1454 | UGCAGGGA A UUAUACAA | 342 | TTGTATAA GGCTAGCTACAACGA TCCCTGCA | 2044 |
| 1457 | AGGGAALTU A UACAAUCU | 343 | AGATTGTA GGCTAGCTACAACGA AATTCCCT | 2045 |
| 1459 | GGAAUUAU A CAAUCUUG | 344 | CAAGATTG GGCTAGCTACAACGA ATAATTCC | 2046 |
| 1462 | AUUAUACA A UCUUGCUG | 345 | CAGGAAGA GGCTAGCTACAACGA TGTATAAT | 2047 |
| 1467 | ACAAUCUU G CUGAGCAU | 346 | ATGCTCAG GGCTAGCTACAACGA AAGATTGT | 2048 |
| 1472 | CUUGCUGA G CAUAAAAC | 347 | GTTTTATG GGCTAGCTACAACGA TCAGCAAG | 2049 |
| 1474 | UGCUGAGC A UAAAACAG | 348 | CTGTTTTA GGCTAGCTACAACGA GCTCAGCA | 2050 |
| 1479 | AGCAUAAA A CAGUCAAA | 349 | TTTGACTG GGCTAGCTACAACGA TTTATGCT | 2051 |
| 1482 | AUAAAACA G UCAAAUGU | 350 | ACATTTGA GGCTAGCTACAACGA TGTTTTAT | 2052 |
| 1487 | ACAGUCAA A UGUGUUUA | 351 | TAAACACA GGCTAGCTACAACGA TTGACTGT | 2053 |
| 1489 | AGUCAAAU G UGUUUAAA | 352 | TTTAAACA GGCTAGCTACAACGA ATTTGACT | 2054 |
| 1491 | UCAAAUGU G UUUAAAAA | 353 | TTTTTAAA GGCTAGCTACAACGA ACATTTGA | 2055 |
| 1499 | GUUUAAAA A CCUCACUG | 354 | CAGTGAGG GGCTAGCTACAACGA TTTTAAAC | 2056 |
| 1504 | AAAACCUC A CUGCCACU | 355 | AGTGGCAG GGCTAGCTACAACGA GAGGTTTT | 2057 |
| 1507 | ACCUCACU G CCACUCUA | 356 | TAGAGTGG GGCTAGCTACAACGA AGTGAGGT | 2058 |
| 1510 | UCACUGCC A CUCUAAUU | 357 | AATTAGAG GGCTAGCTACAACGA GGCAGTGA | 2059 |
| 1516 | CCACUCUA A UUGUCAAU | 358 | ATTGACAA GGCTAGCTACAACGA TAGAGTGG | 2060 |
| 1519 | CUCUAAUU G UCAAUGUG | 359 | CACATTGA GGCTAGCTACAACGA AATTAGAG | 2061 |
| 1523 | AAUUGUCA A UGUGAAAC | 360 | GTTTCACA GGCTAGCTACAACGA TGACAATT | 2062 |
| 1525 | UUGUCAAU G UGAAACCC | 361 | GGGTTTCA GGCTAGCTACAACGA ATTGACAA | 2063 |
| 1530 | AAUGUGAA A CCCCAGAU | 362 | ATCTGGGG GGCTAGCTACAACGA TTCACATT | 2064 |
| 1537 | AACCCCAG A UUUACGAA | 363 | TTCGTAAA GGCTAGCTACAACGA CTGGGGTT | 2065 |
| 1541 | CCAGAUUU A CGAAAAGG | 364 | CCTTTTCG GGCTAGCTACAACGA AAATCTGG | 2066 |
| 1549 | ACGAAAAG G CCGUGUCA | 365 | TGACACGG GGCTAGCTACAACGA CTTTTCGT | 2067 |
| 1552 | AAAAGGCC G UGUCAUCG | 366 | CGATGACA GGCTAGCTACAACGA GGCCTTTT | 2068 |
| 1554 | AAGGCCGU G UCAUCGUU | 367 | AACGATGA GGCTAGCTACAACGA ACGGCCTT | 2069 |
| 1557 | GCCGUGUC A UCGUUUCC | 368 | GGAAACGA GGCTAGCTACAACGA GACACGGC | 2070 |
| 1560 | GUGUCAUC G UUUCCAGA | 369 | TCTGGAAA GGCTAGCTACAACGA GATGACAC | 2071 |
| 1568 | GUUUCCAG Ar CCCGGCUC | 370 | GAGCCGGG GGCTAGCTACAACGA CTGGAAAC | 2072 |
| 1573 | CAGACCCG G CUCUCUAC | 371 | GTAGAGAG GGCTAGCTACAACGA CGGGTCTG | 2073 |
| 1580 | GGCUCUCU A CCCACUGG | 372 | CCAGTGGG GGCTAGCTACAACGA AGAGAGCC | 2074 |
| 1584 | CUCUACCC A CUGGGCAG | 373 | CTGCCCAG GGCTAGCTACAACGA GGGTAGAG | 2075 |
| 1589 | CCCACUGG G CAGCAGAC | 374 | GTCTGCTG GGCTAGCTACAACGA CCAGTGGG | 2076 |
| 1592 | ACUGGGCA G CAGACAAA | 375 | TTTGTCTG GGCTAGCTACAACGA TGCCCAGT | 2077 |
| 1596 | GGCAGCAG A CAAAUCCU | 376 | AGGATTTG GGCTAGCTACAACGA CTGCTGCC | 2078 |
| 1600 | GCAGACAA A UCCUGACU | 377 | AGTCAGGA GGCTAGCTACAACGA TTGTCTGC | 2079 |
| 1606 | AAAUCCUG A CWGUACC | 378 | GGTACAAG GGCTAGCTACAACGA CAGGATTT | 2080 |
| 1610 | CCUGACUU G UACCGCAU | 379 | ATGCGGTA GGCTAGCTACAACGA AAGTCAGG | 2081 |
| 1612 | UGACUUGU A CCGCAUAU | 380 | ATATGCGG GGCTAGCTACAACGA ACAAGTCA | 2082 |
| 1615 | CUUGUACC G CAUAUGGU | 381 | ACCATATG GGCTAGCTACAACGA GGTACAAG | 2083 |
| 1617 | UGUACCGC A UAUGGUAU | 382 | ATACCATA GGCTAGCTACAACGA GCGGTACA | 2084 |
| 1619 | UACCGCAU A UGGUAUCC | 383 | GGATACCA GGCTAGCTACAACGA ATGCGGTA | 2085 |
| 1622 | CGCAUAUG G UAUCCCUC | 384 | GAGGGATA GGCTAGCTACAACGA CATATGCG | 2086 |
| 1624 | CAUAUGGU A UCCCUCAA | 385 | TTGAGGGA GGCTAGCTACAACGA ACCATATG | 2087 |
| 1632 | AUCCCUCA A CCUACAAU | 386 | ATTGTAGG GGCTAGCTACAACGA TGAGGGAT | 2088 |
| 1636 | CUCAACCU A CAAUCAAG | 387 | CTTGATTG GGCTAGCTACAACGA AGGTTGAG | 2089 |
| 1639 | AACCUACA A UCAAGUGG | 388 | CCACTTGA GGCTAGCTACAACGA TGTAGGTT | 2090 |
| 1644 | ACAAUCAA G UGGUUCUG | 389 | CAGAACCA GGCTAGCTACAACGA TTGATTGT | 2091 |
| 1647 | AUCAAGUG G UUCUGGCA | 390 | TGCCAGAA GGCTAGCTACAACGA CACTTGAT | 2092 |
| 1653 | UGGUUCUG G CACCCCUG | 391 | GAGGGGTG GGCTAGCTACAACGA CAGAACCA | 2093 |
| 1655 | GUUCUGGC A CCCCUGUA | 392 | TACAGGGG GGCTAGCTACAACGA GCCAGAAC | 2094 |
| 1661 | GCACCCCU G UAACCAUA | 393 | TATCCTTA GGCTAGCTACAACGA AGGGGTGC | 2095 |
| 1664 | CCCCUGUA A CCAUAAUC | 394 | GATTATGG GGCTAGCTACAACGA TACAGGGG | 2096 |
| 1667 | CUGUAACC A UAAUCAUU | 395 | AATGATTA GGCTAGCTACAACGA GGTTACAG | 2097 |
| 1670 | UAACCAUA A UCAUUCCG | 396 | CGGAATGA GGCTAGCTACAACGA TATGGTTA | 2098 |
| 1673 | CCAUAAUC A UUCCGAAG | 397 | CTTCGGAA GGCTAGCTACAACGA GATTATGG | 2099 |
| 1681 | AUUCCGAA G CAAGGUGU | 398 | ACACCTTG GGCTAGCTACAACGA TTCGGAAT | 2100 |
| 1686 | GAAGCAAG G UGUGACUU | 399 | AAGTCACA GGCTAGCTACAACGA CTTGCTTC | 2101 |
| 1688 | AGCAAGGU G UGACUUUU | 400 | AAAAGTCA GGCTAGCTACAACGA ACCTTGCT | 2102 |
| 1691 | AAGGUGUG A CUUUUGUU | 401 | AACAAAAG GGCTAGCTACAACGA CACACCTT | 2103 |
| 1697 | UGACUUUU G UUCCAAUA | 402 | TATTGGAA GGCTAGCTACAACGA AAAAGTCA | 2104 |
| 1703 | UUGUUCCA A UAAUGAAG | 403 | CTTCATTA GGCTAGCTACAACGA TGGAACAA | 2105 |
| 1706 | UUCCAAUA A UGAAGAGU | 404 | ACTCTTCA GGCTAGCTACAACGA TATTGGAA | 2106 |
| 1713 | AAUGAAGA G UCCUUUAU | 405 | ATAAAGGA GGCTAGCTACAACGA TCTTCATT | 2107 |
| 1720 | AGUCCUUU A UCCUGGAU | 406 | ATCCAGGA GGCTAGCTACAACGA AAAGGACT | 2108 |
| 1727 | UAUCCUGG A UGCUGACA | 407 | TGTCAGCA GGCTAGCTACAACGA CCAGGATA | 2109 |
| 1729 | UCCUGGAU G CUGACAGC | 408 | GCTGTCAG GGCTAGCTACAACGA ATCCAGGA | 2110 |
| 1733 | GGAUGCUG A CAGCAACA | 409 | TGTTGCTG GGCTAGCTACAACGA CAGCATCC | 2111 |
| 1736 | UGCUGACA G CAACAUGG | 410 | CCATGTTG GGCTAGCTACAACGA TGTCAGCA | 2112 |
| 1739 | UGACAGCA A CAUGGGAA | 411 | TTCCCATG GGCTAGCTACAACGA TGCTGTCA | 2113 |
| 1741 | ACAGCAAC A UGGGAAAC | 412 | GTTTCCCA GGCTAGCTACAACGA GTTGCTGT | 2114 |
| 1748 | CAUGGGAA A CAGAAUUG | 413 | CAATTCTG GGCTAGCTACAACGA TTCCCATG | 2115 |
| 1753 | GAAACAGA A UUGAGAGC | 414 | GCTCTCAA GGCTAGCTACAACGA TCTGTTTC | 2116 |
| 1760 | AAUUGAGA G CAUCACUC | 415 | GAGTGATG GGCTAGCTACAACGA TCTCAATT | 2117 |
| 1762 | UUGAGAGC A UCACUCAG | 416 | CTGAGTGA GGCTAGCTACAACGA GCTCTCAA | 2118 |
| 1765 | AGAGCAUC A CUCAGCGC | 417 | GCGCTGAG GGCTAGCTACAACGA GATGCTCT | 2119 |
| 1770 | AUCACUCA G CGCAUGGC | 418 | GCCATGCG GGCTAGCTACAACGA TGAGTGAT | 2120 |
| 1772 | CACUCAGC G CAUGGCAA | 419 | TTGCCATG GGCTAGCTACAACGA GCTGAGTG | 2121 |
| 1774 | CUCAGCGC A UGGCAAUA | 420 | TATTGCCA GGCTAGCTACAACGA GCGCTGAG | 2122 |
| 1777 | AGCGCAUG G CAAUAAUA | 421 | TATTATTG GGCTAGCTACAACGA CATGCGCT | 2123 |
| 1780 | GCAUGGCA A UAAUAGAA | 422 | TTCTATTA GGCTAGCTACAACGA TGCCATGC | 2124 |
| 1783 | UGGCAAUA A UAGAAGGA | 423 | TCCTTCTA GGCTAGCTACAACGA TATTGCCA | 2125 |
| 1796 | AGGAAAGA A UAAGAUGG | 424 | CCATCTTA GGCTAGCTACAACGA TCTTTCCT | 2126 |
| 1801 | AGAAUAAG A UGGCUAGC | 425 | GCTAGCCA GGCTAGCTACAACGA CTTATTCT | 2127 |
| 1804 | AUAAGAUG G CUAGCACC | 426 | GGTGCTAG GGCTAGCTACAACGA CATCTTAT | 2128 |
| 1808 | GAUGGCUA G CACCUUGG | 427 | CCAAGGTG GGCTAGCTACAACGA TAGCCATC | 2129 |
| 1810 | UGGCUAGC A CCUUGGUU | 428 | AACCAAGG GGCTAGCTACAACGA GCTAGCCA | 2130 |
| 1816 | GCACCUUG G UUGUGGCU | 429 | AGCCACAA GGCTAGCTACAACGA CAAGGTGC | 2131 |
| 1819 | CCUUGGUU G UGGCUGAC | 430 | GTCAGCCA GGCTAGCTACAACGA AACCAAGG | 2132 |
| 1822 | UGGUUGUG G CUGACUCU | 431 | AGAGTCAG GGCTAGCTACAACGA CACAACCA | 2133 |
| 1826 | UGUGGCUG A CUCUAGAA | 432 | TTCTAGAG GGCTAGCTACAACGA CAGCCACA | 2134 |
| 1834 | ACUCUAGA A UUUCUGGA | 433 | TCCAGAAA GGCTAGCTACAACGA TCTAGAGT | 2135 |
| 1843 | UUUCUGGA A UCUACAUU | 434 | AATGTAGA GGCTAGCTACAACGA TCCAGAAA | 2136 |
| 1847 | UGGAAUCU A CAUUUGCA | 435 | TGCAAATG GGCTAGCTACAACGA AGATTCCA | 2137 |
| 1849 | GAAUGUAC A UUUGCAUA | 436 | TATGCAAA GGCTAGCTACAACGA GTAGATTC | 2138 |
| 1853 | CUACAUUU G CAUAGCUU | 437 | AAGCTATG GGCTAGCTACAACGA AAATGTAG | 2139 |
| 1855 | ACAUUUGC A UAGCUUCC | 438 | GGAAGCTA GGCTAGCTACAACGA GCAAATGT | 2140 |
| 1858 | UUUGCAUA G CUUCCAAU | 439 | ATTGGAAG GGCTAGCTACAACGA TATGCAAA | 2141 |
| 1865 | AGCUUCCA A UAAAGUUG | 440 | CAACTTTA GGCTAGCTACAACGA TGGAAGCT | 2142 |
| 1870 | CCAAUAAA G UUGGGACU | 441 | AGTCCCAA GGCTAGCTACAACGA TTTATTGG | 2143 |
| 1876 | AAGUUGGG A CUGUGGGA | 442 | TCCCACAG GGCTAGCTACAACGA CCCAACTT | 2144 |
| 1879 | UUGGGACU G UGGGAAGA | 443 | TCTTCCCA GGCTAGCTACAACGA AGTCCCAA | 2145 |
| 1889 | GGGAAGAA A CAUAAGCU | 444 | AGCTTATG GGCTAGCTACAACGA TTCTTCCC | 2146 |
| 1891 | GAAGAAAC A UAAGCUUU | 445 | AAAGCTTA GGCTAGCTACAACGA GTTTCTTC | 2147 |
| 1895 | AAACAUAA G CUUUUAUA | 446 | TATAAAAG GGCTAGCTACAACGA TTATGTTT | 2148 |
| 1901 | AAGCUUUU A UAUCACAG | 447 | CTGTGATA GGCTAGCTACAACGA AAAAGCTT | 2149 |
| 1903 | GCUUUUAU A UCACAGAU | 448 | ATCTGTGA GGCTAGCTACAACGA ATAAAAGC | 2150 |
| 1906 | UUUAUAUC A CAGAUGUG | 449 | CACATCTG GGCTAGCTACAACGA GATATAAA | 2151 |
| 1910 | UAUCACAG A UGUGCCAA | 450 | TTGGGCACA GGCTAGCTACAACGA CTGTGATA | 2152 |
| 1912 | UCACAGAU G UGCCAAAU | 451 | ATTTGGCA GGCTAGCTACAACGA ATCTGTGA | 2153 |
| 1914 | ACAGAUGU G CCAAAUGG | 452 | CCATTTGG GGCTAGCTACAAEGA ACATCTGT | 2154 |
| 1919 | UGUGCCAA A UGGGUUUC | 453 | GAAACCCA GGCTAGCTACAACGA TTGGCACA | 2155 |
| 1923 | CCAAAUGG G UUUCAUGU | 454 | ACATGAAA GGCTAGCTACAACGA CCATTTGG | 2156 |
| 1928 | UGGGUUUC A UGUUAACU | 455 | AGTTAACA GGCTAGCTACAACGA GAAACCCA | 2157 |
| 1930 | GGUUUCAU G UUAACUUG | 456 | CAAGTTAA GGCTAGCTACAACGA ATGAAACC | 2158 |
| 1934 | UCAUGUUA A CUUGGAAA | 457 | TTTCCAAG GGCTAGCTACAACGA TAACATGA | 2159 |
| 1945 | UGGAAAAA A UGCCGACG | 458 | CGTCGGCA GGCTAGCTACAACGA TTTTTCCA | 2160 |
| 1947 | GAAAAAAU G CCGACGGA | 459 | TCCGTCGG GGCTAGCTACAACGA ATTTTTTC | 2161 |
| 1951 | AAAUGCCG A CGGAAGGA | 460 | TCCTTCCG GGCTAGCTACAACGA CGGCATTT | 2162 |
| 1964 | AGGAGAGG A CCUGAAAC | 461 | GTTTCAGG GGCTAGCTACAACGA CCTCTCCT | 2163 |
| 1971 | GACCUGAA A CUGUCUUG | 462 | CAAGACAG GGCTAGCTACAACGA TTCAGGTC | 2164 |
| 1974 | CUGAAACU G UCUUGCAC | 463 | GTGCAAGA GGCTAGCTACAACGA AGTTTCAG | 2165 |
| 1979 | ACUGUCUU G CACAGUUA | 464 | TAACTGTG GGCTAGCTACAACGA AAGACAGT | 2166 |
| 1981 | UGUCUUGC A CAGUUAAC | 465 | GTTAACTG GGCTAGCTACAACGA GCAAGACA | 2167 |
| 1984 | CUUGCACA G UUAACAAG | 466 | CTTGTTAA GGCTAGCTACAACGA TGTGCAAG | 2168 |
| 1988 | CACAGUUA A CAAGUUCU | 467 | AGAACTTG GGCTAGCTACAACGA TAACTGTG | 2169 |
| 1992 | GUUAACAA G UUCUUAUA | 468 | TATAAGAA GGCTAGCTACAACGA TTGTTAAC | 2170 |
| 1998 | AAGUUCUU A UACAGAGA | 469 | TCTCTGTA GGCTAGCTACAACGA AAGAACTT | 2171 |
| 2000 | GUUCUUAU A CAGAGACG | 470 | CGTCTCTG GGCTAGCTACAACGA ATAAGAAC | 2172 |
| 2006 | AUACAGAG A CGUUACUU | 471 | AAGTAACG GGCTAGCTACAACGA CTCTGTAT | 2173 |
| 2008 | ACAGAGAC G UUACUUGG | 472 | CCAAGTAA GGCTAGCTACAACGA GTCTCTGT | 2174 |
| 2011 | GAGACGUU A CUUGGAUU | 473 | AATCCAAG GGCTAGCTACAACGA AACGTCTC | 2175 |
| 2017 | UUACUUGG A UUUUACUG | 474 | CAGTAAAA GGCTAGCTACAACGA CCAAGTAA | 2176 |
| 2022 | UGGAUUUU A CUGCGGAC | 475 | GTCCGCAG GGCTAGCTACAACGA AAAATCCA | 2177 |
| 2025 | AUUUUACU G CGGACAGU | 476 | ACTGTCCG GGCTAGCTACAACGA AGTAAAAT | 2178 |
| 2029 | UACUGCGG A CAGUUAAU | 477 | ATTAACTG GGCTAGCTACAACGA CCGCAGTA | 2179 |
| 2032 | UGCGGACA G UUAAUAAC | 478 | GTTATTAA GGCTAGCTACAACGA TGTCCGCA | 2180 |
| 2035 | GACAGUUA A UAACAGAA | 479 | TTCTGTTA GGCTAGCTACAACGA TAACTGTC | 2181 |
| 2039 | AGUUAAUA A CAGAACAA | 480 | TTGTTCTG GGCTAGCTACAACGA TATTAACT | 2182 |
| 2044 | AUAACAGA A CAAUGCAC | 481 | GTGCATTG GGCTAGCTACAACGA TCTGTTAT | 2183 |
| 2047 | ACAGAACA A UGCACUAC | 482 | GTAGTGCA GGCTAGCTACAACGA TGTTCTGT | 2184 |
| 2049 | AGAACAAU G CACUACAG | 483 | CTGTAGTG GGCTAGCTACAACGA ATTGTTCT | 2185 |
| 2051 | AACAAUGC A CUACAGUA | 484 | TACTGTAG GGCTAGCTACAACGA GCATTGTT | 2186 |
| 2054 | AAUGCACU A CAGUAUUA | 485 | TAATACTG GGCTAGCTACAACGA AGTGCATT | 2187 |
| 2057 | GCACUACA G UAUUAGCA | 486 | TGCTAATA GGCTAGCTACAACGA TGTAGTGC | 2188 |
| 2059 | ACUACAGU A UUAGCAAG | 487 | CTTGCTAA GGCTAGCTACAACGA ACTGTAGT | 2189 |
| 2063 | CAGUAUUA G CAAGCAAA | 488 | TTTGCTTG GGCTAGCTACAACGA TAATACTG | 2190 |
| 2067 | AUUAGCAA G CAAAAAAU | 489 | ATTTTTTG GGCTAGCTACAACGA TTGCTAAT | 2191 |
| 2074 | AGCAAAAA A UGGCCAUC | 490 | GATGGCCA GGCTAGCTACAACGA TTTTTGCT | 2192 |
| 2077 | AAAAAAUG G CCAUCACU | 491 | AGTGATGG GGCTAGCTACAACGA CATTTTTT | 2193 |
| 2080 | AAAUGGCC A UCACUAAG | 492 | CTTAGTGA GGCTAGCTACAACGA GGCCATTT | 2194 |
| 2083 | UGGCCAUC A CUAAGGAG | 493 | CTCCTTAG GGCTAGCTACAACGA GATGGCCA | 2195 |
| 2091 | ACUAAGGA G CACUCCAU | 494 | ATGGAGTG GGCTAGCTACAACGA TCCTTAGT | 2196 |
| 2093 | UAAGGAGC A CUCCAUCA | 495 | TGATGGAG GGCTAGCTACAACGA GCTCCTTA | 2197 |
| 2098 | AGCACUCC A UCACUCUU | 496 | AAGAGTGA GGCTAGCTACAACGA GGAGTGCT | 2198 |
| 2101 | ACUCCAUC A CUCUUAAU | 497 | ATTAAGAG GGCTAGCTACAACGA GATGGAGT | 2199 |
| 2108 | CACUCUUA A UCUUACCA | 498 | TGGTAAGA GGCTAGCTACAACGA TAAGAGTG | 2200 |
| 2113 | UUAAUCUU A CCAUCAUG | 499 | CATGATGG GGCTAGCTACAACGA AAGATTAA | 2201 |
| 2116 | AUCUUACC A UCAUGAAU | 500 | ATTCATGA GGCTAGCTACAACGA GGTAAGAT | 2202 |
| 2119 | UUACCAUC A UGAAUGUU | 501 | AACATTCA GGCTAGCTACAACGA CATGGTAA | 2203 |
| 2123 | CAUCAUGA A UGUUUCCC | 502 | GGGAAACA GGCTAGCTACAACGA TCATGATG | 2204 |
| 2125 | UCAUGAAU G UUUCCCUG | 503 | CAGGGAAA GGCTAGCTACAACGA ATTCATGA | 2205 |
| 2133 | GUUUCCCU G CAAGAUUC | 504 | GAATCTTG GGCTAGCTACAACGA AGGGAAAC | 2206 |
| 2138 | CCUGCAAG A UUCAGGCA | 505 | TGCCTGAA GGCTAGCTACAACGA CTTGCAGG | 2207 |
| 2144 | AGAUUCAG G CACCUAUG | 506 | CATAGGTG GGCTAGCTACAACGA CTGAATCT | 2208 |
| 2146 | AUUCAGGC A CCUAUGCC | 507 | GGCATAGG GGCTAGCTACAACGA GCCTGAAT | 2209 |
| 2150 | AGGCACCU A UGCCUGCA | 508 | TGCAGGCA GGCTAGCTACAACGA AGGTGCCT | 2210 |
| 2152 | GCACCUAU G CCUGCAGA | 509 | TCTGCAGG GGCTAGCTACAACGA ATAGGTGC | 2211 |
| 2156 | CUAUGCCU G CAGAGCCA | 510 | TGGCTCTG GGCTAGCTACAACGA AGGCATAG | 2212 |
| 2161 | CCUGCAGA G CCAGGAAU | 511 | ATTCCTGG GGCTAGCTACAACGA TCTGCAGG | 2213 |
| 2168 | AGCCAGGA A UGUAUACA | 512 | TGTATACA GGCTAGCTACAACGA TCCTGGCT | 2214 |
| 2170 | CCAGGAAU G UAUACACA | 513 | TGTGTATA GGCTAGCTACAACGA ATTCCTGG | 2215 |
| 2172 | AGGAAUGU A UACACAGG | 514 | CCTGTGTA GGCTAGCTACAACGA ACATTCCT | 2216 |
| 2174 | GAAUGUAU A CACAGGGG | 515 | CCCCTGTG GGCTAGCTACAACGA ATACATTC | 2217 |
| 2176 | AUGUAUAC A CAGGGGAA | 516 | TTCCCCTG GGCTAGCTACAACGA GTATACAT | 2218 |
| 2188 | GGGAAGAA A UCCUCCAG | 517 | CTGGAGGA GGCTAGCTACAACGA TTCTTCCC | 2219 |
| 2206 | AGAAAGAA A UUACAAUC | 518 | GATTGTAA GGCTAGCTACAACGA TTCTTTCT | 2220 |
| 2209 | AAGAAAUU A CAAUCAGA | 519 | TCTGATTG GGCTAGCTACAACGA AATTTCTT | 2221 |
| 2212 | AAAUUACA A UCAGAGAU | 520 | ATCTCTGA GGCTAGCTACAACGA TGTAATTT | 2222 |
| 2219 | AAUCAGAG A UCAGGAAG | 521 | CTTCCTGA GGCTAGCTACAACGA CTCTGATT | 2223 |
| 2227 | AUCAGGAA G CACCAUAC | 522 | GTATGGTG GGCTAGCTACAACGA TTCCTGAT | 2224 |
| 2229 | CAGGAAGC A CCAUACCU | 523 | AGGTATGG GGCTAGCTACAACGA GCTTCCTG | 2225 |
| 2232 | GAAGCACC A UACCUCCU | 524 | AGGAGGTA GGCTAGCTACAACGA GGTGCTTC | 2226 |
| 2234 | AGCACCAU A CCUCCUGC | 525 | GCAGGAGG GGCTAGCTACAACGA ATGGTGCT | 2227 |
| 2241 | UACCUCCU G CGAAACCU | 526 | AGGTTTCG GGCTAGCTACAACGA AGGAGGTA | 2228 |
| 2246 | CCUGCGAA A CCUCAGUG | 527 | CACTGAGG GGCTAGCTACAACGA TTCGCAGG | 2229 |
| 2252 | AAACCUCA G UGAUCACA | 528 | TGTGATCA GGCTAGCTACAACGA TGAGGTTT | 2230 |
| 2255 | CCUCAGUG A UCACACAG | 529 | CTGTGTGA GGCTAGCTACAACGA CACTGAGG | 2231 |
| 2258 | CAGUGAUC A CACAGUGG | 530 | CCACTGTG GGCTAGCTACAACGA GATCACTG | 2232 |
| 2260 | GUGAUCAC A CAGUGGCC | 531 | GGCCACTG GGCTAGCTACAACGA GTGATCAC | 2233 |
| 2263 | AUCACACA G UGGCCAUC | 532 | GATGGCCA GGCTAGCTACAACGA TGTGTGAT | 2234 |
| 2266 | ACACAGUG G CCAUCAGC | 533 | GCTGATGG GGCTAGCTACAACGA CACTGTGT | 2235 |
| 2269 | CAGUGGCC A UCAGCAGU | 534 | ACTGCTGA GGCTAGCTACAACGA GGCCACTG | 2236 |
| 2273 | GGCCAUCA G CAGUUCCA | 535 | TGGAACTG GGCTAGCTACAACGA TGATGGCC | 2237 |
| 2276 | CAUCAGCA G UUCCACCA | 536 | TGGTGGAA GGCTAGCTACAACGA TGCTGATG | 2238 |
| 2281 | GCAGUUCC A CCACUUUA | 537 | TAAAGTGG GGCTAGCTACAACGA GGAACTGC | 2239 |
| 2284 | GWCCACC A CUULTAGAC | 538 | GTCTAAAG GGCTA6CTACAACGA GGTGGAAC | 2240 |
| 2291 | CACUUUAG A CUGUCAUG | 539 | CATGACAG GGCTAGCTACAACGA CTAAAGTG | 2241 |
| 2294 | UUUAGACU G UCAUGCUA | 540 | TAGCATGA GGCTAGCTACAACGA AGTCTAAA | 2242 |
| 2297 | AGACUGUC A UGCUAAUG | 541 | CATTAGCA GGCTAGCTACAACGA GACAGTCT | 2243 |
| 2299 | ACUGUCAU G CUAAUGGU | 542 | ACCATTAG GGCTAGCTACAACGA ATGACAGT | 2244 |
| 2303 | UCAUGCUA A UGGUGUCC | 543 | GGACACCA GGCTAGCTACAACGA TAGCATGA | 2245 |
| 2306 | UGCUAAUG G UGUCCCCG | 544 | CGGGGACA GGCTAGCTACAACGA CATTAGCA | 2246 |
| 2308 | CUAAUGGU G UCCCCGAG | 545 | CTCGGGGA GGCTAGCTACAACGA ACCATTAG | 2247 |
| 2316 | GUCCCCGA G CCUCAGAU | 546 | ATCTGAGG GGCTAGCTACAACGA TCGGGGAC | 2248 |
| 2323 | AGCCUCAG A UCACUUGG | 547 | CCAAGTGA GGCTAGCTACAACGA CTGAGGCT | 2249 |
| 2326 | CUCAGAUC A CUUGGUUU | 548 | AAACCAAG GGCTAGCTACAACGA GATCTGAG | 2250 |
| 2331 | AUCACUUG G UUUAAAAA | 549 | TTTTTAAA GGCTAGCTACAACGA CAAGTGAT | 2251 |
| 2339 | GUUUAAAA A CAACCACA | 550 | TGTGGTTG GGCTAGCTACAACGA TTTTAAAC | 2252 |
| 2342 | UAAAAACA A CCACAAAA | 551 | TTTTGTGG GGCTAGCTACAACGA TGTTTTTA | 2253 |
| 2345 | AAACAACC A CAAAAUAC | 552 | GTATTTTG GGCTAGCTACAACGA GGTTGTTT | 2254 |
| 2350 | ACCACAAA A UACAACAA | 553 | TTGTTGTA GGCTAGCTACAACGA TTTGTGGT | 2255 |
| 2352 | CACAAAAU A CAACAAGA | 554 | TCTTGTTG GGCTAGCTACAACGA ATTTTGTG | 2256 |
| 2355 | AAAAUACA A CAAGAGCC | 555 | GGCTCTTG GGCTAGCTACAACGA TGTATTTT | 2257 |
| 2361 | CAACAAGA G CCUGGAAU | 556 | ATTCCAGG GGCTAGCTACAACGA TCTTGTTG | 2258 |
| 2368 | AGCCUGGA A UUAUUUUA | 557 | TAAAATAA GGCTAGCTACAACGA TCCAGGCT | 2259 |
| 2371 | CUGGAAUU A UUUUAGGA | 558 | TCCTAAAA GGCTAGCTACAACGA AATTCCAG | 2260 |
| 2379 | AUUUUAGG A CCAGGAAG | 559 | CTTCCTGG GGCTAGCTACAACGA CCTAAAAT | 2261 |
| 2387 | ACCAGGAA G CAGCACGC | 560 | GCGTGCTG GGCTAGCTACAACGA TTCCTGGT | 2262 |
| 2390 | AGGAAGCA G CACGCUGU | 561 | ACAGCGTG GGCTAGCTACAACGA TGCTTCCT | 2263 |
| 2392 | GAAGCAGC A CGCUGUUU | 562 | AAACAGCG GGCTAGCTACAACGA GCTGCTTC | 2264 |
| 2394 | AGCAGCAC G CUGUUUAU | 563 | ATAAACAG GGCTAGCTACAACGA GTGCTGCT | 2265 |
| 2397 | AGCACGCU G WUAWGA | 564 | TCAATAAA GGCTAGCTACAACGA AGCGTGCT | 2266 |
| 2401 | CGCUGUUU A UUGAAAGA | 565 | TCTTTCAA GGCTAGCTACAACGA AAACAGCG | 2267 |
| 2410 | UUGAAAGA G UCACAGAA | 566 | TTCTGTGA GGCTAGCTACAACGA TCTTTCAA | 2268 |
| 2413 | AAAGAGUC A CAGAAGAG | 567 | CTCTTCTG GGCTAGCTACAACGA GACTCTTT | 2269 |
| 2423 | AGAAGAGG A UGAAGGUG | 568 | CACCTTCA GGCTAGCTACAACGA CCTCTTCT | 2270 |
| 2429 | GGAUGAAG G UGUCUAUC | 569 | GATAGACA GGCTAGCTACAACGA CTTCATCC | 2271 |
| 2431 | AUGAAGGU G UCUAUCAC | 570 | GTGATAGA GGCTAGCTACAACGA ACCTTCAT | 2272 |
| 2435 | AGGUGUCU A UCACUGCA | 571 | TGCAGTGA GGCTAGCTACAACGA AGACACCT | 2273 |
| 2438 | UGUCUAUC A CUGCAAAG | 572 | CTTTGCAG GGCTAGCTACAACGA GATAGACA | 2274 |
| 2441 | CUAUCACU G CAAAGCCA | 573 | TGGCTTTG GGCTAGCTACAACGA AGTGATAG | 2275 |
| 2446 | ACUGCAAA G CCACCAAC | 574 | GTTGGTGG GGCTAGCTACAACGA TTTGCAGT | 2276 |
| 2449 | GCAAAGCC A CCAACCAG | 575 | CTGGTTGG GGCTAGCTACAACGA GGCTTTGC | 2277 |
| 2453 | AGCCACCA A CCAGAAGG | 576 | CCTTCTGG GGCTAGCTACAACGA TGGTGGCT | 2278 |
| 2462 | CCAGAAGG G CUCUGUGG | 577 | CCACAGAG GGCTAGCTACAACGA CCTTCTGG | 2279 |
| 2467 | AGGGCUCU G UGGAAAGU | 578 | ACTTTCCA GGCTAGCTACAACGA AGAGCCCT | 2280 |
| 2474 | UGUGGAAA G UUCAGCAU | 579 | ATGCTGAA GGCTAGCTACAACGA TTTCCACA | 2281 |
| 2479 | AAAGUUCA G CAUACCUC | 580 | GAGGTATG GGCTAGCTACAACGA TGAACTTT | 2282 |
| 2481 | AGUUCAGC A UACCUCAC | 581 | GTGAGGTA GGCTAGCTACAACGA GCTGAACT | 2283 |
| 2483 | UUCAGCAU A CCUCACUG | 582 | CAGTGAGG GGCTAGCTACAACGA ATGCTGAA | 2284 |
| 2488 | CAUACCUC A CUGUUCAA | 583 | TTGAACAG GGCTAGCTACAACGA GAGGTATG | 2285 |
| 2491 | ACCUCACU G UUCAAGGA | 584 | TCCTTGAA GGCTAGCTACAACGA AGTGAGGT | 2286 |
| 2500 | UUCAAGGA A CCUCGGAC | 585 | GTCCGAGG GGCTAGCTACAACGA TCCTTGAA | 2287 |
| 2507 | AACCUCGG A CAAGUCUA | 586 | TAGACTTG GGCTAGCTACAACGA CCGAGGTT | 2288 |
| 2511 | UCGGACAA G UCUAAUCU | 587 | AGATTAGA GGCTAGCTACAACGA TTGTCCGA | 2289 |
| 2516 | CAAGUCUA A UCUGGAGC | 588 | GCTCCAGA GGCTAGCTACAACGA TAGACTTG | 2290 |
| 2523 | AAUCUGGA G CUGAUCAC | 589 | GTGATCAG GGCTAGCTACAACGA TCCAGATT | 2291 |
| 2527 | UGGAGCUG A UCACUCUA | 590 | TAGAGTGA GGCTAGCTACAACGA CAGCTCCA | 2292 |
| 2530 | AGCUGAUC A CUCUAACA | 591 | TGTTAGAG GGCTAGCTACAACGA GATCAGCT | 2293 |
| 2536 | UCACUCUA A CAUGCACC | 592 | GGTGCATG GGCTAGCTACAACGA TAGAGTGA | 2294 |
| 2538 | ACUCUAAC A UGCACCUG | 593 | CAGGTGCA GGCTAGCTACAACGA GTTAGAGT | 2295 |
| 2540 | UGUAACAU G CACCUGUG | 594 | CACAGGTG GGCTAGCTACAACGA ATGTTAGA | 2296 |
| 2542 | UAACAUGC A CCUGUGUG | 595 | CACACAGG GGCTAGCTACAACGA GCATGTTA | 2297 |
| 2546 | AUGCACCU G UGUGGCUG | 596 | CAGCCACA GGCTAGCTACAACGA AGGTGCAT | 2298 |
| 2548 | GCACCUGU G UGGCUGCG | 597 | CGCAGCCA GGCTAGCTACAACGA ACAGGTGC | 2299 |
| 2551 | CCUGUGUG G CUGCGACU | 598 | AGTCGCAG GGCTAGCTACAACGA CACACAGG | 2300 |
| 2554 | GUGUGGCU G CGACUCUC | 599 | GAGAGTCG GGCTAGCTACAACGA AGCCACAC | 2301 |
| 2557 | UGGCUGCG A CUCUCUUC | 600 | GAAGAGAG GGCTAGCTACAACGA CGCAGCCA | 2302 |
| 2568 | CUCUUCUG G CUCCUAUU | 601 | AATAGGAG GGCTAGCTACAACGA CAGAAGAG | 2303 |
| 2574 | UGGCUCCU A UUAACCCU | 602 | AGGGTTAA GGCTAGCTAGAACGA AGGAGCCA | 2304 |
| 2578 | UCCUAUUA A CCCUCCUU | 603 | AAGGAGGG GGCTAGCTACAACGA TAATAGGA | 2305 |
| 2587 | CCCUCCUU A UCCGAAAA | 604 | TTTTCGGA GGCTAGCTACAACGA AAGGAGGG | 2306 |
| 2596 | UCCGAAAA A UGAAAAGG | 605 | CCTTTTCA GGCTAGCTACAACM TTTTCGGA | 2307 |
| 2604 | AUGAAAAG G UCUUCUUC | 606 | GAAGAAGA GGCTAGCTACAACGA CTTTTCAT | 2308 |
| 2617 | CUUCUGAA A UAAAGACU | 607 | AGTCTTTA GGCTAGCTACAACGA TTCAGAAG | 2309 |
| 2623 | AAAUAAAG A CUGACUAC | 608 | GTAGTCAG GGCTAGCTACAACGA CTTTATTT | 2310 |
| 2627 | AAAGACUG A CUACCUAU | 609 | ATAGGTAG GGCTAGCTACAACGA CAGTCTTT | 2311 |
| 2630 | GACUGACU A CCUAUCAA | 610 | TTGATAGG GGCTAGCTACAACGA AGTCAGTC | 2312 |
| 2634 | GACUACCU A UCAAUUAU | 611 | ATAATTGA GGCTAGCTACAACGA AGGTAGTC | 2313 |
| 2638 | ACCUAUCA A UUAUAAUG | 612 | CATTATAA GGCTAGCTACAACGA TGATAGGT | 2314 |
| 2641 | UAUCAAUU A UAAUGGAC | 613 | GTCCATTA GGCTAGCTACAACGA AATTGATA | 2315 |
| 2644 | CAAUUAUA A UGGACCCA | 614 | TGGGTCCA GGCTAGCTACAACGA TATAATTG | 2316 |
| 2648 | UAUAAUGG A CCCAGAUG | 615 | CATCTGGG GGCTAGCTACAACGA CCATTATA | 2317 |
| 2654 | GGACCCAG A UGAAGUUC | 616 | GAACTTCA GGCTAGCTACAACGA CTGGGTCC | 2318 |
| 2659 | CAGAUGAA G UUCCUUUG | 617 | CAAAGGAA GGCTAGCTACAACGA TTCATCTG | 2319 |
| 2669 | UCCUUUGG A UGAGCAGU | 618 | ACTGCTCA GGCTAGCTACAACGA CCAAAGGA | 2320 |
| 2673 | UUGGAUGA G CAGUGUGA | 619 | TCACACTG GGCTAGCTACAACGA TCATCCAA | 2321 |
| 2676 | GAUGAGCA G UGUGAGCG | 620 | CGCTCACA GGCTAGCTACAACGA TGCTCATC | 2322 |
| 2678 | UGAGCAGU G UGAGCGGC | 621 | GCCGCTCA GGCTAGCTACAACGA ACTGCTCA | 2323 |
| 2682 | CAGUGUGA G CGGCUCCC | 622 | GGGAGCCG GGCTAGCTACAACGA TCACACTG | 2324 |
| 2685 | UGUGAGCG G CUCCCUUA | 623 | TAAGGGAG GGCTAGCTACAACGA CGCTCACA | 2325 |
| 2693 | GCUCCCUU A UGAUGCCA | 624 | TGGCATCA GGCTAGCTACAACGA AAGGGAGC | 2326 |
| 2696 | CCCUUAUG A UGCCAGCA | 625 | TGCTGGCA GGCTAGCTACAACGA CATAAGGG | 2327 |
| 2698 | CUUAUGAU G CCAGCAAG | 626 | CTTGCTGG GGCTAGCTACAACGA ATCATAAG | 2328 |
| 2702 | UGAUGCCA G CAAGUGGG | 627 | CCCACTTG GGCTAGCTACAACGA TGGCATCA | 2329 |
| 2706 | GCCAGCAA G UGGGAGUU | 628 | AACTCCCA GGCTAGCTACAACGA TTGCTGGC | 2330 |
| 2712 | AAGUGGGA G UUUGCCCG | 629 | CGGGCAAA GGCTAGCTACAACGA TCCCACTT | 2331 |
| 2716 | GGGAGUUU G CCCGGGAG | 630 | CTCCCGGG GGCTAGCTACAACGA AAACTCCC | 2332 |
| 2727 | CGGGAGAG A CUUAAACU | 631 | AGTTTAAG GGCTAGCTACAACGA CTCTCCCG | 2333 |
| 2733 | AGACUUAA A CUGGGCAA | 632 | TTGCCCAG GGCTAGCTACAACGA TTAAGTCT | 2334 |
| 2738 | UAAACUGG G CAAAUCAC | 633 | GTGATTTG GGCTAGCTACAACGA CCAGTTTA | 2335 |
| 2742 | CUGGGCAA A UCACUUGG | 634 | CCAAGTGA GGCTAGCTACAACGA TTGCCCAG | 2336 |
| 2745 | GGCAAAUC A CUUGGAAG | 635 | CTTCCAAG GGCTAGCTACAACGA GATTTGCC | 2337 |
| 2758 | GAAGAGGG G CUUUUGGA | 636 | TCCAAAAG GGCTAGCTACAACGA CCCTCTTC | 2338 |
| 2770 | UUGGAAAA G UGGUUCAA | 637 | TTGAACCA GGCTAGCTACAACGA TTTTCCAA | 2339 |
| 2773 | GAAAAGUG G UUCAAGCA | 638 | TGCTTGAA GGCTAGCTACAACGA CACTTTTC | 2340 |
| 2779 | UGGUUCAA G CAUCAGCA | 639 | TGCTGATG GGCTAGCTACAACGA TTGAACCA | 2341 |
| 2781 | GUUCAAGC A UCAGCAUU | 640 | AATGCTGA GGCTAGCTACAACGA GCTTGAAC | 2342 |
| 2785 | AAGCAUCA G CAUUUGGC | 641 | GCCAAATG GGCTAGCTACAACGA TGATGCTT | 2343 |
| 2787 | GCAUCAGC A UUUGGCAU | 642 | ATGCCAAA GGCTAGCTACAACGA GCTGATGC | 2344 |
| 2792 | AGCAUUUG G CAUUAAGA | 643 | TCTTAATG GGCTAGCTACAACGA CAAATGCT | 2345 |
| 2794 | CAUUUGGC A UUAAGAAA | 644 | TTTCTTAA GGCTAGCTACAACGA GCCAAATG | 2346 |
| 2802 | AUUAAGAA A UCACCUAC | 645 | GTAGGTGA GGCTAGCTACAACGA TTCTTAAT | 2347 |
| 2805 | AAGAAAUC A CCUACGUG | 646 | CACGTAGG GGCTAGCTACAACGA GATTTCTT | 2348 |
| 2809 | AAUCACCU A CGUGCCGG | 647 | CCGGCACG GGCTAGCTACAACGA AGGTGATT | 2349 |
| 2811 | UCACCUAC G UGCCGGAC | 648 | GTCCGGCA GGCTAGCTACAACGA GTAGGTGA | 2350 |
| 2813 | ACCUACGU G CCGGACUG | 649 | CAGTCCGG GGCTAGCTACAACGA ACGTAGGT | 2351 |
| 2818 | CGUGCCGG A CUGUGGCU | 650 | AGCCACAG GGCTAGCTACAACGA CCGGCACG | 2352 |
| 2821 | GCCGGACU G UGGCUGUG | 651 | CACAGCCA GGCTAGCTACAACGA AGTCCGGC | 2353 |
| 2824 | GGACUGUG G CUGUGAAA | 652 | TTTCACAG GGCTAGCTACAACGA CACAGTCC | 2354 |
| 2827 | CUGUGGCU G UGAAAAUG | 653 | CATTTTCA GGCTAGCTACAACGA AGCCACAG | 2355 |
| 2833 | CUGUGAAA A UGCUGAAA | 654 | TTTCAGCA GGCTAGCTACAACGA TTTCACAG | 2356 |
| 2835 | GUGAAAAU G CUGAAAGA | 655 | TCTTTCAG GGCTAGCTACAACGA ATTTTCAC | 2357 |
| 2848 | AAGAGGGG G CCACGGCC | 656 | GGCCGTGG GGCTAGCTACAACGA CCCCTCTT | 2358 |
| 2851 | AGGGGGCC A CGGCCAGC | 657 | GCTGGCCG GGCTAGCTACAACGA GGCCCCCT | 2359 |
| 2854 | GGGCCACG G CCAGCGAG | 658 | CTCGCTGG GGCTAGCTACAACGA CGTGGCCC | 2360 |
| 2858 | CACGGCCA G CGAGUACA | 659 | TGTACTCG GGCTAGCTACAACGA TGGCCGTG | 2361 |
| 2862 | GCCAGCGA G UACAAAGC | 660 | GCTTTGTA GGCTAGCTACAACGA TCGCTGGC | 2362 |
| 2864 | CAGCGAGU A CAAAGCUC | 661 | GAGCTTTG GGCTAGCTACAACGA ACTCGCTG | 2363 |
| 2869 | AGUACAAA G CUCUGAUG | 662 | CATCAGAG GGCTAGCTACAACGA TTTGTACT | 2364 |
| 2875 | AAGCUCUG A UGACUGAG | 663 | CTCAGTCA GGCTAGCTACAACGA CAGAGCTT | 2365 |
| 2878 | CUCUGAUG A CUGAGCUA | 664 | TAGCTCAG GGCTAGCTACAACGA CATCAGAG | 2366 |
| 2883 | AUGACUGA G CUAAAAAU | 665 | ATTTTTAG GGCTAGCTACAACGA TCAGTCAT | 2367 |
| 2890 | AGCUAAAA A UCUUGACC | 666 | GGTCAAGA GGCTAGCTACAACGA TTTTAGCT | 2368 |
| 2896 | AAAUCUUG A CCCACAUU | 667 | AATGTGGG GGCTAGCTACAACGA CAAGATTT | 2369 |
| 2900 | CUUGACCC A CAUUGGCC | 668 | GGCCAATG GGCTAGCTACAACGA GGGTCAAG | 2370 |
| 2902 | UGACCCAC A UUGGCCAC | 669 | GTGGCCAA GGCTAGCTACAACGA GTGGGTCA | 2371 |
| 2906 | CCACAUUG G CCACCAUC | 670 | GATGGTGG GGCTAGCTACAACGA CAATGTGG | 2372 |
| 2909 | CAUUGGCC A CCAUCUGA | 671 | TCAGATGG GGCTAGCTACAACGA GGCCAATG | 2373 |
| 2912 | UGGCCACC A UCUGAACG | 672 | CGTTCAGA GGCTAGCTACAACGA GGTGGCCA | 2374 |
| 2918 | CCAUCUGA A CGUGGUUA | 673 | TAACCACG GGCTAGCTACAACGA TCAGATGG | 2375 |
| 2920 | AUCUGAAC G UGGUUAAC | 674 | GTTAACCA GGCTAGCTACAACGA GTTCAGAT | 2376 |
| 2923 | UGAACGUG G UUAACCUG | 675 | CAGGTTAA GGCTAGCTACAACGA CACGTTCA | 2377 |
| 2927 | CGUGGUUA A CCUGCUGG | 676 | CCAGCAGG GGCTAGCTACAACGA TAACCACG | 2378 |
| 2931 | GUUAACCU G CUGGGAGC | 677 | GCTCCCAG GGCTAGCTACAACGA AGGTTAAC | 2379 |
| 2938 | UGCUGGGA G CCUGCACC | 678 | GGTGCAGG GGCTAGCTACAACGA TCCCAGCA | 2380 |
| 2942 | GGGAGCCU G CACCAAGC | 679 | GCTTGGTG GGCTAGCTACAACGA AGGCTCCC | 2381 |
| 2944 | GAGCCUGC A CCAAGCAA | 680 | TTGCTTGG GGCTAGCTACAACGA GCAGGCTC | 2382 |
| 2949 | UGCACCAA G CAAGGAGG | 681 | CCTCCTTG GGCTAGCTACAACGA TTGGTGCA | 2383 |
| 2958 | CAAGGAGG G CCUCUGAU | 682 | ATCAGAGG GGCTAGCTACAACGA CCTCCTTG | 2384 |
| 2965 | GGCCUCUG A UGGUGAUU | 683 | AATCACCA GGCTAGCTACAACGA CAGAGGCC | 2385 |
| 2968 | CUCUGAUG G UGAUUGUU | 684 | AACAATCA GGCTAGCTACAACGA CATCAGAG | 2386 |
| 2971 | UGAUGGUG A UUGUUGAA | 685 | TTCAACAA GGCTAGCTACAACGA CACCATCA | 2387 |
| 2974 | UGGUGAUU G UUGAAUAC | 686 | GTATTCAA GGCTAGCTACAACGA AATCACCA | 2388 |
| 2979 | AUUGUUGA A UACUGCAA | 687 | TTGCAGTA GGCTAGCTACAACGA TCAACAAT | 2389 |
| 2981 | UGUUGAAU A CUGCAAAU | 688 | ATTTGCAG GGCTAGCTACAACGA ATTCAACA | 2390 |
| 2984 | UGAAUACU G CAAAUAUG | 689 | CATATTTG GGCTAGCTACAACGA AGTATTCA | 2391 |
| 2988 | UACUGCAA A UAUGGAAA | 690 | TTTCCATA GGCTAGCTAGAACGA TTGCAGTA | 2392 |
| 2990 | CUGCAAAU A UGGAAAUC | 691 | GATTTCCA GGCTAGCTACAACGA ATTTGCAG | 2393 |
| 2996 | AUAUGGAA A UCUCUCCA | 692 | TGGAGAGA GGCTAGCTACAACGA TTCCATAT | 2394 |
| 3005 | UCUCUCCA A CUACCUCA | 693 | TGAGGTAG GGCTAGCTACAACGA TGGAGAGA | 2395 |
| 3008 | CUCCAACU A CCUCAAGA | 694 | TCTTGAGG GGCTAGCTACAACGA AGTTGGAG | 2396 |
| 3017 | CCUCAAGA G CAAACGUG | 695 | CACGTTTG GGCTAGCTACAACGA TCTTGAGG | 2397 |
| 3021 | AAGAGCAA A CGUGACUU | 696 | AAGTCACG GGCTAGCTACAACGA TTGCTCTT | 2398 |
| 3023 | GAGCAAAC G UGACUUAU | 697 | ATAAGTCA GGCTAGCTACAACGA GTTTGCTC | 2399 |
| 3026 | CAAACGUG A CUUAUUUU | 698 | AAAATAAG GGCTAGCTACAACGA CACGTTTG | 2400 |
| 3030 | CGUGACUU A UUUUUUCU | 699 | AGAAAAAA GGCTAGCTACAACGA AAGTCACG | 2401 |
| 3041 | UUUUCUCA A CAAGGAUG | 700 | CATCCTTG GGCTAGCTACAACGA TGAGAAAA | 2402 |
| 3047 | CAACAAGG A UGCAGCAC | 701 | GTGCTGCA GGCTAGCTACAACGA CCTTGTTG | 2403 |
| 3049 | ACAAGGAU G CAGCACUA | 702 | TAGTGCTG GGCTAGCTACAACGA ATCCTTGT | 2404 |
| 3052 | AGGAUGCA G CACUACAC | 703 | GTGTAGTG GGCTAGCTACAACGA TGCATCCT | 2405 |
| 3054 | GAUGCAGC A CUACACAU | 704 | ATGTGTAG GGCTAGCTACAACGA GCTGCATC | 2406 |
| 3057 | GCAGCACU A CACAUGGA | 705 | TCCATGTG GGCTAGCTACAACGA AGTGCTGC | 2407 |
| 3059 | AGCACUAC A CAUGGAGC | 706 | GCTCCATG GGCTAGCTACAACGA GTAGTGCT | 2408 |
| 3061 | CACUACAC A UGGAGCCU | 707 | AGGCTCCA GGCTAGCTACAACGA GTGTAGTG | 2409 |
| 3066 | CACAUGGA G CCUAAGAA | 708 | TTCTTAGG GGCTAGCTACAACGA TCCATGTG | 2410 |
| 3082 | AAGAAAAA A UGGAGCCA | 709 | TGGCTCCA GGCTAGCTACAACGA TTTTTCTT | 2411 |
| 3087 | AAAAUGCA G CCAGGCCU | 710 | AGGCCTGG GGCTAGCTACAACGA TCCATTTT | 2412 |
| 3092 | GGAGCCAG G CCUGGAAC | 711 | GTTCCAGG GGCTAGCTACAACGA CTGGCTCC | 2413 |
| 3099 | GGCCUGGA A CAAGGCAA | 712 | TTGCCTTG GGCTAGCTACAACGA TCCAGGCC | 2414 |
| 3104 | GGAACAAG G CAAGAAAC | 713 | GTTTCTTG GGCTAGCTACAACGA CTTGTTCC | 2415 |
| 3111 | GGCAAGAA A CCAAGACU | 714 | AGTCTTGG GGCTAGCTACAACGA TTCTTGCC | 2416 |
| 3117 | AAACCAAG A CUAGAUAG | 715 | CTATCTAG GGCTAGCTACAACGA CTTGGTTT | 2417 |
| 3122 | AAGACUAG A UAGCGUCA | 716 | TGACGCTA GGCTAGCTACAACGA CTAGTCTT | 2418 |
| 3125 | ACUAGAUA G CGUCACCA | 717 | TGGTGACG GGCTAGCTACAACGA TATCTAGT | 2419 |
| 3127 | UAGAUAGC G UCACCAGC | 718 | GCTGGTGA GGCTAGCTACAACGA GCTATCTA | 2420 |
| 3130 | AUAGCGUC A CCAGCAGC | 719 | GCTGCTGG GGCTAGCTACAACGA GACGCTAT | 2421 |
| 3134 | CGUCACCA G CAGCGAAA | 720 | TTTCGCTG GGCTAGCTACAACGA TGGTGACG | 2422 |
| 3137 | CACCAGCA G CGAAAGCU | 721 | AGCTTTCG GGCTAGCTACAACGA TGCTGGTG | 2423 |
| 3143 | CAGCGAAA G CUUUGCGA | 722 | TCGCAAAG GGCTAGCTACAACGA TTTCGCTG | 2424 |
| 3148 | AAAGCUUU G CGAGCUCC | 723 | GGAGCTCG GGCTAGCTACAACGA AAAGCTTT | 2425 |
| 3152 | CUUUGCGA G CUCCGGCU | 724 | AGCCGGAG GGCTAGCTACAACGA TCGCAAAG | 2426 |
| 3158 | GAGCUCCG G CUUUCAGG | 725 | CCTGAAAG GGCTAGCTACAACGA CGGAGCTC | 2427 |
| 3170 | UCAGGAAG A UAAAAGUC | 726 | GACTTTTA GGCTAGCTACAACGA CTTCCTGA | 2428 |
| 3176 | AGAUAAAA G UCUGAGUG | 727 | CACTCAGA GGCTAGCTACAACGA TTTTATCT | 2429 |
| 3182 | AAGUCUGA G UGAUGUUG | 728 | CAACATCA GGCTAGCTACAACGA TCAGACTT | 2430 |
| 3185 | UCUGAGUG A UGUUGAGG | 729 | CCTCAACA GGCTAGCTACAACGA CACTCAGA | 2431 |
| 3187 | UGAGUGAU G UUGAGGAA | 730 | TTCCTCAA GGCTAGCTACAACGA ATCACTCA | 2432 |
| 3203 | AGAGGAGG A UUCUGACG | 731 | CGTCAGAA GGCTAGCTACAACGA CCTCCTCT | 2433 |
| 3209 | GGAUUCUG A CGGUUUCU | 732 | AGAAACCG GGCTAGCTACAACGA CAGAATCC | 2434 |
| 3212 | UUCUGACG G UUUCUACA | 733 | TGTAGAAA GGCTAGCTACAACGA CGTCAGAA | 2435 |
| 3218 | CGGUUUCU A CAAGGAGC | 734 | GCTCCTTG GGCTAGCTACAACGA AGAAACCG | 2436 |
| 3225 | UACAAGGA G CCCAUCAC | 735 | GTGATGGG GGCTAGCTACAACGA TCCTTGTA | 2437 |
| 3229 | AGGAGCCC A UCACUAUG | 736 | CATAGTGA GGCTAGCTACAACGA GGGCTCCT | 2438 |
| 3232 | AGCCCAUC A CUAUGGAA | 737 | TTCCATAG GGCTAGCTACAACGA GATGGGCT | 2439 |
| 3235 | CCAUCACU A UGGAAGAU | 738 | ATCTTCCA GGCTAGCTACAACGA AGTGATGG | 2440 |
| 3242 | UAUGGAAG A UCUGAUUU | 739 | AAATCAGA GGCTAGCTACAACGA CTTCCATA | 2441 |
| 3247 | AAGAUCUG A UUUCUUAC | 740 | GTAAGAAA GGCTAGCTACAACGA CAGATCTT | 2442 |
| 3254 | GAUUUCUU A CAGUUUUC | 741 | GAAAACTG GGCTAGCTACAACGA AAGAAATC | 2443 |
| 3257 | UUCUUACA G UUUUCAAG | 742 | CTTGAAAA GGCTAGCTACAACGA TGTAAGAA | 2444 |
| 3265 | GUUUUCAA G UGGCCAGA | 743 | TCTGGCCA GGCTAGCTACAACGA TTGAAAAC | 2445 |
| 3268 | UUCAAGUG G CCAGAGGC | 744 | GCCTCTGG GGCTAGCTACAACGA CACTTGAA | 2446 |
| 3275 | GGCCAGAG G CAUGGAGU | 745 | ACTCCATG GGCTAGCTACAACGA CTCTGGCC | 2447 |
| 3277 | CCAGAGGC A UGGAGUUC | 746 | GAACTCCA GGCTAGCTACAACGA GCCTCTGG | 2448 |
| 3282 | GGCAUGGA G UUCCUGUC | 747 | GACAGGAA GGCTAGCTACAACGA TCCATGCC | 2449 |
| 3288 | GAGUUCCU G UCUUCCAG | 748 | CTGGAAGA GGCTAGCTACAACGA AGGAACTC | 2450 |
| 33.00 | UCCAGAAA G UGCAUUCA | 749 | TGAATGCA GGCTAGCTACAACGA TTTCTGGA | 2451 |
| 3302 | CAGAAAGU G CAUUCAUC | 750 | GATGAATG GGCTAGCTACAACGA ACTTTCTG | 2452 |
| 3304 | GAAAGUGC A UUCAUCGG | 751 | CCGATGAA GGCTAGCTACAACGA GCACTTTC | 2453 |
| 3308 | GUGCAUUC A UCGGGACC | 752 | GGTCCCGA GGCTAGCTACAACGA GAATGCAC | 2454 |
| 3314 | UCAUCGGG A CCUGBCAG | 753 | CTGCCAGG GGCTAGCTACAACGA CCCGATGA | 2455 |
| 3319 | GGGACCUG G CAGCGAGA | 754 | TCTCGCTG GGCTAGCTACAACGA CAGGTCCC | 2456 |
| 3322 | ACCUGGCA G CGAGAAAC | 755 | GTTTCTCG GGCTAGCTACAACGA TGCCAGGT | 2457 |
| 3329 | AGCGAGAA A CAUUCUUU | 756 | AAAGAATG GGCTAGCTACAACGA TTCTCGCT | 2458 |
| 3331 | CGAGAAAC A UUCUUUUA | 757 | TAAAAGAA GGCTAGCTACAACGA GTTTCTCG | 2459 |
| 3339 | AUUCUUUU A UCUGAGAA | 758 | TTCTCAGA GGCTAGCTACAACGA AAAAGAAT | 2460 |
| 3347 | AUCUGAGA A CAACGUGG | 759 | CCACGTTG GGCTAGCTACAACGA TCTCAGAT | 2461 |
| 3350 | UGAGAACA A CGUGGUGA | 760 | TCACCACG GGCTAGCTACAACGA TGTTCTCA | 2462 |
| 3352 | AGAACAAC G UGGUGAAG | 761 | CTTCACCA GGCTAGCTACAACGA GTTGTTCT | 2463 |
| 3355 | ACAACGUG G UGAAGAUU | 762 | AATCTTCA GGCTAGCTACAACGA CACGTTGT | 2464 |
| 3361 | UGGUGAAG A UUUGUGAU | 763 | ATCACAAA GGCTAGCTACAACGA CTTCACCA | 2465 |
| 3365 | GAAGAUUU G UGAUUUUG | 764 | CAAAATCA GGCTAGCTACAACGA AAATCTTC | 2466 |
| 3368 | GAUUUGUG A UUUUGGCC | 765 | GGCCAAAA GGCTAGCTACAACGA CACAAATC | 2467 |
| 3374 | UGAUUUUG G CCUUGCCC | 766 | GGGCAAGG GGCTAGCTACAACGA CAAAATCA | 2468 |
| 3379 | UUGGCCUU G CCCGGGAU | 767 | ATCCCGGG GGCTAGCTACAACGA AAGGCCAA | 2469 |
| 3386 | UGCCCGGG A UAUUUAUA | 768 | TATAAATA GGCTAGCTACAACGA CCCGGGCA | 2470 |
| 3388 | CCCGGGAU A UUUAUAAG | 769 | CTTATAAA GGCTAGCTACAACGA ATCCCGGG | 2471 |
| 3392 | GGAUAUUU A UAAGAACC | 770 | GGTTCTTA GGCTAGCTACAACGA AAATATCC | 2472 |
| 3398 | UUAUAAGA A CCCCGAUU | 771 | AATCGGGG GGCTAGCTACAACGA TCTTATAA | 2473 |
| 3404 | GAACCCCG A UUAUGUGA | 772 | TCACATAA GGCTAGCTACAACGA CGGGGTTC | 2474 |
| 3407 | CCCCGAUU A UGUGAGAA | 773 | TTCTCACA GGCTAGCTACAACGA AATCGGGG | 2475 |
| 3409 | CCGAUUAU G UGAGAAAA | 774 | TTTTCTCA GGCTAGCTACAACGA ATAATCGG | 2476 |
| 3422 | AAAAGGAG A UACUCGAC | 775 | GTCGAGTA GGCTAGCTACAACGA CTCCTTTT | 2477 |
| 3424 | AAGGAGAU A CUCGACUU | 776 | AAGTCGAG GGCTAGCTACAACGA ATCTCCTT | 2478 |
| 3429 | GAUACUCG A CUUCCUCU | 777 | AGAGGAAG GGCTAGCTACAACGA CGAGTATC | 2479 |
| 3441 | CCUCUGAA A UGGAUGGC | 778 | GCCATCCA GGCTAGCTACAACGA TTCAGAGG | 2480 |
| 3445 | UGAAAUGG A UGGCUCCC | 779 | GGGAGCCA GGCTAGCTACAACGA CCATTTCA | 2481 |
| 3448 | AAUGGAUG G CUCCCGAA | 780 | TTCGGGAG GGCTAGCTACAACGA CATCCATT | 2482 |
| 3456 | GCUCCCGA A UCUAUCUU | 781 | AAGATAGA GGCTAGCTACAACGA TCGGGAGC | 2483 |
| 3460 | CCGAAUCU A UCUUUGAC | 782 | GTCAAAGA GGCTAGCTACAACGA AGATTCGG | 2484 |
| 3467 | UAUCUUUG A CAAAAUCU | 783 | AGATTTTG GGCTAGCTACAACGA CAAAGATA | 2485 |
| 3472 | UUGACAAA A UCUACAGC | 784 | GCTGTAGA GGCTAGCTACAACGA TTTGTCAA | 2486 |
| 3476 | CAAAAUCU A CAGCACCA | 785 | TGGTGCTG GGCTAGCTACAACGA AGATTTTG | 2487 |
| 3479 | AAUCUACA G CACCAAGA | 786 | TCTTGGTG GGCTAGCTACAACGA TGTAGATT | 2488 |
| 3481 | UCUACAGC A CCAAGAGC | 787 | GCTCTTGG GGCTAGCTACAACGA GCTGTAGA | 2489 |
| 3488 | CACCAAGA G CGACGUGU | 788 | ACACGTCG GGCTAGCTACAACGA TCTTGGTG | 2490 |
| 3491 | CAAGAGCG A CGUGUGGU | 789 | ACCACACG GGCTAGCTACAACGA CGCTCTTG | 2491 |
| 3493 | AGAGCGAC G UGUGGUCU | 790 | AGACCACA GGCTAGCTACAACGA GTCGCTCT | 2492 |
| 3495 | AGCGACGU G UGGUCUUA | 791 | TAAGACCA GGCTAGCTACAACGA ACGTCGCT | 2493 |
| 3498 | GACGUGUG G UCUUACGG | 792 | CCGTAAGA GGCTAGCTACAACGA CACACGTC | 2494 |
| 3503 | GUGGUCUU A CGGAGUAU | 793 | ATACTCCG GGCTAGCTACAACGA AAGACCAC | 2495 |
| 3508 | CUUACGGA G UAUUGCUG | 794 | CAGCAATA GGCTAGCTACAACGA TCCGTAAG | 2496 |
| 3510 | UACGGAGU A UUGCUGUG | 795 | CACAGCAA GGCTAGCTACAACGA ACTCCGTA | 2497 |
| 3513 | GGAGUAUU G CUGUGGGA | 796 | TCCCACAG GGCTAGCTACAACGA AATACTCC | 2498 |
| 3516 | GUAUUGCU G UGGGAAAU | 797 | ATTTCCCA GGCTAGCTACAACGA AGCAATAC | 2499 |
| 3523 | UGUGGGAA A UCUUCUCC | 798 | GGAGAAGA GGCTAGCTACAACGA TTCCCACA | 2500 |
| 3536 | CUCCUUAG G UGGGUCUC | 799 | GAGACCCA GGCTAGCTACAACGA CTAAGGAG | 2501 |
| 3540 | UUAGGUGG G UCUCCAUA | 800 | TATGGAGA GGCTAGCTACAACGA CCACCTAA | 2502 |
| 3546 | GGGUCUCC A UACCCAGG | 801 | CCTGGGTA GGCTAGCTACAACGA GGAGACCC | 2503 |
| 3548 | GUCUCCAU A CCCAGGAG | 802 | CTCCTGGG GGCTAGCTACAACGA ATGGAGAC | 2504 |
| 3556 | ACCCAGGA G UACAAAUG | 803 | CATTTGTA GGCTAGCTACAACGA TCCTGGGT | 2505 |
| 3558 | CCAGGAGU A CAAAUGGA | 804 | TCCATTTG GGCTAGCTACAACGA ACTCCTGG | 2506 |
| 3562 | GAGUACAA A UGGAUGAG | 805 | CTCATCCA GGCTAGCTACAACGA TTGTACTC | 2507 |
| 3566 | ACAAAUGG A UGAGGACU | 806 | AGTCCTCA GGCTAGCTACAACGA CCATTTGT | 2508 |
| 3572 | GGAUGAGG A CUUUUGCA | 807 | TGCAAAAG GGCTAGCTACAACGA CCTCATCC | 2509 |
| 3578 | GGACUUUU G CAGUCGCC | 808 | GGCGACTG GGCTAGCTACAACGA AAAAGTCC | 2510 |
| 3581 | CUUUUGCA G UCGCCUGA | 809 | TCAGGCGA GGCTAGCTACAACGA TGCAAAAG | 2511 |
| 3584 | UUGCAGUC G CCUGAGGG | 810 | CCCTCAGG GGCTAGCTACAACGA GACTGCAA | 2512 |
| 3596 | GAGGGAAG G CAUGAGGA | 811 | TCCTCATG GGCTAGCTACAACGA CTTCCCTC | 2513 |
| 3598 | GGGAAGGC A UGAGGAUG | 812 | CATCCTCA GGCTAGCTACAACGA GCCTTCCC | 2514 |
| 3604 | GCAUGAGG A UGAGAGCU | 813 | AGCTCTCA GGCTAGCTACAACGA CCTCATGC | 2515 |
| 3610 | GGAUGAGA G CUCCUGAG | 814 | CTCAGGAG GGCTAGCTACAACGA TCTCATCC | 2516 |
| 3618 | GCUCCUGA G UACUCUAC | 815 | GTAGAGTA GGCTAGCTACAACGA TCAGGAGC | 2517 |
| 3620 | UCCUGAGU A CUCUACUC | 816 | GAGTAGAG GGCTAGCTACAACGA ACTCAGGA | 2518 |
| 3625 | AGUACUCU A CUCCUGAA | 817 | TTCAGGAG GGCTAGCTACAACGA AGAGTACT | 2519 |
| 3634 | CUCCUGAA A UCUAUCAG | 818 | CTGATAGA GGCTAGCTACAACGA TTCAGGAG | 2520 |
| 3638 | UGAAAUCU A UCAGAUCA | 819 | TGATCTGA GGCTAGCTACAACGA AGATTTCA | 2521 |
| 3643 | UCUAUCAG A UCAUGCUG | 820 | CAGCATGA GGCTAGCTACAACGA CTGATAGA | 2522 |
| 3646 | AUCAGAUC A UGCUGGAC | 821 | GTCCAGCA GGCTAGCTACAACGA GATCTGAT | 2523 |
| 3648 | CAGAUCAU G CUGGACUG | 822 | CAGTCCAG GGCTAGCTACAACGA ATGATCTG | 2524 |
| 3653 | CAUGCUGG A CUGCUGGC | 823 | GCCAGCAG GGCTAGCTACAACGA CCAGCATG | 2525 |
| 3656 | GCUGGACU G CUGGCACA | 824 | TGTGCCAG GGCTAGCTACAACGA AGTCCAGC | 2526 |
| 3660 | GACUGCUG G CACAGAGA | 825 | TCTCTGTG GGCTAGCTACAACGA CAGCAGTC | 2527 |
| 3662 | CUGCUGGC A CAGAGACC | 826 | GGTCTCTG GGCTAGCTACAACGA GCCAGCAG | 2528 |
| 3668 | GCACAGAG A CCCAAAAG | 827 | CTTTTGGG GGCTAGCTACAACGA CTCTGTGC | 2529 |
| 3681 | AAAGAAAG G CCAAGAUU | 828 | AATCTTGG GGCTAGCTACAACGA CTTTCTTT | 2530 |
| 3687 | AGGCCAAG A UUUGCAGA | 829 | TCTGCAAA GGCTAGCTACAACGA CTTGGCCT | 2531 |
| 3691 | CAAGAUUU G CAGAACUU | 830 | AAGTTCTG GGCTAGCTACAACGA AAATCTTG | 2532 |
| 3696 | UUUGCAGA A CUUGUGGA | 831 | TCCACAAG GGCTAGCTACAACGA TCTGCAAA | 2533 |
| 3700 | CAGAACUU G UGGAAAAA | 832 | TTTTTCCA GGCTAGCTACAACGA AAGTTCTG | 2534 |
| 3708 | GUGGAAAA A CUAGGUGA | 833 | TCACCTAG GGCTAGCTACAACGA TTTTCCAC | 2535 |
| 3713 | AAAACUAG G UGAUUUGC | 834 | GCAAATCA GGCTAGCTACAACGA CTAGTTTT | 2536 |
| 3716 | ACUAGGUG A UUUGCUUC | 835 | GAAGCAAA GGCTAGCTACAACGA CACCTAGT | 2537 |
| 3720 | GGUGAUUU G CUUCAAGC | 836 | GCTTGAAG GGCTAGCTACAACGA AAATCACC | 2538 |
| 3727 | UGCUUCAA G CAAAUGUA | 837 | TACATTTG GGCTAGCTACAACGA TTGAAGCA | 2539 |
| 3731 | UCAAGCAA A UGUACAAC | 838 | GTTGTACA GGCTAGCTACAACGA TTGCTTGA | 2540 |
| 3733 | AAGCAAAU G UACAACAG | 839 | CTGTTGTA GGCTAGCTACAACGA ATTTGCTT | 2541 |
| 3735 | GCAAAUGU A CAACAGGA | 840 | TCCTGTTG GGCTAGCTACAACGA ACATTTGC | 2542 |
| 3738 | AAUGUACA A CAGGAUGG | 841 | CCATCCTG GGCTAGCTACAACGA TGTACATT | 2543 |
| 3743 | ACAACAGG A UGGUAAAG | 842 | CTTTACCA GGCTAGCTACAACGA CCTGTTGT | 2544 |
| **3746** | ACAGGAUG G UAAAGACU | 843 | AGTCTTTA GGCTAGCTACAACGA CATCCTGT | 2545 |
| 3752 | UGGUAAAG A CUACAUCC | 844 | GGATGTAG GGCTAGCTACAACGA CTTTACCA | 2546 |
| 3755 | UAAAGACU A CAUCCCAA | 845 | TTGGGATG GGCTAGCTACAACGA AGTCTTTA | 2547 |
| 3757 | AAGACUAC A UCCCAAUC | 846 | GATTGGGA GGCTAGCTACAACGA GTAGTCTT | 2548 |
| 3763 | ACAUCCCA A UCAAUGCC | 847 | GGCATTGA GGCTAGCTACAACGA TGGGATGT | 2549 |
| 3767 | CCCAAUCA A UGCCAUAC | 848 | GTATGGCA GGCTAGCTACAACGA TGATTGGG | 2550 |
| 3769 | CAAUCAAU G CCAUACUG | 849 | CAGTATGG GGCTAGCTACAACGA ATTGATTG | 2551 |
| 3772 | UCAAUGCC A UACUGACA | 850 | TGTCAGTA GGCTAGCTACAACGA GGCATTGA | 2552 |
| 3774 | AAUGCCAU A CUGACAGG | 851 | CCTGTCAG GGCTAGCTACAACGA ATGGCATT | 2553 |
| 3778 | CCAUACUG A CAGGAAAU | 852 | ATTTCCTG GGCTAGCTACAACGA CAGTATGG | 2554 |
| 3785 | GACAGGAA A UAGUGGGU | 853 | ACCCACTA GGCTAGCTACAACGA TTCCTGTC | 2555 |
| 3788 | AGGAAAUA G UGGGUUUA | 854 | TAAACCCA GGCTAGCTACAACGA TATTTCCT | 2556 |
| 3792 | AAUAGUGG G UUUACAUA | 855 | TATGTAAA GGCTAGCTACAACGA CCACTATT | 2557 |
| 3796 | GUGGGUUU A CAUACUCA | 856 | TGAGTATG GGCTAGCTACAACGA AAACCCAC | 2558 |
| 3798 | GGGUUUAC A UACUCAAC | 857 | GTTGAGTA GGCTAGCTACAACGA GTAAACCC | 2559 |
| 3800 | GUUUACAU A CUCAACUC | 858 | GAGTTGAG GGCTAGCTACAACGA ATGTAAAC | 2560 |
| 3805 | CAUACUCA A CUCCUGCC | 859 | GGCAGGAG GGCTAGCTACAACGA TGAGTATG | 2561 |
| 3811 | CAACUCCU G CCUUCUCU | 860 | AGAGAAGG GGCTAGCTACAACGA AGGAGTTG | 2562 |
| 3824 | CUCUGAGG A CUUCUUCA | 861 | TGAAGAAG GGCTAGCTACAACGA CCTCAGAG | 2563 |
| 3839 | CAAGGAAA G UAUUUCAG | 862 | CTGAAATA GGCTAGCTACAACGA TTTCCTTG | 2564 |
| 3841 | AGGAAAGU A UUUCAGCU | 863 | AGCTGAAA GGCTAGCTACAACGA ACTTTCCT | 2565 |
| 3847 | GUAUUUCA G CUCCGAAG | 864 | CTTCGGAG GGCTAGCTACAACGA TGAAATAC | 2566 |
| 3855 | GCUCCGAA G UUUAAUUC | 865 | GAATTAAA GGCTAGCTACAACGA TTCGGAGC | 2567 |
| 3860 | GAAGUUUA A UUCAGGAA | 866 | TTCCTGAA GGCTAGCTACAACGA TAAACTTC | 2568 |
| 3869 | UUCAGGAA G CUCUGAUG | 867 | CATCAGAG GGCTAGCTACAACGA TTCCTGAA | 2569 |
| 3875 | AAGCUCUG A UGAUGUCA | 868 | TGACATCA GGCTAGCTACAACGA CAGAGCTT | 2570 |
| 3878 | CUCUGAUG A UGUCAGAU | 869 | ATCTGACA GGCTAGCTACAACGA CATCAGAG | 2571 |
| 3880 | CUGAUGAU G UCAGAUAU | 870 | ATATCTGA GGCTAGCTACAACGA ATCATCAG | 2572 |
| 3885 | GAUGUCAG A UAUGUAAA | 871 | TTTACATA GGCTAGCTACAACGA CTGACATC | 2573 |
| 3887 | UGUCAGAU A UGUAAAUG | 872 | CATTTACA GGCTAGCTACAACGA ATCTGACA | 2574 |
| 3889 | UCAGAUAU G UAAAUGCU | 873 | AGCATTTA GGCTAGCTACAACGA ATATCTGA | 2575 |
| 3893 | AUAUGUAA A UGCUWCA | 874 | TGAAAGCA GGCTAGCTACAACGA TTACATAT | 2576 |
| 3895 | AUGUAAAU G CUUUCAAG | 875 | CTTGAAAG GGCTAGCTACAACGA ATTTACAT | 2577 |
| 3903 | GCUUUCAA G UUCAUGAG | 876 | CTCATGAA GGCTAGCTACAACGA TTGAAAGC | 2578 |
| 3907 | UCAAGUUC A UGAGCCUG | 877 | CAGGCTCA GGCTAGCTACAACGA GAACTTGA | 2579 |
| 3911 | GUUCAUGA G CCUGGAAA | 878 | TTTCCAGG GGCTAGCTACAACGA TCATGAAC | 2580 |
| 3922 | UGGAAAGA A UCAAAACC | 879 | GGTTTTGA GGCTAGCTACAACGA TCTTTCCA | 2581 |
| 3928 | GAAUCAAA A CCUUUGAA | 880 | TTCAAAGG GGCTAGCTACAACGA TTTGATTC | 2582 |
| 3939 | UUUGAAGA A CUUUUACC | 881 | GGTAAAAG GGCTAGCTACAACGA TCTTCAAA | 2583 |
| 3945 | GAACUUUU A CCGAAUGC | 882 | GCATTCGG GGCTAGCTACAACGA AAAAGTTC | 2584 |
| 3950 | UUUACCGA A UGCCACCU | 883 | AGGTGGCA GGCTAGCTACAACGA TCGGTAAA | 2585 |
| 3952 | UACCGAAU G CCACCUCC | 884 | GGAGGTGG GGCTAGCTACAACGA ATTCGGTA | 2586 |
| 3955 | CGAAUGCC A CCUCCAUG | 885 | CATGGAGG GGCTAGCTACAACGA GGCATTCG | 2587 |
| 3961 | CCACCUCC A UGUUUGAU | 886 | ATCAAACA GGCTAGCTACAACGA GGAGGTGG | 2588 |
| 3963 | ACCUCCAU G UUUGAUGA | 887 | TCATCAAA GGCTAGCTACAACGA ATGGAGGT | 2589 |
| 3968 | CAUGUUUG A UGACUACC | 888 | GGTAGTCA GGCTAGCTACAACGA CAAACATG | 2590 |
| 3971 | GUUUGAUG A CUACCAGG | 889 | CCTGGTAG GGCTAGCTACAACGA CATCAAAC | 2591 |
| 3974 | UGAUGACU A CCAGGGCG | 890 | CGCCCTGG GGCTAGCTACAACGA AGTCATCA | 2592 |
| 3980 | CUACCAGG G CGACAGCA | 891 | TGCTGTCG GGCTAGCTACAACGA CCTGGTAG | 2593 |
| 3983 | CCAGGGCG A CAGCAGCA | 892 | TGCTGCTG GGCTAGCTACAACGA CGCCCTGG | 2594 |
| 3986 | GGGCGACA G CAGCACUC | 893 | GAGTGCTG GGCTAGCTACAACGA TGTCGCCC | 2595 |
| 3989 | CGACAGCA G CACUCUGU | 894 | ACAGAGTG GGCTAGCTACAACGA TGCTGTCG | 2596 |
| 3991 | ACAGCAGC A CUCUGUUG | 895 | CAACAGAG GGCTAGCTACAACGA GCTGCTGT | 2597 |
| 3996 | AGCACUCU G UUGGCCUC | 896 | GAGGCCAA GGCTAGCTACAACGA AGAGTGCT | 2598 |
| 4000 | CUCUGUUG G CCUCUCCC | 897 | GGGAGAGG GGCTAGCTACAACGA CAACAGAG | 2599 |
| 4009 | CCUCUCCC A UGCUGAAG | 898 | CTTCAGCA GGCTAGCTACAACGA GGGAGAGG | 2600 |
| 4011 | UCUCCCAU G CUGAAGOG | 899 | CGCTTCAG GGCTAGCTACAACGA ATGGGAGA | 2601 |
| 4017 | AUGCUGAA G CGCUUCAC | 900 | GTGAAGCG GGCTAGCTACAACGA TTCAGCAT | 2602 |
| 4019 | GCUGAAGG G CUUCACCU | 901 | AGGTGAAG GGCTAGGTACAACGA GCTTCAGC | 2603 |
| 4024 | AGCGCUUC A CCUGGACU | 902 | AGTCCAGG GGCTAGCTACAACGA GAAGCGCT | 2604 |
| 4030 | UCACCUGG A CUGACAGC | 903 | GCTGTCAG GGCTAGCTAGAACGA CCAGGTGA | 2605 |
| 4034 | CUGGACUG A CAGCAAAC | 904 | GTTTGCTG GGCTAGCTACAACGA CAGTCCAG | 2606 |
| 4037 | GACUGACA G CAAACCCA | 905 | TGGGTTTG GGCTAGCTACAACGA TGTCAGTC | 2607 |
| 404L | GACAGCAA A CCCAAGGC | 906 | GCCTTGGG GGCTAGCTACAACGA TTGCTGTC | 2608 |
| 4048 | AACCCAAG G CCUCGCUC | 907 | GAGCGAGG GGCTAGCTACAACGA CTTGGGTT | 2609 |
| 4053 | AAGGCCUC G CUCAAGAU | 908 | ATCTTGAG GGCTAGCTACAACGA GAGGCCTT | 2610 |
| 4060 | CGCUCAAG A UUGACUUG | 909 | CAAGTCAA GGCTAGCTACAACGA CTTGAGCG | 2611 |
| 4064 | CAAGAUUG A CUUGAGAG | 910 | CTCTCAAG GGCTAGCTACAACGA CAATCTTG | 2612 |
| 4072 | ACUUGAGA G UAACCAGU | 911 | ACTGGTTA GGCTAGCTACAACGA TCTCAAGT | 2613 |
| 4075 | UGAGAGUA A CCAGUAAA | 912 | TTTACTGG GGCTAGCTACAACGA TACTCTCA | 2614 |
| 4079 | AGUAACCA G UAAAAGUA | 913 | TACTTTTA GGCTAGCTACAACGA TGGTTACT | 2615 |
| 4085 | CAGUAAAA G UAAGGAGU | 914 | ACTCCTTA GGCTAGCTACAACGA TTTTACTG | 2616 |
| 4092 | AGUAAGGA G UCGGGGCU | 915 | AGCCCCGA GGCTAGCTACAACGA TCCTTACT | 2617 |
| 4098 | GAGUCGGG G CUGUCUGA | 916 | TCAGACAG GGCTAGCTACAACGA CCCGACTC | 2618 |
| 4101 | UCGGGGCU G UCUGAUGU | 917 | ACATCAGA GGCTAGCTACAACGA AGCCCCGA | 2619 |
| 4106 | GCUGUCUG A UGUCAGCA | 918 | TGCTGACA GGCTAGCTACAACGA CAGACAGC | 2620 |
| 4108 | UGUCUGAU G UCAGCAGG | 919 | CCTGCTGA GGCTAGCTACAACGA ATCAGACA | 2621 |
| 4112 | UGAUGUCA G CAGGCCCA | 920 | TGGGCCTG GGCTAGCTACAACGA TGACATCA | 2622 |
| 4116 | GUCAGCAG G CCCAGUUU | 921 | AAACTGGG GGCTAGCTACAACGA CTGCTGAC | 2623 |
| 4121 | CAGGCCCA G UUUCUGCC | 922 | GGCAGAAA GGCTAGCTACAACGA TGGGCCTG | 2624 |
| 4127 | CAGUUUCU G CCAUUCCA | 923 | TGGAATGG GGCTAGCTACAACGA AGAAACTG | 2625 |
| 4130 | UUUCUGCC A UUCCAGCU | 924 | AGCTGGAA GGCTAGCTACAACGA GGCAGAAA | 2626 |
| 4136 | CCAUUCCA G CUGUGGGC | 925 | GCCCACAG GGCTAGCTACAACGA TGGAATGG | 2627 |
| 4139 | UUCCAGCU G UGGGCACG | 926 | CGTGCCCA GGCTAGCTACAACGA AGCTGGAA | 2628 |
| 4143 | AGCUGUGG G CACGUCAG | 927 | CTGACGTG GGCTAGCTACAACGA CCACAGCT | 2629 |
| 4145 | CUGUGGGC A CGUCAGCG | 928 | CGCTGACG GGCTAGCTACAACGA GCCCACAG | 2630 |
| 4147 | GUGGGCAC G UCAGCGAA | 929 | TTCGCTGA GGCTAGCTACAACGA GTGCCCAC | 2631 |
| 4151 | GCACGUCA G CGAAGGCA | 930 | TGCCTTCG GGCTAGCTACAACGA TGACGTGC | 2632 |
| 4157 | CAGCGAAG G CAAGCGCA | 931 | TGCGCTTG GGCTAGCTACAACGA CTTCGCTG | 2633 |
| 4161 | GAAGGCAA G CGCAGGUU | 932 | AACCTGCG GGCTAGCTACAACGA TTGCCTTC | 2634 |
| 4163 | AGGCAAGC G CAGGUUCA | 933 | TGAACCTG GGCTAGCTACAACGA GCTTGCCT | 2635 |
| 4167 | AAGCGCAG G UUCACCUA | 934 | TAGGTGAA GGCTAGCTACAACGA CTGCGCTT | 2636 |
| 4171 | GCAGGUUC A CCUACGAC | 935 | GTCGTAGG GGCTAGCTACAACGA GAACCTGC | 2637 |
| 4175 | GUUCACCU A CGACCACG | 936 | CGTGGTCG GGCTAGCTACAACGA AGGTGAAC | 2638 |
| 4178 | CACCUACG A CCACGCUG | 937 | CAGCGTGG GGCTAGCTACAACGA CGTAGGTG | 2639 |
| 4181 | CUACGACC A CGCUGAGC | 938 | GCTCAGCG GGCTAGCTACAACGA GGTCGTAG | 2640 |
| 4183 | ACGACCAC G CUGAGCUG | 939 | CAGCTCAG GGCTAGCTACAACGA GTGGTCGT | 2641 |
| 4188 | CACGCUGA G CUGGAAAG | 940 | CTTTCCAG GGCTAGCTACAACGA TCAGCGTG | 2642 |
| 4201 | AAAGGAAA A UCGCGUGC | 941 | GCACGCGA GGCTAGCTACAACGA TTTCCTTT | 2643 |
| 4204 | GGAAAAUC G CGUGCUGC | 942 | GCAGCACG GGCTAGCTACAACGA GATTTTCC. | 2644 |
| 4206 | AAAAUCGC G UGCUGCUC | 943 | GAGCAGCA GGCTAGCTACAACGA GCGATTTT | 2645 |
| 4208 | AAUCGGGUT G CUGCUCCC | 944 | GGGAGCAG GGCTAGCTACAACGA ACGCGATT | 2646 |
| 4211 | CGCGUGCU G CUCCCCGC | 945 | GCGGGGAG GGCTAGCTACAACGA AGCACGCG | 2647 |
| 4218 | UGCUCCCC G CCCCCAGA | 946 | TCTGGGGG GGCTAGCTACAACGA GGGGAGCA | 2648 |
| 4226 | GCCCCCAG A CUACAACU | 947 | AGTTGTAG GGCTAGCTACAACGA CTGGGGGC | 2649 |
| 4229 | CCCAGACU A CAACUCGG | 948 | CCGAGTTG GGCTAGCTACAACGA AGTCTGGG | 2650 |
| 4232 | AGACUACA A CUCGGUGG | 949 | CCACCGAG GGCTAGCTACAACGA TGTAGTCT | 2651 |
| 4237 | ACAACUCG G UGGUCCUG | 950 | CAGGACCA GGCTAGCTACAACGA CGAGTTGT | 2652 |
| 4240 | ACUCGGUG G UCCUGUAC | 951 | GTACAGGA GGCTAGCTACAACGA CACCGAGT | 2653 |
| 4245 | GUGGUCCU G UACUCCAC | 952 | GTGGAGTA GGCTAGCTACAACGA AGGACCAC | 2654 |
| 4247 | GGUCCUGU A CUCCACCC | 953 | GGGTGGAG GGCTAGCTACAACGA ACAGGACC | 2655 |
| 4252 | UGUACUCC A CCCCACCC | 954 | GGGTGGGG GGCTAGCTACAACGA GGAGTACA | 2656 |
| 4257 | UCCACCCC A CCCAUCUA | 955 | TAGATGGG GGCTAGCTACAACGA GGGGTGGA | 2657 |
| 4261 | CCCCACCC A UCUAGAGU | 956 | ACTCTAGA GGCTAGCTACAACGA GGGTGGGG | 2658 |
| 4268 | CAUCUAGA G UUUGACAC | 957 | GTGTCAAA GGCTAGCTACAACGA TCTAGATG | 2659 |
| 4273 | AGAGUUUG A CACGAAGC | 958 | GCTTCGTG GGCTAGCTACAACGA CAAACTCT | 2660 |
| 4275 | AGUUUGAC A CGAAGCCU | 959 | AGGCTTCG GGCTAGCTACAACGA GTCAAACT | 2661 |
| 4280 | GACACGAA G CCUUAUUU | 960 | AAATAAGG GGCTAGCTACAACGA TTCGTGTC | 2662 |
| 4285 | GAAGCCUU A UUUCUAGA | 961 | TCTAGAAA GGCTAGCTACAACGA AAGGCTTC | 2663 |
| 4295 | UUCUAGAA G CACAUGUG | 962 | CACATGTG GGCTAGCTACAACGA TTCTAGAA | 2664 |
| 4297 | CUAGAAGC A CAUGUGUA | 963 | TACACATG GGCTAGCTACAACGA GCTTCTAG | 2665 |
| 4299 | AGAAGCAC A UGUGUAUU | 964 | AATACACA GGCTAGCTACAACGA GTGCTTCT | 2666 |
| 4301 | AAGCACAU G UGUAUUUA | 965 | TAAATACA GGCTABCTACAACGA ATGTGCTT | 2667 |
| 4303 | GCACAUGU G UAUUUAUA | 966 | TATAAATA GGCTAGCTACAACGA ACATGTGC | 2668 |
| 4305 | ACAUGUGU A UUUAUACC | 967 | GGTATAAA GGCTAGCTACAACGA ACACATGT | 2669 |
| 4309 | GUGUAUUU A UACCCCCA | 968 | TGGGGGTA GGCTAGCTACAACGA AAATACAC | 2670 |
| 4311 | GUAUUUAU A CCCCCAGG | 969 | CCTGGGGG GGCTAGCTACPACGA ATAAATAC | 2671 |
| 4322 | CCCAGGAA A CUAGCUUU | 970 | AAAGCTAG GGCTAGCTACAACGA TTCCTGGG | 2672 |
| 4326 | GGAAACUA G CUUUUGCC | 971 | GGCAAAAG GGCTAGCTACAACGA TAGTTTCC | 2673 |
| 4332 | UAGCUUUU G CCAGUAUU | 972 | AATACTGG GGCTAGCTACAACGA AAAAGCTA | 2674 |
| 4336 | UUUUGCCA G UAUUAUGC | 973 | GCATAATA GGCTAGCTACAACGA TGGCAAAA | 2675 |
| 4338 | UUGCCAGU A UUAUGCAU | 974 | ATGCATAA GGCTAGCTACAACGA ACTGGCAA | 2676 |
| 4341 | CCAGUAUU A UGCAUAUA | 975 | TATATGCA GGCTAGCTACAACGA AATACTGG | 2677 |
| 4343 | AGUAUUAU G CAUAUAUA | 976 | TATATATG GGCTAGCTACAACGA ATAATACT | 2678 |
| 4345 | UAUUAUGC A UAUAUAAG | 977 | CTTATATA GGCTAGCTACAACGA GCATAATA | 2679 |
| 4347 | UUAUGCAU A UAUAAGUU | 978 | AACTTATA GGCTAGCTACAACGA ATGCATAA | 2680 |
| 4349 | AUGCAUAU A UAAGUUUA | 979 | TAAACTTA GGCTAGCTACAACGA ATATGCAT | 2681 |
| 4353 | AUAUAUAA G UUUACACC | 980 | GGTGTAAA GGCTAGCTACAACGA TTATATAT | 2682 |
| 4357 | AUAAGUUU A CACCUUUA | 981 | TAAAGGTG GGCTAGCTACAACGA AAACTTAT | 2683 |
| 4359 | AAGUUUAC A CCUUUAUC | 982 | GATAAAGG GGCTAGCTACAACGA GTAAACTT | 2684 |
| 4365 | ACACCUUU A UCUUUCCA | 983 | TGGAAAGA GGCTAGCTACAACGA AAAGGTGT | 2685 |
| 4373 | AUCUUUCC A UGGGAGCC | 984 | GGCTCCCA GGCTAGCTACAACGA GGAAAGAT | 2686 |
| 4379 | CCAUGGGA G CCAGCUGC | 985 | GCAGCTGG GGCTAGCTACAACGA TCCCATGG | 2687 |
| 4383 | GGGAGCCA G CUGCUUUU | 986 | AAAAGCAG GGCTAGCTACAACGA TGGCTCCC | 2688 |
| 4386 | AGCCAGCU G CUUUUUGU | 987 | ACAAAAAG GGCTAGCTACAACGA AGCTGGCT | 2689 |
| 4393 | UGCUUUUU G UGAUUUUU | 988 | AAAAATCA GGCTAGCTACAACGA AAAAAGCA | 2690 |
| 4396 | UUUUUGUG A UUUUUUUA | 989 | TAAAAAAA GGCTAGCTACAACGA CACAAAAA | 2691 |
| 4405 | UUUUUUUA A UAGUGCW | 990 | AAGCACTA GGCTAGCTACAACGA TAAAAAAA | 2692 |
| 4408 | UUUUAAUA G UGCUUUUU | 991 | AAAAAGCA GGCTAGCTACAACGA TATTAAAA | 2693 |
| 4410 | UUAAUAGU G CUUUUUUU | 992 | AAAAAAAG GGCTAGCTACAACGA ACTATTAA | 2694 |
| 4424 | UUUUUUUG A CUAACAAG | 993 | CTTGTTAG GGCTAGCTACAACGA CAAAAAAA | 2695 |
| 4428 | UUUGACUA A CAAGAAUG | 994 | CATTCTTG GGCTAGCTACAACGA TAGTCAAA | 2696 |
| 4434 | UAACAAGA A UGUAACUC | 995 | GAGTTACA GGCTAGCTACAACGA TCTTGTTA | 2697 |
| 4436 | ACAAGAAU G UAACUCCA | 916 | TGGAGTTA GGCTAGCTACAACGA ATTCTTGT | 2698 |
| 4439 | AGAAUGUA A CUCCAGAU | 997 | ATCTGGAG GGCTAGCTACAACGA TACATTCT | 2699 |
| 4446 | AACUCCAG A UAGAGAAA | 998 | TTTCTCTA GGCTAGCTACAACGA CTGGAGTT | 2700 |
| 4454 | AUAGAGAA A UAGUGACA | 999 | TGTCACTA GGCTAGCTACAACGA TTCTCTAT | 2701 |
| 4457 | GAGAAAUA G UGACAAGU | 1000 | ACTTGTCA GGCTAGCTACAACGA TATTTCTC | 2702 |
| 4460 | AAAUAGUG A CAAGUGAA | 1001 | TTCACTTG GGCTAGCTACAACGA CACTATTT | 2703 |
| 4464 | AGUGACAA G UGAAGAAC | 1002 | GTTCTTCA GGCTAGCTACAACGA TTGTCACT | 2704 |
| 4471 | AGUGAAGA A CACUACUG | 1003 | CAGTAGTG GGCTAGCTACAACGA TCTTCACT | 2705 |
| 4473 | UGAAGAAC A CUACUGCU | 1004 | AGCAGTAG GGCTAGCTACAACGA GTTCTTCA | 2706 |
| 4476 | AGAACACU A CUGCUAAA | 1005 | TTTAGCAG GGCTAGCTACAACGA AGTGTTCT | 2707 |
| 4479 | ACACUACU G CUAAAUCC | 1006 | GGATTTAG GGCTAGCTACAACGA AGTAGTGT | 2708 |
| 4484 | ACUGCUAA A UCCUCAUG | 1007 | CATGAGGA GGCTAGCTACAACGA TTAGCAGT | 2709 |
| 4490 | AAAUCCUC A UGUUACUC | 1008 | GAGTAACA GGCTAGCTACAACGA GAGGATTT | 2710 |
| 4492 | AUCCUCAU G UUACUCAG | 1009 | CTGAGTAA GGCTAGCTACAACGA ATGAGGAT | 2711 |
| 4495 | CUCAUGUU A CUCAGUGU | 1010 | ACACTGAG GGCTAGCTACAACGA AACATGAG | 2712 |
| 4500 | GUUACUCA G UGUUAGAG | 1011 | CTCTAACA GGCTAGCTACAACGA TGAGTAAC | 2713 |
| 4502 | UACUCAGU G WAGAGAA | 1012 | TTACTCTAA GGCTAGCTACAACGA ACTGAGTA | 2714 |
| 4511 | UUAGAGAA A UCCUUCCU | 1013 | AGGAAGGA GGCTAGCTACAACGA TTCTCTAA | 2715 |
| 4522 | CUUCCUAA A CCCAAUGA | 1014 | TCATTGGG GGCTAGCTACAACGA TTAGGAAG | 2716 |
| 4527 | UAAACCCA A UGACUUCC | 1015 | GGAAGTCA GGCTAGCTACAACGA TGGGTTTA | 2717 |
| 4530 | ACCCAAUG A CUUCCCUG | 1016 | CAGGGAAG GGCTAGCTACAACGA CATTGGGT | 2718 |
| 4538 | ACUUCCCU G CUCCAACC | 1017 | GGTTGGAG GGCTAGCTACAACGA AGGGAAGT | 2719 |
| 4544 | CUGCUCCA A CCCCCGCC | 1018 | GGCGGGGG GGCTAGCTACAACGA TGGAGCAG | 2720 |
| 4550 | CAACCCCC G CCACCUCA | 1019 | TGAGGTGG GGCTAGCTACAACGA GGGGGTTG | 2721 |
| 4553 | CCCCCGCC A CCUCAGGG | 1020 | CCCTGAGG GGCTAGCTACAACGA GGCGGGGG | 2722 |
| 4561 | ACCUCAGG G CACGCAGG | 1021 | CCTGCGTG GGCTAGCTACAACGA CCTGAGGT | 2723 |
| 4563 | CUCAGGGC A CGCAGGAC | 1022 | GTCCTGCG GGCTAGCTACAACGA GCCCTGAG | 2724 |
| 4565 | CAGGGCAC G CAGGACCA | 1023 | TGGTCCTG GGCTAGCTACAACGA GTGCCCTG | 2725 |
| 4570 | CACGCAGG A CCAGUUUG | 1024 | CAAACTGG GGCTAGCTACAACGA CCTGCGTG | 2726 |
| 4574 | CAGGACCA G UUUGAUUG | 1025 | CAATCAAA GGCTAGCTACAACGA TGGTCCTG | 2727 |
| 4579 | CCAGUUUG A UUGAGGAG | 1026 | CTCCTCAA GGCTAGCTACAACGA CAAACTGG | 2728 |
| 4587 | AUUGAGGA G CUGCACUG | 1027 | CAGTGCAG GGCTAGCTACAACGA TCCTCAAT | 2729 |
| 4590 | GAGGAGCU G CACUGAUC | 1028 | GATCAGTG GGCTAGCTACAACGA AGCTCCTC | 2730 |
| 4592 | GGAGCUGC A CUGAUCAC | 1029 | GTGATCAG GGCTAGCTACAACGA GCAGCTCC | 2731 |
| 4596 | CUGCACUG A UCACCCAA | 1030 | TTGGGTGA GGCTAGCTACAACGA CAGTGCAG | 2732 |
| 4599 | CACUGAUC A CCCAAUGC | 1031 | GCATTGGG GGCTAGCTACAACGA GATCAGTG | 2733 |
| 4604 | AUCACCCA A UGCAUCAC | 1032 | GTGATGCA GGCTAGCTACAACGA TGGGTGAT | 2734 |
| 4606 | CACCCAAU G CAUCACGU | 1033 | ACGTGATG GGCTAGCTACAACGA ATTGGGTG | 2735 |
| 4608 | CCCAAUGC A UCACGUAC | 1034 | GTACGTGA GGCTAGCTACAACGA GCATTGGG | 2736 |
| 4611 | AAUGCAUC A CGUACCCC | 1035 | GGGGTACG GGCTAGCTACAACGA GATGCATT | 2737 |
| 4613 | UGCAUCAC G UACCCCAC | 1036 | GTGGGGTA GGCTAGCTACAACGA GTGATGCA | 2738 |
| 4615 | CAUCACGU A CCCCACUG | 1037 | CAGTGGGG GGCTAGCTACAACGA ACGTGATG | 2739 |
| 4620 | CGUACCCC A CUGGGCCA | 1038 | TGGCCCAG GGCTAGCTACAACGA GGGGTACG | 2740 |
| 4625 | CCCACUGG G CCAGCCCU | 1039 | AGGGCTGG GGCTAGCTACAACGA CCAGTGGG | 2741 |
| 4629 | CUGGGCCA G CCCUGCAG | 1040 | CTGCAGGG GGCTAGCTACAACGA TGGCCCAG | 2742 |
| 4634 | CCAGCCCU G CAGCCCAA | 1041 | TTGGGCTG GGCTAGCTACAACGA AGGGCTGG | 2743 |
| 4637 | GCCCUGCA G CCCAAAAC | 1042 | GTTTTGGG GGCTAGCTACAACGA TGCAGGGC | 2744 |
| 4644 | AGCCCAAA A CCCAGGGC | 1043 | GCCCTGGG GGCTAGCTACAACGA TTTGGGCT | 2745 |
| 4651 | AACCCAGG G CAACAAGC | 1044 | GCTTGTTG GGCTAGCTACAACGA CCTGGGTT | 2746 |
| 4654 | CCAGGGCA A CAAGCCCG | 1045 | CGGGCTTG GGCTAGCTACAACGA TGCCCTGG | 2747 |
| 4658 | GGCAACAA G CCCGUUAG | 1046 | CTAACGGG GGCTAGCTACAACGA TTGTTGCC | 2748 |
| 4662 | ACAAGCCC G UUAGCCCC | 1047 | GGGGCTAA GGCTAGCTACAACGA GGGCTTGT | 2749 |
| 4666 | GCCCGUUA G CCCCAGGG | 1048 | CCCTGGGG GGCTAGCTACAACGA TAACGGGC | 2750 |
| 4676 | CCCAGGGG A UCACUGGC | 1049 | GCCAGTGA GGCTAGCTACAACGA CCCCTGGG | 2751 |
| 4679 | AGGGGAUC A CUGGCUGG | 1050 | CCAGCCAG GGCTAGCTACAACGA GATCCCCT | 2752 |
| 4683 | GAUCACUG G CUGGCCUG | 1051 | CAGGCCAG GGCTAGCTACAACGA CAGTGATC | 2753 |
| 4687 | ACUGGCUG G CCUGAGCA | 1052 | TGCTCAGG GGCTAGCTACAACGA CAGCCAGT | 2754 |
| 4693 | UGGCCUGA G CAACAUCU | 1053 | AGATGTTG GGCTAGCTACAACGA TCAGGCCA | 2755 |
| 4696 | CCUGAGCA A CAUCUCGG | 1054 | CCGAGATG GGCTAGCTACAACGA TGCTCAGG | 2756 |
| 4698 | UGAGCAAC A UCUCGGGA | 1055 | TCCCGAGA GGCTAGCTACAACGA GTTGCTCA | 2757 |
| 4707 | UCUCGGGA G UCCUCUAG | 1056 | CTAGAGGA GGCTAGCTACAACGA TCCCGAGA | 2758 |
| 4715 | GUCCUCUA G CAGGCCUA | 1057 | TAGGCCTG GGCTAGCTACAACGA TAGAGGAC | 2759 |
| 4719 | UCUAGCAG G CCUAAGAC | 1058 | GTCTTAGG GGCTAGCTACAACGA CTGCTAGA | 2760 |
| 4726 | GGCCUAAG A CAUGUGAG | 1059 | CTCACATG GGCTAGCTACAACGA CTTAGGCC | 2761 |
| 4728 | CCUAAGAC A UGUGAGGA | 1060 | TCCTCACA GGCTAGCTACAACGA GTCTTAGG | 2762 |
| 4730 | UAAGACAU G UGAGGAGG | 1061 | CCTCCTCA GGCTAGCTACAACGA ATGTCTTA | 2763 |
| 4752 | GAAAAAAA G CAAAAAGC | 1062 | GCTTTTTG GGCTAGCTACAACGA TTTTTTTC | 2764 |
| 4759 | AGCAAAAA G CAAGGGAG | 1063 | CTCCCTTG GGCTAGCTACAACGA TTTTTGCT | 2765 |
| 4777 | AAAGAGAA A CCGGGAGA | 1064 | TCTCCCGG GGCTAGCTACAACGA TTCTCTTT | 2766 |
| 4788 | GGGAGAAG G CAUGAGAA | 1065 | TTCTCATG GGCTAGCTACAACGA CTTCTCCC | 2767 |
| 4790 | GAGAAGGC A UGAGAAAG | 1066 | CTTTCTCA GGCTAGCTACAACGA GCCTTCTC | 2768 |
| 4800 | GAGAAAGA A UUUGAGAC | 1067 | GTCTCAAA GGCTAGCTACAACGA TCTTTCTC | 2769 |
| 4807 | AAUUUGAG A CGCACCAU | 1068 | ATGGTGCG GGCTAGCTACAACGA CTCAAATT | 2770 |
| 4809 | UUUGAGAC G CACCAUGU | 1069 | ACATGGTG GGCTAGCTACAACGA STCTCAAA | 2771 |
| 4811 | UGAGACGC A CCAUGUGG | 1070 | CCACATGG GGCTAGCTACAACGA GCGTCTCA | 2772 |
| 4814 | GACGCACC A UGUGGGCA | 1071. | TGCCCACA GGCTAGCTACAACGA GGTGCGTC | 2773 |
| 4816 | CGCACCAU G UGGGCACG | 1072 | CGTGCCCA GGCTAGCTACAACGA ATGGTGCG | 2774 |
| 4820 | CCAUGUGG G CACGGAGG | 1073 | CCTCCGTG GGCTAGCTACAACGA CCACATGG | 2775 |
| 4822 | AUGUGGGC A CGGAGGGG | 1074 | CCCCTCCG GGCTAGCTACAACGA GCCCACAT | 2776 |
| 4832 | GGAGGGGG A CGGGGCUC | 1075 | GAGCCCCG GGCTAGCTACAACGA CCCCCTCC | 2777 |
| 4837 | GGGACGGG G CUCAGCAA | 1076 | TTGCTGAG GGCTAGCTACAACGA CCCGTCCC | 2778 |
| 4842 | GGGGCUCA G CAAUGCCA | 1077 | TGGCATTG GGCTAGCTACAACGA TGAGCCCC | 2779 |
| 4845 | GCUCAGCA A UGCCAUUU | 1078 | AAATGGCA GGCTAGCTACAACGA TGCTGAGC | 2780 |
| 4847 | UCAGCAAU G CCAUUUCA | 1079 | TGAAATGG GGCTAGCTACAACGA ATTGCTGA | 2781 |
| 4850 | GCAAUGCC A UUUCAGUG | 1080 | CACTGAAA GGCTAGCTACAACGA GGCATTGC | 2782 |
| 4856 | CCAUUUCA G UGGCUUCC | 1081 | GGAAGCCA GGCTAGCTACAACGA TGAAATGG | 2783 |
| 4859 | UUUCAGUG G CUUCCCAG | 1082 | CTGGGAAG GGCTAGCTACAACGA CACTGAAA | 2784 |
| 4867 | GCUUCCCA G CUCUGACC | 1083 | GGTCAGAG GGCTAGCTACAACGA TGGGAAGC | 2785 |
| 4873 | CAGCUCUG A CCCUUCUA | 1084 | TAGAAGGG GGCTAGCTACAACGA CAGAGCTG | 2786 |
| 4881 | ACCCUUCU A CAUUUGAG | 1085 | CTCAAATG GGCTAGCTACAACGA AGAAGGGT | 2787 |
| 4883 | CCUUCUAC A UUUGAGGG | 1086 | CCCTCAAA GGCTAGCTACAACGA GTAGAAGG | 2788 |
| 4891 | AUUUGAGG G CCCAGCCA | 1087 | TGGCTGGG GGCTAGCTACAACGA CCTCAAAT | 2789 |
| 4896 | AGGGCCCA G CCAGGAGC | 1088 | GCTCCTGG GGCTAGCTACAACGA TGGGCCCT | 2790 |
| 4903 | AGCCAGGA G CAGAUGGA | 1089 | TCCATCTG GGCTAGCTACAACGA TCCTGGCT | 2791 |
| 4907 | AGGAGCAG A UGGACAGC | 1090 | GCTGTCCA GGCTAGCTACAACGA CTGCTCCT | 2792 |
| 4911 | GCAGAUGG A CAGCGAUG | 1091 | CATCGCTG GGCTAGCTACAACGA CCATCTGC | 2793 |
| 4914 | GAUGGACA G CGAUGAGG | 1092 | CCTCATCG GGCTAGCTACAACGA TGTCCATC | 2794 |
| 4917 | GGACAGCG A UGAGGGGA | 1093 | TCCCCTCA GGCTAGCTACAACGA CGCTGTCC | 2795 |
| 4925 | AUGAGGGG A CAUUUUCU | 1094 | AGAAAATG GGCTAGCTACAACGA CCCCTCAT | 2796 |
| 4927 | GAGGGGAC A UUUUCUGB | 1095 | CCAGAAAA GGCTAGCTACAACGA GTCCCCTC | 2797 |
| 4936 | UUUUCUGG A UUCUGGGA | 1096 | TCCCAGAA GGCTAGCTACAACGA CCAGAAAA | 2798 |
| 4946 | UCUGGGAG G CAAGAAAA | 1097 | TTTTCTTG GGCTAGCTACAACGA CTCCCAGA | 2799 |
| 4957 | AGAAAAGG A CAAAUAUC | 1098 | GATATTTG GGCTAGCTACAACGA CCTTTTCT | 2800 |
| 4961 | AAGGACAA A UAUCUUUU | 1099 | AAAAGATA GGCTAGCTACAACGA TTGTCCTT | 2801 |
| 4963 | GGACAAAU A UCUUUUUU | 1100 | AAAAAAGA GGCTAGCTACAACGA ATTTGTCC | 2802 |
| 4975 | UUUUUGGA A CUAAAGCA | 1101 | TGCTTTAG GGCTAGCTACAACGA TCCAAAAA | 2803 |
| 4981 | GAACUAAA G CAAAUUUU | 1102 | AAAATTTG GGCTAGCTACAACGA TTTAGTTC | 2804 |
| 4985 | UAAAGCAA A UUUUAGAC | 1103 | GTCTAAAA GGCTAGCTACAACGA TTGCTTTA | 2805 |
| 4992 | AAUUUUAG A CCUUUACC | 1104 | GGTAAAGG GGCTAGCTACAACGA CTAAAATT | 2806 |
| 4998 | AGACCUUU A CCUAUGGA | 1105 | TCCATAGG GGCTAGCTACAACGA AAAGGTCT | 2807 |
| 5002 | CUUUACCU A UGGAAGUG | 1106 | CACTTCCA GGCTAGCTACAACGA AGGTAAAG | 2808 |
| 5008 | CUAUGGAA G UGGUUCUA | 1107 | TAGAACCA GGCTAGCTACAACGA TTCCATAG | 2809 |
| 5011 | UGGAAGUG G UUCUAUGU | 1108 | ACATAGAA GGCTAGCTACAACGA CACTTCCA | 2810 |
| 5016 | GUGGUUCU A UGUCCAUU | 1109 | AATGGACA GGCTAGCTACAACGA AGAACCAC | 2811 |
| 5018 | GGUUCUAU G UCCAUUCU | 1110 | AGAATGGA GGCTAGCTACAACGA ATAGAACC | 2812 |
| 5022 | CUAUGUCC A UUCUCAUU | 1111 | AATGAGAA GGCTAGCTACAACGA GGACATAG | 2813 |
| 5028 | CCAUUCUC A UUCGUGGC | 1112 | GCCACGAA GGCTAGCTACAACGA GAGAATGG | 2814 |
| 5032 | UCUCAUUC G UGGCAUGU | 1113 | ACATGCCA GGCTAGCTACAACGA GAATGAGA | 2815 |
| 5035 | CAUUCGUG G CAUGUUUU | 1114 | AAAACATG GGCTAGCTACAACGA CACGAATG | 2816 |
| 5037 | UUCGUGGC A UGUUUUGA | 1115 | TCAAAACA GGCTAGCTACAACGA GCCACGAA | 2817 |
| 5039 | CGUGGCAU G UUUUGAUU | 1116 | AATCAAAA GGCTAGCTACAACGA ATGCCACG | 2818 |
| 5045 | AUGUUUUG A UUUGUAGC | 1117 | GCTACAAA GGCTAGCTACAACGA CAAAACAT | 2819 |
| 5049 | UUUGAUUU G UAGCACUG | 1118 | CAGTGCTA GGCTAGCTACAACGA AAATCAAA | 2820 |
| 5052 | GAUUUGUA G CACUGAGG | 1119 | CCTCAGTG GGCTAGCTACAACGA TACAAATC | 2821 |
| 5054 | UUUGUAGC A CUGAGGGU | 1120 | ACCCTCAG GGCTAGCTACAACGA GCTACAAA | 2822 |
| 5061 | CACUGAGG G UGGCACUC | 1121 | GACTGCCA GGCTAGCTACAACGA CCTCAGTG | 2823 |
| 5064 | UGAGGGUG G CACUCAAC | 1122 | GTTGAGTG GGCTAGCTACAACGA CACCCTCA | 2824 |
| 5066 | AGGGUGGC A CUCAACUC | 1123 | GAGTTGAG GGCTAGCTACAACGA GCCACCCT | 2825 |
| 5071 | GGCACUCA A CUCUGAGC | 1124 | GCTCAGAG GGCTAGCTACAACGA TGAGTGCC | 2826 |
| 5078 | AACUCUGA G CCCAUACU | 1125 | AGTATGGG GGCTAGCTACAACGA TCAGAGTT | 2827 |
| 5082 | CUGAGCCC A UACUUUUG | 1126 | CAAAAGTA GGCTAGCTACAACGA GGGCTCAG | 2828 |
| 5084 | GAGCCCAU A CUUUUGBC | 1127 | GCCAAAAG GGCTAGGTACAACGA ATGGGCTC | 2829 |
| 5091 | UACUUUUG G CUCCUCUA | 1128 | TAGAGGAG GGCTAGCTACAACGA CAAAAGTA | 2830 |
| 5100 | CUCCUCUA G UAAGAUGC | 1129 | GCATCTTA GGCTAGCTACAACGA TAGAGGAG | 2831 |
| 5105 | CUAGUAAG A UGCACUGA | 1130 | TCAGTGCA GGCTAGCTACAACGA CTTACTAG | 2832 |
| 5107 | AGUAAGAU G CACUGAAA | 1131 | TTTCAGTG GGCTAGCTACAACGA ATCTTACT | 2833 |
| 5109 | UAAGAUGC A CUGAAAAC | 1132 | GTTTTCAG GGCTAGCTACAACGA GCATCTTA | 2834 |
| 5116 | CACUGAAA A CUUAGCCA | 1133 | TGGCTAAG GGCTAGCTACAACGA TTTCAGTG | 2835 |
| 5121 | AAAACUUA G CCAGAGUU | 1134 | AACTCTGG GGCTAGCTACAACGA TAAGTTTT | 2836 |
| 5127 | UAGCCAGA G UUAGGUUG | 1135 | CAACCTAA GGCTAGCTACAACGA TCTGGCTA | 2837 |
| 5132 | AGAGUUAG G UUGUCUCC | 1136 | GGAGACAA GGCTAGCTACAACGA CTAACTCT | 2838 |
| 5135 | GUUAGGUU G UCUCCAGG | 1137 | CCTGGAGA GGCTAGCTACAACGA AACCTAAC | 2839 |
| 5143 | GUCUCCAG G CCAUGAUG | 1138 | CATCATGG GGCTAGCTACAACGA CTGGAGAC | 2840 |
| 5146 | UCCAGGCC A UGAUGGCC | 1139 | GGCCATCA GGCTAGCTACAACGA GGCCTGGA | 2841 |
| 5149 | AGGCCAUG A UGGCCUUA | 1140 | TAAGGCCA GGCTAGCTACAACGA CATGGCCT | 2842 |
| 5152 | CCAUGAUG G CCUUACAC | 1141 | GTGTAAGG GGCTAGCTACAACGA CATCATGG | 2843 |
| 5157 | AUGGCCUU A CACUGAAA | 1142 | TTTCAGTG GGCTAGCTACAACGA AAGGCCAT | 2844 |
| 5159 | GGCCUUAC A CUGAAAAU | 1143 | ATTTTCAG GGCTAGCTACAACGA GTAAGGCC | 2845 |
| 5166 | CACUGAAA A UGUCACAU | 1144 | ATGTGACA GGCTAGCTACAACGA TTTCAGTG | 2846 |
| 5168 | CUGAAAAU G UCACAUUC | 1145 | GAATGTGA GGCTAGCTACAACGA ATTTTCAG | 2847 |
| 5171 | AAAAUGUC A CAUUCUAU | 1146 | ATAGAATG GGCTAGCTACAACGA GACATTTT | 2848 |
| 5173 | AAUGUCAC A UUCUAUUU | 1147 | AAATAGAA GGCTAGCTACAACGA GTGACATT | 2849 |
| 5178 | CACAUUCU A UUUUGGGU | 1148 | ACCCAAAA GGCTAGCTACAACGA AGAATGTG | 2850 |
| 5185 | UAUUUUGG G UAUUAAUA | 1149 | TATTAATA GGCTAGCTACAACGA CCAAAATA | 2851 |
| 5187 | UUUUGGGU A UUAAUAUA | 1150 | TATATTAA GGCTAGCTACAACGA ACCCAAAA | 2852 |
| 5191 | GGGUAUUA A UAUAUAGU | 1151 | ACTATATA GGCTAGCTACAACGA TAATACCC | 2853 |
| 5193 | GUAUUAAU A UAUAGUCC | 1152 | GGACTATA GGCTAGCTACAACGA ATTAATAC | 2854 |
| 5195 | AUUAAUAU A UAGUCCAG | 1153 | CTGGACTA GGCTAGCTACAACGA ATATTAAT | 2855 |
| 5198 | AAUAUAUA G UCCAGACA | 1154 | TGTCTGGA GGCTAGCTACAACGA TATATATT | 2856 |
| 5204 | UAGUCCAG A CACUUAAC | 1155 | GTTAAGTG GGCTAGCTACAACGA CTGGACTA | 2857 |
| 5206 | GUCCAGAC A CUUAACUC | 1156 | GAGTTAAG GGCTAGCTACAACGA GTCTGGAC | 2858 |
| 5211 | GACACUUA A CUCAAUUU | 1157 | AAATTGAG GGCTAGCTACAACGA TAAGTGTC | 2859 |
| 5216. | UUAACUCA A UUUCULTGG | 1158 | CCAAGAAA GGCTAGCTACAACGA TGAGTTAA | 2860 |
| 5224 | AUUUCUUG G UAUUAUUC | 1159 | GAATAATA GGCTAGCTACAACGA CAAGAAAT | 2861 |
| 5226 | UUCUUGGU A UUAUUCUG | 1160 | CAGAATAA GGCTAGCTACAACGA ACCAAGAA | 2862 |
| 5229 | UUGGUAUU A UUCUGUUU | 1161 | AAACAGAA GGCTAGCTACAACGA AATACCAA | 2863 |
| 5234 | AUUAUUCU G UUUUGCAC | 1162 | GTGCAAAA GGCTAGCTACAACGA AGAATAAT | 2864 |
| 5239 | UCUGUUUU G CACAGUUA | 1163 | TAACTGTG GGCTAGCTACAACGA AAAACAGA | 2865 |
| 5241 | UGUUUUGC A CAGUUAGU | 1164 | ACTAACTG GGCTAGCTACAACGA GCAAAACA | 2866 |
| 5244 | UUUGCACA G UUAGUUGU | 1165 | ACAACTAA GGCTAGCTACAACGA TGTGCAAA | 2867 |
| 5248 | CACAGUUA G UUGUGAAA | 1166 | TTTCACAA GGCTAGCTACAACGA TAACTGTG | 2868 |
| 5251 | AGUUAGUU G UGAAAGAA | 1167 | TTCTTTCA GGCTAGCTACAACGA AACTAACT | 2869 |
| 5261 | GAAAGAAA G CUGAGAAG | 1168 | CTTCTCAG GGCTAGCTACAACGA TTTCTTTC | 2870 |
| 5271 | UGAGAAGA A UGAAAAUG | 1169 | CATTTTCA GGCTAGCTACAAGGA TCTTCTCA | 2871 |
| 5277 | GAAUGAAA A UGCAGUCC | 1170 | GGACTGCA GGCTAGCTACAACGA TTTCATTC | 2872 |
| 5279 | AUGAAAAU G CAGUCCUG | 1171 | CAGGACTG GGCTAGCTACAACGA ATTTTCAT | 2873 |
| 5282 | AAAAUGCA G UCCUGAGG | 1172 | CCTCAGGA GGCTAGCTACAACGA TGCATTTT | 2874 |
| 5294 | UGAGGAGA G UUUUCUCC | 1173 | GGAGAAAA GGCTAGCTACAACGA TCTCCTGA | 2875 |
| 5303 | UUUUCUCC A UAUCAAAA | 1174 | TTTTGATA GGCTAGCTACAACGA GGAGAAA | 2876 |
| 5305 | UUCUCCAU A UCAAAACG | 1175 | CGTTTTGA GGCTAGCTACAACGA ATGGAGAA | 2877 |
| 5311 | AUAUCAAA A CGAGGGCU | 1176 | AGCCCTCG GGCTAGCTACAACGA TTTGATAT | 2878 |
| 5317 | AAACGAGG G CUGAUGGA | 1177 | TCCATCAG GGCTAGCTACAACGA CCTCGTTT | 2879 |
| 5321 | GAGGGCUG A UGGAGGAA | 1178 | TTCCTCCA GGCTAGCTACAACGA CAGCCCTC | 2880 |
| 5334 | GGAAAAAG G UCAAUAAG | 1179 | CTTATTGA GGCTAGCTACAACGA CTTTTTCC | 2881 |
| 5338 | AAAGGUCA A UAAGGUCA | 1180 | TGACCTTA GGCTAGCTACAACGA TGACCTTT | 2882 |
| 5343 | UCAAUAAG G UCAAGGGA | 1181 | TCCCTTGA GGCTAGCTACAACGA CTTATTGA | 2883 |
| 5354 | AAGGGAAG A CCCCGUCU | 1182 | AGACGGGG GGCTAGCTACAACGA CTTCCCTT | 2884 |
| 5359 | AAGACCCC G UCUCUAUA | 1183 | TATAGAGA GGCTAGCTACAACGA GGGGTCTT | 2885 |
| 5365 | CCGUCUCU A UACCAACC | 1184 | GGTTGGTA GGCTAGCTACAACGA AGAGACGG | 2886 |
| 5367 | GUCUCUAU A CCAACCAA | 1185 | TTGGTTGG GGCTAGCTACAACGA ATAGAGAC | 2887 |
| 5371 | CUAUACCA A CCAAACCA | 1186 | TGGTTTGG GGCTAGCTACAACGA TGGTATAG | 2888 |
| 5376 | CCAACCAA A CCAAUUCA | 1187 | TGAATTGG GGCTAGCTACAACGA TTGGTTGG | 2889 |
| 5380 | CCAAACCA A UUCACCAA | 1188 | TTGGTGAA GGCTAGCTACAACGA TGGTTTGG | 2890 |
| 5384 | ACCAAUUC A CCAACACA | 1189 | TGTGTTGG GGCTAGCTACAACGA GAATTGGT | 2891 |
| 5388 | AUUCACCA A CACAGUUG | 1190 | CAACTGTG GGCTAGCTACAACGA TGGTGAAT | 2892 |
| 5390 | UCACCAAC A CAGUUGGG | 1191 | CCCAACTG GGCTAGCTACAACGA GTTGGTGA | 2893 |
| 5393 | CCAACACA G UUGGGACC | 1192 | GGTCCCAA GGCTAGCTACAACGA TGTGTTGG | 2894 |
| 5399 | CAGUUGGG A CCCAAAAC | 1193 | GTTTTGGG GGCTAGCTACAACGA CCCAACTG | 2895 |
| 5406 | GACCCAAA A CACAGGAA | 1194 | TTCCTGTG GGCTAGCTACAACGA TTTGGGTC | 2896 |
| 5408 | CCCAAAAC A CAGGAAGU | 1195 | ACTTCCTG GGCTAGCTACAACGA GTTTTGGG | 2897 |
| 5415 | CACAGGAA G UCAGUCAC | 1196 | GTGACTGA GGCTAGCTACAACGA TTCCTGTG | 2898 |
| 5419 | GGAAGUCA G UCACGUUU | 1197 | AAACGTGA GGCTAGCTACAACGA TGACTTCC | 2899 |
| 5422 | AGUCAGUC A CGUUUCCU | 1198 | AGGAAACG GGCTAGCTACAACGA GACTGACT | 2900 |
| 5424 | UCAGUCAC G UUUCCUUU | 1199 | AAAGGAAA GGCTAGCTACAACGA GTGACTGA | 2901 |
| 5435 | UCCUUUUC A UUUAAUGG | 1200 | CCATTAAA GGCTAGCTACAACGA GAAAAGGA | 2902 |
| 5440 | UUCAUUUA A UGGGGAUU | 1201 | AATCCCCA GGCTAGCTACAACGA TAAATGAA | 2903 |
| 5446 | UAAUGGGG A UUCCACUA | 1202 | TAGTGGAA GGCTAGCTACAACGA CCCCATTA | 2904 |
| 5451 | GGGAUUCC A CUAUCUCA | 1203 | TGAGATAG GCTAGCTACAACGA GGAATCCC | 2905 |
| 5454 | AUUCCACU A UCUCACAC | 1204 | GTGTGAGA GGCTAGCTACAACGA AGTGGAAT. | 2906 |
| 5459 | ACUAUCUC A CACUAAUC | 1205 | GATTAGTG GGCTAGCTACAACGA GAGATAGT | 2907 |
| 5461 | UAUCUCAC A CUAAUCUG | 1206 | CAGATTAG GGCTAGCTACAACGA GTGAGATA | 2908 |
| 5465 | UCACACUA A UCUGAAAG | 1207 | CTTTCAGA GGCTAGCTACAACGA TAGTGTGA | 2909 |
| 5475 | CUGAAAGG A UGUGGAAG | 1208 | CTTCCACA GGCTAGCTACAACGA CCTTTCAG | 2910 |
| 5477 | GAAAGGAU G UGGAAGAG | 1209 | CTCTTCCA GGCTAGCTACAACGA ATCCTTTC | 2911 |
| 5485 | GUGGAAGA G CAUUAGCU | 1210 | AGCTAATG GGCTAGGTACA&CGA TCTTCCAC | 2912 |
| 5487 | GGAAGAGC A UUAGCUGG | 1211 | CCAGCTAA GGCTAGCTACAACGA GCTCTTCC | 2913 |
| 5491 | GAGCAUUA G CUGGCGCA | 1212 | TGCGCCAG GGCTAGCTACAACGA TAATGCTC | 2914 |
| 5495 | AUUAGCUG G CGCAUAUU | 1213 | AATATGCG GGCTAGCTACAACGA CAGCTAAT | 2915 |
| 5497 | UAGCUGGC G CAUAUUAA | 1214 | TTAATATG GGCTAGCTACAACGA GCCAGCTA | 2916 |
| 5499 | GCUGGCGC A UAUUAAGC | 1215 | GCTTAATA GGCTAGCTACAACGA GCGCCAGC | 2917 |
| 5501 | UGGCGCAU A UUAAGCAC | 1216 | GTGCTTAA GGCTAGCTACAACGA ATGCGCCA | 2918 |
| 5506 | CAUAUUAA G CACUUUAA | 1217 | TTAAAGTG GGCTAGCTACAACGA TTAATATG | 2919 |
| 5508 | UAUUAAGC A CUUUAAGC | 1218 | GCTTAAAG GGCTAGCTACAACGA GCTTAATA | 2920 |
| 5515 | CACUUUAA G CUCCUUGA | 1219 | TCAAGGAG GGCTAGCTACAACGA TTAAAGTG | 2921 |
| 5524 | CUCCUUGA G UAAAAAGG | 1220 | CCTTTTTA GGCTAGCTACAACGA TCAAGGAG | 2922 |
| 5532 | GUAAAAAG G UGGUAUGU | 1221 | ACATACCA GGCTAGCTACAACGA CTTTTTAC | 2923 |
| 5535 | AAAAGGUG G UAUGUAAU | 1222 | ATTACATA GGCTAGCTACAACGA CACCTTTT | 2924 |
| 5537 | AAGGUGGU A UGUAAUUU | 1223 | AAATTACA GGCTAGCTACAACG AACCACCT | 2925 |
| 5539 | GGUGGUAU G UAAUUUAU | 1224 | ATAAATTA GGCTAGCTACAACGA ATACCACC | 2926 |
| 5542 | GGUAUGUA A UUUAUGCA | 1225 | TGCATAAA GGCTAGCTACAACGA TACATACC | 2927 |
| 5546 | UGUAAUUU A UGCAAGGU | 1226 | ACCTTGCA GGCTAGCTACAACGA AAATTACA | 2928 |
| 5548 | UAAUUUAU G CAAGGUAU | 1227 | ATACCTTG GGCTAGCTACAACGA ATAAATTA | 2929 |
| 5553 | UAUGCAAG G UAUUUCUC | 1228 | GAGAAATA GGCTAGCTACAACGA CTTGCATA | 2930 |
| 5555 | UGCAAGGU A UUUCUCCA | 1229 | TGGAGAAA GGCTAGCTACAACGA ACCTTGCA | 2931 |
| 5564 | UUUCUCCA G UUGGGACU | 1230 | AGTCCCAA GGCTAGCTACAACGA TGGAGAAA | 2932 |
| 5570 | CAGUUGGG A CUCAGGAU | 1231 | ATCCTGAG GGCTAGCTACAACGA CCCAACTG | 2933 |
| 5577 | GACUCAGG A UAUUAGUU | 1232 | AACTAATA GGCTAGCTACAACGA CCTGAGTC | 2934 |
| 5579 | CUCAGGAU A UUAGUUAA | 1233 | TTAACTAA GGCTAGCTACAACGA ATCCTGAG | 2935 |
| 5583 | GGAUAUUA G UUAAUGAG | 1234 | CTCATTAA GGCTAGCTACAACGA TAATATCC | 2936 |
| 5587 | AUUAGUUA A UGAGCCAU | 1235 | ATGGCTCA GGCTAGCTACAACGA TAACTAAT | 2937 |
| 5591 | GUUAAUGA G CCAUCACU | 1236 | AGTGATGG GGCTAGCTACAACGA TCATTAAC | 2938 |
| 5594 | AAUGAGCC A UCACUAGA | 1237 | TCTAGTGA GGCTAGCTACAACGA GGCTCATT | 2939 |
| 5597 | GAGCCAUC A CUAGAAGA | 1238 | TCTTCTAG GGCTAGCTACAACGA GATGGCTC | 2940 |
| 5609 | GAAGAAAA G CCCAUUUU | 1239 | AAAATGGG GGCTAGCTACAACGA TTTTCTTC | 2941 |
| 5613 | AAAAGCCC A UUUUCAAC | 1240 | GTTGAAAA GGCTAGCTACAACGA GGGCTTTT | 2942 |
| 5620 | CAUUUUCA A CUGCUUUG | 1241 | CAAAGCAG GGCTAGCTACAACGA TGAAAATG | 2943 |
| 5623 | UUUCAACU G CUUUGAAA | 1242 | TTTCAAAG GGCTAGCTACAACGA AGTTGAAA | 2944 |
| 5631 | GCUUUGAA A CUUGCCUG | 1243 | CAGGCAAG GGCTAGCTACAACGA TTCAAAGC | 2945 |
| 5635 | UGAAACUU G CCUGGGGU | 1244 | ACCCCAGG GGCTAGCTACAACGA AAGTTTCA | 2946 |
| 5642 | UGCCUGGG G UCUGAGCA | 1245 | TGCTCAGA GGCTAGCTACAACGA CCCAGGCA | 2947 |
| 5648 | GGGUCUGA G CAUGAUGG | 1246 | CCATCATG GGCTAGCTACAACGA TCAGACCC | 2948 |
| 5650 | GUCUGAGC A UGAUGGGA | 1247 | TCCCATCA GGCTAGCTACAACGA GCTCAGAC | 2949 |
| 5653 | UGAGCAUG A UGGGAAUA | 1248 | TATTCCCA GGCTAGCTACAACGA CATGCTCA | 2950 |
| 5659 | UGAUGGGA A UAGGGAGA | 1249 | TCTCCCTA GGCTAGCTACAACGA TCCCATCA | 2951 |
| 5667 | AUAGGGAG A CAGGGUAG | 1250 | CTACCCTG GGCTAGCTACAACGA CTCCCTAT | 2952 |
| 5672 | GAGACAGG G UAGGAAAG | 1251 | CTTTCCTA GGCTAGCTACAACGA CCTGTCTC | 2953 |
| 5682 | AGGAAAGG G CGCCUACU | 1252 | AGTAGGCG GGCTAGCTACAACGA CCTTTCCT | 2954 |
| 5684 | GAAAGGGC G CCUACUCU | 1253 | AGAGTAGG GGCTAGCTACAACGA GCCCTTTC | 2955 |
| 5688 | GGGCGCCU A CUCUUCAG | 1254 | CTGAAGAG GGCTAGCTACAACGA AGGCGCCC | 2956 |
| 5698 | UCUUCAGG G UCUAAAGA | 1255 | TCTTTAGA GGCTAGCTACAACGA CCTGAAGA | 2957 |
| 5706 | GUCUAAAG A UCAAGUGG | 1256 | CCACTTGA GGCTAGCTACAACGA CTTTAGAC | 2958 |
| 5711 | AAGAUCAA G UGGGCCUU | 1257 | AAGGCCCA GGCTAGCTACAACGA TTGATCTT | 2959 |
| 5715 | UCAAGUGG G CCUUGGAU | 1258 | ATCCAAGG GGCTAGCTACAACGA CCACTTGA | 2960 |
| 5722 | GGCCUUGG A UCGCUAAG | 1259 | CTTAGCGA GGCTAGCTACAACGA CCAAGGCC | 2961 |
| 5725 | CUUGGAUC G CUAAGCUG | 1260 | CAGCTTAG GGCTAGCTACAACGA GATCCAAG | 2962 |
| 5730 | AUCGCUAA G CUGGCUCU | 1261 | AGAGCCAG GGCTAGCTACAACGA TTAGCGAT | 2963 |
| 5734 | CUAAGCUG G CUCUGUUU | 1262 | AAACAGAG GGCTAGCTACAACGA CAGCTTAG | 2964 |
| 5739 | CUGGCUCU G UUUGAUGC | 1263 | GCATCAAA GGCTAGCTACAACGA AGAGCCAG | 2965 |
| 5744 | UCUGUUUG A UGCUAUUU | 1264 | AAATAGCA GGCTAGCTACAACGA CAAACAGA | 2966 |
| 5746 | UGUUUGAU G CUAUUUAU | 1265 | ATAAATAG GGCTAGCTACAACGA ATCAAACA | 2967 |
| 5749 | UUGAUGCU A UUUAUGCA | 1266 | TGCATAAA GGCTAGCTACAACGA AGCATCAA | 2968 |
| 5753 | UGCUAUUU A UGCAAGUU | 1267 | AACTTGCA GGCTAGCTACAACGA AAATAGCA | 2969 |
| 5755 | CUAUUUAU G CAAGUUAG | 1268 | CTAACTTG GGCTAGCTACAACGA ATAAATAG | 2970 |
| 5759 | UUAUGCAA G UUAGGGUC | 1269 | GACCCTAA GGCTAGCTACAACGA TTGCATAA | 2971 |
| 5765 | AAGUUAGG G UCUAUGUA | 1270 | TACATAGA GGCTAGCTACAACGA CCTAACTT | 2372 |
| 5769 | UAGGGUCU A UGUAUUUA | 1271 | TAAATACA GGCTAGCTACAACGA AGACCCTA | 2973 |
| 5771 | GGGUCUAU G UAUUUAGG | 1272 | CCTAAATA GGCTAGCTACAACGA ATAGACCC | 2974 |
| 5773 | GUCUAUGU A UUUAGGAU | 1273 | ATCCTAAA GGCTAGCTACAACGA ACATAGAC | 2975 |
| 5780 | UAUUUAGG A UGCGCCUA | 1274 | TAGGCGCA GGCTAGCTACAACGA CCTAAATA | 2976 |
| 5782 | UUUAGGAU G CGCCUACU | 1275 | AGTAGGCG GGCTAGCTACAACGA ATCCTAAA | 2977 |
| 5784 | UAGGAUGC G CCUACUCU | 1276 | AGAGTAGG GGCTAGCTACAACGA GCATCCTA | 2978 |
| 5788 | AUGCGCCU A CUCUUCAG | 1277 | CTGAAGAG GGCTAGCTACAACGA AGGCGCAT | 2979 |
| 5798 | UCUUCAGG G UCUAAAGA | 1278 | TCTTTAGA GGCTAGCTACAACGA CCTGAAGA | 2980 |
| 5806 | GUCUAAAG A UCAAGUGG | 1279 | CCACTTGA GGCTAGCTACAACGA CTTTAGAC | 2981 |
| 5811 | AAGAUCAA G UGGGCCUU | 1280 | AAGGCCCA GGCTAGCTACAACGA TTGATCTT | 2982 |
| 5815 | UCAAGUGG G CCUUGGAU | 1281 | ATCCAAGG GGCTAGCTACAACGA CCACTTGA | 2983 |
| 5822 | GGCCUUGG A UCGCUAAG | 1282 | CTTAGCGA GGCTAGCTACAACGA CCAAGGCC | 2984 |
| 5825 | CUUGGAUC G CUAAGCUG | 1283 | CAGCTTAG GGCTAGCTACAACGA GATCCAAG | 2985 |
| 5830 | AUCGCUAA G CUGGCUCU | 1284 | AGAGCCAG GGCTAGCTACAACGA TTAGCGAT | 2986 |
| 5834 | CUAAGCUG G CUCUGUW | 1285 | AAACAGAG GGCTAGCTACAACGA CAGCTTAG | 2987 |
| 5839 | CUGGCUCU G UUUGAUGC | 1286 | GCATCAAA GGCTAGCTACAACGA AGAGCCAG | 2988 |
| 5844 | UCUGUUUG A UGCUAUUU | 1287 | AAATAGCA GGCTAGCTACAACGA CAAACAGA | 2989 |
| 5846 | UGUUUGAU G CUAUUUAU | 1288 | ATAAATAG GGCTAGCTACAACGA ATCAAACA | 2990 |
| 5849 | UUGAUGCU A UUUAUGCA | 1289 | TGCATAAA GGCTAGCTACAACGA AGCATCAA | 2991 |
| 5853 | UGCUAUUU A UGCAAGUU | 1290 | AACTTGCA GGCTAGCTACAACGA AAATAGCA | 2992 |
| 5855 | CUAUUUAU G CAAGUUAG | 1291 | CTAACTTG GGCTAGCTACAACGA ATAAATAG | 2993 |
| 5859 | UUAUGCAA G UUAGGGUC | 1292 | GACCCTAA GGCTAGCTACAACGA TTGCATAA | 2994 |
| 5865 | AAGUUAGG G UCUAUGUA | 1293 | TACATAGA GGCTAGCTACAACGA CCTAACTT | 2995 |
| 5869 | UAGGGUCU A UGUAUUUA | 1294 | TAAATACA GGCTAGCTACAACGA AGACCCTA | 2996 |
| 5871 | GGGUCUAU G UAUUUAGG | 1295 | CCTAAATA GGCTAGCTACAACGA ATAGACCC | 2997 |
| 5873 | GUCUAUGU A UUUAGGAU | 1296 | ATCCTAAA GGCTAGCTACAACGA ACATAGAC | 2998 |
| 5880 | UAUUUAGG A UGUCUGCA | 1297 | TGCAGACA GGCTAGCTACAACGA CCTAAATA | 2999 |
| 5882 | UUUAGGAU G UCUGCACC | 1298 | GGTGCAGA GGCTAGCTACAACGA ATCCTAAA | 3000 |
| 5886 | GGAUGUCU G CACCUUCU | 1299 | AGAAGGTG GGCTAGCTACAACGA AGACATCC | 3001 |
| 5888 | AUGUCUGC A CCUUCUGC | 1300 | GCAGAAGG GGCTAGCTACAACGA GCAGACAT | 3002 |
| 5895 | CACCUUCU G CAGCCAGU | 1301 | ACTGGCTG GGCTAGCTACAACGA AGAAGGTG | 3003 |
| 5898 | CUUCUGCA G CCAGUCAG | 1302 | CTGACTGG GGCTAGCTACAACGA TGCAGAAG | 3004 |
| 5902 | UGCAGCCA G UCAGAAGC | 1303 | GCTTCTGA GGCTAGCTACAACGA TGGCTGCA | 3005 |
| 5909 | AGUCAGAA G CUGGAGAG | 1304 | CTCTCCAG GGCTAGCTACAACGA TTCTGACT | 3006 |
| 5918 | CUGGAGAG G CAACAGUG | 1305 | CACTGTTG GGCTAGCTACAACGA CTCTCCAG | 3007 |
| 5921 | GAGAGGCA A CAGUGGAU | 1306 | ATCCACTG GGCTAGCTACAACGA TGCCTCTC | 3008 |
| 5924 | AGGCAACA G UGGAUUGC | 1307 | GCAATCCA GGCTAGCTACAACGA TGTTGCCT | 3009 |
| 5928 | AACAGUGG A UUGCUGCU | 1308 | AGCAGCAA GGCTAGCTACAACGA CCACTGTT | 3010 |
| 5931 | AGUGGAUU G CUGCUUCU | 1309 | AGAAGCAG GGCTAGCTACAACGA AATCCACT | 3011 |
| 5934 | GGAUUGCU G CUUCUUGG | 1310 | CCAAGAAG GGCTAGCTACAACGA AGCAATCC | 3012 |
| 5951 | GGAGAAGA G UAUGCUUC | 1311 | GAAGCATA GGCTAGCTACAACGA TCTTCTCC | 3013 |
| 5953 | AGAAGAGU A UGCUUCCU | 1312 | AGGAAGCA GGCTAGCTACAACGA ACTCTTCT | 3014 |
| 5955 | AAGAGUAU G CUUCCUUU | 1313 | AAAGGAAG GGCTAGCTACAACGA ATACTCTT | 3015 |
| 5965 | UUCCUUUU A UCCAUGUA | 1314 | TACATGGA GGCTAGCTACAACGA AAAAGGAA | 3016 |
| 5969 | UUUUAUCC A UGUAAUUU | 1315 | AAATTACA GGCTAGCTACAACGA GGATAAAA | 3017 |
| 5971 | UUAUCCAU G UAAUUUAA | 1316 | TTAUTTA GGCTAGCTACAACGA ATGGATAA | 3018 |
| 5974 | UCCAUGUA A UUUAACUG | 1317 | CAGTTAAA GGCTAGCTACAACGA TACATGGA | 3019 |
| 5979 | GUAAUUUA A CUGUAGAA | 1318 | TTCTACAG GGCTAGCTACAACGA TAAATTAC | 3020 |
| 5982 | AUUUAACU G UAGAACCU | 1319 | AGGTTCTA GGCTAGCTACAACGA AGTTAAAT | 3021 |
| 5987 | ACUGUAGA A CCUGAGCU | 1320 | AGCTCAGG GGCTAGCTACAACGA TCTACAGT | 3022 |
| 5993 | GAACCUGA G CUCUAAGU | 1321 | ACTTAGAG GGCTAGCTACAACGA TCAGGTTC | 3023 |
| 6000 | AGCUCUAA G UAACCGAA | 1322 | TTCGGTTA GGCTAGCTACAACGA TTAGAGCT | 3024 |
| 6003 | UCUAAGUA A CCGAAGAA | 1323 | TTCTTCGG. GGCTAGCTACAACGA TACTTAGA | 3025 |
| 6011 | ACCGAAGA A UGUAUGCC | 1324 | GGCATACA GGCTAGCTACAACGA TCTTCGGT | 3026 |
| 6013 | CGAAGAAU G UAUGCCUC | 1325 | GAGGCATA GGCTAGCTACAACGA ATTCTTCG | 3027 |
| 6015 | AAGAAUGU A UGCCUCUG | 1326 | CAGAGGCA GGCTAGCTACAACGA ACATTCTT | 3028 |
| 6017 | GAAUGUAU G CCUCUGUU | 1327 | AACAGAGG GGCTAGCTACAACGA ATACATTC | 3029 |
| 6023 | AUGCCUCU G UUCUUAUG | 1328 | CATAAGAA GGCTAGCTACAACGA AGAGGCAT | 3030 |
| 6029 | CUGUUCUU A UGUGCCAC | 1329 | GTGGCACA GGCTAGCTACAACGA AAGAACAG | 3031 |
| 60.31 | GUUCUUAU G UGCCACAU | 1330 | ATGTGGCA GGCTAGCTACAACGA ATAAGAAC | 3032 |
| 6033 | UCUUAUGU G CCACAUCC | 1331 | GGATGTGG GGCTAGCTACAACGA ACATAAGA | 3033 |
| 6036 | UAUGUGCC A CAUCCUUG | 1332 | CAAGGATG GGCTAGCTACAACGA GGCACATA | 3034 |
| 6038 | UGUGCCAC A UCCUUGUU | 1333 | AACAAGGA GGCTAGCTACAACGA GTGGCACA | 3035 |
| 6044 | ACAUCCUU G UUUAAAGG | 1334 | CCTTTAAA GGCTAGCTACAACGA AAGGATGT | 3036 |
| 6052 | GUUUAAAG G CUCUCUGU | 1335 | ACAGAGAG GGCTAGCTACAACGA CTTTAAAC | 3037 |
| 6059 | GGCUCUCU G UAUGAAGA | 1336 | TCTTCATA GGCTAGCTACAACGA AGAGAGCC | 3038 |
| 6061 | CUCUCUGU A UGAAGAGA | 1337 | TCTCTTCA GGCTAGCTACAACGA ACAGAGAG | 3039 |
| 6069 | AUGAAGAG A UGGGACCG | 1338 | CGGTCCCA GGCTAGCTACAACGA CTCTTCAT | 3040 |
| 6074 | GAGAUGGG A CCGUCAUC | 1339 | GATGACGG GGCTAGCTACAACGA CCCATCTC | 3041 |
| 6077 | AUGGGACC G UCAUCAGC | 1340 | GCTGATGA GGCTAGCTACAACGA GGTCCCAT | 3042 |
| 6080 | GGACCGUC A UCAGCACA | 1341 | TGTGCTGA GGCTAGCTACAACGA GACGGTCC | 3043 |
| 6084 | CGUCAUCA G CACAUUCC | 1342 | GGAATGTG GGCTAGCTACAACGA TGATGACG | 3044 |
| 6086 | UCAUCAGC A CAUUCCCU | 1343 | AGGGAATG GGCTAGCTACAACGA GCTGATGA | 3045 |
| 6088 | AUCAGCAC A UUCCCUAG | 1344 | CTAGGGAA GGCTAGCTACAACGA GTGCTGAT | 3046 |
| 6096 | AUUCCCUA G UGAGCCUA | 1345 | TAGGCTCA GGCTAGCTACAACGA TAGGGAAT | 3047 |
| 6100 | CCUAGUGA G CCUACUGG | 1346 | CCAGTAGG GGCTAGCTACAACGA TCACTAGG | 3048 |
| 6104 | GUGAGCCU A CUGGCUCC | 1347 | GGAGCCAG GGCTAGCTACAACGA AGGCTCAC | 3049 |
| 6108 | GCCUACUG G CUCCUGGC | 1348 | GCCAGGAG GGCTAGCTACAACGA CAGTAGGC | 3050 |
| 6115 | GGCUCCUG G CAGCGGCU | 1349 | AGCCGCTG GGCTAGCTACAACGA CAGGAGCC | 3051 |
| 6118 | UCCUGGCA G CGGCUUUU | 1350 | AAAAGCCG GGCTAGCTACAACGA TGCCAGGA | 3052 |
| 6121 | UGGCAGCG G CUUUUGUG | 1351 | CACAAAAG GGCTAGCTACAACGA CGCTGCCA | 3053 |
| 6127 | CGGCUUUU G UGGAAGAC | 1352 | GTCTTCCA GGCTAGCTACAACGA AAAAGCCG | 3054 |
| 6134 | UGUGGAAG A CUCACUAG | 1353 | CTAGTGAG GGCTAGCTACAACGA CTTCCACA | 3055 |
| 6138 | GAAGACUC A CUAGCCAG | 1354 | CTGGCTAG GGCTAGCTACAACGA GAGTCTTC | 3056 |
| 6142 | ACUCACUA G CCAGAAGA | 1355 | TCTTCTGG GGCTAGCTACAACGA TAGTGAGT | 3057 |
| 6156 | AGAGAGGA G UGGGACAG | 1356 | CTGTCCCA GGCTAGCTACAACGA TCCTCTCT | 3058 |
| 6161 | GGAGUGGG A CAGUCCUC | 1357 | GAGGACTG GGCTAGCTACAACGA CCCACTCC | 3059 |
| 6164 | GUGGGACA G UCCUCUCC | 1358 | GGAGAGGA GGCTAGCTACAACGA TGTCCCAC | 3060 |
| 6173 | UCCUCUCC A CCAAGAUC | 1359 | GATCTTGG GGCTAGCTACAACGA GGAGAGGA | 3061 |
| 6179 | CCACCAAG A UCUAAAUC | 1360 | GATTTAGA GGCTAGCTACAACGA CTTGGTGG | 3062 |
| 6185 | AGAUCUAA A UCCAAACA | 1361 | TGTTTGGA GGCTAGCTACAACGA TTAGATCT | 3063 |
| 6191 | AAAUCCAA A CAAAAGCA | 1362 | TGCTTTTG GGCTAGCTACAACGA TTGGATTT | 3064 |
| 6197 | AAACAAAA G CAGGCUAG | 1363 | CTAGCCTG GGCTAGCTACAACGA TTTTGTTT | 3065 |
| 6201 | AAAAGCAG G CUAGAGCC | 1364 | GGCTCTAG GGCTAGCTACAACGA CTGCTTTT | 3066 |
| 6207 | AGGCUAGA G CCAGAAGA | 1365 | TCTTCTGG GGCTAGCTACAACGA TCTAGCCT | 3067 |
| 6220 | AAGAGAGG A CAAAUCUU | 1366 | AAGATTTG GGCT AGCTACAACGA CCTCTCTT | 3068 |
| 6224 | GAGGACAA A UCUUUGUU | 1367 | AACAAAGA GGCTAGCTACAACGA TTGTCCTC | 3069 |
| 6230 | AAAUCUUU G UUGUUCCU | 1368 | AGGAACAA GGCTAGCTACAACGA AAAGATTT | 3070 |
| 6233 | UCUUUGUU G UUCCUCUU | 1369 | AAGAGGAA GGCTAGCTACAACGA AACAAAGA | 3071 |
| 6246 | UCUUCUUU A CACAUACG | 1370 | CGTATGTG GGCTAGCTACAACGA AAAGAAGA | 3072 |
| 6248 | UUCUUUAC A CAUACGCA | 1371 | TGCGTATG GGCTAGCTACAACGA GTAAAGAA | 3073 |
| 6250 | CUUUACAC A UACGCAAA | 1372 | TTTGCGTA GGCTAGCTACAACGA GTGTAAAG | 3074 |
| 6252 | UUACACAU A CGCAAACC | 1373 | GGTTTGCG GGCTAGCTACAACGA ATGTGTAA | 3075 |
| 6254 | ACACAUAC G CAAACCAC | 1374 | GTGGTTTG GGCTAGCTACAACGA GTATGTGT | 3076 |
| 6258 | AUACGCAA A CCACCUGU | 1375 | ACAGGTGG GGCTAGCTACAACGA TTGCGTAT | 3077 |
| 6261 | CGCAAACC A CCUGUGAC | 1376 | GTCACAGG GGCTAGCTACAACGA GGTTTGCG | 3078 |
| 6265 | AACCACCU G UGAGAGCU | 1377 | AGCTGTCA GGCTAGCTACAACGA AGGTGGTT | 3079 |
| 6268 | CACCUGUG A CAGCUGGC | 1378 | GCCAGCTG GGCTAGCTACAACGA CACAGGTG | 3080 |
| 6271 | CUGUGACA G CUGGCAAU | 1379 | ATTGCCAG GGCTAGCTACAACGA TGTCACAG | 3081 |
| 6275 | GACAGCUG G CAAUUUUA | 1380 | TAAAATTG GGCTAGCTACAACGA CAGCTGTC | 3082 |
| 6278 | AGCUGGCA A UUUUAUAA | 1381 | TTATAAAA GGCTAGCTACAACGA TGCCAGCT | 3083 |
| 6283 | GCAAUUUU A UAAAUCAG | 1382 | CTGATTTA GGCTAGCTACAACGA AAAATTGC | 3084 |
| 6287 | UUUUAUAA A UCAGGUAA | 1383 | TTACCTGA GGCTAGCTACAACGA TTATAAAA | 3085 |
| 6292 | UAAAUGAG G UAACUGGA | 1384 | TCCAGTTA GGCTAGCTACAACGA CTGATTTA | 3086 |
| 6295 | AUCAGGUA A CUGGAAGG | 1385 | CCTTCCAG GGCTAGCTACAACGA TACCTGAT | 3087 |
| 6306 | GGAAGGAG G UUAAACUC | 1386 | GAGTTTAA GGCTAGCTACAAGGA CTCCTTCC | 3088 |
| 6311 | GAGGUUAA A CUCAGAAA | 1387 | TTTCTGAG GGCTAGCTACAACGA TTAACCTC | 3089 |
| 6327 | AAAAGAAG A CCUCAGUC | 1388 | GACTGAGG GGCTAGCTACAACGA CTTCTTTT | 3090 |
| 6333 | AGACCUCA G UCAAUUCU | 1389 | AGAATTGA GGCTAGCTACAACGA TGAGGTCT | 3091 |
| 6337 | CUCAGUCA A UUCUCUAC | 1390 | GTAGAGAA GGCTAGCTACAACGA TGACTGAG | 3092 |
| 6344 | AAUUCUCU A CUUUUUUU | 1391 | AAAAAAAG GGCTAGCTACAACGA AGAGAATT | 3093 |
| 6366 | UUUUCCAA A UCAGAUAA | 1392 | TTATCTGA GGCTAGCTACAACGA TTGGAAAA | 3094 |
| 6371 | CAAAUCAG A UAAUAGCC | 1393 | GGCTATTA GGCTAGCTACAACGA CTGATTTG | 3095 |
| 6374 | AUCAGAUA A UAGCCCAG | 1394 | CTGGGCTA GGCTAGCTACAACGA TATCTGAT | 3096 |
| 6377 | AGAUAAUA G CCCAGCAA | 1395 | TTGCTGGG GGCTAGCTACAACGA TATTATCT | 3097 |
| 6382 | AUAGCCCA G CAAAUAGU | 1396 | ACTATTTG GGCTAGCTACAACGA TGGGCTAT | 3098 |
| 6386 | CCCAGCAA A UAGUGAUA | 1397 | TATCACTA GGCTAGCTACAACGA TTGCTGGG | 3099 |
| 6389 | AGCAAAUA G UGAUAACA | 1398 | TGTTATCA GGCTAGCTACAACGA TATTTGCT | 3100 |
| 6392 | AAAUAGUG A UAACAAAU | 1399 | ATTTGTTA GGCTAGCTACAACGA CACTATTT | 3101 |
| 6395 | UAGUGAUA A CAAAUAAA | 1400 | TTTATTTG GGCTAGCTACAACGA TATCACTA | 3102 |
| 6399 | GAUAACAA A UAAAACCU | 1401 | AGGTTTTA GGCTAGCTACAACGA TTGTTATC | 3103 |
| 6404 | CAAAUAAA A CCUUAGCU | 1402 | AGCTAAGG GGCTAGCTACAACGA TTTATTTG | 3104 |
| 6410 | AAACCUUA G CUGUUCAU | 1403 | ATGAACAG GGCTAGCTACAACGA TAAGGTTT | 3105 |
| 6413 | CCUUAGCU G UUCAUGUC | 1404 | GACATGAA GGCTAGCTACAACGA AGCTAAGG | 3106 |
| 6417 | AGCUGUUC A UGUCUUGA | 1405 | TCAAGACA GGCTAGCTACAACGA GAACAGCT | 3107 |
| 6419 | CUGUUCAU G UCUUGAUU | 1406 | AATCAAGA GGCTAGCTACAACGA ATGAACAG | 3108 |
| 6425 | AUGUCUUG A UUUCAAUA | 1407 | TATTGAAA GGCTAGCTACAACGA CAAGACAT | 3109 |
| 6431 | UGAUUUCA A UAAUUAAU | 1408 | ATTAATTA GGCTAGCTACAACGA TGAAATCA | 3110 |
| 6434 | UUUCAAUA A UUAAUUCU | 1409 | AGAATTAA GGCTAGCTACAACGA TATTGAAA | 3111 |
| 6438 | AAUAAUUA A UUCUUAAU | 1410 | ATTAAGAA GGCTAGCTACAACGA TAATTATT | 3112 |
| 6445 | AAUUCUUA A UCAUUAAG | 1411 | CTTAATGA GGCTAGCTACAACGA TAAGAATT | 3113 |
| 6448 | UCUUAAUC A UUAAGAGA | 1412 | TCTCTTAA GGCTAGCTACAACGA GATTAAGA | 3114 |
| 6456 | AUUAAGAG A CCAUAAUA | 1413 | TATTATGG GGCTAGCTACAACGA CTCTTAAT | 3115 |
| 6459 | AAGAGACC A UAAUAAAU | 1414 | ATTTATTA GGCTAGCTACAACGA GGTCTCTT | 3116 |
| 6462 | AGACCAUA A UAAAUACU | 1415 | AGTATTTA GGCTAGCTACAACGA TATGGTCT | 3117 |
| 6466 | CAUAAUAA A UACUCCUU | 1416 | AAGGAGTA GGCTAGCTACAACGA TTATTATG | 3118 |
| 6468 | UAAUAAAU A CUCCUUUU | 1417 | AAAAGGAG GGCTAGCTACAACGA ATTTATTA | 3119 |
| 6487 | AGAGAAAA G CAAAACCA | 1418 | TGGTTTTG GGCTAGCTACAACGA TTTTCTCT | 3120 |
| 6492 | AAAGCAAA A CCAUUAGA | 1419 | TCTAATGG GGCTAGCTACAACGA TTTGCTTT | 3121 |
| 6495 | GCAAAACC A UUAGAAUU | 1420 | AATTCTAA GGCTAGCTACAACGA GGTTTTGC | 3122 |
| 6501 | CCAUUAGA A UUGUUACU | 1421 | AGTAACAA GGCTAGCTACAACGA TCTAATGG | 3123 |
| 6504 | UUAGAAUU G UUACUCAG | 1422 | CTGAGTAA GGCTAGCTACAACGA AATTCTAA | 3124 |
| 6507 | GAAUUGUU A CUCAGCUC | 1423 | GAGCTGAG GGCTAGCTACAACGA AACAATTC | 3125 |
| 6512 | GUUACUCA G CUCCUUCA | 1424 | TGAAGGAG GGCTAGCTACAACGA TGAGTAAC | 3126 |
| 6522 | UCCUUCAA A CUCAGGUU | 1425 | AACCTGAG GGCTAGCTACAACGA TTGAAGGA | 3127 |
| 6528 | AAACUCAG G UUUGUAGC | 1426 | GCTACAAA GGCTAGCTACAACGA CTGAGTTT | 3128 |
| 6532 | UCAGGUUU G UAGCAUAC | 1427 | GTATGCTA GGCTAGCTACAACGA AAACCTGA | 3129 |
| 6535 | GGUUUGUA G CAUACAUG | 1428 | CATGTATG GGCTAGCTACAACGA TACAAACC | 3130 |
| 6537 | UUUGUAGC A UACAUGAG | 1429 | CTCATGTA GGCTAGCTACAACGA GCTACAAA | 3131 |
| 6539 | UGUAGCAU A CAUGAGUC | 1430 | GACTCATG GGCTAGCTACAACGA ATGCTACA | 3132 |
| 6541 | UAGCAUAC A UGAGUCCA | 1431 | TGGACTCA GGCTAGCTACAACGA GTATGCTA | 3133 |
| 6545 | AUACAUGA G UCCAUCCA | 1432 | TGGATGGA GGCTAGCTACAACGA TCATGTAT | 3134 |
| 6549 | AUGAGUCC A UCCAUCAG | 1433 | CTGATGGA GGCTAGCTACAACGA GGACTCAT | 3135 |
| 6553 | GUCCAUCC A UCAGUCAA | 1434 | TTGACTGA GGCTAGCTACAACGA GGATGGAC | 3136 |
| 6557 | AUCCAUCA G UCAAAGAA | 1435 | TTCTTTGA GGCTAGCTACAACGA TGATGGAT | 3137 |
| 6565 | GUCAAAGA A UGGUUCCA | 1436 | TGGAACCA GGCTAGCTACAACGA TCTTTGAC | 3138 |
| 6568 | AAAGAAUG G UUCCAUCU | 1437 | AGATGGAA GGCTAGCTACAACGA CATTCTTT | 3139 |
| 6573 | AUGGUUCC A UCUGGAGU | 1438 | ACTCCAGA GGCTAGCTACAACGA GGAACCAT | 3140 |
| 6580 | CAUCUGGA G UCUUAAUG | 1439 | CATTAAGA GGCTAGCTACAACGA TCCAGATG | 3141 |
| 6586 | GAGUCUUA A UGUAGAAA | 1440 | TTTCTACA GGCTAGCTACAACGA TAAGACTC | 3142 |
| 6588 | GUCUUAAU G UAGAAAGA | 1441 | TCTTTCTA GGCTAGCTACAACGA ATTAAGAC | 3143 |
| 6600 | AAAGAAAA A UGGAGACU | 1442 | AGTCTCCA GGCTAGCTACAACGA TTTTCTTT | 3144 |
| 6606 | AAAUGGAG A CUUGUAAU | 1443 | ATTACAAG GGCTAGCTACAACGA CTCCATTT | 3145 |
| 6610 | GGAGACUU G UAAUAAUG | 1444 | CATTATTA GGCTAGCTACAACGA AAGTCTCC | 3146 |
| 6613 | GACUUGUA A UAAUGAGC | 1445 | GCTCATTA GGCTAGCTACAACGA TACAAGTC | 3147 |
| 6616 | UUGUAAUA A UGAGCUAG | 1446 | CTAGCTCA GGCTAGCTACAACGA TATTACAA | 3148 |
| 6620 | AAUAAUGA G CUAGUUAC | 1447 | GTAACTAG GGCTAGCTACAACGA TCATTATT | 3149 |
| 6624 | AUGAGCUA G UUACAAAG | 1448 | CTTTGTAA GGCTAGCTACAACGA TAGCTCAT | 3150 |
| 6627 | AGCUAGUU A CAAAGUGC | 1449 | GCACTTTG GGCTAGCTACAACGA AACTAGCT | 3151 |
| 6632 | GUUACAAA G UGCUUGUU | 1450 | AACAAGCA GGCTAGCTACAACGA TTTGTAAC | 3152 |
| 6634 | UACAAAGU G CUUGUUCA | 1451 | TGAACAAG GGCTAGCTACAACGA ACTTTGTA | 3153 |
| 6638 | AAGUGCUU G UUCAUUAA | 1452 | TTAATGAA GGCTAGCTACAACGA AAGCACTT | 3154 |
| 6642 | GCUUGUUC A UUAAAAUA | 1453 | TATTTTAA GGCTAGCTACAACGA GAACAAGC | 3155 |
| 6648 | UCAUUAAA A UAGCACUG | 1454 | CAGTGCTA GGCTAGCTACAACGA TTTAATGA | 3156 |
| 6651 | UUAAAAUA G CACUGAAA | 1455 | TTTCAGTG GGCTAGCTACAACGA TATTTTAA | 3157 |
| 6653 | AAAAUAGC A CUGAAAAU | 1456 | ATTTTCAG GGCTAGCTACAACGA GCTATTTT | 3158 |
| 6660 | CACUGAAA A UUGAAACA | 1457 | TGTTTCAA GGCTAGCTACAACGA TTTCAGTG | 3159 |
| 6666 | AAAUUGAA A CAUGAAUU | 1458 | AATTCATG GGCTAGCTACAACGA TTCAATTT | 3160 |
| 6668 | AUUGAAAC A UGAAUUAA | 1459 | TTAATTCA GGCTAGCTACAACGA GTTTCAAT | 3161 |
| 6672 | AAACAUGA A UUAACUGA | 1460 | TCAGTTAA GGCTAGCTACAACGA TCATGTTT | 3162 |
| 6676 | AUGAAUUA A CUGAUAAU | 1461 | ATTATCAG GGCTAGCTACAACGA TAATTCAT | 3163 |
| 6680 | AUUAACUG A UAAUAUUC | 1462 | GAATATTA GGCTAGCTACAACGA CAGTTAAT | 3164 |
| 6683 | AACUGAUA A UAUUCCAA | 1463 | TTGGAATA GGCTAGCTACAACGA TATCAGTT | 3165 |
| 6685 | CUGAUAAU A UUCCAAUC | 1464 | GATTGGAA GGCTAGCTACAACGA ATTATCAG | 3166 |
| 6691 | AUAUUCCA A UCAUUUGC | 1465 | GCAAATGA GGCTAGCTACAACGA TGGAATAT | 3167 |
| 6694 | UUCCAAUC A UUUGCCAU | 1466 | ATGGCAAA GGCTAGCTACAACGA GATTGGAA | 3168 |
| 6698 | AAUCAUUU G CCAUUUAU | 1467 | ATAAATGG GGCTAGCTACAACGA AAATGATT | 3169 |
| 6701 | CAUUUGCC A UUUAUGAC | 1468 | GTCATAAA GGCTAGCTACAACGA GGCAAATG | 3170 |
| 6705 | UGCCAUUU A UGACAAAA | 1469 | TTTTGTCA GGCTAGCTACAACGA AAATGGCA | 3171 |
| 6708 | CAUUUAUG A CAAAAAUG | 1470 | CATTTTTG GGCTAGCTACAACGA CATAAATG | 3172 |
| 6714 | UGACAAAA A UGGUUGGC | 1471 | GCCAACCA GGCTAGCTACAACGA TTTTGTCA | 3173 |
| 6717 | CAAAAAUG G UUGGCACU | 1472 | AGTGCCAA GGCTAGCTACAACGA CATTTTTG | 3174 |
| 6721 | AAUGGUUG G CACUAACA | 1473 | TGTTAGTG GGCTAGCTACAACGA CAACCATT | 3175 |
| 6723 | UGGUUGGC A CUAACAAA | 1474 | TTTGTTAG GGCTAGCTACAACGA GCCAACCA | 3176 |
| 6727 | UGGCACUA A CAAAGAAC | 1475 | GTTCTTTG GGCTAGCTACAACGA TAGTGCCA | 3177 |
| 6734 | AACAAAGA A CGAGCACU | 1476 | AGTGCTCG GGCTAGCTACAACGA TCTTTGTT | 3178 |
| 6738 | AAGAACGA G CACUUCCU | 1477 | AGGAAGTG GGCTAGCTACAACGA TCGTTCTT | 3179 |
| 6740 | GAACGAGC A CUUCCUUU | 1478 | AAAGGAAG GGCTAGCTACAACGA GCTCGTTC | 3180 |
| 6753 | CUUUCAGA G UUUCUGAG | 1479 | CTCAGAAA GGCTAGCTACAACGA TCTGAAAG | 3181 |
| 6762 | UUUCUGAG A UAAUGUAC | 1480 | GTACATTA GGCTAGCTACAACGA CTCAGAAA | 3182 |
| 6765 | CUGAGAUA A UGUACGUG | 1481 | CACGTACA GGCTAGCTACAACGA TATCTCAG | 3183 |
| 6767 | GAGAUAAU G UACGUGGA | 1482 | TCCACGTA GGCTAGCTACAACGA ATTATCTC | 3184 |
| 6769 | GAUAAUGU A CGUGGAAC | 1483 | GTTCCACG GGCTAGCTACAACGA ACATTATC | 3185 |
| 6771 | UAAUGUAC G UGGAACAG | 1484 | CTGTTCCA GGCTAGCTACAACGA GTACATTA | 3186 |
| 6776 | UACGUGGA A CAGUCUGG | 1485 | CCAGACTG GGCTAGCTACAACGA TCCACGTA | 3187 |
| 6779 | GUGGAACA G UCUGGGUG | 1486 | CACCCAGA GGCTAGCTACAAGGA TGTTCCAC | 3188 |
| 6785 | CAGUCUGG G UGGAAUGG | 1487 | CCATTCCA GGCTAGCTACAACGA CCAGACTG | 3189 |
| 6790 | UGGGUGGA A UGGGGCUG | 1488 | CAGCCCGA GGCTAGCTACAACGA TCCACCCA | 3190 |
| 6795 | GGAAUGGG G CUGAAACC | 1489 | GGTTTCAG GGCTAGCTACAACGA CCCATTCC | 3191 |
| 6801 | GGGCUGAA A CCAUGUGC | 1490 | GCACATGG GGCTAGCTACAACGA TTCAGCCC | 3192 |
| 6804 | CUGAAACC A UGUGCAAG | 1491 | CTTGCACA GGCTAGCTACAACGA GGTTTCAG | 3193 |
| 6806 | GAAACCAU G UGCAAGUC | 1492 | GACTTGCA GGCTAGCTACAACGA ATGGTTTC | 3194 |
| 6808 | AACCAUGU G CAAGUCUG | 1493 | CAGACTTG GGCTAGCTACAACGA ACATGGTT | 3195 |
| 6812 | AUGUGCAA G UCUGUGUC | 1494 | GACACAGA GGCTAGCTACAACGA TTGCACAT | 3196 |
| 6816 | GCAAGUCU G UGUCUUGU | 1495 | ACAAGACA GGCTAGCTACAACGA AGACTTGC | 3197 |
| 6818 | AAGUCUGU G UCUUGUCA | 1496 | TGACAAGA GGCTAGCTACAACGA ACAGACTT | 3198 |
| 6823 | UGUGUCUU G UCAGUCCA | 1497 | TGGACTGA GGCTAGCTACAACGA AAGACACA | 3199 |
| 6827 | UCUUGUCA G UCCAAGAA | 1498 | TTCTTGGA GGCTAGCTACAACGA TGACAAGA | 3200 |
| 6836 | UCCAAGAA G UGACACCG | 1499 | CGGTGTCA GGCTAGCTACAACGA TTCTTGGA | 3201 |
| 6839 | AAGAAGUG A CACCGAGA | 1500 | TCTCGGTG GGCTAGCTACAACGA CACTTCTT | 3202 |
| 6841 | GAAGUGAC A CCGAGAUG | 1501 | CATCTCGG GGCTAGCTACAACGA GTCACTTC | 3203 |
| 6847 | ACACCGAG A UGUUAAUU | 1502 | AATTAACA GGCTAGCTACAACGA CTCGGTGT | 3204 |
| 6849 | ACCGAGAU G UUAAUUUU | 1503 | AAAATTAA GGCTAGCTACAACGA ATCTCGGT | 3205 |
| 6853 | AGAUGUUA A UUUUAGGG | 1504 | CCCTAAAA GGCTAGCTACAACGA TAACATCT | 3206 |
| 6862 | UUUUAGGG A CCCGUGCC | 1505 | GGCACGGG GGCTAGCTACAACGA CCCTAAAA | 3207 |
| 6866 | AGGGACCC G UGCCUUGU | 1506 | ACAAGGCA GGCTAGCTACAACGA GGGTCCCT | 3208 |
| 6868 | GGACCCGU G CCUUGUUU | 1507 | AAACAAGG GGCTAGCTACAACGA ACGGGTCC | 3209 |
| 6873 | CGUGCCUU G UUUCCUAG | 1508 | CTAGGAAA GGCTAGCTACAACGA AAGGCACG | 3210 |
| 6881 | GUUUCCUA G CCCACAAG | 1509 | CTTGTGGG GGCTAGCTACAACGA TAGGAAAC | 3211 |
| 6885 | CCUAGCCC A CAAGAAUG | 1510 | CATTCTTG GGCTAGCTACAACGA GGGCTAGG | 3212 |
| 6891 | CCACAAGA A UGCAAACA | 1511 | TGTTTGCA GGCTAGCTACAACGA TCTTGTGG | 3213 |
| 6893 | ACAAGAAU G CAAACAUC | 1512 | GATGTTTG GGCTAGCTACAACGA ATTCTTGT | 3214 |
| ' 6897 | GAAUGCAA A CAUCAAAC | 1513 | GTTTGATG GGCTAGGTACAACGA TTGCATTC | 3215 |
| 6899 | AUGCAAAC A UCAAACAG | 1514 | CTGTTTGA GGCTAGCTACAACGA GTTTGCAT | 3216 |
| 6904 | AACAUCAA A CAGAUACU | 1515 | AGTATCTG GGCTAGCTACAACGA TTGATGTT | 3217 |
| 6908 | UCAAACAG A UACUCGCU | 1516 | AGCGAGTA GGCTAGCTACAACGA CTGTTTGA | 3218 |
| 6910 | AAACAGAU A CUCGCUAG | 1517 | CTAGCGAG GGCTAGCTACAACGA ATCTGTTT | 3219 |
| 6914 | AGAUACUC G CUAGCCUC | 1518 | GAGGCTAG GGCTAGCTACAACGA GAGTATCT | 3220 |
| 6918 | ACUCGCUA G CCUCAUUU | 1519 | AAATGAGG GGCTAGCTACAACGA TAGCGAGT | 3221 |
| 6923 | CUAGCCUC A UUUAAAUU | 1520 | AATTTAAA GGCTAGCTACAACGA GAGGCTAG | 3222 |
| 6929 | UCAUUUAA A UUGAUUAA | 1521 | TTAATCAA GGCTAGCTACAACGA TTAAATGA | 3223 |
| 6933 | UUAAAUUG A UUAAAGGA. | 1522 | TCCTTTAA GGCTAGCTACAACGA CAATTTAA | 3224 |
| 6945 | AAGGAGGA G UGCAUCUU | 1523 | AAGATGCA GGCTAGCTACAACGA TCCTCCTT | 3225 |
| 6947 | GGAGGAGU G CAUCUUUG | 1524 | CAAAGATG GGCTAGCTACAACGA ACTCCTCC | 3226 |
| 6949 | AGGAGUGC A UCUUUGGC | 1525 | GCCAAAGA GGCTAGCTACAACGA GCACTCCT | 3227 |
| 6956 | CAUCUUUG G CCGACAGU | 1526 | ACTGTCGG GGCTAGCTACAACGA CAAAGATG | 3228 |
| 6960 | UUUGGCCG A CAGUGGUG | 1527 | CACCACTG GGCTAGCTACAACGA CGGCCAAA | 3229 |
| 6963 | GGCCGACA G UGGUGUAA | 1528 | TTACACCA GGCTAGCTACAACGA TGTCGGCC | 3230 |
| 6966 | CGACAGUG G UGUAACUG | 1529 | CAGTTACA GGCTAGCTACAACGA CACTGTCG | 3231 |
| 6968 | ACAGUGGU G UAACUGUG | 1530 | CAGAGTTA GGCTAGCTACAACGA ACCACTGT | 3232 |
| 6971 | GUGGUGUA A CUGUGUGU | 1531 | ACACACAG GGCTAGCTACAACGA TACACCAC | 3233 |
| 6974 | GUGUAACU G UGUGUGUG | 1532 | CACACACA GGCTAGCTACAACGA AGTTACAC | 3234 |
| 6976 | GUAACUGU G UGUGUGUG | 1533 | CACACACA GGCTAGCTACAACGA ACAGTTAC | 3235 |
| 6978 | AACUGUGU G UGUGUGUG | 1534 | CACACACA GGCTAGCTACAACGA ACACAGTT | 3236 |
| 6980 | CUGUGUGU G UGUGUGUG | 1535 | CACACACA GGCTAGCTACAACGA ACACACAG | 3237 |
| 6982 | GUGUGUGU G UGUGUGUG | 1536 | CACACACA GGCTAGCTACAACGA ACACACAC | 3238 |
| 6984 | GUGUGUGU G UGUGUGUG | 1537 | CACACACA GGCTAGCTACAACGA ACACACAC | 3239 |
| 6986 | GUGUGUGU G UGUGUGUG | 1538 | CACACACA GGCTAGCTACAACGA ACACACAC | 3240 |
| 6988 | GUGUGUGU G UGUGUGUG | 1539 | CACACACA GGCTAGCTACAACGA ACACACAC | 3241 |
| 6990 | GUGUGUGU G UGUGUGUG | 1540 | CACACACA GGCTAGCTACAACGA ACACACAC | 3242 |
| 6992 | GUGUGUGU G UGUGUGUG | 1541 | CACACACA GGCTAGCTACAACGA ACACACAC | 3243 |
| 6994 | GUGUGUGU G UGUGUGUG | 1542 | CACACACA GGCTAGCTACAACGA ACACACAC | 3244 |
| 6996 | GUGUGUGU G UGUGUGUG | 1543 | CACACACA GGCTAGCTACAACGA ACACACAC | 3245 |
| 6998 | GUGUGUGU G UGUGUGUG | 1544 | CACACACA GGCTAGCTACAACGA ACACACAC | 3246 |
| 7000 | GUGUGUGU G UGUGUGUG | 1545 | CACACACA GGCTAGCTACAACGA ACACACAC | 3247 |
| 7002 | GUGUGUGU G UGUGUGUG | 1546 | CACACACA GGCTAGCTACAACGA ACACACAC | 3248 |
| 7004 | GUGUGUGU G UGUGUGUG | 1547 | CACACACA GGCTAGCTACAACGA ACACACAC | 3249 |
| 7006 | GUGUGUGU G UGUGUGUG | 1548 | CACACACA GGCTAGCTACAACGA ACACACAC | 3250 |
| 7008 | GUGUGUGU G UGUGUGGG | 1549 | CCCACACA GGCTAGCTACAACGA ACACACAC | 3251 |
| 7010 | GUGUGUGU G UGUGGGUG | 1550 | CACCCACA GGCTAGCTACAACGA ACACACAC | 3252 |
| 7012 | GUGUGUGU G UGGGUGUG | 1551 | CACACCCA GGCTAGCTACAACGA ACACACAC | 3253 |
| 7016 | GUGUGUGG G UGUGGGUG | 1552 | CACCCACA GGCTAGCTACAACGA CCACACAC | 3254 |
| 7018 | GUGUGGGU G UGGGUGUA | 1553 | TACACCCA GGCTAGCTACAACGA ACCCACAC | 3255 |
| 7022 | GGGUGUGG G UGUAUGUG | 1554 | CACATACA GGCTAGCTACAACGA CCACACCC | 3256 |
| 7024 | GUGUGGGU G UAUGUGUG | 1555 | CACACATA GGCTAGCTACAACGA ACCCACAC | 3257 |
| 7026 | GUGGGUGU A UGUGUGUU | 1556 | AACACACA GGCTAGCTACAACGA ACACCCAC | 3258 |
| 7028 | GGGUGUAU G UGUGUUUU | 1557 | AAAACACA GGCTAGCTACAACGA ATACACCC | 3259 |
| 7030 | GUGUAUGU G UGUUUUGU | 1558 | ACAAAACA GGCTAGCTACAACGA ACATACAC | 3260 |
| 7032 | GUAUGUGU G UUUUGUGC | 1559 | GCACAAAA GGCTAGCTACAACGA ACACATAC | 3261 |
| 7037 | UGUGUUUU G UGCAUAAC | 1560 | GTTATGCA GGCTAGCTACAACGA AAAACACA | 3262 |
| 7039 | UGUUUUGU G CAUAACUA | 1561 | TAGTTATG GGCTAGCTACAACGA ACAAAACA | 3263 |
| 7041 | UUUUGUGC A UAACUAUU | 1562 | AATAGTTA GGCTAGCTACAACGA GCACAAAA | 3264 |
| 7044 | UGUGCAUA A CUAUUUAA | 1563 | TTAAATAG GGCTAGCTACAACGA TATGCACA | 3265 |
| 7047 | GCAUAACU A UUUAAGGA | 1564 | TCCTTAAA GGCTAGCTACAACGA AGTTATGC | 3266 |
| 7057 | UOAAGGAA A CUGGAAUU | 1565 | AATTCCAG GGCTAGCTACAACGA TTCCTTAA | 3267 |
| 7063 | AAACUGGA A UUUUAAAG | 1566 | CTTTAAAA GGCTAGCTACAACGA TCCAGTTT | 3268 |
| 7071 | AUUUUAAA G UUACUUUU | 1567 | AAAAGTAA GGCTAGCTACAACGA TTTAAAAT | 3269 |
| 7074 | UUAAAGUU A CUUUUAUA | 1568 | TATAAAAG GGCTAGCTACAACGA AACTTTAA | 3270 |
| 7080 | UUACUUUU A UACAAACC | 1569 | GGTTTGTA GGCTAGCTACAACGA AAAAGTAA | 3271 |
| 7082 | ACUUUUAU A CAAACCAA | 1570 | TTGGTTTG GGCTAGCTACAACGA ATAAAAGT | 3272 |
| 7086 | UUAUACAA A CCAAGAAU | 1571 | ATTCTTGG GGCTAGCTACAACGA TTGTATAA | 3273 |
| 7093 | AACCAAGA A UAUAUGCU | 1572 | AGCATATA GGCTAGCTACAACGA TCTTGGTT | 3274 |
| 7095 | CCAAGAAU A UAUGCUAC | 1573 | GTAGCATA GGCTAGCTACAACGA ATTCTTGG | 3275 |
| 7097 | AAGAAUAU A UGCUACAG | 1574 | CTGTAGCA GGCTAGCTACAACGA ATATTCTT | 3276 |
| 7099 | GAAUAUAU G CUACAGAU | 1575 | ATCTGTAG GGCTAGCTACAACGA ATATATTC | 3277 |
| 7102 | UAUAUGCU A CAGAUAUA | 1576 | TATATCTG GGCTAGCTACAACGA AGCATATA | 3278 |
| 7106 | UGCUACAG A UAUAAGAC | 1577 | GTCTTATA GGCTAGCTACAACGA CTGTAGCA | 3279 |
| 7108 | CUACAGAU A UAAGACAG | 1578 | CTGTCTTA GGCTAGCTACAACGA ATCTGTAG | 3280 |
| 7113 | GAUAUAAG A CAGACAUG | 1579 | CATGTCTG GGCTAGCTACAACGA CTTATATC | 3281 |
| 7117 | UAAGACAG A CAUGGUUU | 1580 | AAACCATG GGCTAGCTACAACGA CTGTCTTA | 3282 |
| 7119 | AGACAGAC A UGGUUUGG | 1581 | CCAAACCA GGCTAGCTACAACGA GTCTGTCT | 3283 |
| 7122 | CAGACAUG G UUUGGUCC | 1582 | GGACCAAA GGCTAGCTACAACGA CATGTCTG | 3284 |
| 7127 | AUGGUUUG G UCCUAUAU | 1583 | ATATAGGA GGCTAGCTACAACGA CAAACCAT | 3285 |
| 7132 | UUGGUCCU A UAUUUCUA | 1584 | TAGAAATA GGCTAGCTACAACGA AGGACCAA | 3286 |
| 7134 | GGUCCUAU A UUUCUAGU | 1585 | ACTAGAAA GGCTAGCTACAACGA ATAGGACC | 3287 |
| 7141 | UAUUUCUA G UCAUGAUG | 1586 | CATCATGA GGCTAGCTACAACGA TAGAAATA | 3288 |
| 7144 | UUCUAGUC A UGAUGAAU | 1587 | ATTCATCA GGCTAGCTACAACGA GACTAGAA | 3289 |
| 7147 | UAGUCAUG A UGAAUGUA | 1588 | TACATTCA GGCTAGCTACAACGA CATGACTA | 3290 |
| 7151 | CAUGAUGA A UGUAUUUU | 1589 | AAAATACA GGCTAGCTACAACGA TCATCATG | 3291 |
| 7153 | UGAUGAAU G UAUUUUGU | 1590 | ACAAAATA GGCTAGCTACAACGA ATTCATCA | 3292 |
| 7155 | AUGAAUGU A UUUUGUAU | 1591 | ATACAAAA GGCTAGCTACAACGA ACATTCAT | 3293 |
| 7160 | UGUAUUUU G UAUACCAU | 1592 | ATGGTATA GGCTAGCTACAACGA AAAATACA | 3294 |
| 7162 | UAUUUUGU A UACCAUCU | 1593 | AGATGGTA GGCTAGCTACAACGA ACAAAATA | 3295 |
| 7164 | UUUUGUAU A CCAUCUUC | 1594 | GAAGATGG GGCTAGCTACAACGA ATACAAAA | 3296 |
| 7167 | UGUAUACC A UCUUCAUA | 1595 | TATGAAGA GGCTAGCTACAACGA GGTATACA | 3297 |
| 7173 | CCAUCUUC A UAUAAUAU | 1596 | ATATTATA GGCTAGCTACAACGA GAAGATGG | 3298 |
| 7175 | AUCUUCAU A UAAUAUAC | 1597 | GTATATTA GGCTAGCTACAACGA ATGAAGAT | 3299 |
| 7178 | UUCAUAUA A UAUACUUA | 1598 | TAAGTATA GGCTAGCTACAACGA TATATGAA | 3300 |
| 7180 | CAUAUAAU A UACUUAAA | 1599 | TTTAAGTA GGCTAGCTACAACGA ATTATATG | 3301 |
| 7182 | UAUAAUAU A CUUAAAAA | 1600 | TTTTTAAG GGCTAGCTACAACGA ATATTATA | 3302 |
| 7190 | ACUUAAAA A UAUUUCUU | 1601 | AAGAAATA GGCTAGCTACAACGA TTTTAAGT | 3303 |
| 7192 | UUAAAAAU A UUUCUUAA | 1602 | TTAAGAAA GGCTAGCTACAACGA ATTTTTAA | 3304 |
| 7200 | AUUUCUUA A UUGGGAUU | 1603 | AATCCCAA GGCTAGCTACAACGA TAAGAAAT | 3305 |
| 7206 | UAAUUGGG A UUUGUAAU | 1604 | ATTACAAA GGCTAGCTACAACGA CCCAATTA | 3306 |
| 7210 | UGGGAUUU G UAAUCGUA | 1605 | TACGATTA GGCTAGCTACAACGA AAATCCCA | 3307 |
| 7213 | GAUUUGUA A UCGUACCA | 1606 | TGGTACGA GGCTAGCTACAACGA TACAAATC | 3308 |
| 7216 | UUGUAAUC G UACCAACU | 1607 | AGTTGGTA GGCTAGCTACAACGA GATTACAA | 3309 |
| 7218 | GUAAUCGU A CCAACUUA | 1608 | TAAGTTGG GGCTAGCTACAACGA ACGATTAC | 3310 |
| 7222 | UCGUACCA A CUUAAUUG | 1609 | CAATTAAG GGCTAGCTACAACGA TGGTACGA | 3311 |
| 7227 | CCAACUUA A UUGAUAAA | 1610 | TTTATCAA GGCTAGCTACAACGA TAAGTTGG | 3312 |
| 7231 | CUUAAUUG A UAAACUUG | 1611 | CAAGTTTA GGCTAGCTACAACGA CAATTAAG | 3313 |
| 7235 | AUUGAUAA A CUUGGCAA | 1612 | TTGCCAAG GGCTAGCTACAACGA TTATCAAT | 3314 |
| 7240 | UAAACUUG G CAACUGCU | 1613 | AGCAGTTG GGCTAGCTACAACGA CAAGTTTA | 3315 |
| 7243 | ACUUGGCA A CUGCUUUU | 1614 | AAAAGCAG GGCTAGCTACAACGA TGCCAAGT | 3316 |
| 7246 | UGGCAACU G CUUUUAUG | 1615 | CATAAAAG GGCTAGCTACAACGA AGTTGCCA | 3317 |
| 7252 | CUGCUUUU A UGUUCUGU | 1616 | ACAGAACA GGCTAGCTACAACGA AAAAGCAG | 3318 |
| 7254 | GCUUUUAU G UUCUGUCU | 1617 | AGACAGAA GGCTAGCTACAACGA ATAAAAGC | 3319 |
| 7259 | UAUGUUCU G UCUCCUUC | 1618 | GAAGGAGA GGCTAGCTACAACGA AGAACATA | 3320 |
| 7269 | CUCCUUCC A UAAAUUUU | 1619 | AAAATTTA GGCTAGCTACAACGA GGAAGGAG | 3321 |
| 7273 | UUCCAUAA A UUUUUCAA | 1620 | TTGAAAAA GGCTAGCTACAACGA TTATGGAA | 3322 |
| 7283 | UUUUCAAA A UACUAAUU | 1621 | AATTAGTA GGCTAGCTACAACGA TTTGAAAA | 3323 |
| 7285 | UUCAAAAU A CUAAUUCA | 1622 | TGAATTAG GGCTAGCTACAACGA ATTTTGAA | 3324 |
| 7289 | AAAUACUA A UUCAACAA | 1623 | TTGTTGAA GGCTAGCTACAACGA TAGTATTT | 3325 |
| 7294 | CUAAUUCA A CAAAGAAA | 1624 | TTTCTTTG GGCTAGCTACAACGA TGAATTAG | 3326 |
| 7305 | AAGAAAAA G CUCUUUUU | 1625 | AAAAAGAG GGCTAGCTACAACGA TTTTTCTT | 3327 |
| 7323 | UUCCUAAA A UAAACUCA | 1626 | TGAGTTTA GGCTAGCTACAACGA TTTAGGAA | 3328 |
| 7327 | UAAAAUAA A CUCAAAUU | 1627 | AATTTGAG GGCTAGCTACAACGA TTATTTTA | 3329 |
| 7333 | AAACUCAA A UUUAUCCU | 1628 | AGGATAAA GGCTAGCTACAACGA TTGAGTTT | 3330 |
| 7337 | UCAAAUUU A UCCUUGUU | 1629 | AACAAGGA GGCTAGCTACAACGA AAATTTGA | 3331 |
| 7343 | UUAUCCUU G UUUAGAGC | 1630 | GCTCTAAA GGCTAGCTACAACGA AAGGATAA | 3332 |
| 7350 | UGUUUAGA G CAGAGAAA | 1631 | TTTCTCTG GGCTAGCTACAACGA TCTAAACA | 3333 |
| 7360 | AGAGAAAA A UUAAGAAA | 1632 | TTTCTTAA GGCTAGCTACAACGA TTTTCTCT | 3334 |
| 7370 | UAAGAAAA A CUUUGAAA | 1633 | TTTCAAAG GGCTAGCTACAACGA TTTTCTTA | 3335 |
| 7378 | ACUUUGAA A UGGUCUCA | 1634 | TGAGACCA GGCTAGCTACAACGA TTCAAAGT | 3336 |
| 7381 | UUGAAAUG G UCUCAAAA | 1635 | TTTTGAGA GGCTAGCTACAACGA CATTTCAA | 3337 |
| 7391 | CUCAAAAA A UUGCUAAA | 1636 | TTTAGCAA GGCTAGCTACAACGA TTTTTGAG | 3338 |
| 7394 | AAAAAAUU G CUAAAUAU | 1637 | ATATTTAG GGCTAGCTACAACGA AATTTTTT | 3339 |
| 7399 | AUUGCUAA A UAUUUUCA | 1638 | TGAAAATA GGCTAGCTACAACGA TTAGCAAT | 3340 |
| 7401 | UGCUAAAU A UUUUGAAU | 1639 | ATTGAAAA GGCTAGCTACAACGA ATTTAGCA | 3341 |
| 7408 | UAUUUUCA A UGGAAAAC | 1640 | GTTTTCCA GGCTAGCTACAACGA TGAAAATA | 3342 |
| 7415 | AAUGGAAA A CUAAAUGU | 1641 | ACATTTAG GGCTAGCTACAACGA TTTCCATT | 3343 |
| 7420 | AAAACUAA A UGUUAGUU | 1642 | AACTAACA GGCTAGCTACAACGA TTAGTTTT | 3344 |
| 7422 | AACUAAAU G UUAGUUUA | 1643 | TAAACTAA GGCTAGCTACAACGA ATTTAGTT | 3345 |
| 7426 | AAAUGUUA G UUUAGCUG | 1644 | CAGCTAAA GGCTAGCTACAACGA TAACATTT | 3346 |
| 7431 | UUAGUUUA G CUGAUUGU | 1645 | ACAATCAG GGCTAGCTACAACGA TAAACTAA | 3347 |
| 7435 | UUUAGCUG A UUGUAUGG | 1646 | CCATACAA GGCTAGCTACAACGA CAGCTAAA | 3348 |
| 7438 | AGCUGAUU G UAUGGGGU | 1647 | ACCCCATA GGCTAGCTACAACGA AATCAGCT | 3349 |
| 7440 | CUGAUUGU A UGGGGUUU | 1648 | AAACCCCA GGCTAGCTACAACGA ACAATCAG | 3350 |
| 7445 | UGUAUGGG G UUUUCGAA | 1649 | TTCGAAAA GGCTAGCTACAACGA CCCATACA | 3351 |
| 7453 | GUUUUCGA A CCUUUCAC | 1650 | GTGAAAGG GGCTAGCTACAACGA TCGAAAAC | 3352 |
| 7460 | AACCUUUC A CUUUUUGU | 1651 | ACAAAAAG GGCTAGCTACAACGA GAAAGGTT | 3353 |
| 7467 | CACUUUUU G UUUGUUUU | 1652 | AAAACAAA GGCTAGCTACAACGA AAAAAGTG | 3354 |
| 7471 | UUUUGUUU G UUUUACCU | 1653 | AGGTAAAA GGCTAGCTACAACGA AAACAAAA | 3355 |
| 7476 | UUUGUUUU A CCUAUUUC | 1654 | GAAATAGG GGCTAGCTACAACGA AAAACAAA | 3356 |
| 7480 | UUUUACCU A UUUCACAA | 1655 | TTGTGAAA GGCTAGCTACAACGA AGGTAAAA | 3357 |
| 7485 | CCUAUUUC A CAACUGUG | 1656 | CACAGTTG GGCTAGCTACAACGA GAAATAGG | 3358 |
| 7488 | AUUUCACA A CUGUGUAA | 1657 | TTACACAG GGCTAGCTACAACGA TGTGAAAT | 3359 |
| 7491 | UCACAACU G UGUAAAUU | 1658 | AATTTACA GGCTAGCTACAACGA AGTTGTGA | 3360 |
| 7493 | ACAACUGU G UAAAUUGC | 1659 | GCAATTTA GGCTAGCTACAACGA ACAGTTGT | 3361 |
| 7497 | CUGUGUAA A UUGCCAAU | 1660 | ATTGGCAA GGCTAGCTACAACGA TTACACAG | 3362 |
| 7500 | UGUAAAUU G CCAAUAAU | 1661 | ATTATTGG GGCTAGCTACAACGA AATTTACA | 3363 |
| 7504 | AAUUGCCA A UAAUUCCU | 1662 | AGGAATTA GGCTAGCTACAACGA TGGCAATT | 3364 |
| 7507 | UGCCAAUA A UUCCUGUC | 1663 | GACAGGAA GGCTAGCTACAACGA TATTGGCA | 3365 |
| 7513 | UAAUUCCU G UCCAUGAA | 1664 | TTCATGGA GGCTAGCTACAACGA AGGAATTA | 3366 |
| 7517 | UCCUGUCC A UGAAAAUG | 1665 | CATTTTCA GGCTAGCTACAACGA GGACAGGA | 3367 |
| 7523 | CCAUGAAA A UGCAAAUU | 1666 | AATTTGCA GGCTAGCTACAACGA TTTCATGG | 3368 |
| 7525 | AUGAAAAU G CAAAUUAU | 1667 | ATAATTTG GGCTAGCTACAACGA ATTTTCAT | 3369 |
| 7529 | AAAUGCAA A UUAUCCAG | 1668 | CTGGATAA GGCTAGCTACAACGA TTGCATTT | 3370 |
| 7532 | UGCAAAUU A UCCAGUGU | 1669 | ACACTGGA GGCTAGCTACAACGA AATTTGCA | 3371 |
| 7537 | AUUAUCCA G UGUAGAUA | 1670 | TATCTACA GGCTAGCTACAACGA TGGATAAT | 3372 |
| 7539 | UAUCCAGU G UAGAUAUA | 1671 | TATATCTA GGCTAGCTACAACGA ACTGGATA | 3373 |
| 7543 | CAGUGUAG A UAUAUUUG | 1672 | CAAATATA GGCTAGCTACAACGA CTACACTG | 3374 |
| 7545 | GUGUAGAU A UAUUUGAC | 1673 | GTCAAATA GGCTAGCTACAACGA ATCTACAC | 3375 |
| 7547 | GUAGAUAU A UUUGACCA | 1674 | TGGTCAAA GGCTAGCTACAACGA ATATCTAC | 3376 |
| 7552 | UAUAUUUG A CCAUCACC | 1675 | GGTGATGG GGCTAGCTACAACGA CAAATATA | 3377 |
| 7555 | AUUUGACC A UCACCCUA | 1676 | TAGGGTGA GGCTAGCTACAACGA GGTCAAAT | 3378 |
| 7558 | UGACCAUC A CCCUAUGG | 1677 | CCATAGGG GGCTAGCTACAACGA GATGGTCA | 3379 |
| 7563 | AUCACCCU A UGGAUAUU | 1678 | AATATCCA GGCTAGCTACAACGA AGGGTGAT | 3380 |
| 7567 | CCCUAUGG A UAUUGGCU | 1679 | AGCCAATA GGCTAGCTACAACGA CCATAGGG | 3381 |
| 7569 | CUAUGGAU A UUGGCUAG | 1680 | CTAGCCAA GGCTAGCTACAACGA ATCCATAG | 3382 |
| 7573 | GGAUAUUG G CUAGUUUU | 1681 | AAAACTAG GGCTAGCTACAACGA CAATATCC | 3383 |
| 7577 | AUUGGCUA G UUUUGCCU | 1682 | AGGCAAAA GGCTAGCTACAACGA TAGCCAAT | 3384 |
| 7582 | CUAGUUUU G CCUUUAUU | 1683 | AATAAAGG GGCTAGCTACAACGA AAAACTAG | 3385 |
| 7588 | UUGCCUUU A UUAAGCAA | 1684 | TTGCTTAA GGCTAGCTACAACGA AAAGGCAA | 3386 |
| 7593 | UUUAUUAA G CAAAUUCA | 1685 | TGAATTTG GGCTAGCTACAACGA TTAATAAA | 3387 |
| 7597 | UUAAGCAA A UUCAUUUC | 1686 | GAAATGAA GGCTAGCTACAACGA TTGCTTAA | 3388 |
| 7601 | GCAAAUUC A UUUCAGCC | 1687 | GGCTGAAA GGCTAGCTACAACGA GAATTTGC | 3389 |
| 7607 | UCAUUUCA G CCUGAAUG | 1688 | CATTCAGG GGCTAGCTACAACGA TGAAATGA | 3390 |
| 7613 | CAGCCUGA A UGUCUGCC | 1689 | GGCAGACA GGCTAGCTACAACGA TCAGGCTG | 3391 |
| 7615 | GCCUGAAU G UCUGCCUA | 1690 | TAGGCAGA GGCTAGCTACAACGA ATTCAGGC | 3392 |
| 7619 | GAAUGUCU G CCUAUAUA | 1691 | TATATAGG GGCTAGCTACAACGA AGACATTC | 3393 |
| 7623 | GUCUGCCU A UAUAUUCU | 1692 | AGAATATA GGCTAGCTACAACGA AGGCAGAC | 3394 |
| 7625 | CUGCCUAU A UAUUCUCU | 1693 | AGAGAATA GGCTAGCTACAACGA ATAGGCAG | 3395 |
| 7627 | GCCUAUAU A UUCUCUGC | 1694 | GCAGAGAA GGCTAGCTACAACGA ATATAGGC | 3396 |
| 7634 | UAUUCUCU G CUCUUUGU | 1695 | ACAAAGAG GGCTAGCTACAACGA AGAGAATA | 3397 |
| 7641 | UGCUCUUU G UAUUCUCC | 1696 | GGAGAATA GGCTAGCTACAACGA AAAGAGCA | 3398 |
| 7643 | CUCUUUGU A UUCUCCUU | 1697 | AAGGAGAA GGCTAGCTACAACGA ACAAAGAG | 3399 |
| 7655 | UCCUUUGA A CCCGUUAA | 1698 | TTAACGGG GGCTAGCTACAACGA TCAAAGGA | 3400 |
| 7659 | UUGAACCC G UUAAAACA | 1699 | TGTTTTAA GGCTAGCTACAACGA GGGTTCAA | 3401 |
| 7665 | CCGUUAAA A CAUCCUGU | 1700 | ACAGGATG GGCTAGCTACAACGA TTTAACGG | 3402 |
| 7667 | GUUAAAAC A UCCUGUGG | 1701 | CCACAGGA GGCTAGCTACAACGA GTTTTAAC | 3403 |
| 7672 | AACAUCCU G UGGCACUC | 1702 | GAGTGCCA GGCTAGCTACAACGA AGGATGTT | 3404. |

| | | | | |
|---|---|---|---|---|
| Input Sequence = HSFLT. Cut Site = R/Y Arm Length = 8. Core Sequence = GGCTAGCTACAACGA HSFLT (Human fit mRNA for receptor-related tyrosine kinase.; Acc# X51602; 7680 bp) | | | | |

**Table VI: Human KDR DNAzyme and Substrate sequence**

| **Pos** | **Substrate** | **Seq ID No** | **DNAzyme** | **Seq ID No** |
|---|---|---|---|---|
| 14 | GUCCCGGG A CCCCGGGA | 3405 | TCCCGGGG GGCTAGCTACAACGA CCCGGGAC | 4691 |
| 25 | CCGGGAGA G CGGUCAGU | 3406 | ACTGACCG GGCTAGCTACAACGA TCTCCCGG | 4692 |
| 28 | GGAGAGCG G UCAGUGUG | 3407 | CACACTGA GGCTAGCTACAACGA CGCTCTCC | 4693 |
| 32 | AGCGGUCA G UGUGUGGU | 3408 | ACCACACA GGCTAGCTACAACGA TGACCGCT | 4694 |
| 34 | CGGUCAGU G UGUGGUCG | 3409 | CGACCACA GGCTAGCTACAACGA ACTGACCG. | 4695 |
| 36 | GUCAGUGU G UGGUCGCU | 3410 | AGCGACCA GGCTAGCTACAACGA ACACTGAC | 4696 |
| 39 | AGUGUGUG G UCGCUGCG | 3411 | CGCAGCGA GGCTAGCTACAACGA CACACACT | 4697 |
| 42 | GUGUGGUC G CUGCGUUU | 3412 | AAACGCAG GGCTAGCTACAACGA GACCACAC | 4698 |
| 45 | UGGUCGCU G CGUUUCCU | 3413 | AGGAAACG GGCTAGCTACAACGA AGCGACCA | 4699 |
| 47 | GUCGCUGC G UUUCCUCU | 3414 | AGAGGAAA GGCTAGCTACAACGA GCAGCGAC | 4700 |
| 56 | UUUCCUCU G CCUGCGCC | 3415 | GGCGCAGG GGCTAGCTACAACGA AGAGGAAA | 4701 |
| 60 | CUCUGCCU G CGCCGGGC | 3416 | GCCCGGCG GGCTAGCTACAACGA AGGCAGAG | 4702 |
| 62 | CUGCCUGC G CCGGGCAU | 3417 | ATGCCCGG GGCTAGCTACAACGA GCAGGCAG | 4703 |
| 67 | UGCGCCGG G CAUCACUU | 3418 | AAGTGATG GGCTAGCTACAACGA CCGGCGCA | 4704 |
| 69 | CGCCGGGC A UCACUUGC | 3419 | GCAAGTGA GGCTAGCTACAACGA GCCCGGCG | 4705 |
| 72 | CGGGCAUC A CUUGCGCG | 3420 | CGCGCAAG GGCTAGCTACAACGA GATGCCCG | 4706 |
| 76 | AUCACUU G CGCGCCGC | 3421 | GCGGCGCG GGCTAGCTACAACGA AAGTGATG | 4707 |
| 78 | UCACUUGC G CGCCGCAG | 3422 | CTGCGGCG GGCTAGCTACAACGA GCAAGTGA | 4708 |
| 80 | ACUUGCGC G CCGCAGAA | 3423 | TTCTGCGG GGCTAGCTACAACGA GCGCAAGT | 4709 |
| 83 | UGCGCGCC G CAGAAAGU | 3424 | ACTTTCTG GGCTAGCTACAACGA GGCGCGCA | 4710 |
| 90 | CGCAGAAA G UCCGUCUG | 34.25 | CAGACGGA GGCTAGCTACAACGA TTTCTGCG | 4711 |
| 94 | GAAAGUCC G UCUGGCAG | 3426 | CTGCCAGA GGCTAGCTACAACGA GGACTTTC | 4712 |
| 99 | UCCGUCUG G CAGCCUGG | 3427 | CCAGGCTG GGCTAGCTACAACGA CAGACGGA | 4713 |
| 102 | GUCUGGCA G CCUGGAUA | 3428 | TATCCAGG GGCTAGCTACAACGA TGCCAGAC | 4714 |
| 108 | CAGCCUGG A UAUCCUCU | 3429 | AGAGGATA GGCTAGCTACAACGA CCAGGCTG | 4715 |
| 110 | GCCUGGAU A UCCUCUCC | 3430 | GGAGAGGA GGCTAGCTACAACGA ATCCAGGC | 4716 |
| 120 | CCUCUCCU A CCGGCACC | 3431 | GGTGCCGG GGCTAGCTACAACGA AGGAGAGG | 4717 |
| 124 | UCCUACCG G CACCCGCA | 3432 | TGCGGGTG GGCTAGCTACAACGA CGGTAGGA | 4718 |
| 126 | CUACCGGC A CCCGCAGA | 3433 | TCTGCGGG GGCTAGCTACAACGA GCCGGTAG | 4719 |
| 130 | CGGCACCC G CAGACGCC | 3434 | GGCGTCTG GGCTAGCTACAACGA GGGTGCCG | 4720 |
| 134 | ACCCGCAG A CGCCCCUG | 3435 | CAGGGGCG GGCTAGCTACAACGA CTGCGGGT | 4721 |
| 136 | CCGCAGAC G CCCCUGCA | 3436 | TGCAGGGG GGCTAGCTACAACGA GTCTGCGG | 4722 |
| 142 | ACGCCCCU G CAGCGGCC | 3437 | GGCGGCTG GGCTAGCTACAACGA AGGGGCGT | 4723 |
| 145 | CCCCUGCA G CCGCCGGU | 3438 | ACCGGCGG GGCTAGCTACAACGA TGCAGGGG | 4724 |
| 148 | CUGCAGCC G CCGGUCGG | 3439 | CCGACCGG GGCTAGCTACAACGA GGCTGCAG | 4725 |
| 152 | AGCCGCCG G UCGGCGCC | 3440 | GGCGCCGA GGCTAGCTACAACGA CGGCGGCT | 4726 |
| 156 | GCCGGUCG G CGCCCGGG | 3441 | CCCGGGCG GGCTAGCTAGAACGA CGACCGGC | 4727 |
| 158 | CGGUCGGC G CCCGGGCU | 3442 | AGCCCGGG GGCTAGCTACAACGA GCCGACCG | 4728 |
| 164 | GCGCCCGG G CUCCCUAG | 3443 | CTAGGGAG GGCTAGCTACAACGA CCGGGCGC | 4729 |
| 172 | GCUCCCUA G CCCUGUGC | 3444 | GCACAGGG GGCTAGCTACAACGA TAGGGAGC | 4730 |
| 177 | CUAGCCCU G UGCGCUCA | 3445 | TGAGCGCA GGCTAGCTACAACGA AGGGCTAG | 4731 |
| 179 | AGCCCUGU G CGCUCAAC | 3446 | GTTGAGCG GGCTAGCTACAACGA ACAGGGCT | 4732 |
| 181 | CCCUGUGC G CUCAACUG | 3447 | CAGTTGAG GGCTAGCTACAACGA GCACAGGG | 4733 |
| 186 | UGCGCUCA A CUGUCCUG | 3448 | CAGGACAG GGCTAGCTACAACGA TGAGCGCA | 4734 |
| 189 | GCUCAACU G UCCUGCGC | 3449 | GCGCAGGA GGCTAGCTACAACGA AGTTGAGC | 4735 |
| 194 | ACUGUCCU G CGCUGCGG | 3450 | CCGCAGCG GGCTAGCTACAACGA AGGACAGT | 4736 |
| 196 | UGUCCUGC G CUGCGGGG | 3451 | CCCCGCAG GGCTAGCTACAACGA GCAGGACA | 4737 |
| 199 | CCUGCGCU G CGGGGUGC | 3452 | GCACCCCG GGCTAGCTACAACGA AGCGCAGG | 4738 |
| 204 | GCUGCGGG G UGCCGCGA | 3453 | TCGCGGCA GGCTAGCTACAACGA CCCGCAGC | 4739 |
| 206 | UGCGGGGU G CCGCGAGU | 3454 | ACTCGCGG GGCTAGCTACAACGA ACCCCGCA | 4740 |
| 209 | GGGGUGCC G CGAGUUCC | 3455 | GGAACTCG GGCTAGCTACAACGA GGCACCCC | 4741 |
| 213 | UGCCGCGA G UUCCACCU | 3456 | AGGTGGAA GGCTAGCTACAACGA TCGCGGCA | 4742 |
| 218 | CGAGUUCC A CCUCCGCG | 3457 | CGCGGAGG GGCTAGCTACAACGA GGAACTCG | 4743 |
| 224 | CCACCUCC G CGCCUCCU | 3458 | AGGAGGCG GGCTAGCTACAACGA GGAGGTGG | 4744 |
| 226 | ACCUCCGC G CCUCCUUC | 3459 | GAAGGAGG GGCTAGCTACAACGA GCGGAGGT | 4745 |
| 240 | UUCUCUAG A CAGGCGCU | 3460 | AGCGCCTG GGCTAGCTACAACGA CTAGAGAA | 4746 |
| 244 | CUAGACAG G CGCUGGGA | 3461 | TCCCAGCG GGCTAGCTACAACGA CTGTCTAG | 4747 |
| 246 | AGACAGGC G CUGGGAGA | 3462 | TCTCCCAG GGCTAGCTACAACGA GCCTGTCT | 4748 |
| 259 | GAGAAAGA A CCGGCUCC | 3463 | GGAGCCGG GGCTAGCTACAACGA TCTTTCTC | 4749 |
| 263 | AAGAACCG G CUCCCGAG | 3464 | CTCGGGAG GGCTAGCTACAACGA CGGTTCTT | 4750 |
| 271 | GCUCCCGA G UUCUGGGC | 3465 | GCCCAGAA GGCTAGCTACAACGA TCGGGAGC | 4751 |
| 278 | AGUUCUGG G CAUUUCGC | 3466 | GCGAAATG GGCTAGCTACAACGA CCAGAACT | 4752 |
| 280 | UUCUGGGC A UUUCGCCC | 3467 | GGGCGAAA GGCTAGCTACAACGA GCCCAGAA | 4753 |
| 285 | GGCAUUUC G CCCGGCUC | 3468 | GAGCCGGG GGCTAGCTACAACGA GAAATGCC | 4754 |
| 290 | UUCGCCCG G CUCGAGGU | 3469 | ACCTCGAG GGCTAGCTACAACGA CGGGCGAA | 4755 |
| 297 | GGCUCGAG G UGCAGGAU | 3470 | ATCCTGCA GGCTAGCTACAACGA CTCGAGCC | 4756 |
| 299 | CUCGAGGU G CAGGAUGC | 3471 | GCATCCTG GGCTAGCTACAACGA ACCTCGAG | 4757 |
| 304 | GGUGCAGG A UGCAGAGC | 3472 | GCTCTGCA GGCTAGCTACAACGA CCTGCACC | 4758 |
| 306 | UGCAGGAU G CAGAGCAA | 3473 | TTGCTCTG GGCTAGCTACAACGA ATCCTGCA | 4759 |
| 311 | GAUGCAGA G CAAGGUGC | 3474 | GCACCTTG GGCTAGGTACAACGA TCTGCATC | 4760 |
| 316 | AGAGCAAG G UGCUGCUG | 3475 | CAGCAGCA GGCTAGCTACAACGA CTTGCTCT | 4761 |
| 318 | AGCAAGGU G CUGCUGGC | 3476 | GCCAGCAG GGCTAGCTACAACGA ACCTTGCT | 4762 |
| 321 | AAGGUGCU G CUGGCCGU | 3477 | ACGGCCAG GGCTAGCTACAACGA AGCACCTT | 4763 |
| 325 | UGCUGCUG G CCGUCGCC | 3478 | GGCGACGG GGCTAGCTACAACGA CAGCAGCA | 4764 |
| 328 | UGCUGGCC G UCGCCCUG | 3479 | CAGGGCGA GGCTAGCTACAACGA GGCCAGCA | 4765 |
| 331 | UGGCCGUC G CCCUGUGG | 3480 | CCACAGGG GGCTAGCTACAACGA GACGGCCA | 4766 |
| 336 | GUCGCCCU G UGGCUCUG | 3481 | CAGAGCCA GGCTAGCTACAACGA AGGGCGAC | 4767 |
| 339 | GCCCUGUG G CUCUGCGU | 3482 | ACGCAGAG GGCTAGCTACAACGA CACAGGGC | 4768 |
| 344 | GUGGCUCU G CGUGGAGA | 3483 | TCTCCACG GGCTAGCTACAACGA AGAGCCAC | 4769 |
| 346 | GGCUCUGC G UGGAGACC | 3484 | GGTCTCCA GGCTAGCTACAACGA GCAGAGCC | 4770 |
| 352 | GCGUGGAG A CCCGGGCC | 3485 | GGCCCGGG GGCTAGCTACAACGA CTCCACGC | 4771 |
| 358 | AGACCCGG G CCGCCUCU | 3486 | AGAGGCGG GGCTAGCTACAACGA CCGGGTCT | 4772. |
| 361 | CCCGGGCC G CCUCUGUG | 3487 | CACAGAGG GGCTAGCTACAACGA GGCCCGGG | 4773 |
| 367 | CCGCCUCU G UGGGUUUG | 3488 | CAAACCCA GGCTAGCTACAACGA AGAGGCGG | 4774 |
| 371 | CUCUGUGG G UUUGCCUA | 3489 | TAGGCAAA GGCTAGCTACAACGA CCACAGAG | 4775 |
| 375 | GUGGGUUU G CCUAGUGU | 3490 | ACACTAGG GGCTAGCTACAACGA AAACCCAC | 4776 |
| 380 | UUUGCCUA G UGUUUCUC | 3491 | GAGAAACA GGCTAGCTACAACGA TAGGCAAA | 4777 |
| 382 | UGCCUAGU G UUUCUGUU | 3492 | AAGAGAAA GGCTAGCTACAACSA ASTAGGCA | 4778 |
| 392 | UUCUCUUG A UCUGCCCA | 3493 | TGGGCAGA GGCTAGCTACAACGA CAAGAGAA | 4779 |
| 396 | CUUGAUCU G CCCAGGCU | 3494 | AGCCTGGG GGCTAGCTACAACGA AGATCAAG | 4780 |
| 402 | CUGCCCAG G CUCAGCAU | 3495 | ATGCTGAG GGCTAGCTACAACGA CTGGGCAG | 4781 |
| 407 | CAGGCUCA G CAUACAAA | 3496 | TTTGTATG GGCTAGCTACAACGA TGAGCCTG | 4782 |
| 409 | GGCUCAGC A UACAAAAA | 3497 | TTTTTGTA GGCTAGCTACAACGA GCTGAGCC | 4783 |
| 411 | CUCAGCAU A CAAAAAGA | 3498 | TCTTTTTG GGCTAGCTACAACGA ATGCTGAG | 4784 |
| 419 | ACAAAAAG A CAUACUUA | 3499 | TAAGTATG GGCTAGCTACAACGA CTTTTTGT | 4785 |
| 421 | AAAAAGAC A UACUUACA | 3500 | TGTAAGTA GGCTAGCTACAACGA GTCTTTTT | 4786 |
| 423 | AAAGACAU A CUUACAAU | 3501 | ATTGTAAG GGCTAGCTACAACGA ATGTCTTT | 4787 |
| 427 | ACAUACUU A CAAUUAAG | 3502 | CTTAATTG GGCTAGCTACAACGA AAGTATGT | 4788 |
| 430 | UACUUACA A UUAAGGCU | 3503 | AGCCTTAA GGCTAGCTACAACGA TGTAAGTA | 4789 |
| 436 | CAAUUAAG G CUAAUACA | 3504 | TGTATTAG GGCTAGCTACAACGA CTTAATTG | 4790 |
| 440 | UAAGGCTA A UACAACUC | 3505 | GAGTTGTA GGCTAGCTACAACGA TAGCCTTA | 4791 |
| 442 | AGGCUAAU A CAACUCUU | 3506 | AAGAGTTG GGCTAGCTACAACGA ATTAGCCT | 4792 |
| 445 | CUAAUACA A CUCUUCAA | 3507 | TTGAAGAG GGCTAGCTACAACGA TGTATTAG | 4793 |
| 454 | CUCUUCAA A UUACUUGC | 3508 | GCAAGTAA GGCTAGCTACAACGA TTGAAGAG | 4794 |
| 457 | UUCAAAUU A CUUGCAGG | 3509 | CCTGCAAG GGCTAGCTACAACGA AATTTGAA | 4795 |
| 461 | AAUUACUU G CAGGGGAC | 3510 | GTCCCCTG GGCTAGCTACAACGA AAGTAATT | 4796 |
| 468 | UGCAGGGG A CAGAGGGA | 3511 | TCCCTCTG GGCTAGCTACAACGA CCCCTGCA | 4797 |
| 476 | ACAGAGGG A CUUGGACU | 3512 | AGTCCAAG GGCTAGCTACAACGA CCCTCTGT | 4798 |
| 482 | GGACUUGG A CUGGCUUU | 3513 | AAAGCCAG GGCTAGCTACAACGA CCAAGTCC | 4799 |
| 486 | UUGGACUG G CUUUGGCC | 3514 | GGCCAAAG GGCTAGCTACAACGA CAGTCCAA | 4800 |
| 492 | UGGCCUUUG G CCCAAUAA | 3515 | TTATTGGG GGCTAGCTACAACGA CAAAGCCA | 4801 |
| 497 | UUGGCCCA A UAAUCAGA | 3516 | TCTGATTA GGCTAGCTACAACGA TGGGCCAA | 4802 |
| 500 | GCCCAAUA A UCAGAGUG | 3517 | CACTCTGA GGCTAGCTACAACGA TATTGGGC | 4803 |
| 506 | UAAUCAGA G UGGCAGUG | 3518 | CACTGCCA GGCTAGCTACAACGA TCTGATTA | 4804 |
| 509 | UCAGAGUG G GAGUGAGC | 3519 | GCTCACTG GGCTAGCTACAACGA CACTCTGA | 4805 |
| 512 | GAGUGGCA G UGAGCAAA | 3520 | TTTGCTCA GGCTAGCTACAACGA TGCCACTC | 4806 |
| 516 | GGCAGUGA G CAAAGGGU | 3521 | ACCCTTTG GGCTAGCTACAACGA TCACTGCC | 4807 |
| 523 | AGCAAAGG G UGGAGGUG | 3522 | CACCTCCA GGCTAGCTACAACGA CCTTTGCT. | 4808 |
| 529 | GGGUGGAG G UGACUGAG | 3523 | CTCAGTCA GGCTAGCTACAACGA CTCCACCC | 4809 |
| 532 | UGGAGGUG A CUGAGUGC | 3524 | GCACTCAG GGCTAGCTACAACGA CACCTCCA | 4810 |
| 537 | GUGACUGA G UGCAGCGA | 3525 | TCGCTGCA GGCTAGCTACAACGA TCAGTCAC | 4811 |
| 539 | GACUGAGU G CAGCGAUG | 3526 | CATCGCTG GGCTAGCTACAACGA ACTCAGTC | 4812 |
| 542 | UGAGUGCA G CGAUGGCC | 3527 | GGCCATCG GGCTAGCTACAACGA TGCACTCA | 4813 |
| 545 | GUGCAGCG A UGGCCUCU | 3528 | AGAGGCCA GGCTAGCTACAACGA CGCTGCAC | 4814 |
| 548 | CAGCGAUG G CCUCUUCU | 3529 | AGAAGAGG GGCTAGCTACAACGA CATCGCTG | 4815 |
| 557 | CCUCUUCU G UAAGACAC | 3530 | GTGTCTTA GGCTAGCTACAACGA AGAAGAGG | 4816 |
| 562 | UCUGUAAG A CACUCACA | 3531 | TGTGAGTG GGCTAGCTACAACGA CTTACAGA | 4817 |
| 564 | UGUAAGAC A CUCACAAU | 3532 | ATTGTGAG GGCTAGCTACAACGA GTCTTACA | 4818 |
| 568 | AGACACUC A CAAUUCCA | 3533 | TGGAATTG GGCTAGCTACAACGA GAGTGTCT | 4819 |
| 571 | CACUCACA A UUCCAAAA | 3534 | TTTTGGAA GGCTAGCTACAACGA TGTGAGTG | 4820 |
| 580 | UUCCAAAA G UGAUCGGA | 3535 | TCCGATCA GGCTAGCTACAACGA TTTTGGAA | 4821 |
| 583 | CAAAAGUG A UCGGAAAU | 3536 | ATTTCCGA GGCTAGCTACAACGA CACTTTTG | 4822 |
| 590 | GAUCGGAA A UGACACUG | 3537 7 | CAGTGTCA GGCTAGCTACAACGA TTCCGATC | 4823 |
| 593 | CGGAAAUG A CACUGGAG | 3538 | CTCCAGTG GGCTACCTACAACGA CATTTCCG | 4824 |
| 595 | GAAAUGAC A CUGGAGCC | 3539 | GGCTCCAG GGCTAGCTACAACGA GTCATTTC | 4825 |
| 601 | ACACUGGA G CCUACAAG | 3540 | CTTGTAGG GGCTAGCTACAACGA TCCAGTGT | 4826 |
| 605 | UGGAGCCU A CAAGUGCU | 3541 | AGCACTTG GGCTAGCTACAACGA AGGCTCCA | 4827 |
| 609 | GCCUACAA G UGCUUCUA | 3542 | TAGAAGCA GGCTAGCTACAACGA TTGTAGGC | 4828 |
| 611 | CUACAAGU G CUUCUACC | 3543 | GGTAGAAG GGCTAGCTACAACGA ACTTGTAG | 4829 |
| 617 | GUGCUUCU A CCGGGAAA | 3544 | TTTCCCGG GGCTAGCTACAACGA AGAAGCAC | 4830 |
| 625 | ACCGGGAA A CUGACUUG | 3545 | CAAGTCAG GGCTAGCTACAACGA TTCCCGGT | 4831 |
| 629 | GGAAACUG A CUUGGCCU | 3546 | AGGCCAAG GGCTAGCTACAACGA CAGTTTCC | 4832 |
| 634 | CUGACUUG G CCUCGGUC | 3547 | GACCGAGG GGCTAGCTACAACGA CAAGTCAG | 4833 |
| 640 | UGGCCUCG G UCAUUUAU | 3548 | ATAAATGA GGCTAGCTACAACGA CGAGGCCA | 4834 |
| 643 | CCUCGGUC A UUUAUGUC | 3549 | GACATAAA GGCTAGCTACAACGA GACCGAGG | 4835 |
| 647 | GGUCAUUU A UGUCUAUG | 3550 | CATAGACA GGCTAGCTACAACGA AAATGACC | 4836 |
| 649 | UCAUUUAU G UCUAUGUU | 3551 | AACATAGA GGCTAGCTACAACGA ATAAATGA | 4837 |
| 653 | UUAUGUCU A UGUUCAAG | 3552 | CTTGAACA GGCTAGCTACAACGA AGACATAA | 4838 |
| 655 | AUGUCUAU G UUCAAGAU | 3553 | ATCTTGAA GGCTAGCTACAACGA ATAGACAT | 4839 |
| 662 | UGUUCAAG A UUACAGAU | 3554 | ATCTGTAA GGCTAGCTACAACGA CTTGAACA | 4840 |
| 665 | UCAAGAUU A CAGAUCUC | 3555 | GAGATCTG GGCTAGCTACAACGA AATCTTGA | 4841 |
| 669 | GAUUACAG A UCUCCAUU | 3556 | AATGGAGA GGCTAGCTACAACGA CTGTAATC | 4842 |
| 675 | AGAUCUCC A UUUAUUGC | 3557 | GCAATAAA GGCTAGCTACAACGA GGAGATCT | 4843 |
| 679 | CUCCAUUU A UUGCUUCU | 3558 | AGAAGCAA GGCTAGCTACAACGA AAATGGAG | 4844 |
| 682 | CAUUUAUU G CUUCUGUU | 3559 | AACAGAAG GGCTAGCTACAACGA AATAAATG | 4845 |
| 688 | UUGCUUCU G UUAGUGAC | 3560 | GTCACTAA GGCTAGCTACAACGA AGAAGCAA | 4846 |
| 692 | UUCUGUUA G UGACCAAC | 3561 | GTTGGTCA GGCTAGCTACAACGA TAACAGAA | 4847 |
| 695 | UGUUAGUG A CCAACAUG | 3562 | CATGTTGG GGCTAGCTACAACGA CACTAACA | 4848 |
| 699 | AGUGACCA A CAUGGAGU | 3563 | ACTCCATG GGCTAGCTACAACGA TGGTCACT | 4849 |
| 701 | UGACCAAC A UGGAGUCG | 3564 | CGACTCCA GGCTAGCTACAACGA GTTGGTCA | 4850 |
| 706 | AACAUGGA G UCGUGUAC | 3565 | GTACACGA GGCTAGCTACAACGA TCCATGTT | 4851 |
| 709 | AUGGAGUC G UGUACAUU | 3566 | AATGTACA GGCTAGCTACAACGA GACTCCAT | 4852 |
| 711 | GGAGUCGU G UACAUUAC | 3567 | GTAATGTA GGCTAGCTACAACGA ACGACTCC | 4853 |
| 713 | AGUCGUGU A CAUUACUG | 3568 | CAGTAATG GGCTAGCTACAACGA ACACGACT | 4854 |
| 715 | UCGUGUAC A UUACUGAG | 3569 | CTCAGTAA GGCTAGCTACAACGA GTACACGA | 4855 |
| 718 | UGUACAUU A CUGAGAAC | 3570 | GTTCTCAG GGCTAGCTACAACGA AATGTACA | 4856 |
| 725 | UACUGAGA A CAAAAACA | 3571 | TGTTTTTG GGCTAGCTACAACGA TCTCAGTA | 4857 |
| 731 | GAACAAAA A CAAAACUG | 3572 | CAGTTTTG GGCTAGCTACAACGA. TTTTGTTC | 4858 |
| 736 | AAAACAAA A CUGUGGUG | 3573 | CACCACAG GGCTAGCTACAACGA TTTGTTTT | 4859 |
| 739 | ACAAAACU G UGGUGAUU | 3574 | AATCACCA GGCTAGCTACAACGA AGTTTTGT | 4860 |
| 742 | AAACUGUG G UGAUUCCA | 3575 | TGGAATCA GGCTAGCTACAACGA CACAGTTT | 4861 |
| 745 | CUGUGGUG A UUCCAUGU | 3576 | ACATGGAA GGCTAGCTACAACGA CACCACAG | 4862 |
| 750 | GUGAUUCC A UGUCUCGG | 3577 | CCGAGACA GGCTAGCTACAACGA GGAATCAC | 4863 |
| 752 | GAUUCCAU G UCUCGGGU | 3578 | ACCCGAGA GGCTAGCTACAACGA ATGGAATC | 4864 |
| 759 | UGUCUCGG G UCCAUUUC | 3579 | GAAATGGA GGCTAGCTACAACGA CCGAGAGA | 4865 |
| 763 | UCGGGUCC A UUUCAAAU | 3580 | ATTTGAAA GGCTAGCTACAACGA GGACCCGA | 4866 |
| 770 | CAUUUCAA A UCUCAACG | 3581 | CGTTGAGA GGCTAGCTACAACGA TTGAAATG | 4867 |
| 776 | AAAUCUCA A CGUGUCAC | 3582 | GTGACACG GGCTAGCTACAACGA TGAGATTT | 4868 |
| 778 | AUCUCAAC G UGUCACUU | 3583 | AAGTGACA GGCTAGCTACAACGA GTTGAGAT | 4869 |
| 780 | CUCAACGU G UCACUUUG | 3584 | CAAAGTGA GGCTAGCTACAACGA ACGTTGAG | 4870 |
| 783 | AACGUGUC A CUUUGUGC | 3585 | GCACAAAG GGCTAGCTACAACGA GACACGTT | 4871 |
| 788 | GUCACUUU G UGCAAGAU | 3586 | ATCTTGCA GGCTAGCTACAACGA AAAGTGAC | 4872 |
| 790 | CACUUUGU G CAAGAUAC | 3587 | GTATCTTG GGCTAGCTACAACGA ACAAAGTG | 4873 |
| 795 | UGUGCAAG A UACCCAGA | 3588 | TCTGGGTA GGCTAGCTACAACGA CTTGCACA | 4874 |
| 797 | UGCAAGAU A CCCAGAAA | 3589 | TTTCTGGG GGCTAGCTACAACGA ATCTTGCA | 4875 |
| 810 | GAAAAGAG A UUUGUUCC | 3590 | GGAACAAA GGCTAGCTACAACGA CTCTTTTC | 4876 |
| 814 | AGAGAUUU G UUCCUGAU | 3591 | ATCAGGAA GGCTAGCTACAACGA AAATCTCT | 4877 |
| 821 | UGUUCCUG A UGGUAACA | 3592 | TGTTACCA GGCTAGCTACAACGA CAGGAACA | 4878 |
| 824 | UCCUGAUG G UAACAGAA | 3593 | TTCTGTTA GGCTAGCTACAACGA CATCAGGA | 4879 |
| 827 | UGAUGGUA A CAGAAUUU | 3594 | AAATTCTG GGCTAGCTACAACGA TACCATCA | 4880 |
| 832 | GUAACAGA A UUUCCUGG | 3595 | CCAGGAAA GGCTAGCTACAACGA TCTGTTAC | 4881 |
| 842 | UUCCUGGG A CAGCAAGA | 3596 | TCTTGCTG GGCTAGCTACAACGA CCCAGGAA | 4882 |
| 845 | CUGGGACA G CAAGAAGG | 3597 | CCTTCTTG GGCTAGCTACAACGA TGTCCCAG | 4883 |
| 854 | CAAGAAGG G CUUUACUA | 3598 | TAGTAAAG GGCTAGCTACAACGA CCTTCTTG | 4884 |
| 859 | AGGGCUUU A CUAUUCCC | 3599 | GGGAATAG GGCTAGCTACAACGA AAAGCCCT | 4885 |
| 862 | GCUUUACU A UUCCGAGC | 3600 | GCTGGGAA GGCTAGCTACAACGA AGTAAAGC | 4886 |
| 869 | UAUUCCCA G CUACAUGA | 3601 | TCATGTAG GGCTAGCTACAACGA TGGGAATA | 4887 |
| 872 | UCCCAGCU A CAUGAUCA | 3602 | TGATCATG GGCTAGCTACAACGA AGCTGGGA | 4888 |
| 874 | CCAGCUAC A UGAUCAGC | 3603 | GCTGATCA GGCTAGCTACAACGA GTAGCTGG | 4889 |
| 877 | GCUACAUG A UCAGCUAU | 3604 | ATAGCTGA GGCTAGCTACAACGA CATGTAGC | 4890 |
| 881 | CAUGAUCA G CUAUGCUG | 3605 | CAGCATAG GGCTAGCTACAACGA TGATCATG | 4891 |
| 884 | GAUCAGCU A UGCUGGCA | 3606 | TGCCAGCA GGCTAGCTACAACGA AGCTGATC | 4892 |
| 886 | UCAGCUAU G CUGGCAUG | 3607 | CATGCCAG GGCTAGCTACAACGA ATAGCTGA | 4893 |
| 890 | CUAUGCUG G CAUGGUCU | 3608 | AGACCATG GGCTAGCTACAACGA CAGCATAG | 4894 |
| 892 | AUGCUGGC A UGGUCUUC | 3609 | GAAGACCA GGCTAGCTACAACGA GCCAGCAT | 4895 |
| 895 | CUGGCAUG G UCUUCUGU | 3610 | ACAGAAGA GGCTAGCTACAACGA CATGCCAG | 4896 |
| 902 | GGUCUUCU G UGAAGCAA | 3611 | TTGCTTCA GGCTAGCTACAACGA AGAAGACC | 4897 |
| 907 | UCUGUGAA G CAAAAAUU | 3612 | AATTTTTG GGCTAGCTACAACGA TTCACAGA | 4898 |
| 913 | AAGCAAAA A UUAAUGAU | 3613 | ATCATTAA GGCTAGCTACAACGA TTTTGCTT | 4899 |
| 917 | AAAAAUUA A UGAUGAAA | 3614 | TTTCATCA GGCTAGCTACAACGA TAATTTTT | 4900 |
| 920 | AAUUAAUG A UGAAAGUU | 3615 | AACTTTCA GGCTAGCTACAACGA CATTAATT | 4901 |
| 926 | UGAUGAAA G UUACCAGU | 3616 | ACTGGTAA GGCTAGCTACAACGA TTTCATCA | 4902 |
| 929 | UGAAAGUU A CCAGUCUA | 3617 | TAGACTGG GGCTAGCTACAACGA AACTTTCA | 4903 |
| 933 | AGUUACCA G UCUAUUAU | 3618 | ATAATAGA GGCTAGCTACAACGA TGGTAACT | 4904 |
| 937 | ACCAGUCU A UUAUGUAC | 3619 | GTACATAA GGCTAGCTACAACGA AGACTGGT | 4905 |
| 940 | AGUCUAUU A UGUACAUA | 3620 | TATGTACA GGCTAGCTACAACGA AATAGACT | 4906 |
| 942 | UCUAUUAU G UACAUAGU | 3621 | ACTATGTA GGCTAGCTACAACGA ATAATAGA | 4907 |
| 944 | UAUUAUGU A CAUAGUUG | 3622 | CAACTATG GGCTAGCTACAACGA ACATAATA | 4908 |
| 946 | UUAUGUAC A UAGUUGUC | 3623 | GACAACTA GGCTAGCTACAACGA GTACATAA | 4909 |
| 949 | UGUACAUA G UUGUCGUU | 3624 | AACGACAA GGCTAGCTACAACGA TATGTACA | 4910 |
| 952 | ACAUAGUU G UCGUUGUA | 3625 | TACAACGA GGCTAGCTACAACGA AACTATGT | 4911 |
| 955 | UAGUUGUC G UUGUAGGG | 3626 | CCCTACAA GGCTAGCTACAACGA GACAACTA | 4912 |
| 958 | UUGUCGUU G UAGGGUAU ATACCCTA | 3627 | GGCTAGCTACAACGA AACGACAA | 4913 |
| 963 | GUUGUAGG G UAUAGGAU | 3628 | ATCCTATA GGCTAGCTACAACGA CCTACAAC | 4914 |
| 965 | UGUAGGGU A UAGGAUUU | 3629 | AAATCCTA GGCTAGCTACAACGA ACCCTACA | 4915 |
| 970 | GGUAUAGG A UUUAUGAU | 3630 | ATCATAAA GGCTAGCTACAACGA CCTATACC | 4916 |
| 974 | UAGGAUUU A UGAUGUGG | 3631 | CCACATCA GGCTAGCTACAACGA AAATCCTA | 4917 |
| 977 | GAZIWAUG A UGUGGWC | 3632 | GAACCACA GGCTAGCTACAACGA CATAAATC | 4918 |
| 979 | UUUAUGAU G UGGUUCUG | 3633 | CAGAACCA GGCTAGCTACAACGA ATCATAAA | 4919 |
| 982 | AUGAUGUG G UUCUGAGU | 3634 | ACTCAGAA GGCTAGCTACAACGA CACATCAT | 4920 |
| 989 | GGUUCUGA G UCCGUCUC | 3635 | GAGACGGA GGCTAGCTACAACGA TCAGAACC | 4921 |
| 993 | CUGAGUCC G UCUCAUGG | 3636 | CCATGAGA GGCTAGCTACAACGA GGACTCAG | 4922 |
| 998 | UCCGUCUC A UGGAAUUG | 3637 | CAATTCCA GGCTAGCTACAACGA GAGACGGA | 4923 |
| 1003 | CUCAUGGA A UUGAACUA | 3638 | TAGTTCAA GGCTAGCTACAACGA TCCATGAG | 4924 |
| 1008 | GGAAUUGA A CUAUCUGU | 3639 | ACAGATAG GGCTAGCTACAACGA TCAATTCC | 4925 |
| 1011 | AUUGAACU A UCUGUUGG | 3640 | CCAACAGA GGCTAGCTACAACGA AGTTCAAT | 4926 |
| 1015 | AACUAUCU G UUGGACAA | 3641 | TTCTCCAA GGCTAGCTACAACGA AGATAGTT | 4927 |
| 1026 | GGAGAAAA G CUUGUCUU | 3642 | AAGACAAG GGCTAGCTACAACGA TTTTCTCC | 4928 |
| 1030 | AAAAGCUU G UCUUAAAU | 3643 | ATTTAAGA GGCTAGCTACAACGA AAGCTTTT | 4929 |
| 1037 | UGUCUUAA A UUGUACAG | 3644 | CTGTACAA GGCTAGCTACAACGA TTAAGACA | 4930 |
| 1040 | CUUAAAUU G UACAGCAA | 3645 | TTGCTGTA GGCTAGCTACAACGA AATTTAAG | 4931 |
| 1042 | UAAAUUGU A CAGCAAGA | 3646 | TCTTGCTG GGCTAGCTACAACGA ACAATTTA | 4932 |
| 1045 | AUUGUACA G CAAGAACU | 3647 | AGTTCTTG GGCTAGCTACAACGA TGTACAAT | 4933 |
| 1051 | CAGCAAGA A CUGAACUA | 3648 | TAGTTCAG GGCTAGCTACAACGA TCTTGCTG | 4934 |
| 1056 | AGAACUGA A CUAAAUGU | 3649 | ACATTTAG GGCTAGCTACAACGA TCAGTTCT | 4935 |
| 1061 | UGPACUAA A UGUGGGGA | 3650 | TCCCCACA GGCTAGCTACAACGA TTAGTTCA | 4936 |
| 1063 | AACUAAAU G UGGGGAUU | 3651 | AATCCCCA GGCTAGCTACAACGA ATTTAGTT | 4937 |
| 1069 | AUGUGGGG A UUGACUUC | 3652 | GAAGTCAA GGCTAGCTACAACGA CCCCACAT | 4938 |
| 1073 | GGGGAUUG A CUUCAACU | 3653 | AGTTGAAG GGCTAGCTACAACGA CAATCCCC | 4939 |
| 1079 | UGACUUCA A CUGGGAAU | 3654 | ATTCCCAG GGCTAGCTACAACGA TGAAGTCA | 4940 |
| 1086 | AACUGGGA A UACCCUUC | 3655 | GAAGGGTA GGCTAGCTACAACGA TCCCAGTT | 4941 |
| 1088 | CUGGGAAU A CCCUUCUU | 3656 | AAGAAGGG GGCTAGCTACAACGA ATTCCCAG | 4942 |
| 1101 | UCUUCGAA G CAUCAGCA | 3657 | TGCTGATG GGCTAGCTACAACGA TTCGAAGA | 4943 |
| 1103 | UUCGAAGC A UCAGCAUA | 3658 | TATGCTGA GGCTAGCTACAACGA GCTTCGAA | 4944 |
| 1107 | AAGCAUCA G CAUAAGAA | 3659 | TTCTTATG GGCTAGCTACAACGA TGATGCTT | 4945 |
| 1109 | GCAUCAGC A UAAGAAAC | 3660 | GTTTCTTA GGCTAGCTACAACGA GCTGATGC | 4946 |
| 1116 | CAUAAGAA A CUUGUAAA | 3661 | TTTACAAG GGCTAGCTACAACGA TTCTTATG | 4947 |
| 1120 | AGAAACUU G UAAACCGA | 3662 | TCGGTTTA GGCTAGCTACAACGA AAGTTTCT | 4948 |
| 1124 | ACUUGUAA A CCGAGACC | 3663 | GGTCTCGG GGCTAGCTACAACGA TTACAAGT | 4949 |
| 1130 | AAACCGAG A CCUAAAAA | 3664 | TTTTTAGG GGCTAGCTACAACGA CTCGGTTT | 4950 |
| 1138 | ACCUAAAA A CCCAGUCU | 3665 | AGACTGGG GGCTAGCTACAACGA TTTTAGGT | 4951 |
| 1143 | AAAACCCA G UCUGGGAG | 3666 | CTCCCAGA GGCTAGCTACAACGA TGGGTTTT | 4952 |
| 1151 | GUCUGGGA G UGAGAUGA | 3667 | TCATCTCA GGCTAGCTACAACGA TCCCAGAC | 4953 |
| 1156 | GGAGUGAG A UGAAGAAA | 3668 | TTTCTTCA GGCTAGCTACAACGA CTCACTCC | 4954 |
| 1164 | AUGAAGAA A UUUUUGAG | 3669 | CTCAAAAA GGCTAGCTACAACGA TTCTTCAT | 4955 |
| 1172 | AUUUUUGA G CACCUUAA | 3670 | TTAAGGTG GGCTAGCTACAACGA TCAAAAAT | 4956 |
| 1174 | UUUUGAGC A CCUUAACU | 3671 | AGTTAAGG GGCTAGCTACAACGA GCTCAAAA | 4957 |
| 1180 | GCACCUUA A CUAUAGAU | 3672 | ATCTATAG GGCTAGCTACAACGA TAAGGTGC | 4958 |
| 1183 | CCUUAACU A UAGAUGGU | 3673 | ACCATCTA GGCTAGCTACAACGA AGTTAAGG | 4959 |
| 1187 | AACUAUAG A UGGUGUAA | 3674 | TTACACCA GGCTAGCTACAACGA CTATAGTT | 4960 |
| 1190 | UAUAGAUG G UGUAACCC | 3675 | GGGTTACA GGCTAGCTACAACGA CATCTATA | 4961 |
| 1192 | UAGAUGGU G UAACCCGG | 3676 | CCGGGTTA GGCTAGCTACAACGA ACCATCTA. | 4962 |
| 1195 | AUGGUGUA A CCCGGAGU | 3677 | ACTCCGGG GGCTAGCTACAACGA TACACCAT | 4963 |
| 1202 | AACCCGGA G UGACCAAG | 3678 | CTTGGTCA GGCTAGCTACAACGA TCCGGGTT | 4964 |
| 1205 | CCGGAGUG A CCAAGGAU | 3679 | ATCCTTGG GGCTAGCTACAACGA CACTCCGG | 4965 |
| 1212 | GACCAAGG A UUGUACAC | 3680 | GTGTACAA GGCTAGCTACAACGA CCTTGGTC | 4966 |
| 1215 | CAAGGAUU G UACACCUG | 3681 | CAGGTGTA GGCTAGCTACAACGA AATCCTTG | 4967 |
| 1217 | AGGAUUGU A CACCUGUG | 3682 | CACAGGTG GGCTAGCTACAACGA ACAATCCT | 4968 |
| 1219 | GAUUGUAC A CCUGUGCA | 3683 | TGCACAGG GGCTAGCTACAACGA GTACAATC | 4969 |
| 1223 | GUACACCU G UGCAGCAU | 3684 | ATGCTGCA GGCTAGCTACAACGA AGGTGTAC | 4970 |
| 1225 | ACACCUGU G CAGCAUCC | 3685 | GGATGCTG GGCTAGCTACAACGA ACAGGTGT | 4971 |
| 1228 | CCUGUGCA G CAUCCAGU | 3686 | ACTGGATG GGCTAGCTACAACGA TGCACAGG | 4972 |
| 1230 | UGUGCAGC A UCCAGUGG | 3687 | CCACTGGA GGCTAGCTACAACGA GCTGCACA | 4973 |
| 1235 | AGCAUCCA G UGGGCUGA | 3688 | TCAGCCCA GGCTAGCTACAACGA TGGATGCT | 4974 |
| 1239 | UCCAGUGG G CUGAUGAC | 3689 | GTCATCAG GGCTAGCTACAACGA CCACTGGA | 4975 |
| 1243 | GUGGGCUG A UGACCAAG | 3690 | CTTGGTCA GGCTAGCTACAACGA CAGCCCAC | 4976 |
| 1246 | GGCUGAUG A CCAAGAAG | 3691 | CTTCTTGG GGCTAGCTACAACGA CATCAGCC | 4977 |
| 1256 | CAAGAAGA A CAGCACAU | 3692 | ATGTGCTG GGCTAGCTACAACGA TCTTCTTG | 4978 |
| 1259 | GAAGAACA G CACAUUUG | 3693 | CAAATGTG GGCTAGCTACAACGA TGTTCTTC | 4979 |
| 1261 | AGAACAGC A CAUUUGUC | 3694 | GACAAATG GGCTAGCTACAACGA GCTGTTCT | 4980 |
| 1263 | AACAGCAC A UUUGUCAG | 3695 | CTGACAAA GGCTAGCTACAACGA GTGCTGTT | 4981 |
| 1267 | GCACAUUU G UCAGGGUC | 3696 | GACCCTGA GGCTAGCTACAACGA AAATGTGC | 4982 |
| 1273 | UUGUCAGG G UCCAUGAA | 3697 | TTCATGGA GGCTAGCTACAACGA CCTGACAA | 4983 |
| 1277 | CAGGGUCC A UGAAAAAC | 3698 | GTTTTTCA GGCTAGCTACAACGA GGACCCTG | 4984 |
| 1284 | CAUGAAAA A CCUUUUGU | 3699 | ACAAAAGG GGCTAGCTACAACGA TTTTCATG | 4985 |
| 1291 | AACCUUUU G UUGCUUUU | 3700 | AAAAGCAA GGCTAGCTACAACGA AAAAGGTT | 4986 |
| 1294 | CUUUUGUU G CUUUUGGA | 3701 | TCCAAAAG GGCTAGCTACAACGA AACAAAAG | 4987 |
| 1304 | UUUUGGAA G UGGCAUGG | 3702 | CCATGCCA GGCTAGCTACAACGA TTCCAAAA | 4988 |
| 1307 | UGGAAGUG G CAUGGAAU | 3703 | ATTCCATG GGCTAGCTACAACGA CACTTCCA | 4989 |
| 1309 | GAAGUGGC A UGGAAUCU | 3704 | AGATTCCA GGCTAGCTACAACGA GCCACTTC | 4990 |
| 1314 | GGCAUGGA A UCUCUGGU | 3705 | ACCAGAGA GGCTAGCTACAACGA TCCATGCC | 4991 |
| 1321 | AAUCUCUG G UGGAAGCC | 3706 | GGCTTCCA GGCTAGCTACAACGA CAGAGATT | 4992 |
| 1327 | UGGUGGAA G CCAGGGUG | 3707 | CACCGTGG GGCTAGCTACAACGA TTCCACCA | 4993 |
| 1330 | UGGAAGCC A CGGUGGGG | 3708 | CCCCACCG GGCTAGCTACAACGA GGCTTCCA | 4994 |
| 1333 | AAGCCACG G UGGGGGAG | 3709 | CTCCCCCA GGCTAGCTACAACGA CGTGGCTT | 4995 |
| 1341 | GUGGGGGA G CGUGUCAG | 3710 | CTGACACG GGCTAGCTACAACGA TCCCCCAC | 4996 |
| 1343 | GGGGGAGC G UGUCAGAA | 3711 | TTCTGACA GGCTAGCTACAACGA GCTCCCCC | 4997 |
| 1345 | GGGAGCGU G UCAGAAUC | 3712 | GATTCTGA GGCTAGCTACAACGA ACGCTCCC | 4998 |
| 1351 | GUGUCAGA A UCCCUGCG | 3713 | CGCAGGGA GGCTAGCTACAACGA TCTGACAC | 4999 |
| 1357 | GAAUCCCU G CGAAGUAC | 3714 | GTACTTCG GGCTAGCTACAACGA AGGGATTC | 5000 |
| 1362 | CCUGCGAA G UACCUUGG | 3715 | CCAAGGTA GGCTAGCTACAACGA TTCGCAGG | 5001 |
| 1364 | UGCGAAGU A CCUUGGUU | 3716 | AACCAAGG GGCTAGCTACAACGA ACTTCGCA | 5002 |
| 1370 | GUACCUUG G UUACCCAC | 3717 | GTGGGTAA GGCTAGCTACAACGA CAAGGTAC | 5003 |
| 1373 | CCUUGGUU A CCCACCCC | 3718 | GGGGTGGG GGCTAGCTACAACGA AACCAAGG | 5004 |
| 1377 | GGUUACCC A CCCCCAGA | 3719 | TCTGGGGG GGCTAGCTACAACGA GGGTAACC | 5005 |
| 1387 | CCCCAGAA A UAAAAUGG | 3720 | CCATTTTA GGCTAGCTACAACGA TTCTGGGG | 5006 |
| 1392 | GAAAUAAA A UGGUAUAA | 3721 | TTATACCA GGCTAGCTACAACGA TTTATTTC | 5007 |
| 1395 | AUAAAAUG G UAUAAAAA | 3722 | TTTTTATA GGCTAGCTACAACGA CATTTTAT | 5008 |
| 1397 | AAAAUGGU A UAAAAAUG | 3723 | CATTTTTA GGCTAGCTACAACGA ACCATTTT | 5009 |
| 1403 | GUAUAAAA A UGGAAUAC | 3724 | GTATTCCA GGCTAGCTACAACGA TTTTATAC | 5010 |
| 1408 | AAAAUGGA A UACCCCUU | 3725 | AAGGGGTA GGCTAGCTACAACGA TCCATTTT | 5011 |
| 1410 | AAUGGAAU A CCCCUUGA | 3726 | TCAAGGGG GGCTAGCTACAACGA ATTCCATT | 5012 |
| 1419 | CCCCUUGA G UCCAAUCA | 3727 | TGATTGGA GGCTAGCTACAACGA TCAAGGGG | 5013 |
| 1424 | UGAGUCCA A UCACACAA | 3728 | TTGTGTGA GGCTAGCTACAACGA TGGACTCA | 5014 |
| 1427 | GUCCAAUC A CACAAUUA | 3729 | TAATTGTG GGCTAGCTACAACGA GATTGGAC | 5015 |
| 1429 | CCAAUCAC A CAAUUAAA | 3730 | TTTAATTG GGCTAGCTACAACGA GTGATTGG | 5016 |
| 1432 | AUCACACA A UUAAAGCG | 3731 | CGCTTTAA GGCTAGCTACAACGA TGTGTGAT | 5017 |
| 1438 | CAAUUAAA G CGGGGCAU | 3732 | ATGCCCCG GGCTAGCTACAACGA TTTAATTG | 5018 |
| 1443 | AAAGCGGG G CAUGUACU | 3733 | AGTACATG GGCTAGCTACAACGA CCCGCTTT | 5019 |
| 1445 | AGCGGGGC A UGUACUGA | 3734 | TCAGTACA GGCTAGCTACAACGA GCCCCGCT | 5020 |
| 1447 | CGGGGCAU G UACUGACG | 3735 | CGTCAGTA GGCTAGCTACAACGA ATGCCCCG | 5021 |
| 1449 | GGGCAUGU A CUGACGAU | 3736 | ATCGTCAG GGCTAGCTACAACGA ACATGCCC | 5022 |
| 1453 | AUGUACUG A CGAUUAUG | 3737 | CATAATCG GGCTAGCTACAACGA CAGTACAT | 5023 |
| 1456 | UACUGACG A UUAUGGAA | 3738 | TTCCATAA GGCTAGCTACAACGA CGTCAGTA | 5024 |
| 1459 | UGACGAUU A UGGAAGUG | 3739 | CACTTCCA GGCTAGCTACAACGA AATCGTCA | 5025 |
| 1465 | UUAUGGAA G UGAGUGAA | 3740 | TTCACTCA GGCTAGCTACAACGA TTCCATAA | 5026 |
| 1469 | GGAAGUGA G UGAAAGAG | 3741 | CTCTTTCA GGCTAGCTACAACGA TCACTTCC | 5027 |
| 1478 | UGAAAGAG A CACAGGAA | 3742 | TTCCTGTG GGCTAGCTACAACGA CTCTTTCA | 5028 |
| 1480 | AAAGAGAC A CAGGAAAU | 3743 | ATTTCCTG GGCTAGCTACAACGA GTCTCTTT | 5029 |
| 1487 | CACAGGAA A UUACACUG | 3744 | CAGTGTAA GGCTAGCTACAACGA TTCCTGTG | 5030 |
| 1490 | AGGAAAUU A CACUGUCA | 3745 | TGACAGTG GGCTAGCTACAACGA AATTTCCT | 5031 |
| 1492 | GAAAUUAC A CUGUCAUC | 3746 | GATGACAG GGCTAGCTACAACGA GTAATTTC | 5032 |
| 1495 | AUUACACU G UCAUCCUU | 3747 | AAGGATGA GGCTAGCTACAACGA AGTGTAAT | 5033 |
| 1498 | ACACUGUC A UCCUUACC | 3748 | GGTAAGGA GGCTAGCTACAACGA GACAGTGT | 5034 |
| 1504 | UCAUCCUU A CCAAUCCC | 3749 | GGGATTGG GGCTAGCTACAACGA AAGGATGA | 5035 |
| 1508 | CCUUACCA A UCCCAUUU | 3750 | AAATGGGA GGCTAGCTACAACGA TGGTAAGG | 5036 |
| 1513 | CCAAUCCC A UUUCAAAG | 3751 | CTTTGAAA GGCTAGCTACAACGA GGGATTGG | 5037 |
| 1527 | AAGGAGAA G CAGAGCCA | 3752 | TGGCTCTG GGCTAGCTACAACGA TTCTCCTT | 5038 |
| 1532 | GAAGCAGA G CCAUGUGG | 3753 | CCACATGG GGCTAGCTACAACGA TCTGCTTC | 5039 |
| 1535 | GCAGAGCC A UGUGGUCU | 3754 | AGACCACA GGCTAGCTACAACGA GGCTCTGC | 5040 |
| 1537 | AGAGCCAU G UGGUCUCU | 3755 | AGAGACCA GGCTAGCTACAACGA ATGGCTCT | 5041 |
| 1540 | GCCAUGUG G UCUCUCUG | 3756 | CAGAGAGA GGCTAGCTACAACGA CACATGGC | 5042 |
| 1549 | UCUCUCUG G UUGUGUAU | 3757 | ATACACAA GGCTAGCTACAACGA CAGAGAGA | 5043 |
| 1552 | CUCUGGUU G UGUAUGUC | 3758 | GACATACA GGCTAGCTACAACGA AACCAGAG | 5044 |
| 1554 | CUGGUUGU G UAUGUCCC | 3759 | GGGACATA GGCTAGCTACAACGA ACAACCAG | 5045 |
| 1556 | GGUUGUGU A UGUCCCAC | 3760 | GTGGGACA GGCTAGCTACAACGA ACACAACC | 5046 |
| 1558 | UUGUGUAU G UCCCACCC | 3761 | GGGTGGGA GGCTAGCTACAACGA ATACACAA | 5047 |
| 1563 | UAUGUCCC A CCCCAGAU | 3762 | ATCTGGGG GGCTAGCTACAACGA GGGACATA | 5048 |
| 1570 | CACCCCAG A UUGGUGAG | 3763 | CTCACCAA GGCTAGCTACAACGA CTGGGGTG | 5049 |
| 1574 | CCAGAUUG G UGAGAAAU | 3764 | ATTTCTCA GGCTAGCTACAACGA CAATCTGG | 5050 |
| 1581 | GGUGAGAA A UCUCUAAU | 3765 | ATTAGAGA GGCTAGCTACAACGA TTCTCACC | 5051 |
| 1588 | AAUCUCUA A UCUCUCCU | 3766 | AGGAGAGA GGCTAGCTACAACGA TAGAGATT | 5052 |
| 1597 | UCUCUCCU G UGGAUUCC | 3767 | GGAATCCA GGCTAGCTACAACGA AGGAGAGA | 5053 |
| 1601 | UCCUGUGG A UUCCUACC | 3768 | GGTAGGAA GGCTAGCTACAACGA CCACAGGA | 5054 |
| 1607 | GGAUUCCU A CCAGUACG | 3769 | CGTACTGG GGCTAGCTACAACGA AGGAATCC | 5055 |
| 1611 | UCCUACCA G UACGGCAC | 3770 | GTGCCGTA GGCTAGCTACAACGA TGGTAGGA | 5056 |
| 1613 | CUACCAGU A CGGCACCA | 3771 | TGGTGCCG GGCTAGCTACAACGA ACTGGTAG | 5057 |
| 1616 | CCAGUACG G CACCACUC | 3772 | GAGTGGTG GGCTAGCTACAACGA CGTACTGG | 5058 |
| 1618 | AGUACGGC A CCACUCAA | 3773 | TTGAGTGG GGCTAGCTACAACGA GCCGTACT | 5059 |
| 1621 | ACGGCACC A CUCAAACG | 3774 | CGTTTGAG GGCTAGCTACAACGA GGTGCCGT | 5060 |
| 1627 | CCACUCAA A CGCUGACA | 3775 | TGTCAGCG GGCTAGCTACAACGA TTGAGTGG | 5061 |
| 1629 | ACUCAAAC G CUGACAUG | 3776 | CATGTCAG GGCTAGCTACAACGA GTTTGAGT | 5062 |
| 1633 | AAACGCUG A CAUGUACG | 3777 | CGTACATG GGCTAGCTACAACGA CAGCGTTT | 5063 |
| 1635 | ACGCUGAC A UGUACGGU | 3778 | ACCGTACA GGCTAGCTACAACGA GTCAGCGT | 5064 |
| 1637 | GCUGACAU G UACGGUCU | 3779 | AGACCGTA GGCTAGCTACAACGA ATGTCAGC | 5065 |
| 1639 | UGACAUGU A CGGUCUAU | 3780 | ATAGACCG GGCTAGCTACAACGA ACATGTCA | 5066 |
| 1642 | CAUGUACG G UCUAUGCC | 3781 | GGCATAGA GGCTAGCTACAACGA CGTACATG | 5067 |
| 1646 | UACGGUCU A UGCCAUUC | 3782 | GAATGGCA GGCTAGCTACAACGA AGACCGTA | 5068 |
| 1648 | CGGUCUAU G CCAUUCCU | 3783 | AGGAATGG GGCTAGCTACAACGA ATAGACCG | 5069 |
| 1651 | UCUAUGCC A UUCCUCCC | 3784 | GGGAGGAA GGCTAGCTACAACGA GGCATAGA | 5070 |
| 1662 | CCUCCCCC G CAUCACAU | 3785 | ATGTGATG GGCTAGCTACAACGA GGGGGAGG | 5071 |
| 1664 | UCCCCCGC A UCACAUCC | 3786 | GGATGTGA GGCTAGCTACAACGA GCGGGGGA | 5072 |
| 1667 | CCCGCAUC A CAUCCACU | 3787 | AGTGGATG GGCTAGCTACAACGA GATGCGGG | 5073 |
| 1669 | CGCAUCAC A UCCACUGG | 3788 | CCAGTGGA GGCTAGCTACAACGA GTGATGCG | 5074 |
| 1673 | UCACAUCC A CUGGUAUU | 3789 | AATACCAG GGCTAGCTACAACGA GGATGTGA | 5075 |
| 1677 | AUCCACUG G UAUUGGCA | 3790 | TGCCAATA GGCTAGCTACAACGA CAGTGGAT | 5076 |
| 1679 | CCACUGGU A UUGGCAGU | 3791 | ACTGCCAA GGCTAGCTACAACGA ACCAGTGG | 5077 |
| 1683 | UGGUAUUG G CAGUUGGA | 3792 | TCCAACTG GGCTAGCTACAACGA CAATACCA | 5078 |
| 1686 | UAUUGGCA G UUGGAGGA | 3793 | TCCTCCAA GGCTAGCTACAACGA TGCCAATA | 5079 |
| 1698 | GAGGAAGA G UGCGCCAA | 3794 | TTGGCGCA GGCTAGCTACAACGA TCTTCCTC | 5080 |
| 1700 | GGAAGAGU G CGCCAACG | 3795 | CGTTGGCG GGCTAGCTACAACGA ACTCTTCC | 5081 |
| 1702 | AAGAGUGC G CCAACGAG | 3796 | CTCGTTGG GGCTAGCTACAACGA GCACTCTT | 5082 |
| 1706 | GUGCGCCA A CGAGCCCA | 3797 | TGGGCTCG GGCTAGCTACAACGA TGGCGCAC | 5083 |
| 1710 | GCCAACGA G CCCAGCCA | 3798 | TGGCTGGG GGCTAGCTACAACGA TCGTTGGC | 5084 |
| 1715 | CGAGCCCA G CCAAGCUG | 3799 | CAGCTTGG GGCTAGCTACAACGA TGGGCTCG | 5085 |
| 1720 | CCAGCCAA G CUGUCUCA | 3800 | TGAGACAG GGCTAGCTACAACGA TTGGCTGG | 5086 |
| 1723 | GCCAAGCU G UCUCAGUG | 3801 | CACTGAGA GGCTAGCTACAACGA AGCTTGGC | 5087 |
| 1729 | CUGUCUCA G UGACAAAC | 3802 | GTTTGTCA GGCTAGCTACAACGA TGAGACAG | 5088 |
| 1732 | UCUCAGUG A CAAACCCA | 3803 | TGGGTTTG GGCTAGCTACAACGA CACTGAGA | 5089 |
| 1736 | AGUGACAA A CCCAUACC | 3804 | GGTATGGG GGCTAGCTACAACGA TTGTCACT | 5090 |
| 1740 | ACAAACCC A UACCCUUG | 3805 | CAAGGGTA GGCTAGCTACAACGA GGGTTTGT. | 5091 |
| 1742 | AAACCCAU A CCCUUGUG | 3806 | CACAAGGG GGCTAGCTACAACGA ATGGGTTT | 5092 |
| 1748 | AUACCCUU G UGAAGAAU | 3807 | ATTCTTCA GGCTAGCTACAACGA AAGGGTAT | 5093 |
| 1755 | UGUGAAGA A UGGAGAAG | 3808 | CTTCTCCA GGCTAGCTACAACGA TCTTCACA | 5094 |
| 1763 | AUGGAGAA G UGUGGAGG | 3809 | CCTCCACA GGCTAGCTACAACGA TTCTCCAT | 5095 |
| 1765 | GGAGAAGU G UGGAGGAC | 3810 | GTCCTCCA GGCTAGCTACAACGA ACTTCTCC | 5096 |
| 1772 | UGUGGAGG A CUUCCAGG | 3811 | CCTGGAAG GGCTAGCTACAAGGA CCTCCACA | 5097 |
| 1787 | GGGAGGAA A UAAAAUUG | 3812 | CAATTTTA GGCTAGCTACAACGA TTCCTCCC | 5098 |
| 1792 | GAAAUAAA A UUGAAGUU | 3813 | AACTTCAA GGCTAGCTACAACGA TTTATTTC | 5099 |
| 1798 | AAAUUGAA G UUAAUAAA | 3814 | TTTATTAA GGCTAGCTACAACGA TTCAATTT | 5100 |
| 1802 | UGAAGUUA A UAAAAAUC | 3815 | GATTTTTA GGCTAGCTACAACGA TAACTTCA | 5101 |
| 1808 | UAAUAAAA A UCAAUUUG | 3816 | CAAATTGA GGCTAGCTACAACGA TTTTATTA | 5102 |
| 1812 | AAAAAUCA A UUUGCUCU | 3817 | AGAGCAAA GGCTAGCTACAACGA TGATTTTT | 5103 |
| 1816 | AUCAAUUU G CUCUAAUU | 3818 | AATTAGAG GGCTAGCTACAACGA AAATTGAT | 5104 |
| 1822 | UUGCUCUA A UUGAAGGA | 3819 | TCCTTCAA GGCTAGCTACAACGA TAGAGCAA | 5105 |
| 1835 | AGGAAAAA A CAAAACUG | 3820 | CAGTTTTG GGCTAGCTACAACGA TTTTTCCT | 5106 |
| 1840 | AAAACAAA A CUGUAAGU | 3821 | ACTTACAG GGCTAGCTACAACGA TTTGTTTT | 5107 |
| 1843 | ACAAAACU G UAAGUACC | 3822 | GGTACTTA GGCTAGCTACAACGA AGTTTTGT | 5108 |
| 1847 | AACUGUAA G UACCCUUG | 3823 | CAAGGGTA GGCTAGCTACAACGA TTACAGTT | 5109 |
| 1849 | CUGUAAGU A CCCUUGUU | 3824 | AACAAGGG GGCTAGCTACAACGA ACTTACAG | 5110 |
| 1855 | GUACCCUU G UUAUCCAA | 3825 | TTGGATAA GGCTAGCTACAACGA AAGGGTAC | 5111 |
| 1858 | CCCUUGUU A UCCAAGCG | 3826 | CGCTTGGA GGCTAGCTACAACGA AACAAGGG | 5112 |
| 1864 | UUAUCCAA G CGGCAAAU | 3827 | ATTTGCCG GGCTAGCTACAACGA TTGGATAA | 5113 |
| 1867 | UCCAAGCG G CAAAUGUG | 3828 | CACATTTG GGCTAGCTACAACGA CGCTTGGA | 5114 |
| 1871 | AGCGGCAA A UGUGUCAG | 3829 | CTGACACA GGCTAGCTACAACGA TTGCCGCT | 5115 |
| 1873 | CGGCAAAU G UGUCAGCU | 3830 | AGCTGACA GGCTAGCTACAACGA ATTTGCCG | 5116 |
| 1875 | GCAAAUGU G UCAGCUUU | 3831 | AAAGCTGA GGCTAGCTACAACGA ACATTTGC | 5117 |
| 1879 | AUGUGUCA G CUUUGUAC | 3832 | GTACAAAG GGCTAGCTACAACGA TGACACAT | 5118 |
| 1884 | UCAGCUUU G UACAAAUG | 3833 | CATTTGTA GGCTAGCTACAACGA AAAGCTGA | 5119 |
| 1886 | AGCUUUGU A CAAAUGUG | 3834 | CACATTTG GGCTAGCTACAACGA ACAAAGCT | 5120 |
| 1890 | UUGUACAA A UGUGAAGC | 3835 | GCTTCACA GGCTAGCTACAACGA TTGTACAA | 5121 |
| 1892 | GUACAAAU G UGAAGCGG | 3836 | CCGCTTCA GGCTAGCTACAACGA ATTTGTAC | 5122 |
| 1897 | AAUGUGAA G CGGUCAAC | 3837 | GTTGACCG GGCTAGCTACAACGA TTCACATT | 5123 |
| 1900 | GUGAAGCG G UCAACAAA | 3838 | TTTGTTGA GGCTAGCTACAACGA CGCTTCAC | 5124 |
| 1904 | AGCGGUCA A CAAAGUCG | 3839 | CGACTTTG GGCTAGCTACAACGA TGACCGCT | 5125 |
| 1909 | UCAACAAA G UCGGGAGA | 3840 | TCTCCCGA GGCTAGCTACAACGA TTTGTTGA | 5126 |
| 1927 | GAGAGAGG G UGAUCUCC | 3841 | GGAGATCA GGCTAGCTACAACGA CCTCTCTC | 5127 |
| 1930 | AGAGGGUG A UCUCCUUC | 3842 | GAAGGAGA GGCTAGCTACAACGA CACCCTCT | 5128 |
| 1940 | CUCCUUCC A CGUGACCA | 3843 | TGGTCACG GGCTAGCTACAACGA GGAAGGAG | 5129 |
| 1942 | CCUUCCAC G UGACCAGG | 3844 | GGCTAGCTACAACGA GTGGAAGG CCTGGTCA | 5130 |
| 1945 | UCCACGUG A CCAGGGGU | 3845 | ACCCCTGG GGCTAGCTACAACGA CACGTGGA | 5131 |
| 1952 | GACCAGGG G UCCUGAAA | 3846 | TTTCAGGA GGCTAGCTACAACGA CCCTGGTC | 5132 |
| 1960 | GUCCUGAA A UUACUUUG | 3847 | CAAAGTAA GGCTAGCTACAACGA TTCAGGAC | 5133 |
| 1963 | CUGAAAUU A CUUUGCAA | 3848 | TTGCAAAG GGCTAGCTACAACGA AATTTCAG | 5134 |
| 1968 | AUUACUUU G CAACCUGA | 3849 | TCAGGTTG GGCTAGCTACAACGA AAAGTAAT | 5135 |
| 1971 | AGUUUGCA A CCUGACAU | 3850 | ATGTCAGG GGCTAGCTACAACGA TGCAAAGT | 5136 |
| 1976 | GCAACCUG A CAUGCAGC | 3851 | GCTGCATG GGCTAGCTACAACGA CAGGTTGC | 5137 |
| 1978 | AACCUGAC A UGCAGCCC | 3852 | GGGCTGCA GGCTAGCTACAACGA GTCAGGTT | 5138 |
| 1980 | CCUGACAU G CAGCCCAC | 3853 | GTGGGCTG GGCTAGCTACAACGA ATGTCAGG | 5139 |
| 1983 | GACAUGCA G CCCACUGA | 3854 | TCAGTGGG GGCTAGCTACAACGA TGCATGTC | 5140 |
| 1987 | UGCAGCCC A CUGAGCAG | 3855 | CTGCTCAG GGCTAGCTACAACGA GGGCTGCA | 5141 |
| 1992 | CCCACUGA G CAGGAGAG | 3856 | CTCTCCTG GGCTAGCTACAACGA TCAGTGGG | 5142 |
| 2000 | GCAGGAGA G CGUGUCUU | 3857 | AAGACACG GGCTAGCTACAACGA TCTCCTGC | 5143 |
| 2002 | AGGAGAGC G UGUCUUUG | 3858 | CAAAGACA GGCTAGCTACAACGA GCTCTCCT | 5144 |
| 2004 | GAGASCGU G UCUUUGUG | 3859 | CACAAAGA GGCTAGCTACAACGA ACGCTCTC | 5145 |
| 2010 | GUGUCUUU G UGGUGCAC | 3860 | GTGCACCA GGCTAGCTACAACGA AAAGACAC | 5146 |
| 2013 | UCUUUGUG G UGCACUGC | 3861 | GCAGTGCA GGCTAGCTACAACGA CACAAAGA | 5147 |
| 2015 | UUUGUGGU G CACUGCAG | 3862 | CTGCAGTG GGGTAGCTACAACGA ACCACAAA | 5148 |
| 2017 | UGUGGUGC A CUGCAGAC | 3863 | GTCTGCAG GGCTAGCTACAACGA GCACCACA | 5149 |
| 2020 | GGUGCACU G CAGACAGA | 3864 | TCTGTCTG GGCTAGCTACAACGA AGTGCACC | 5150 |
| 2024 | CACUGCAGA CAGAUCUA | 3865 | TAGATCTG GGCTAGCTACAACGA CTGCAGTG | 5151 |
| 2028 | GCAGACAG A UCUACGUU | 3866 | AACGTAGA GGCTAGCTACAACGA CTGTCTGC | 5152 |
| 2032 | ACAGAUCU A CGUUUGAG | 3867 | CTCAAACG GGCTAGCTACAACGA AGATCTGT | 5153 |
| 2034 | AGAUCUAC G UUUGAGAA | 3868 | TTCTCAAA GGCTAGCTACAACGA GTAGATCT | 5154 |
| 2042 | GUUUGAGA A CCUCACAU | 3869 | ATGTGAGG GGCTAGCTACAACGA TCTCAAAC | 5155 |
| 2047 | AGAACCUC A CAUGGUAC | 3870 | GTACCATG GGCTAGCTACAACGA GAGGTTCT | 5156 |
| 2049 | AACCUCAC A UGGUACAA | 3871 | TTGTACCA GGCTAGCTACAACGA GTGAGGTT | 5157 |
| 2052 | CUCACAUG G UACAAGCU | 3872 | AGCTTGTA GGCTAGCTACAACGA CATGTGAG | 5158 |
| 2054 | CACAUGGU A CAAGCUUG | 3873 | CAAGCTTG GGCTAGCTACAACGA ACCATGTG | 5159 |
| 2058 | UGGUACA A G CUUGGCCC | 3874 | GGGCCAAG GGCTAGCTACAACGA TTGTACCA | 5160 |
| 2063 | CAAGCUUG G CCCACAGC | 3875 | GCTGTGGG GGCTAGCTACAACGA CAAGCTTG | 5161 |
| 2067 | CUUGGCCC A CAGCCUCU | 3876 | AGAGGCTG GGCTAGCTACAACGA GGGCCAAG | 5162 |
| 2070 | GGCCCACA G CCUCUGCC | 3877 | GGCAGAGG GGCTAGCTACAACGA TGTGGGCC | 5163 |
| 2076 | CAGCCUCU G CCAAUCCA | 3878 | TGGATTGG GGCTAGCTACAACGA AGAGGCTG | 5164 |
| 2080 | CUCUGCCA A UCCAUGUG | 3879 | CACATGGA GGCTAGCTACAACGA TGGCAGAG | 5165 |
| 2084 | GCCAAUCC A UGUGGGAG | 3880 | CTCCCACA GGCTAGCTACAACGA GGATTGGC | 5166 |
| 2086 | CAAUCCAU G UGGGAGAG | 3881 | CTCTCCCA GGCTAGCTACAACGA ATGGATTG | 5167 |
| 2094 | GUGGGAGA G UUGCCCAC | 3882 | GTGGGCAA GGCTAGCTACAACGA TCTCCCAC | 5168 |
| 2097 | GGAGAGUU G CCCACACC | 3883 | GGTGTGGG GGCTAGCTACAACGA AACTCTCC | 5169 |
| 2101 | AGUUGCCC A CACCUGUU | 3884 | AACAGGTG GGCTAGCTACAACGA GGGCAACT | 5170 |
| 2103 | UUGCCCAC A CCUSUUUG | 3885 | CAAACAGG GGCTAGCTACAACGA GTGGGCAA | 5171 |
| 2107 | CCACACCU G UUUGCAAG | 3886 | CTTGCAAA GGCTAGCTACAACGA AGGTGTGG | 5172 |
| 2111 | ACCUGUUU G CAAGAACU | 3887 | AGTTCTTG GGCTAGCTACAACGA AAACAGGT | 5173 |
| 2117 | UUGCAAGA A CUUGGAUA | 3888 | TATCCAAG GGCTAGCTACAACGA TCTTGCAA | 5174 |
| 2123 | GAACUUGG A UACUCUUU | 3889 | AAAGAGTA GGCTAGCTACAACGA CCAAGTTC | 5175 |
| 2125 | ACUUGGAU A CUCUUUGG | 3890 | CCAAAGAG GGCTAGCTACAACGA ATCCAAGT | 5176 |
| 2136 | CUUUGGAA A UUGAAUGC | 3891 | GCATTCAA GGCTAGCTACAACGA TTCCAAAG | 5177 |
| 2141 | GAAAUUGA A UGCCACCA | 3892 | TGGTGGCA GGCTAGCTACAACGA TCAATTTC | 5178 |
| 2143 | AAUUGAAU G CCACCAUG | 3893 | CATGGTGG GGCTAGCTACAACGA ATTCAATT | 5179 |
| 2146 | UGAAUGCC A CCAUGUUC | 3894 | GAACATGG GGCTAGCTACAACGA GGCATTCA | 5180 |
| 2149 | AUGCCACC A UGUUCUCU | 3895 | AGAGAACA GGCTAGCTACAACGA GGTGGCAT | 5181 |
| 2151 | GCCACCAU G UUCUCUAA | 3896 | TTAGAGAA GGCTAGCTACAACGA ATGGTGGC | 5182 |
| 2159 | GUUCUCUA A UAGCACAA | 3897 | TTGTGCTA GGCTAGCTACAACGA TAGAGAAC | 5183 |
| 2162 | CUCUAAUA G CACAAAUG | 3898 | CATTTGTG GGCTAGCTACAACGA TATTAGAG | 5184 |
| 2164 | CUAAUAGC A CAAAUGAC | 3899 | GTCATTTG GGCTAGCTACAACGA GCTATTAG | 5185 |
| 2168 | UAGCACAA A UGACAUUU | 3900 | AAATGTCA GGCTAGCTACAACGA TTGTGCTA | 5186 |
| 2171 | CACAAAUG A CAUUUUGA | 3901 | TCAAAATG GGCTAGCTACAACGA CATTTGTG | 5187 |
| 2173 | CAAAUGAC A UUUUGAUC | 3902 | GATCAAAA GGCTAGCTACAACGA GTCATTTG | 5188 |
| 2179 | ACAUULTUG A UCAUGGAG | 3903 | CTCCATGA GGCTAGCTACAACGA CAAAATGT | 5189 |
| 2182 | UUUUGAUC A UGGAGCUU | 3904 | AAGCTCCA GGCTAGCTACAACGA GATCAAAA | 5190 |
| 2187 | AUCAUGGA G CUUAAGAA | 3905 | TTCTTAAG GGCTAGCTACAACGA TCCATGAT | 5191 |
| 2195 | GCUUAAGA A UGCAUCCU | 3906 | AGGATGCA GGCTAGCTACAACGA TCTTAAGC | 5192 |
| 2197 | UUAAGAAU G CAUCCUUG | 3907 | CAAGGATG GGCTAGCTACAACGA ATTCTTAA | 5193 |
| 2199 | AAGAAUGC A UCCUUGCA | 3908 | TGCAAGGA GGCTAGCTACAACGA GCATTCTT | 5194 |
| 2205 | GCAUCCUU G CAGGACCA | 3909 | TGGTCCTG GGCTAGCTACAACGA AAGGATGC | 5195 |
| 2210 | CUUGCAGG A CCAKGGAG | 3910 | CTCCTTGG GGCTAGCTACAACGA CCTGCAAG | 5196 |
| 2219 | CCAAGGAG A CUAUGUCU | 3911 | AGACATAG GGCTAGCTACAACGA CTCCTTGG | 5197 |
| 2222 | AGGAGACU A UGUCUGCC | 3912 | GGCAGACA GGCTAGCTACAACGA AGTCTCCT | 5198 |
| 2224 | GAGACUAU G UCUGCCUU | 3913 | AAGGCAGA GGCTAGCTACAACGA ATAGTCTC | 5199 |
| 2228 | CUAUGUCU G CCUUGCUC | 3914 | GAGCAAGG GGCTAGCTACAACGA AGACATAG | 5200 |
| 2233 | UCUGCCUU G CUCAAGAC | 3915 | GTCTTGAG GGCTAGCTACAACGA AAGGCAGA | 5201 |
| 2240 | UGCUCAAG A CAGGAAGA | 3916 | TCTTCCTG GGCTAGCTACAACGA CTTGAGCA | 5202 |
| 2248 | ACAGGAAG A CCAAGAAA | 3917 | TTTCTTGG GGCTAGCTACAACGA CTTCCTGT | 5203 |
| 2259 | AAGAAAAG A CAUUGCGU | 3918 | ACGCAATG GGCTAGCTACAACGA CTTTTCTT | 5204 |
| 2261 | GAAAAGAC A UUGCGUGG | 3919 | CCACGCAA GGCTAGCTACAACGA GTCTTTTC | 5205 |
| 2264 | AAGACAUU G CGUGGUCA | 3920 | TGACCACG GGCTAGCTACAACGA AATGTCTT | 5206 |
| 2266 | GACAUUGC G UGGUCAGG | 3921 | CCTGACCA GGCTAGCTACAACGA GCAATGTC | 5207 |
| 2269 | AUUGCGUG G UCAGGCAG | 3922 | CTGCCTGA GGCTAGCTACAACGA CACGCAAT | 5208 |
| 2274 | GUGGUCAG G CAGCUCAC | 3923 | GTGAGCTG GGCTAGCTACAACGA CTGACCAC | 5209 |
| 2277 | GUCAGGCA G CUCACAGU | 3924 | ACTGTGAG GGCTAGCTACAACGA TGCCTGAC | 5210 |
| 2281 | GGCAGCUC A CAGUCCUA | 3925 | TAGGACTG GGCTAGCTACAACGA GAGCTGCC | 5211 |
| 2284 | AGCUCACA G UCCUAGAG | 3926 | CTCTAGGA GGCTAGCTACAACGA TGTGAGCT | 5212 |
| 2292 | GUCCUAGA G CGUGUGGC | 3927 | GCCACACG GGCTAGCTACAACGA TCTAGGAC | 5213 |
| 2294 | CCUAGAGC G UGUGGCAC | 3928 | GTGCCACA GGCTAGCTACAACGA GCTCTAGG | 5214 |
| 2296 | UAGAGCGU G UGGCACCC | 3929 | GGGTGCCA GGCTAGCTACAACGA ACGCTCTA | 5215 |
| 2299 | AGCGUGUG G CACCCACG | 3930 | CGTGGGTG GGCTAGCTACAACGA CACACGCT | 5216 |
| 2301 | CGUGUGGC A CCCACGAU | 3931 | ATCGTGGG GGCTAGCTACAACGA GCCACACG | 5217 |
| 2305 | UGGCACCC A CGAUCACA | 3932 | TGTGATCG GGCTAGCTACAACGA GGGTGCCA | 5218 |
| 2308 | CACCCACG A UCACAGGA | 3933 | TCCTGTGA GGCTAGCTACAACGA CGTGGGTG | 5219 |
| 2311 | CCACGAUC A CAGGAAAC | 3934 | GTTTCCTG GGCTAGCTACAACGA GATCGTGG | 5220 |
| 2318 | CACAGGAA A CCUGGAGA | 3935 | TCTCCAGG GGCTAGCTACAACGA TTCCTGTG | 5221 |
| 2327 | CCUGGAGA A UCAGACGA | 3936 | TCGTCTGA GGCTAGCTACAACGA TCTCCAGG | 5222 |
| 2332 | AGAAUCAG A CGACAAGU | 3937 | ACTTGTCG GGCTAGCTACAACGA CTGATTCT | 5223 |
| 2335 | AUCAGACG A CAAGUAUU | 3938 | AATACTTG GGCTAGCTACAACGA CGTCTGAT | 5224 |
| 2339 | GACGACAA G UAUUGGGG | 3939 | CCCCAATA GGCTAGCTACAACGA TTGTCGTC | 5225 |
| 2341 | CGACAAGU A UUGGGGAA | 3940 | TTCCCCAA GGCTAGCTACAACGA ACTTGTCG | 5226 |
| 2351 | UGGGGAAA G CAUCGAAG | 3941 | CTTCGATG GGCTAGCTACAACGA TTTCCCCA | 5227 |
| 2353 | GCGAAAGC A UCGAAGUC | 3942 | GACTTCGA GGCTAGCTACAACGA GCTTTCCC | 5228 |
| 2359 | GCAUCGAA G UCUCAUGC | 3943 | GCATGAGA GGCTAGCTACAACGA TTCGATGC | 5229 |
| 2364 | GAAGUCUC A UGCACGGC | 3944 | GCCGTGCA GGCTAGCTACAACGA GAGACTTC | 5230 |
| 2366 | AGUCUCAU G CACGGCAU | 3945 | ATGCCGTG GGCTAGCTACAACGA ATGAGACT | 5231 |
| 2368 | UCUCAUGC A CGGCAUCC | 3946 | AGATGCCG GGCTAGCTACAACGA GCATGAGA | 5232 |
| 2371 | CAUGCACG G CAUCUGGG | 3947 | CCCAGATG GGCTAGCTACAACGA CGTGCATG | 5233 |
| 2373 | UGCACGGC A UCUGGGAA | 3948 | TTCCCAGA GGCTAGCTACAACGA GCCGTGCA | 5234 |
| 2381 | AUCUGGGA A UCCCCCUC | 3949 | GAGGGGGA GGCTAGCTACAACGA TCCCAGAT | 5235 |
| 2391 | CCCCCUCC A CAGAUCAU | 3950 | ATGATCTG GGCTAGCTACAACGA GGAGGGGG | 5236 |
| 2395 | CUCCACAG A UCAUGUGG | 3951 | CCACATGA GGCTAGCTACAACGA CTGTGGAG | 5237 |
| 2398 | CACAGAUC A UGUGGUUU | 3952 | AAACCACA GGCTAGCTACAACGA GATCTGTG | 5238 |
| 2400 | CAGAUCAU G UGGUUUAA | 3953 | TTAAACCA GGCTAGCTACAACGA ATGATCTG | 5239 |
| 2403 | AUCAUGUG G UUUAAAGA | 3954 | TCTTTAAA GGCTAGCTACAACGA CACATGAT | 5240 |
| 2411 | GUUUAAAG A UAAUGAGA | 3955 | TCTCATTA GGCTAGCTACAACGA CTTTAAAC | 5241 |
| 2414 | UAAAGAUA A UGAGACCC | 3956 | GGGTCTCA GGCTAGCTACAACGA TATCTTTA | 5242 |
| 2419 | AUAAUGAG A CCCUUGUA | 3957 | TACAAGGG GGCTAGCTACAACGA CTCATTAT | 5243 |
| 2425 | AGACCCUU G UAGAAGAC | 3958 | GTCTTCTA GGCTAGCTACAACGA AAGGGTCT | 5244 |
| 2432 | UGUAGAAG A CUCAGGCA | 3959 | TGCCTGAG GGCTAGCTACAACGA CTTCTACA | 5245 |
| 2438 | AGACUCAG G CAUUGUAU | 3960 | ATACAATG GGCTAGCTACAACGA CTGAGTCT | 5246 |
| 2440 | ACUCAGGC A UUGUAUUG | 3961 | CAATACAA GGCTAGCTACAACGA GCCTGAGT | 5247 |
| 2443 | CAGGCAUU G UAUUGAAG | 3962 | CTTCAATA GGCTAGCTACAACGA AATGCCTG | 5248 |
| 2445 | GGCAUUGU A UUGAAGGA | 3963 | TCCTTCAA GGCTAGCTACAACGA ACAATGCC | 5249 |
| 2453 | AUUGAAGG A UGGGAACC | 3964 | GGTTCCCA GGCTAGCTACAACGA CCTTCAAT | 5250 |
| 2459 | GGAUGGGA A CCGGAACC | 3965 | GGTTCCGG GGCTAGCTACAACGA TCCCATCC | 5251 |
| 2465 | GAACCGGA A CCUCACUA | 3966 | TAGTGAGG GGCTAGCTACAACGA TCCGGTTC | 5252 |
| 2470 | GGAACCUC A CUAUCCGC | 3967 | GCGGATAG GGCTAGCTACAACGA GAGGTTCC | 5253 |
| 2473 | ACCUCACU. A UCCGCAGA | 3968 | TCTGCGGA GGCTAGCTACAACGA AGTGAGGT | 5254 |
| 2477 | CACUAUCC G CAGAGUGA | 3969 | TCACTCTG GGCTAGCTACAACGA GGATAGTG | 5255 |
| 2482 | UCCGCAGA G UGAGGAAG | 3970 | CTTCCTCA GGCTAGCTACAACGA TCTGCGGA | 5256 |
| 2495 | GAAGGAGG A CGAAGGCC | 3971 | GGCCTTCG GGCTAGCTACAACGA CCTCCTTC | 5257 |
| 2501 | GGACGAAG G CCUCUACA | 3972 | TGTAGAGG GGCTAGCTACAACGA CTTCGTCC | 5258 |
| 2507 | AGGCCUCU A CACCUGCC | 3973 | GGCAGGTG GGCTAGCTACAACGA AGAGGCCT | 5259 |
| 2509 | GCCUCUAC A CCUGCCAG | 3974 | CTGGCAGG GGCTAGCTACAACGA GTAGAGGC | 5260 |
| 2513 | CUACACCU G CCAGGCAU | 3975 | ATGCCTGG GGCTAGCTACAACGA AGGTGTAG | 5261 |
| 2518 | CCUGCCAG G CAUGCAGU | 3976 | ACTGCATG GGCTAGCTACAACGA CTGGCAGG | 5262 |
| 2520 | UGCCAGGC A UGCAGUGU | 3977 | ACACTGCA GGCTAGCTACAACGA GCCTGGCA | 5263 |
| 2522 | CCAGGCAU G CAGUGUUC | 3978 | GAACACTG GGCTAGCTACAACGA ATGCCTGG | 5264 |
| 2525 | GGCAUGCA G UGUUCUUG | 3979 | CAAGAACA GGCTAGCTACAACGA TGCATGCC | 5265 |
| 2527 | CAUGCAGU G UUCUUGGC | 3980 | GCCAAGAA GGCTAGCTACAACGA ACTGCATG | 5266 |
| 2534 | UGUUCUUG G CUGUGCAA | 3981 | TTGCACAG GGCTAGCTACAACGA CAAGAACA | 5267 |
| 2537 | UCUUGGCU G UGCAAAAG | 3982 | CTTTTGCA GGCTAGCTACAACGA AGCCAAGA | 5268 |
| 2539 | UUGGCUGU G CAAAAGUG | 3983 | CACTTTTG GGCTAGCTACAACGA ACAGCCAA | 5269 |
| 2545 | GUGCAAAA G UGGAGGCA | 3984 | TGCCTCCA GGCTAGCTACAACGA TTTTGCAC | 5270 |
| 2551 | AAGUGGAG G CAUUUUUC | 3985 | GAAAAATG GGCTAGCTACAACGA CTCCACTT | 5271 |
| 2553 | GUGGAGGC A UUUUUCAU | 3986 | ATGAAAAA GGCTAGCTACAACGA GCCTCCAC | 5272 |
| 2560 | CAUUUUUC A UAAUAGAA | 3987 | TTCTATTA GGCTAGCTACAACGA GAAAAATG | 5273 |
| 2563 | UUUUCAUA A UAGAAGGU | 3988 | ACCTTCTA GGCTAGCTACAACGA TATGAAAA | 5274 |
| 2570 | AAUAGAAG G UGCCCAGG | 3989 | CCTGGGCA GGCTAGCTACAACGA CTTCTATT | 5275 |
| 2572 | UAGAAGGU G CCCAGGAA | 3990 | TTCCTGGG GGCTAGCTACAACGA ACCTTCTA | 5276 |
| 2584 | AGGAAAAG A CGAACUUG | 3991 | CAAGTTCG GGCTAGCTACAACGA CTTTTCCT | 5277 |
| 2588 | AAAGACGA A CUUGGAAA | 3992 | TTTCCAAG GGCTAGCTACAACGA TCGTCTTT | 5278 |
| 2596 | ACUUGGAA A UCAUUAUU | 3993 | AATAATGA GGCTAGCTACAACGA TTCCAAGT | 5279 |
| 2599 | UGGAAAUC A UUAUUCUA | 3994 | TAGAATAA GGCTAGCTACAACGA GATTTCCA | 5280 |
| 2602 | AAAUCAUU A UUCUAGUA | 3995 | TACTAGAA GGCTAGCTACAACGA AATGATTT | 5281 |
| 2608 | UUAUUCUA G UAGGCACG | 3996 | CGTGCCTA GGCTAGCTACAACGA TAGAATAA | 5282 |
| 2612 | UCUAGUAG G CACGGCGG | 3997 | CCGCCGTG GGCTAGCTACAACGA CTACTAGA | 5283 |
| 2614 | UAGUAGGC A CGGCGGUG | 3998 | CACCGCCG GGCTAGCTACAACGA GCCTACTA | 5284 |
| 2617 | UAGGCACG G CGGUGAUU | 3999 | AATCACCG GGCTAGCTACAACGA CGTGCCTA | 5285 |
| 2620 | GCACGGCG G UGAUUGCC | 4000 | GGCAATCA GGCTAGCTACAACGA CGCCGTGC | 5286 |
| 2623 | CGGCGGUG A UUGCCAUG | 4001 | CATGGCAA GGCTAGCTACAACGA CACCGCCG | 5287 |
| 2626 | CGGUGAUU G CCAUGUUC | 4002 | GAACATGG GGCTAGCTACAACGA AATCACCG | 5288 |
| 2629 | UGAUUGCC A UGUUCUUC | 4003 | GAAGAACA GGCTAGCTACAACGA GGCAATCA | 5289 |
| 2631 | AUUGCCAU G UUCUUCUG | 4004 | CAGAAGAA GGCTAGCTACAACGA ATGGCAAT | 5290 |
| 2640 | UUCUUCUG G CUACUUCU | 4005 | AGAAGTAG GGCTAGCTACAACGA CAGAAGAA | 5291 |
| 2643 | UUCUGGCU A CUUCUUGU | 4006 | ACAAGAAG GGCTAGCTACAACGA AGCCAGAA | 5292 |
| 2650 | UACUUCUU G UCAUCAUC | 4007 | GATGATGA GGCTAGCTACAACGA AAGAAGTA | 5293 |
| 2653 | UUCUUGUC A UCAUCCUA | 4008 | TAGGATGA GGCTAGCTACAACGA GACAAGAA | 5294 |
| 2656 | UUGUCAUC A UCCUACGG | 4009 | CCGTAGGA GGCTAGCTACAACGA GATGACAA | 5295 |
| 2661 | AUCAUCCU A CGGACCGU | 4010 | ACGGTCCG GGCTAGCTACAACGA AGGATGAT | 5296 |
| 2665 | UCCUACGG A CCGUUAAG | 4011 | CTTAACGG GGCTAGCTACAACGA CCGTAGGA | 5297 |
| 2668 | UACGGACC G UUAAGCGG | 4012 | CCGCTTAA GGCTAGCTACAACGA GGTCCGTA | 5298 |
| 2673 | ACCGUUAA G CGGGCCAA | 4013 | TTGGCCCG GGCTAGCTACAACGA TTAACGGT | 5299 |
| 2677 | UUAAGCGG G CCAAUGGA | 4014 | TCCATTGG GGCTAGCTACAACGA CCGCTTAA | 5300 |
| 2681 | GCGGGCCA A UGGAGGGG | 4015 | CCCCTCCA GGCTAGCTACAACGA TGGCCCGC | 5301 |
| 2691 | GGAGGGGA A CUGAAGAC | 4016 | GTCTTCAG GGCTAGCTACAACGA TCCCCTCC | 5302 |
| 2698 | AACUGAAG A CAGGCUAC | 4017 | GTAGCCTG GGCTAGCTACAACGA CTTCAGTT | 5303 |
| 2702 | CAAGACAG G CUACUUGU | 4018 | ACAAGTAG GGCTAGCTACAACGA CTGTCTTC | 5304 |
| 2705 | GACAGGCU A CUUGUCCA | 4019 | TGGACAAG GGCTAGCTACAACGA AGCCTGTC | 5305 |
| 2709 | GGCUACUU G UCCAUCGU | 4020 | ACGATGGA GGCTAGCTACAACGA AAGTAGCC | 5306 |
| 2713 | ACUUGUCC A UCGUCAUG | 4021 | CATGACGA GGCTAGCTACAACGA GGACAAGT | 5307 |
| 2716 | UGUCCAUC G UCAUGGAU | 4022 | ATCCATGA GGCTAGCTACAACGA GATGGACK | 5308 |
| 2719 | CCAUCGUC A UGGAUCCA | 4023 | TGGATCCA GGCTAGCTACAACGA GACGATGG | 5309 |
| 2723 | CGUCAUGG A UCCAGAUG | 4024 | CATCTGGA GGCTAGCTACAACGA CCATGACG | 5310 |
| 2729 | GGAUCCAG A UGAACUCC | 4025 | GGAGTTCA GGCTAGCTACAACGA CTGGATCC | 5311 |
| 2733 | CCAGAUGA A CUCCCAUU | 4026 | AATGGGAG GGCTAGCTACAACGA TCATCTGG | 5312 |
| 2739 | GAACUCCC A UUGGAUGA | 4027 | TCATCCAA GGCTAGCTACAACGA GGGAGTTC | 5313 |
| 2744 | CCCAUUGG A UGAACAUU | 4028 | AATGTTCA GGCTAGCTACAACGA CCAATGGG | 5314 |
| 2748 | UUGGAUGA A CAUUGUGA | 4029 | TCACAATG GGCTAGCTACAACGA TCATCCAA | 5315 |
| 2750 | GGAUGAAC A UUGUGAAC | 4030 | GTTCACAA GGCTAGCTACAACGA GTTCATCC | 5316 |
| 2753 | UGAACAUU G UGAACGAC | 4031 | GTCGTTCA GGCTAGCTACAACGA AATGTTCA | 5317 |
| 2757 | CAUUGUGA A CGACUGCC | 4032 | GGCAGTCG GGCTAGCTACAACGA TCACAATG | 5318 |
| 2760 | UGUGAACG A CUGCCUUA | 4033 | TAAGGCAG GGCTAGCTACAACGA CGTTCACA | 5319 |
| 2763 | GAACGACU G CCUUAUGA | 4034 | TCATAAGG GGCTAGCTACAACGA AGTCGTTC | 5320 |
| 2768 | ACUGCCUU A UGAUGCCA | 4035 | TGGCATCA GGCTAGCTACAACGA AAGGCAGT | 5321 |
| 2771 | GCCUUAUG A UGCCAGCA | 4036 | TGCTGGCA GGCTAGCTACAACGA CATAAGGC | 5322 |
| 2773 | CWAUGAU G CCAGCAAA | 4037 | TTTGCTGG GGCTAGCTACAACGA ATCATAAG | 5323 |
| 2777 | UGAUGCCA G CAAAUGGG | 4038 | CCCATTTG GGCTAGCTACAACGA TGGCATCA | 5324 |
| 2781 | GCCAGCAA A UGGGAAUU | 4039 | AATTCCCA GGCTAGCTACAACGA TTGCTGGC | 5325 |
| 2787 | AAAUGGGA A UUCCCCAG | 4040 | CTGGGGAA GGCTAGCTACAACGA TCCCATTT | 5326 |
| 2798 | CCCCAGAG A CCGGCUGA | 4041 | TCAGCCGG GGCTAGCTACAACGA CTCTGGGG | 5327 |
| 2802 | AGAGACCG G CUGAAGCU | 4042 | AGCTTCAG GGCTAGCTACAACGA CGGTCTCT | 5328 |
| 2808 | CGGCUGAA G CUAGGUAA | 4043 | TTACCTAG GGCTAGCTACAACGA TTCAGCCG | 5329 |
| 2813 | GAAGCUAG G UAAGCCUC | 4044 | GAGGCTTA GGCTAGCTACAACGA CTAGCTTC | 5330 |
| 2817 | CUAGGUAA G CCUCUUGG | 4045 | CCAAGAGG GGCTAGCTACAACGA TTACCTAG | 5331 |
| 2825 | GCCUCUUG G CCGUGGUG | 4046 | CACCACGG GGCTAGCTACAACGA CAAGAGGC | 5332 |
| 2828 | UCUUGGCC G UGGUGCCU | 4047 | AGGCACCA GGCTAGCTACAACGA GGCCAAGA | 5333 |
| 2831 | UGGCCGUG G UGCCUWG | 4048 | CAAAGGCA GGCTAGCTACAACGA CACGGCCA | 5334 |
| 2833 | GCCGUGGU G CCUUUGGC | 4049 | GCCAAAGG GGCTAGCTACAACGA ACCACGGC | 5335 |
| 2840 | UGCCUUUG G CCAAGUGA | 4050 | TCACTTGG GGCTAGCTACAACGA CAAAGGCA | 5336 |
| 2845 | UUGGCCAA G UGAUUGAA | 4051 | TTCAATCA GGCTAGCTACAACGA TTGGCCAA | 5337 |
| 2848 | GCCAAGUG A UUGAAGCA | 4052 | TGCTTCAA GGCTAGCTACAACGA CACTTGGC | 5338 |
| 2854 | UGAWGAA G CAGAUGCC | 4053 | GGCATCTG GGCTAGCTACAACGA TTCAATCA | 5339 |
| 2858 | UGAAGCAG A UGCCUUUG | 4054 | CAAAGGCA GGCTAGCTACAACGA CTGCTTCA | 5340 |
| 2860 | AAGCAGAU G CCUUUGGA | 4055 | TCCAAAGG GGCTAGCTACAACGA ATCTGCTT | 5341 |
| 2869 | CCUUUGGA A UUGACAAG | 4056 | CTTGTCAA GGCTAGCTACAACGA TCCAAAGG | 5342 |
| 2873 | UGGAAUUG A CAAGACAG | 4057 | CTGTCTTG GGCTAGCTACAACGA CAATTCCA | 5343 |
| 2878 | UUGACAAG A CAGCAACU | 4058 | AGTTGCTG GGCTAGCTACAACGA CTTGTCAA | 5344 |
| 2881 | ACAAGACA G CAACUUGC | 4059 | GCAAGTTG GGCTAGCTACAACGA TGTCTTGT | 5345 |
| 2884 | AGACAGCA A CUUGCAGG | 4060 | CCTGCAAG GGCTAGCTACAACGA TGCTGTCT | 5346 |
| 2688 | AGCAACUU G CAGGACAG | 4061 | CTGTCCTG GGCTAGCTACAACGA AAGTTGCT | 5347 |
| 2893 | CUUGCAGG A CAGUAGCA | 4062 | TGCTACTG GGCTAGCTACAACGA CCTGCAAG | 5348 |
| 2896 | GCAGGACA G UAGCAGUC | 4063 | GACTGCTA GGCTAGCTACAACGA TGTCCTGC | 5349 |
| 2899 | GGACAGUA G CAGUCAAA | 4064 | TTTGACTG GGCTAGCTACAACGA TACTGTCC | 5350 |
| 2902 | CAGUAGCA G UCAAAAUG | 4065 | CATTTTGA GGCTAGCTACAACGA TGCTACTG | 5351 |
| 2908 | CAGUCAAA A UGUUGAAA | 4066 | TTTCAACA GGCTAGCTACAACGA TTTGACTG | 5352 |
| 2910 | GUCAAAAU G UUGAAAGA | 4067 | TCTTTCAA GGCTAGCTACAACGA ATTTTGAC | 5353 |
| 2923 | AAGAAGGA G CAACACAC | 4068 | GTGTGTTG GGCTAGCTACAACGA TCCTTCTT | 5354 |
| 2926 | AAGGAGCA A CACACAGU | 4069 | ACTGTGTG GGCTAGCTACAACGA TGCTCCTT | 5355 |
| 2928 | GGAGCAAC A CACAGUGA | 4070 | TCACTGTG GGCTAGCTACAACGA GTTGCTCC | 5356 |
| 2930 | AGCAACAC A CAGUGAGC | 4071 | GCTCACTG GGCTAGCTACAACGA GTGTTGCT | 5357 |
| 2933 | AACACACA G UGAGCAUC | 4072 | GATGCTCA GGCTAGCTACAACGA TGTGTGTT | 5358 |
| 2937 | CACAGUGA G CAUCGAGC | 4073 | GCTCGATG GGCTAGCTACAACGA TCACTGTG | 5359 |
| 2939 | CAGUGAGC A UCGAGCUC | 4074 | GAGCTCGA GGCTAGCTACAACGA GGTCACTG | 5360 |
| 2944 | AGCAUCGA G CUCUCAUG | 4075 | CATGAGAG GGCTAGCTACAACGA TCGATGCT | 5361 |
| 2950 | GAGCUCUC A UGUCUGAA | 4076 | TTCAGACA GGCTAGCTACAACGA GAGAGCTC | 5362 |
| 2952 | GCUCUCAU G UCUGAACU | 4077 | AGTTCAGA GGCTAGCTACAACGA ATGAGAGC | 5363 |
| 2958 | AUGUCUGA A CUCAAGAU | 4078 | ATCTTGAG GGCTAGCTACAACGA TCAGACAT | 5364 |
| 2965 | AACUCAAG A UCCUCAUU | 4079 | AATGAGGA GGCTAGCTACAACGA CTTGAGTT | 5365 |
| 2971 | AGAUCCUC A UUCAUAUU | 4080 | AATATGAA GGCTAGCTACAACGA GAGGATCT | 5366 |
| 2975 | CCUCAWC A UAUUGGUC | 4081 | GACCAATA GGCTAGCTACAACGA GAATGAGG | 5367 |
| 2977 | UCAWCAU A WGGUCAC | 4082 | GTGACCAA GGCTAGCTACAACGA ATGAATGA | 5368 |
| 2981 | UCAUAWG G UCACCAUC | 4083 | GATGGTGA GGCTAGCTACAACGA CAATATGA | 5369 |
| 2984 | UAUUGGUC A CCAUCUCA | 4084 | TGAGATGG GGCTAGCTACAACGA GACCAATA | 5370 |
| 2987 | UGGUCACC A UCUCAAUG | 4085 | CATTGAGA GGCTAGCTACAACGA GGTGACCA | 5371 |
| 2993 | CCAUCUCA A UGUGGUCA | 4086 | TGACCACA GGCTAGCTACAACGA TGAGATGG | 5372 |
| 2995 | AUCUCAAU G UGGUCAAC | 4087 | GTTGACCA GGCTAGCTACAACGA ATTGAGAT | 5373 |
| 2998 | UCAAUGUG G UCAACCUU | 4088 | AAGGTTGA GGCTAGCTACAACGA CACATTGA | 5374 |
| 3002 | UGUGGUCA A CCUUCUAG | 4089 | CTAGAAGG GGCTAGCTACAACGA TGACCACA | 5375 |
| 3011 | CCUUCUAG G UGCCUGUA | 4090 | TACAGGCA GGCTAGCTACAACGA CTAGAAGG | 5376 |
| 3013 | UUCUAGGU G CCUGUACC | 4091 | GGTACAGG GGCTAGCTACAACGA ACCTAGAA | 5377 |
| 3017 | AGGUGCCU G UACCAAGC | 4092 | GCTTGGTA GGCTAGCTACAACGA AGGCACCT | 5378 |
| 3019 | GUGCCUGU A CCAAGCCA | 4093 | TGGCTTGG GGCTAGCTACAACGA ACAGGCAC | 5379 |
| 3024 | UGUACCAA G CCAGGAGG | 4094 | CCTCCTGG GGCTAGCTACAACGA TTGGTACA | 5380 |
| 3033 | CCAGGAGG G CCACUCAU | 4095 | ATGAGTGG GGCTAGCTACAACGA CCTCCTGG | 5381 |
| 3036 | GGAGGGCC A CUCAUGGU | 4096 | ACCATGAG GGCTAGCTACAACGA GGCCCTCC | 5382 |
| 3040 | GGCCACUC A UGGUGAUU | 4097 | AATCACCA GGCTAGCTACAACGA GAGTGGCC | 5383 |
| 3043 | CACUCAUG G UGAUUGUG | 4098 | CACAATCA GGCTAGCTACAACGA CATGAGTG | 5384 |
| 3046 | UCAUGGUG A UUGUGGAA | 4099 | TTCCACAA GGCTAGCTACAACGA CACCATGA | 5385 |
| 3049 | UGGUGAUU G UGGAAUUC | 4100 | GAATTCCA. GGCTAGCTACAACGA AATCACCA | 5386 |
| 3054 | AUUGUGGA A UUCUGCAA | 4101 | TTGCAGAA GGCTAGCTACAACGA TCCACAAT | 5387 |
| 3059 | GGAAUUCU G CAAAUUUG | 4102 | CARATTTG GGCTAGCTACAACGA AGAATTCC | 5388 |
| 3063 | UUCUGCAA A UUUGGAAA | 4103 | TTTCCAAA GGCTAGCTACAACGA TTGCAGAA | 5389 |
| 3071 | AUUUGGAA A CCUGUCCA | 4104 | TGGACAGG GGCTAGCTACAACGA TTCCAAAT | 5390 |
| 3075 | GGAAACCU G UCCACUUA | 4105 | TAAGTGGA GGCTAGCTACAACGA AGGTTTCC | 5391 |
| 3079 | ACCUGUCC A CUUACCUG | 4106 | CAGGTAAG GGCTAGCTACAACGA GGACAGGT | 5392 |
| 3083 | GUCCACUU A CCUGAGGA | 4107 | TCCTCAGG GGCTAGCTACAACGA AAGTGGAC | 5393 |
| 3092 | CCUGAGGA G CAAGAGAA | 4108 | TTCTCTTG GGCTAGCTACAACGA TCCTCAGG | 5394 |
| 3101 | CAAGAGAA A UGAAUUUG | 4109 | CAAATTCA .GGCTAGCTACAACGA TTCTCTTG | 5395 |
| 3105 | AGAAAUGA A UUUGUCCC | 4110 | GGGACAAA GGCTAGCTACAACGA TCATTTCT | 5396 |
| 3109 | AUGAAUUU G UCCCCUAC | 4111 | GTAGGGGA GGCTAGCTACAACGA AAATTCAT | 5397 |
| 3116 | UGUCCCCU A CAAGACCA | 4112 | TGGTCTTG GGCTAGCTACAACGA AGGGGACA | 5398 |
| 3121 | CCUACAAG A CCAAAGGG | 4113 | CCCTTTGG GGCTAGCTACAACGA CTTGTAGG | 5399 |
| 3130 | CCAAAGGG G CACGAUUC | 4114 | GAATCGTG GGCTAGCTACAACGA CCCTTTGG | 5400 |
| 3132 | AAAGGGGC A CGAUUCCG | 4115 | CGGAATCG GGCTAGCTACAACGA GCCCCTTT | 5401 |
| 3135 | GGGGCACG A UUCCGUCA | 4116 | TGACGGAA GGCTAGCTACAACGA CGTGCCCC | 5402 |
| 3140 | ACGAUUCC G UCAAGGGA | 4117 | TCCCTTGA GGCTAGCTACAACGA GGAATCGT | 5403 |
| 3152 | AGGGAAAG A CUACGUUG | 4118 | CAACGTAG GGCTAGCTACAACGA CTTTCCCT | 5404 |
| 3155 | GAAAGACU A CGUUGGAG | 4119 | CTCCAACG GGCTAGCTACAACGA AGTCTTTC | 5405 |
| 3157 | AAGACUAC G UUGGAGCA | 4120 | TGCTCCAA GGCTAGCTACAACGA GTAGTCTT | 5406 |
| 3163 | ACGUUGGA G CAAUCCCU | 4121 | AGGGATTG GGCTAGCTACAACGA TCCAACGT | 5407 |
| 3166 | UUGGAGCA A UCCCUGUG | 4122 | CACAGGGA GGCTAGCTACAACGA TGCTCCAA | 5408 |
| 3172 | CAAUCCCU G UGGAUCUG | 4123 | CAGATCCA GGCTAGCTACAACGA AGGGATTG | 5409 |
| 3176 | CCCUGUGG A UCUGAAAC | 4124 | GTTTCAGA GGCTAGCTACAACGA CCACAGGG | 5410 |
| 3183 | GAUCUGAA A CGGCGCUU | 4125 | AAGCGCCG GGCTAGCTACAACGA TTCAGATC | 5411 |
| 3186 | CUGAAACG G CGCUUGGA | 4126 | TCCAAGCG GGCTAGCTACAACGA CGTTTCAG | 5412 |
| 3188 | GAAACGGC G CUUGGACA | 4127 | TGTCCAAG GGCTAGCTACAACGA GCCGTTTC | 5413 |
| 3194 | GCGCUUGG A CAGCAUCA | 4128 | TGATGCTG GGCTAGCTACAACGA CCAAGCGC | 5414 |
| 3197 | CUUGGACA G CAUCACCA | 4129 | TGGTGATG GGCTAGCTACAACGA TGTCCAAG | 5415 |
| 3199 | UGGACAGC A UCACCAGU | 4130 | ACTGGTGA GGCTAGCTACAACGA GCTGTCCA | 5416 |
| 3202 | ACAGCAUC A CCAGUAGC | 4131 | GCTACTGG GGCTAGCTACAACGA GATGCTGT | 5417 |
| 3206 | CAUCACCA G UAGCCAGA | 4132 | TCTGGCTA GGCTAGCTACAACGA TGGTGATG | 5418 |
| 3209 | CACCAGUA G CCAGAGCU | 4133 | AGCTCTGG GGCTAGCTACAACGA TACTGGTG | 5419 |
| 3215 | UAGCCAGA G CUCAGCCA | 4134 | TGGCTGAG GGCTAGCTACAACGA TCTGGCTA | 5420 |
| 3220 | AGAGCUCA G CCAGCUCU | 4135 | AGAGCTGG GGCTAGCTACAACGA TGAGCTCT | 5421 |
| 3224 | CUCAGCCA G CUCUGGAU | 4136 | ATCCAGAG GGCTAGCTACAACGA TGGCTGAG | 5422 |
| 3231 | AGCUCUGG A UUUGUGGA | 4137 | TCCACAAA GGCTAGCTACAACGA CCAGAGCT | 5423 |
| 3235 | CUGGAUUU G UGGAGGAG | 4138 | CTCCTCCA GGCTAGCTACAACGA AAATCCAG | 5424 |
| 3246 | GAGGAGAA G UCCCUCAG | 4139 | CTGAGGGA GGCTAGCTACAACGA TTCTCCTC | 5425 |
| 3254 | GUCCCUCA G UGAUGUAG | 4140 | CTACATCA GGCTAGCTACAACGA TGAGGGAC | 5426 |
| 3257 | CCUCAGUG A UGUAGAAG | 4141 | CTTCTACA GGCTAGCTACAACGA CACTGAGG | 5427 |
| 3259 | UCAGUGAU G UAGAAGAA | 4142 | TTCTTCTA GGCTAGCTACAACGA ATCACTGA | 5428 |
| 3274 | AAGAGGAA G CUCCUGAA | 4143 | TTCAGGAG GGCTAGCTACAACGA TTCCTCTT | 5429 |
| 3284 | UCCUGAAG A UCUGUAUA | 4144 | TATACAGA GGCTAGCTACAACGA CTTCAGGA | 5430 |
| 3288 | GAAGAUCU G UAUAAGGA | 4145 | TCCTTATA GGCTAGCTACAACGA AGATCTTC | 5431 |
| 3290 | AGAUCUGU A UAAGGACU | 4146 | AGTCCTTA GGCTAGCTACAACGA ACAGATCT | 5432 |
| 3296 | GUAUAAGG A CUUCCUGA | 4147 | TCAGGAAG GGCTAGCTACAACGA CCTTATAC | 5433 |
| 3304 | ACUUCCUG A CCUUGGAG | 4148 | CTCCAAGG GGCTAGCTACAACGA CAGGAAGT | 5434 |
| 3312 | ACCUUGGA G CAUCUCAU | 4149 | ATGAGATG GGCTAGCTACAACGA TCCAAGGT | 5435 |
| 3314 | CUUGGAGC A UCUCAUCU | 4150 | AGATGAGA GGCTAGCTACAACGA GCTCCAAG | 5436 |
| 3319 | AGCAUCUC A UCUGUUAC | 4151 | GTAACAGA GGCTAGCTACAACGA GAGATGCT | 5437 |
| 3323 | UCUCAUCU G UUACAGCU | 4152 | AGCTGTAA GGCTAGCTACAACGA AGATGAGA | 5438 |
| 3326 | CAUCUGUU A CAGCUUCC | 4153 | GGAAGCTG GGCTAGCTACAACGA AACAGATG | 5439 |
| 3329 | CUGUUACA G CUUCCAAG | 4154 | CTTGGAAG GGCTAGCTACAACGA TGTAACAG | 5440 |
| 3337 | GCUUCCAA G UGGCUAAG | 4155 | CTTAGCCA GGCTAGCTACAACGA TTGGAAGC | 5441 |
| 3340 | UCCAAGUG G CUAAGGGC | 4156 | GCCCTTAG GGCTAGCTACAACGA CACTTGGA | 5442 |
| 3347 | GGCUAAGG G CAUGGAGU | 4157 | ACTCCATG GGCTAGCTACAACGA CCTTAGCC | 5443 |
| 3345 | CUAAGGGC A UGGAGUUC | 4158 | GAACTCCA GGCTAGCTACAACGA GCCCTTAG | 5444 |
| 3354 | GGCAUGGA G UUCUUGGC | 4159 | GCCAAGAA GGCTAGCTACAACGA TCCATGCC | 5445 |
| 3361 | AGUUCUUG G CAUCGCGA | 4160 | TCGCGATG GGCTAGCTACAACGA CAAGAACT | 5446 |
| 3363 | UUCUUGGC A UCGCGAAA | 4161 | TTTCGCGA GGCTAGCTACAACGA GCCAAGAA | 5447 |
| 3366 | UUGGCAUC G CGAAAGUG | 4162 | CACTTTCG GGCTAGCTACAACGA GATGCCAA | 5448 |
| 3372 | UCGCGAAA G UGUAUCCA | 4163 | TGGATACA GGCTAGCTACAACGA TTTCGCGA | 5449 |
| 3374 | GCGAAAGU G UAUCCACA | 4164 | TGTGGATA GGCTAGCTACAACGA ACTTTCGC | 5450 |
| 3376 | GAAAGUGU A UCCACAGG | 4165 | CCTGTGGA GGCTAGCTACAACGA ACACTTTC | 5451 |
| 3380 | GUGUAUCC A CAGGGACC | 4166 | GGTCCCTG GGCTAGCTACAACGA GGATACAC | 5452 |
| 3386 | CCACAGGG A CCUGGCGG | 4167 | CCGCCAGG GGCTAGCTACAACGA CCCTGTGG | 5453 |
| 3391 | GGGACCUG G CGGCACGA | 4168 | TCGTGCCG GGCTAGCTACAACGA CAGGTCCC | 5454 |
| 3394 | ACCUGGCG G CACGAAAU | 4169 | ATTTCGTG GGCTAGCTACAACGA CGCCAGGT | 5455 |
| 3396 | CUGGCGGC A CGAAAUAU | 4170 | ATATTTCG GGCTAGCTACAACGA GCCGCCAG | 5456 |
| 3401 | GGCACGAA A UAUCCUCU | 4171 | AGAGGATA GGCTAGCTACAACGA TTCGTGCC | 5457 |
| 3403 | CACGAAAU A UCCUCUUA | 4172 | TAAGAGGA GGCTAGCTACAACGA ATTTCGTG | 5458 |
| 3411 | AUCCUCUU A UCGGAGAA | 4173 | TTCTCCGA GGCTAGCTACAACGA AAGAGGAT | 5459 |
| 3422 | GGAGAAGA A CGUGGUUA | 4174 | TAACCACG GGCTAGCTACAACGA TCTTCTCC | 5460 |
| 3424 | AGAAGAAC G UGGUUAAA | 4175 | TTTAACCA GGCTAGCTACAACGA GTTCTTCT | 5461 |
| 3427 | AGAACGUG G UUAAAAUC | 4176 | GATTTTAA GGCTAGCTACAACGA CACGTTCT | 5462 |
| 3433 | UGGUUAAA A UCUGUGAC | 4177 | GTCACAGA GGCTAGCTACAACGA TTTAACCA | 5463 |
| 3437 | UAAAAUCU G UGACUUUG | 4178 | CAAAGTCA GGCTAGCTACAACGA AGATTTTA | 5464 |
| 3440 | AAUCUGUG A CUUUGGCU | 4179 | AGCCAAAG GGCTAGCTACAACGA CACAGATT | 5465 |
| 3446 | UGACUUUG G CUUGGCCC | 4180 | GGGCCAAG GGCTAGCTACAACGA CAAAGTCA | 5466 |
| 3451 | UUGGCUUG G CCCGGGAU | 4181 | ATCCCGGG GGCTAGCTACAACGA CAAGCCAA | 5467 |
| 3458 | GGCCCGGG A UAUUUAUA | 4182 | TATAAATA GGCTAGCTACAACGA CCCGGGCC | 5468 |
| 3460 | CCCGGGAU A UUUAUAAA | 4183 | TTTATAAA GGCTAGCTACAACGA ATCCCGGG | 5469 |
| 3464 | GGAUAUUU A UAAAGAUC | 4184 | GATCTTTA GGCTAGCTACAACGA AAATATCC | 5470 |
| 3470 | UUAUAAAG A UCCAGAUU | 4185 | AATCTGGA GGCTAGCTACAACGA CTTTATAA | 5471 |
| 3476 | AGAUCCAG A UUAUGUCA | 4186 | TGACATAA GGCTAGCTACAACGA CTGGATCT | 5472 |
| 3479 | UCCAGAUU A UGUCAGAA | 4187 | TTCTGACA GGCTAGCTACAACGA AATCTGGA | 5473 |
| 3481 | CAGAUUAU G UCAGAAAA | 4188 | TTTTCTGA GGCTAGCTACAACGA ATAATCTG | 5474 |
| 3494 | AAAAGGAG A UGCUCGCC | 4189 | GGCGAGCA GGCTAGCTACAACGA CTCCTTTT | 5475 |
| 3496 | AAGGAGAU G CUCGCCUC | 4190 | GAGGCGAG GGCTAGCTACAACGA ATCTCCTT | 5476 |
| 3500 | AGAUGCUC G CCUCCCUU | 4191 | AAGGGAGG GGCTAGCTACAACGA GAGCATCT | 5477 |
| 3513 | CCUUUGAA A UGGAUGGC | 4192 | GCCATCCA GGCTAGCTACAACGA TTCAAAGG | 5478 |
| 3517 | UGAAAUGG A UGGCCCCA | 4193 | TGGGGCCA GGCTAGCTACAACGA CCATTTCA | 5479 |
| 3520 | AAUGGAUG G CCCCAGAA | 4194 | TTCTGGGG GGCTAGCTACAACGA CATCCATT | 5480 |
| 3529 | CCCCAGAA A CAAUUUUU | 4195 | AAAAATTG GGCTAGCTACAACGA TTCTGGGG | 5481 |
| 3532 | CAGAAACA A UUUUUGAC | 4196 | GTCAAAAA GGCTAGCTACAACGA TGTTTCTG | 5482 |
| 3539 | AAUUUUUG A CAGAGUGU | 4197 | ACACTCTG GGCTAGCTACAACGA CAAAAATT | 5483 |
| 3544 | UUGACAGA G UGUACACA | 4198 | TGTGTACA GGCTAGCTACAACGA TCTGTCAA | 5484 |
| 3546 | GACAGAGU G UACACAAU | 4199 | ATTGTGTA GGCTAGCTACAACGA ACTCTGTC | 5485 |
| 3548 | CAGAGUGU A CACAAUCC | 4200 | GGATTGTG GGCTAGCTACAACGA ACACTCTG | 5486 |
| 3550 | GAGUGUAC A CAAUCCAG | 4201 | CTGGATTG GGCTAGCTACAACGA GTACACTC | 5487 |
| 3553 | UGUACACA A UCCAGAGU | 4202 | ACTCTGGA GGCTAGCTACAACGA TGTGTACA | 5488 |
| 3560 | AAUCCAGA G UGACGUCU | 4203 | AGACGTCA GGCTAGCTACAACGA TCTGGATT | 5489 |
| 3563 | CCAGAGUG A CGUCUGGU | 4204 | ACCAGACG GGCTAGCTACAACGA CACTCTGG | 5490 |
| 3565 | AGAGUGAC G UCUGGUCU | 4205 | AGACCAGA GGCTAGCTACAACGA GTCACTCT | 5491 |
| 3570 | GACGUCUG G UCUUUUGG | 4206 | CCAAAAGA GGCTAGCTACAACGA CAGACGTC | 5492 |
| 3578 | GUCUUUUG G UGUUUUGC | 4207 | GCAAAACA GGCTAGCTACAACGA CAAAAGAC | 5493 |
| 3580 | CUUUUGGU G UUUUGCUG | 4208 | CAGCAAAA GGCTAGCTACAACGA ACCAAAAG | 5494 |
| 3585 | GGUGUUUU G CUGUGGGA | 4209 | TCCCACAG GGCTAGCTACAACGA AAAACACC | 5495 |
| 3588 | GUUUUGCU G UGGGAAAU | 4210 | ATTTCCCA GGCTAGCTACAACGA AGCAAAAC | 5496 |
| 3595 | UGUGGGAA A UAUUUUCC | 4211 | GGAAAATA GGCTAGCTACAACGA TTCCCACA | 5497 |
| 3597 | UGGGAAAU A UUUUCCUU | 4212 | AAGGAAAA GGCTAGCTACAACGA ATTTCCCA | 5498 |
| 3608 | UUCCUUAG G UGCUUCUC | 4213 | GAGAAGCA GGCTAGCTACAACGA CTAAGGAA | 5499 |
| 3610 | CCUUAGGU G CUUCUCCA | 4214 | TGGAGAAG GGCTAGCTACAACGA ACCTAAGG | 5500 |
| 3618 | GCUUCUCC A UAUCCUGG | 4215 | CCAGGATA GGCTAGCTACAACGA GGAGAAGC | 5501 |
| 3620 | UUCUCCAU A UCCUGGGG | 4216 | CCCCAGGA GGCTAGCTACAACGA ATGGAGAA | 5502 |
| 3628 | AUCCUGGG G UAAAGAUU | 4217 | AATCTTTA GGCTAGCTACAACGA CCCAGGAT | 5503 |
| 3634 | GGGUAAAG A UUGAUGAA | 4218 | TTCATCAA GGCTAGCTACAACGA CTTTACCC | 5504 |
| 3638 | AAAGAUUG A UGAAGAAU | 4219 | ATTCTTCA GGCTAGCTACAACGA CAATCTTT | 5505 |
| 3645 | GAUGAAGA A UUUUGUAG | 4220 | CTACAAAA GGCTAGCTACAACGA TCTTCATC | 5506 |
| 3650 | AGAAUUUU G UAGGCGAU | 4221 | ATCGCCTA GGCTAGCTACAACGA AAAATTCT | 5507 |
| 3654 | UUUUGUAG G CGAUUGAA | 4222 | TTCAATCG GGCTAGCTACAACGA CTACAAAA | 5508 |
| 3657 | UGUAGGCG A UUGAAAGA | 4223 | TCTTTCAA GGCTAGCTACAACGA CGCCTACA | 5509 |
| 3670 | AAGAAGGA A CUAGAAUG | 4224 | CATTCTAG GGCTAGCTACAACGA TCCTTCTT | 5510 |
| 3676 | GAACUAGA A UGAGGGCC | 4225 | GGCCCTCA GGCTAGCACAACGA TCTAGTTC | 5511 |
| 3682 | GAAUGAGG G CCCCUGAU | 4226 | ATCAGGGG GGCTAGCTACAACGA CCTCATTC | 5512 |
| 3689 | GGCCCCUG A UUAUACUA | 4227 | TAGTATAA GGCTAGCTACAACGA CAGGGGCC | 5513 |
| 3692 | CCCUGAUU A UACUACAC | 4228 | GTGTAGTA GGCTAGCTACAACGA AATCAGGG | 5514 |
| 3694 | CUGAUUAU A CUACACCA | 4229 | TGGTGTAG GGCTAGCTACAACGA ATAATCAG | 5515 |
| 3697 | AUUAUACU A CACCAGAA | 4230 | TTCTGGTG GGCTAGCTACAACGA AGTATAAT | 5516 |
| 3699 | UAUACUAC A CCAGAAAU | 4231 | ATTTCTGG GGCTAGCTACAACGA GTAGTATA | 5517 |
| 3706 | CACCAGAA A UGUACCAG | 4232 | CTGGTACA GGCTAGCTACAACGA TTCTGGTG | 5518 |
| 3708 | CCAGAAAU G UACCAGAC | 4233 | GTCTGGTA GGCTAGCTACAACGA ATTTCTGG | 5519 |
| 3710 | AGAAAUGU A CCAGACCA | 4234 | TGGTCTGG GGCTAGCTACAACGA ACATTTCT | 5520 |
| 3715 | UGUACCAG A CCAUGCUG | 4235 | CAGCATGG GGCTAGCTACAACGA CTGGTACA | 5521 |
| 3718 | ACCAGACC A UGCUGGAC | 4236 | GTCCAGCA GGCTAGCTACAACGA GGTCTGGT | 5522 |
| 3720 | CAGACCAU G CUGGACUG | 4237 | CAGTCCAG GGCTAGCTACAACGA ATGGTCTG | 5523 |
| 3725 | CAUGCUGG A CUGCUGGC | 4238 | GCCAGCAG GGCTAGCTACAACGA CCAGCATG | 5524 |
| 3728 | GCUGGACU G CUGGCACG | 4239 | CGTGCCAG GGCTAGCTACAACGA AGTCCAGC | 5525 |
| 3732 | GACUGCUG G CACGGGGA | 4240 | TCCCCGTG GGCTAGCTACAACGA CAGCAGTC | 5526 |
| 3734 | CUGCUGGC A CGGGGAGC | 4241 | GCTCCCCG GGCTAGCTACAACGA GCCAGCAG | 5527 |
| 3741 | CACGGGGA G CCCAGUCA | 4242 | TGACTGGG GGCTAGCTACAACGA TCCCCGTG | 5528 |
| 3746 | GGAGCCCA G UCAGAGAC | 4243 | GTCTCTGA GGCTAGCTACAACGA TGGGCTCC | 5529 |
| 3753 | AGUCAGAG A CCCACGUU | 4244 | AACGTGGG GGCTAGCTACAACGA CTCTGACT | 5530 |
| 3757 | AGAGACCC A CGUUUUCA | 4245 | TGAAAACG GGCTAGCTACAACGA GGGTCTCT | 5531 |
| 3759 | AGACCCAC G UUUUCAGA | 4246 | TCTGAAAA GGCTAGCTACAACGA GTGGGTCT | 5532 |
| 3768 | UUUUCAGA G UUGGUGGA | 4247 | TCCACCAA GGCTAGCTACAACGA TCTGAAAA | 5533 |
| 3772 | CAGAGUUG G UGGAACAU | 4248 | ATGTTCCA GGCTAGCTACAACGA CAACTCTG | 5534 |
| 3777 | UUGGUGGA A CAUUUGGG | 4249 | CCCAAATG GGCTAGCTACAACGA TCCACCAA | 5535 |
| 3779 | GGUGGAAC A UUUGGGAA | 4250 | TTCCCAAA GGCTAGCTACAACGA GTTCCACC | 5536 |
| 3788 | UUUGGGAA A UCUCUUGC | 4251 | GCAAGAGA GGCTAGCTACAACGA TTCCCAAA | 5537 |
| 3795 | AAUCUCUU G CAAGCUAA | 4252 | TTAGCTTG GGCTAGCTACAACGA AAGAGATT | 5538 |
| 3799 | UCUUGCAA G CUAAUGCU | 4253 | AGCATTAG GGCTAGCTACAACGA TTGCAAGA | 5539 |
| 3803 | GCAAGCUA A UGCUCAGC | 4254 | GCTGAGCA GGCTAGCTACAACGA TAGCTTGC | 5540 |
| 3805 | AAGCUAAU G CUCAGCAG | 4255 | CTGCTGAG GGCTAGCTACAACGA ATTAGCTT | 5541 |
| 3810 | AAUGCUCA G CAGGAUGG | 4256 | CCATCCTG GGCTAGCTACAACGA TGAGCATT | 5542 |
| 3815 | UCAGCAGG A UGGCAAAG | 4257 | CTTTGCCA GGCTAGCTACAACGA CCTGCTGA | 5543 |
| 3818 | GCAGGAUG G CAAAGACU | 4258 | AGTCTTTG GGCTAGCTACAACGA CATCCTGC | 5544 |
| 3824 | UGGCAAAG A CUACAUUG | 4259 | CAATGTAG GGCTAGCTACAACGA CTTTGCCA | 5545 |
| 3827 | CAAAGACU A CAUUGUUC | 4260 | GAACAATG GGCTAGCTACAACGA AGTCTTTG | 5546 |
| 3829 | AAGACUAC A UUGUUCUU | 4261 | AAGAACAA GGCTAGCTACAACGA GTAGTCTT | 5547 |
| 3832 | ACUACAUU G UUCUUCCG | 4262 | CGGAAGAA GGCTAGCTACAACGA AATGTAGT | 5548 |
| 3841 | UUCUUCCG A UAUCAGAG | 4263 | CTCTGATA GGCTAGCTACAACGA CGGAAGAA | 5549 |
| 3843 | CUUCCGAU A UCAGAGAC | 4264 | GTCTCTGA GGCTAGCTAGAACGA ATCGGAAG | 5550 |
| 3850 | UAUCAGAG A CUUUGAGC | 4265 | GCTCAAAG GGCTAGCTACAACGA CTCTGATA | 5551 |
| 3857 | GACUUUGA G CAUGGAAG | 4266 | CTTCCATG GGCTAGCTACAACGA TCAAAGTC | 5552 |
| 3859 | CUUUGAGC A UGGAAGAG | 4267 | CTCTTCCA GGCTAGCTACAACGA GCTCAAAG | 5553 |
| 3869 | GGAAGAGG A UUCUGGAC | 4268 | GTCCAGAA GGCTAGCTACAACGA CCTCTTCC | 5554 |
| 3876 | GAUUCUGG A CUCUCUCU | 4269 | AGAGAGAG GGCTAGCTACAACGA CCAGAATC | 5555 |
| 3885 | CUCUCUCU G CCUACCUC | 4270 | GAGGTAGG GGCTAGCTACAACGA AGAGAGAG | 5556 |
| 3889 | CUCUGCCU A CCUCACCU | 4271 | AGGTGAGG GGCTAGCTACAACGA AGGCAGAG | 5557 |
| 3894 | CCUACCUC A CCUGUUUC | 4272 | GAAACAGG GGCTAGCTACAACGA GAGGTAGG | 5558 |
| 3898 | CCUCACCU G UUUCCUGU | 4273 | ACAGGAAA GGCTAGCTACAACGA AGGTGAGG | 5559 |
| 3905 | UGUUUCCU G UAUGGAGG | 4274 | CCTCCATA GGCTAGCTACAACGA AGGAAACA | 5560 |
| 3907 | UUUCCUGU A UGGAGGAG | 4275 | CTCCTCCA GGCTAGCTACAACGA ACAGGAAA | 5561 |
| 3922 | AGGAGGAA G UAUGUGAC | 4276 | GTCACATA GGCTAGCTACAACGA TTCCTCCT | 5562 |
| 3924 | GAGGAAGU A UGUGACCC | 4277 | GGGTCACA GGCTAGCTACAACGA ACTTCCTC | 5563 |
| 3926 | GGAAGUAU G UGACCCCA | 4278 | TGGGGTCA GGCTAGCTACAACGA ATACTTCC | 5564 |
| 3929 | AGUAUGUG A CCCCAAAU | 4279 | ATTTGGGG GGCTAGCTACAACGA CACATACT | 5565 |
| 3936 | GACCCCAA A UUCCAUUA | 4280 | TAATGGAA GGCTAGCTACAACGA TTGGGGTC | 5566 |
| 3941 | CAAAUUCC A UUAUGACA | 4281 | TGTCATAA GGCTAGCTACAACGA GGAATTTG | 5567 |
| 3944 | AUUCCAUU A UGACAACA | 4282 | TGTTGTCA GGCTAGCTACAACGA AATGGAAT | 5568 |
| 3947 | CCAUUAUG A CAACACAG | 4283 | CTGTGTTG GGCTAGCTACAACGA CATAATGG | 5569 |
| 3950 | UUAUGACK A CACAGCAG | 4284 | CTGCTGTG GGCTAGCTACAACGA TGTCATAA | 5570 |
| 3952 | AUGACAAC A CAGCAGGA | 4285 | TCCTGCTG GGCTAGCTACAACGA GTTGTCAT | 5571 |
| 3955 | ACAACACA G CAGGAAUC | 4286 | GATTCCTG GGCTAGCTACAACGA TGTGTTGT | 5572 |
| 3961 | CAGCAGGA A UCAGUCAG | 4287 | CTGACTGA GGCTAGCTACAACGA TCCTGCTG | 5573 |
| 3965 | AGGAAUCA G UCAGUAUC | 4288 | GATACTGA GGCTAGCTACAACGA TGATTCCT | 5574 |
| 3969 | AUCAGUCA G UAUCUGCA | 4289 | TGCAGATA GGCTAGCTACAACGA TGACTGAT | 5575 |
| 3971 | CAGUCAGU A UCUGCAGA | 4290 | TCTGCAGA GGCTAGCTACAACGA ACTGACTG | 5576 |
| 3975 | CAGUAUCU G CAGAACAG | 4291 | CTGTTCTG GGCTAGCTACAACGA AGATACTG | 5577 |
| 3980 | UCUGCAGA A CAGUAAGC | 4292 | GCTTACTG GGCTAGCTACAACGA TCTGCAGA | 5578 |
| 3983 | GCAGAACA G UAAGCGAA | 4293 | TTCGCTTA GGCTAGCTACAACGA TGTTCTGC | 5579 |
| 3987 | AACAGUAA G CGAAAGAG | 4294 | CTCTTTCG GGCTAGCTACAACGA TTACTGTT | 5580 |
| 3995 | GCGAAAGA G CCGGCCUG | 4295 | CAGGCCGG GGCTAGCTACAACGA TCTTTCGC | 5581 |
| 3999 | AAGAGCCG G CCUGUGAG | 4296 | CTCACAGG GGCTAGCTACAACGA CGGCTCTT | 5582 |
| 4003 | GCCGGCCU G UGAGUGUA | 4297 | TACACTCA GGCTAGCTACAACGA AGGCCGGC | 5583 |
| 4007 | GCCUGUGA G UGUAAAAA | 4298 | TTTTTACA GGCTAGCTACAACGA TCACAGGC | 5584 |
| 4009 | CUGUGAGU G UAAAAACA | 4299 | TGTTTTTA GGCTAGCTACAACGA ACTCACAG | 5585 |
| 4015 | GUGUAAAA A CAUUUGAA | 4300 | TTCAAATG GGCTAGCTACAACGA TTTTACAC | 5586 |
| 4017 | GUAAAAAC A UUUGAAGA | 4301 | TCTTCAAA GGCTAGCTACAACGA GTTTTTAC | 5587 |
| 4025 | AUUUGAAG A UAUCCCGU | 4302 | ACGGGATA GGCTAGCTACAACGA CTTCAAAT | 5588 |
| 4027 | UUGAAGAU A UCCCGUUA | 4303 | TAACGGGA GGCTAGCTACAACGA ATCTTCAA | 5589 |
| 4032 | GAUAUCCC G UUAGAAGA | 4304 | TCTTCTAA GGCTAGCTACAACGA GGGATATC | 5590 |
| 4041 | UUAGAAGA A CCAGAAGU | 4305 | ACTTCTGG GGCTAGCTACAACGA TCTTCTAA | 5591 |
| 4048 | AACCAGAA G UAAAAGUA | 4306 | TACTTTTA GGCTAGCTACAACGA TTCTGGTT | 5592 |
| 4054 | AAGUAAAA G UAAUCCCA | 4307 | TGGGATTA GGCTAGCTACAACGA TTTTACTT | 5593 |
| 4057 | UAAAAGUA A UCCCAGAU | 4308 | ATCTGGGA GGCTAGCTACAACGA TACTTTTA | 5594 |
| 4064 | AAUCCCAG A UGACAACC | 4309 | GGTTGTCA GGCTAGCTACAACGA CTGGGATT | 5595 |
| 4067 | CCCAGAUG A CAACCAGA | 4310 | TCTGGTTG GGCTAGCTACAACGA CATCTGGG | 5596 |
| 4070 | AGAUGACA A CCAGACGG | 4311 | CCGTCTGG GGCTAGCTACAACGA TGTCATCT | 5597 |
| 4075 | ACAACCAG A CGGACAGU | 4312 | ACTGTCCG GGCTAGCTACAACGA CTGGTTGT | 5598 |
| 4079 | CCAGACGG A CAGUGGUA | 4313 | TACCACTG GGCTAGCTACAACGA CCGTCTGG | 5599 |
| 4082 | GACGGACA G UGGUAUGG | 4314 | CCATACCA GGCTAGCTACAACGA TGTCCGTC | 5600 |
| 4085 | GGACAGUG G UAUGGUUC | 4315 | GAACCATA GGCTAGCTACAACGA CACTGTCC | 5601 |
| 4087 | ACAGUGGU A UGGUUCUU | 4316 | AAGAACCA GGCTAGCTACAACGA ACCACTGT | 5602 |
| 4090 | GUGGUAUG G UUCUUGCC | 4317 | GGCAAGAA GGCTAGCTACAACGA CATACCAC | 5603 |
| 4096 | UGGUUCUU G CCUCAGAA | 4318 | TTCTGAGG GGCTAGCTACAACGA AAGAACCA | 5604 |
| 4107 | UCAGAAGA G CUGAAAAC | 4319 | GTTTTCAG GGCTAGCTACAACGA TCTTCTGA | 5605 |
| 4114 | AGCUGAAA A CUUUGGAA | 4320 | TTCCAAAG GGCTAGCTACAACGA TTTCAGCT | 5606 |
| 4124 | UUUGGAAG A CAGAACCA | 4321 | TGGTTCTG GGCTAGCTACAACGA CTTCCAAA | 5607 |
| 4129 | AAGACAGA A CAAAUUA | 4322 | TAATTTGG GGCTAGCTACAACGA TCTGTCTT | 5608 |
| 4134 | AGAACCAA A UUAUCUCC | 4323 | GGAGATAA GGCTAGCTACAACGA TTGGTTCT | 5609 |
| 4137 | ACCAAAUU A UCUCCAUC | 4324 | GATGGAGA GGCTAGCTACAACGA AATTTGGT | 5610 |
| 4143 | UUAUCUCC A UCUUUUGG | 4325 | CCAAAAGA GGCTAGCTACAACGA GGAGATAA | 5611 |
| 4151 | AUCUUUUG G UGGAAUGG | 4326 | CCATTCCA GGCTAGCTACAACGA CAAAAGAT | 5612 |
| 4156 | UUGGUGGA A UGGUGCCC | 4327 | GGGCACCA GGCTAGCTACAACGA TCCACCAA | 5613 |
| 4159 | GUGGAAUG G UGCCCAGC | 4328 | GCTGGGCA GGCTAGCTACAACGA CATTCCAC | 5614 |
| 4161 | GGAAUGGU G CCCAGCAA | 4329 | TTGCTGGG GGCTAGCTACAACGA ACCATTCC | 5615 |
| 4166 | GGUGCCCA G CAAAAGCA | 4330 | TGCTTTTG GGCTAGCTACAACGA TGGGCACC | 5616 |
| 4172 | CAGCAAAA G CAGGGAGU | 4331 | ACTCCCTG GGCTAGCTACAACGA TTTTGCTG | 5617 |
| 4179 | AGCAGGGA G UCUGUGGC | 4332 | GCCACAGA GGCTAGCTACAACGA TCCCTGCT | 5618 |
| 4183 | GGGAGUCU G UGGCAUCU | 4333 | AGATGCCA GGCTAGCTACAACGA AGACTCCC | 5619 |
| 4186 | AGUCUGUG G CAUCUGAA | 4334 | TTCAGATG GGCTAGCTACAACGA CACAGACT | 5620 |
| 4188 | UCUGUGGC A UCUGAAGG | 4335 | CCTTCAGA GGCTAGCTACAACGA GCCACAGA | 5621 |
| 4196 | AUCUGAAG G CUCAAACC | 4336 | GGTTTGAG GGCTAGCTACAACGA CTTCAGAT | 5622 |
| 4202 | AGGCUCAA A CCAGACAA | 4337 | TTGTCTGG GGCTAGCTACAACGA TTGAGCCT | 5623 |
| 4207 | CAAACCAG A CAAGCGGC | 4338 | GCCGCTTG GGCTAGCTACAACGA CTGGTTTG | 5624 |
| 4211 | CCAGACAA G CGGCUACC | 4339 | GGTAGCCG GGCTAGCTACAACGA TTGTCTGG | 5625 |
| 4214 | GACAAGCG G CUACCAGU | 4340 | ACTGGTAG GGCTAGCTACAACGA CGCTTGTC | 5626 |
| 4217 | AAGCGGCU A CCAGUCCG | 4341 | CGGACTGG GGCTAGCTACAACGA AGCCGCTT | 5627 |
| 4221 | GGCUACCA G UCCGGAUA | 4342 | TATCCGGA GGCTAGCTACAACGA TGGTAGCC | 5628 |
| 4227 | CAGUCCGG A UAUCACUC | 4343 | GAGTGATA GGCTAGCTACAACGA CCGGACTG | 5629 |
| 4229 | GUCCGGAU A UCACUCCG | 4344 | CGGAGTGA GGCTAGCTACAACGA ATCCGGAC | 5630 |
| 4232 | CGGAUAUC A CUCCGAUG | 4345 | CATCGGAG GGCTAGCTACAACGA GATATCCG | 5631 |
| 4238 | UCACUCCG A UGACACAG | 4346 | CTGTGTCA GGCTAGCTACAACGA CGGAGTGA | 5632 |
| 4241 | CUCCGAUG A CACAGACA | 4347 | TGTCTGTG GGCTAGCTACAACGA CATCGGAG | 5633 |
| 4243 | CCGAUGAC A CAGACACC | 4348 | GGTGTCTG GGCTAGCTACAACGA GTCATCGG | 5634 |
| 4247 | UGACACAG A CACCACCG | 4349 | CGGTGGTG GGCTAGCTACAACGA CTGTGTCA | 5635 |
| 4249 | ACACAGAC A CCACCGUG | 4350 | CACGGTGG GGCTAGCTACAACGA GTCTGTGT | 5636 |
| 4252 | CAGACACC A CCGUGUAC | 4351 | GTACACGG GGCTAGCTACAACGA GGTGTCTG | 5637 |
| 4255 | ACACCACC G UGUACUCC | 4352 | GGAGTACA GGCTAGCTACAACGA GGTGGTGT | 5638 |
| 4257 | ACCACCGU G UACUCCAG | 4353 | CTGGAGTA GGCTAGCTACAACGA ACGGTGGT | 5639 |
| 4259 | CACCGUGU A CUCCAGUG | 4354 | CACTGGAG GGCTAGCTACAACGA ACACGGTG | 5640 |
| 4265 | GUACUCCA G UGAGGAAG | 4355 | CTTCCTCA GGCTAGCTACAACGA TGGAGTAC | 5641 |
| 4273 | GUGAGGAA G CAGAACUU | 4356 | AAGTTCTG GGCTAGCTACAACGA TTCCTCAC | 5642 |
| 4278 | GAAGCAGA A CUUUUAAA | 4357 | TTTAAAAG GGCTAGCTACAACGA TCTGCTTC | 5643 |
| 4287 | CUUUUAAA G CUGAUAGA | 4358 | TCTATCAG GGCTAGCTACAACGA TTTAAAAG | 5644 |
| 4291 | UAAAGCUG A UAGAGAUU | 4359 | AATCTCTA GGCTAGCTACAACGA CAGCTTTA | 5645 |
| 4297 | UGAUAGAG A UUGGAGUG | 4360 | CACTCCAA GGCTAGCTACAACGA CTCTATCA | 5646 |
| 4303 | AGAUUGGA G UGCAAACC | 4361 | GGTTTGCA GGCTAGCTACAACGA TCCAATCT | 5647 |
| 4305 | AUUGGAGU G CAAACCGG | 4362 | CCGGTTTG GGCTAGCTACAACGA ACTCCAAT | 5648 |
| 4309 | GAGUGCAA A CCGGUAGC | 4363 | GCTACCGG GGCTAGCTACAACGA TTGCACTC | 5649 |
| 4313 | GCAAACCG G UAGCACAG | 4364 | CTGTGCTA GGCTAGCTACAACGA CGGTTTGC | 5650 |
| 4316 | AACCGGUA G CACAGCCC | 4365 | GGGCTGTG GGCTAGCTACAACGA TACCGGTT | 5651 |
| 4318 | CCGGUAGC A CAGCCCAG | 4366 | CTGGGCTG GGCTAGCTACAACGA GCTACCGG | 5652 |
| 4321 | GUAGCACA G CCCAGAUU | 4367 | AATCTGGG GGCTAGCTACAACGA TGTGCTAC | 5653 |
| 4327 | CAGCCCAG A UUCUCCAG | 4368 | CTGGAGAA GGCTAGCTACAACGA CTGGGCTG | 5654 |
| 4335 | AUUCUCCA G CCUGACUC | 4369 | GAGTCAGG GGCTAGCTACAACGA TGGAGAAT | 5655 |
| 4340 | CCAGCCUG A CUCGGGGA | 4370 | TCCCCGAG GGCTAGCTACAACGA CAGGCTGG | 5656 |
| 4348 | ACUCGGGG A CCACACUG | 4371 | CAGTGTGG GGCTAGCTACAACGA CCCCGAGT | 5657 |
| 4351 | CGGGGACC A CACUGAGC | 4372 | GCTCAGTG GGCTAGCTACAACGA GGTCCCCG | 5658 |
| 4353 | GGGACCAC A CUGAGCUC | 4373 | GAGCTCAG GGCTAGCTACAACGA GTGGTCCC | 5659 |
| 4358 | CACACUGA G CUCUCCUC | 4374 | GAGGAGAG GGCTAGCTACAACGA TCAGTGTG | 5660 |
| 4369 | CUCCUCCU G UUUAAAAG | 4375 | CTTTTAAA GGCTAGCTACAACGA AGGAGGAG | 5661 |
| 4381 | AAAAGGAA G CAUCCACA | 4376 | TGTGGATG GGCTAGCTACAACGA TTCCTTTT | 5662 |
| 4383 | AAGGAAGC A UCCACACC | 4377 | GGTGTGGA GGCTAGCTACAACGA GCTTCCTT | 5663 |
| 4387 | AAGCAUCC A CACCCCAA | 4378 | TTGGGGTG GGCTAGCTACAACGA GGATGCTT | 5664 |
| 4389 | GCAUCCAC A CCCCAACU | 4379 | AGTTGGGG GGCTAGCTACAACGA GTGGATGC | 5665 |
| 4395 | ACACCCCA A CUCCCGGA | 4380 | TCCGGGAG GGCTAGCTACAACGA TGGGGTGT | 5666 |
| 4403 | ACUCCCGG A CAUCACAU | 4381 | ATGTGATJG GGCTAGCTACAACGA CCGGGAGT | 5667 |
| 4405 | UCCCGGAC A UCACAUGA | 4382 | TCATGTGA GGCTAGCTACAACGA GTCCGGGA | 5668 |
| 4408 | CGGACAUC A CAUGAGAG | 4383 | CTCTCATG GGCTAGCTACAACGA GATGTCCG | 5669 |
| 4410 | GACAUCAC A UGAGAGGU | 4384 | ACCTCTCA GGCTAGCTACAACGA GTGATGTC | 5670 |
| 4417 | CAUGAGAG G UCUGCUCA | 4385 | TGAGCAGA GGCTAGCTACAACGA CTCTCATG | 5671 |
| 4421 | AGAGGUCU G CUCAGAUU | 4386 | AATCTGAG GGCTAGCTACAACGA AGACCTCT | 5672 |
| 4427 | CUGCUCAG A UUUUGAAG | 4381 | CTTCAAAA GGCTAGCTACAACGA CTGAGCAG | 5673 |
| 4435 | AUUUUGAA G UGUUGUUC | 4388 | GAACAACA GGCTAGCTACAACGA TTCAAAAT | 5674 |
| 4437 | UUUGAAGU G UUGUUCUU | 4389 | AAGAACAA GGCTAGCTACAACGA ACTTCAAA | 5675 |
| 4440 | GAAGUGUU G UUCUUUCC | 4390 | GGAAAGAA GGCTAGCTACAACGA AACACTTC | 5676 |
| 4449 | UUCUUUCC A CCAGCAGG | 4391 | CCTGCTGG GGCTAGCTACAACGA GGAAAGAA | 5677 |
| 4453 | UUCCACCA G CAGGAAGU | 4392 | ACTTCCTG GGCTAGCTACAACGA TGGTGGAA | 5678 |
| 4460 | AGCAGGAA G UAGCCGCA | 4393 | TGCGGCTA GGCTAGCTACAACGA TTCCTGCT | 5679 |
| 4463 | AGGAAGUA G CCGCAUUU | 4394 | AAATGCGG GGCTAGCTACAACGA TACTTCCT | 5680 |
| 4466 | AAGUAGCC G CAUUUGAU | 4395 | ATGAAATG GGCTAGCTACAACGA GGCTACTT | 5681 |
| 4468 | GUAGCCGC A UUUGAUUU | 4396 | AAATCAAA GGCTAGCTACAACGA GCGGCTAC | 5682 |
| 4473 | CGCAUUUG A UUUUCAUU | 4397 7 | AATGAAAA GGCTAGCTACAACGA CAAATGCG | 5683 |
| 4479 | UGAUUUUC A UUUCGACA | 4398 | TGTCGAAA GGCTAGCTACAACGA GAAAATCA | 5684 |
| 4485 | UCAUUUCG A CAACAGAA | 4399 | TTCTGTTG GGCTAGCTACAACGA CGAAATGA | 5685 |
| 4488 | UUUCGACA A CAGAAAAA | 4400 | TTTTTCTG GGCTAGCTACAACGA TGTCGAAA | 5686 |
| 4499 | GAAAAAGG A CCUCGGAC | 4401 | GTCCGAGG GGCTAGCTACAACGA CCTTTTTC | 5687 |
| 4506 | GACCUCGG A CUGCAGGG | 4402 | CCCTGCAG GGCTAGCTACAACGA CCGAGGTC | 5688 |
| 4509 | CUCGGACU G CAGGGAGC | 4403 | GCTCCCTG GGCTAGCTACAACGA AGTCCGAG | 5689 |
| 4516 | UGCAGGGA G CCAGUCUU | 4404 | AAGACTGG GGCTAGCTACAACGA TCCCTGCA | 5690 |
| 4520 | GGGAGCCA G UCUUCUAG | 4405 | CTAGAAGA GGCTAGCTACAACGA TGGCTCCC | 5691 |
| 4529 | UCUUCUAG G CAUAUCCU | 4406 | AGGATATG GGCTAGCTACAACGA CTAGAAGA | 5692 |
| 4531 | UUCUAGGC A UAUCCUGG | 4407 | CCAGGATA GGCTAGCTACAACGA GCCTAGAA | 5693 |
| 4533 | CUAGGCAU A UCCUGGAA | 4408 | TTCCAGGA GGCTAGCTACAACGA ATGCCTAG | 5694 |
| 4545 | UGGAAGAG G CUUGUGAC | 4409 | GTCACAAG GGCTAGCTACAACGA CTCTTCCA | 5695 |
| 4549 | AGAGGCUU G UGACCCAA | 4410 | TTGGGTCA GGCTAGCTACAACGA AAGCCTCT | 5696 |
| 4552 | GGCUUGUG A CCCAAGAA | 4411 | TTCTTGGG GGCTAGCTACAACGA CACAAGCC | 5697 |
| 4560 | ACCCAAGA A UGUGUCUG | 4412 | CAGACACA GGCTAGCTACAACGA TCTTGGGT | 5698 |
| 4562 | CCAAGAAU G UGUCUGUG | 4413 | CACAGACA GGCTAGCTACAACGA ATTCTTGG | 5699 |
| 4564 | AAGAAUGU G UCUGUGUC | 4414 | GACACAGA GGCTAGCTACAACGA ACATTCTT | 5700 |
| 4568 | AUGUGUCU G UGUCUUCU | 4415 | AGAAGACA GGCTAGCTACAACGA AGACACAT | 5701 |
| 4570 | GUGUCUGU G UCUUCUCC | 4416 | GGAGAAGA GGCTAGCTACAACGA ACAGACAC | 5702 |
| 4581 | UUCUCCCA G UGUUGACC | 4417 | GGTCAACA GGCTAGCTACAACGA TGGGAGAA | 5703 |
| 4583 | CUCCCAGU G UUGACCUG | 4418 | CAGGTCAA GGCTAGCTACAACGA ACTGGGAG | 5704 |
| 4587 | CAGUGUUG A CCUGAUCC | 4419 | GGATCAGG GGCTAGCTACAACGA CAACACTG | 5705 |
| 4592 | UUGACCUG A UCCUCUUU | 4420 | AAAGAGGA GGCTAGCTACAACGA CAGGTCAA | 5706 |
| 4605 | CUUUUUUC A UUCAUUUA | 4421 | TAAATGAA GGCTAGCTACAACGA GAAAAAAG | 5707 |
| 4609 | UUUCAUUC A UUUAAAAA | 4422 | TTTTTAAA GGCTAGCTACAACGA GAATGAAA | 5708 |
| 4618 | UUUAAAAA G CAUUAUCA | 4423 | TGATAATG GGCTAGCTACAACGA TTTTTAAA | 5709 |
| 4620 | UAAAAAGC A WAUCAUG | 4424 | CATGATAA GGCTAGCTACAACGA GCTTTTTA | 5710 |
| 4623 | AAAGCAUU A UCAUGCCC | 4425 | GGGCATGA GGCTAGCTACAACGA AATGCTTT | 5711 |
| 4626 | GCAUUAUC A UGCCCCUG | 4426 | CAGGGGCA GGCTAGCTACAACGA GATAATGC | 5712 |
| 4628 | AUUAUCAU G CCCCUGCU | 4427 | AGCAGGGG GGCTAGCTACAACGA ATGATAAT | 5713 |
| 4634 | AUGCCCCU G CUGCGGGU | 4428 | ACCCGCAG GGCTAGCTACAACGA AGGGGCAT | 5714 |
| 4637 | CCCCUGCU G CGGGUCUC | 4429 | GAGACCCG GGCTAGCTACAACGA AGCAGGGG | 5715 |
| 4641 | UGCUGCGG G UCUCACCA | 4430 | TGGTGAGA GGCTAGCTACAACGA CCGCAGCA | 5716 |
| 4646 | CGGGUCUC A CCAUGGGU | 4431 | ACCCATGG GGCTAGCTACAACGA GAGACCCG | 5717 |
| 4649 | GUCUCACC A UGGGUUUA | 4432 | TAAACCCA GGCTAGCTACAACGA GGTGAGAC | 5718 |
| 4653 | CACCAUGG G UWAGAAC | 4433 | GTTCTAAA GGCTAGCTACAACGA CCATGGTG | 5719 |
| 4660 | GGUUUAGA A CAAAGAGC | 4434 | GCTCTTTG GGCTAGCTACAACGA TCTAAACC | 5720 |
| 4667 | AACAAAGA G CUUCAAGC | 4435 | GCTTGAAG GGCTAGCTACAACGA TCTTTGTT | 5721 |
| 4674 | AGCUUCAA G CAAUGGCC | 4436 | GGCCATTG GGCTAGCTACAACGA TTGAAGCT | 5722 |
| 4677 | UUCAAGCA A UGGCCCCA | 4437 | TGGGGCCA GGCTAGCTACAACGA TGCTTGAA | 5723 |
| 4680 | AAGCAAUG G CCCCAUCC | 4438 | GGATGGGG GGCTAGCTACAACGA CATTGCTT | 5724 |
| 4685 | AUGGCCCC A UCCUCAAA | 4439 | TTTGAGGA GGCTAGCTACAACGA GGGGCCAT | 5725 |
| 4697 | UCAAAGAA G UAGCAGUA | 4440 | TACTGCTA GGCTAGCTACAACGA TTCTTTGA | 5726 |
| 4700 | AAGAAGUA G CAGUACCU | 4441 | AGGTACTG GGCTAGCTACAACGA TACTTCTT | 5727 |
| 4703 | AAGUAGCA G UACCUGGG | 4442 | CCCAGGTA GGCTAGCTACAACGA TGCTACTT | 5728 |
| 4705 | GUAGCAGU A CCUGGGGA | 4443 | TCCCCAGG GGCTAGCTACAACGA ACTGCTAC | 5729 |
| 4714 | CCUGGGGA G CUGACACU | 4444 | AGTGTCAG GGCTAGCTACAACGA TCCCCAGG | 5730 |
| 4718 | GGGAGCUG A CACUUCUG | 4445 | CAGAAGTG GGCTAGCTACAACGA CAGCTCCC | 5731 |
| 4720 | GAGCUGAC A CUUCUGUA | 4446 | TACAGAAG GGCTAGCTACAACGA GTCAGCTC | 5732 |
| 4726 | ACACUUCU G UAAAACUA | 4447 | TAGTTTTA GGCTAGCTACAACGA AGAAGTGT | 5733 |
| 4731 | UCUGUAAA A CUAGAAGA | 4448 | TCTTCTAG GGCTAGCTACAACGA TTTACAGA | 5734 |
| 4739 | ACUAGAAG A UAAACCAG | 4449 | CTGGTTTA GGCTAGCTACAACGA CTTCTAGT | 5735 |
| 4743 | GAAGAUAA A CCAGGCAA | 4450 | TTGCCTGG GGCTAGCTACAACGA TTATCTTC | 5736 |
| 4748 | UAAACCAG G CAACGUAA | 4451 | TTACGTTG GGCTAGCTACAACGA CTGGTTTA | 5737 |
| 4751 | ACCAGGCA A CGUAAGUG | 4452 | CACTTACG GGCTAGCTACAACGA TGCCTGGT | 5738 |
| 4753 | CAGGCAAC G UAAGUGUU | 4453 | AACACTTA GGCTAGCTACAACGA GTTGCCTG | 5739 |
| 4757 | CAACGUAA G UGUUCGAG | 4454 | CTCGAACA GGCTAGCTACAACGA TTACGTTG | 5740 |
| 4759 | ACGUAAGU G UUCGAGGU | 4455 | ACCTCGAA GGCTAGCTACAACGA ACTTACGT | 5741 |
| 4766 | UGUUCGAG G UGUUGAAG | 4456 | CTTCAACA GGCTAGCTACAACGA CTCGAACA | 5742 |
| 4768 | UUCGAGGU G UUGAAGAU | 4457 | ATCTTCAA GGCTAGCTACAACGA ACCTCGAA | 5743 |
| 4775 | UGUUGAAG A UGGGAAGG | 4458 | CCTTCCCA GGCTAGCTACAACGA CTTCAACA | 5744 |
| 4784 | UGGGAAGG A UUUGCAGG | 4459 | CCTGCAAA GGCTAGCTACAACGA CCTTCCCA | 5745 |
| 4788 | AAGGAUUU G CAGGGCUG | 4460 | CAGCCCTG GGCTAGCTACAACGA AAATCCTT | 5746 |
| 4793 | UUUGCAGG G CUGAGUCU | 4461 | AGACTCAG GGCTAGCTACAACGA CCTGCAAA | 5747 |
| 4798 | AGGGCUGA G UCUAUCCA | 4462 | TGGATAGA GGCTAGCTACAACGA TCAGCCCT | 5748 |
| 4802 | CUGAGUCU A UCCAAGAG | 4463 | CTCTTGGA GGCTAGCTACAACGA AGACTCAG | 5749 |
| 4811 | UCCAAGAG G CUUUGUUU | 4464 | AAACAAAG GGCTAGCTACAACGA CTCTTGGA | 5750 |
| 4816 | GAGGCUUU G UUUAGGAC | 4465 | GTCCTAAA GGCTAGCTACAACGA AAAGCCTC | 5751 |
| 4823 | UGUUUAGG A CGUGGGUC | 4466 | GACCCACG GGCTAGCTACAACGA CCTAAACA | 5752 |
| 4825 | UUUAGGAC G UGGGUCCC | 4467 | GGGACCCA GGCTAGCTACAACGA GTCCTAAA | 5753 |
| 4829 | GGACGUGG G UCCCAAGC | 4468 | GCTTGGGA GGCTAGCTACAACGA CCACGTCC | 5754 |
| 4836 | GGUCCCAA G CCAAGCCU | 4469 | AGGCTTGG GGCTAGCTACAACGA TTGGGACC | 5755 |
| 4841 | CAAGCCAA G CCUUAAGU | 4470 | ACTTAAGG GGCTAGCTACAACGA TTGGCTTG | 5756 |
| 4848 | AGCCUUAA G UGUGGAAU | 4471 | ATTCCACA GGCTAGCTACAACGA TTAAGGCT | 5757 |
| 4850 | CCUUAAGU G UGGAAUUC | 4472 | GAATTCCA GGCTAGCTACAACGA ACTTAAGG | 5758 |
| 4855 | AGUGUGGA A UUCGGAUU | 4473 | AATCCGAA GGCTAGCTACAACGA TCCACACT | 5759 |
| 4861 | GAAUUCGG A UUGAUAGA | 4474 | TCTATCAA GGCTAGCTACAACGA CCGAATTC | 5760 |
| 4865 | UCGGAUUG A UAGAAAGG | 4475 | CCTTTCTA GGCTAGCTACAACGA CAATCCGA | 5761 |
| 4877 | AAAGGAAG A CUAACGUU | 4476 | AACGTTAG GGCTAGCTACAACGA CTTCCTTT | 5762 |
| 4881 | GAAGACUA A CGUUACCU | 4477 | AGGTAACG GGCTAGCTACAACGA TAGTCTTC | 5763 |
| 4883 | AGACUAAC G UUACCUUG | 4478 | CAAGGTAA GGCTAGCTACAACGA GTTAGTCT | 5764 |
| 4886 | CUAACGUU A CCUUGCUU | 4479 | AAGCAAGG GGCTAGCTACAACGA AACGTTAG | 5765 |
| 4891 | GUUACCUU G CUUUGGAG | 4480 | CTCCAAAG GGCTAGCTACAACGA AAGGTAAC | 5766 |
| 49.01 | UUUGGAGA G UACUGGAG | 4481 | CTCCAGTA GGCTAGCTACAACGA TCTCCAAA | 5767 |
| 4903 | UGGAGAGU A CUGGAGCC | 4482 | GGCTCCAG GGCTAGCTACAACGA ACTCTCCA | 5768 |
| 4909 | GUACUGGA G CCUGCAAA | 4483 | TTTGCAGG GGCTAGCTACAACGA TCCAGTAC | 5769 |
| 4913 | UGGAGCCU G CAAAUGCA | 4484 | TGCATTTG GGCTAGCTACAACGA AGGCTCCA | 5770 |
| 4917 | GCCUGCAA A UGCAUUGU | 4485 | ACAATGCA GGCTAGCTACAACGA TTGCAGGC | 5771 |
| 4919 | CUGCAAAU G CAUUGUGU | 4486 | ACACAATG GGCTAGCTACAACGA ATTTGCAG | 5772 |
| 4921 | GCAAAUGC A UUGUGUUU | 4487 | AAACACAA GGCTAGCTACAACGA GCATTTGC | 5773 |
| 4924 | AAUGCAUU G UGUUUGCU | 4488 | AGCAAACA GGCTAGCTACAACGA AATGCATT | 5774 |
| 4926 | UGCAUUGU G UUUGCUCU | 4489 | AGAGCAAA GGCTAGCTACAACGA ACAATGCA | 5775 |
| 4930 | UUGUGUUU G CUCUGGUG | 4490 | CACCAGAG GGCTAGCTACAACGA AAACACAA | 5776 |
| 4936 | UUGCUCUG G UGGAGGUG | 4491 | CACCTCCA GGCTAGCTACAACGA CAGAGCAA | 5777 |
| 4942 | UGGUGGAG G UGGGCAUG | 4492 | CATGCCCA GGCTAGCTACAACGA CTCCACCA | 5778 |
| 4946 | GGAGGUGG G CAUGGGGU | 4493 | ACCCCATG GGCTAGCTACAACGA CCACCTCC | 5779 |
| 4948 | AGGUGGGC A UGGGGUCU | 4494 | AGACCCCA GGCTAGCTACAACGA GCCCACCT | 5780 |
| 4953 | GGCAUGGG G UCUGUUCU | 4495 | AGAACAGA GGCTAGCTACAACGA CCCATGCC | 5781 |
| 4957 | UGGGGUCU G UUCUGAAA | 4496 | TTTCAGAA GGCTAGCTACAACGA AGACCCCA | 5782 |
| 4965 | GUUCUGAA A UGUAAAGG | 4497 | CCTTTACA GGCTAGCTACAACGA TTCAGAAC | 5783 |
| 4967 | UCUGAAAU G UAAAGGGU | 4498 | ACCCTTTA GGCTAGCTACAACGA ATTTCACA | 5784 |
| 4974 | UGUAAAGG G UUCAGACG | 4499 | CGTCTGAA GGCTAGCTACAACGA CCTTTACA | 5785 |
| 4980 | GGGUUCAG A CGGGGUUU | 4500 | AAACCCCG GGCTAGCTACAACGA CTGAACCC | 5786 |
| 4985 | CAGACGGG G UUUCUGGU | 4501 | ACCAGAAA GGCTAGCTACAACGA CCCGTCTG | 5787 |
| 4992 | GGUUUCUG G UUUUAGAA | 4502 | TTCTAAAA GGCTAGCTACAACGA CAGAAACC | 5788 |
| 5002 | UUUAGAAG G UUGCGUGU | 4503 | ACACGCAA GGCTAGCTACAACGA CTTCTAAA | 5789 |
| 5005 | AGAAGGUU G CGUGUUCU | 4504 | AGAACACG GGCTAGCTACAACGA AACCTTCT | 5790 |
| 5007 | AAGGUUGC G UGUUCUUC | 4505 | GAAGAACA GGCTAGCTACAACGA GCAACCTT | 5791 |
| 5009 | GGUUGCGU G UUCUUCGA | 4506 | TCGAAGAA GGCTAGCTACAACGA ACGCAACC | 5792 |
| 5018 | UUCUUCGA G UUGGGCUA | 4507 | TAGCCCAA GGCTAGCTACAACGA TCGAAGAA | 5793 |
| 5023 | CGAGUUGG G CUAAAGUA | 4508 | TACTTTAG GGCTAGCTACAACGA CCAACTCG | 5794 |
| 5029 | GGGCUAAA G UAGAGUUC | 4509 | GAACTCTA GGCTAGCTACAACGA TTTAGCCC | 5795 |
| 5034 | AAAGUAGA G UUCGUUGU | 4510 | ACAACGAA GGCTAGCTACAACGA TCTACTTT | 5796 |
| 5038 | UAGAGUUC G UUGUGCUG | 4511 | CAGCACAA GGCTAGCTACAACGA GAACTCTA | 5797 |
| 5041 | AGUUCGUU G UGCUGUUU | 4512 | AAACAGCA GGCTAGCTACAACGA AACGAACT | 5798 |
| 5043 | UUCGUUGU G CUGUUUCU | 4513 | AGAAACAG GGCTAGCTACAACGA ACAACGAA | 5799 |
| 5046 | GWGUGCU G UWCUGAC | 4514 | GTCAGAAA GGCTAGCTACAACGA AGCACAAC | 5800 |
| 5053 | UGUUUCUG A CUCCUAAU | 4515 | ATTAGGAG GGCTAGCTACAACGA CAGAAACA | 5801 |
| 5060 | GACUCCUA A UGAGAGW | 4516 | AACTCTCA GGCTAGCTACAACGA TAGGAGTC | 5802 |
| 5066 | UAAUGAGA G UUCCWCC | 4517 | GGAAGGAA GGCTAGCTACAACGA TCTCATTA | 5803 |
| 5077 | CCUUCCAG A CCGUUAGC | 4518 | GCTAACGG GGCTAGCTACAACGA CTGGAAGG | 5804 |
| 5080 | UCCAGACC G UUAGCUGU | 4519 | ACAGCTAA GGCTAGCTACAACGA GGTCTGGA | 5805 |
| 5084 | GACCGWA G CUGUCUCC | 4520 | GGAGACAG GGCTAGCTACAACGA TAACGGTC | 5806 |
| 5087 | CGUUAGCU G UCUCCUUG | 4521 | CAAGGAGA GGCTAGCTACAACGA AGCTAACG | 5807 |
| 5095 | GUCUCCW G CCAAGCCC | 4522 | GGGCTTGG GGCTAGCTACAACGA AAGGAGAC | 5808 |
| 5100 | CWGCCAA G CCCCAGGA | 4523 | TCCTGGGG GGCTAGCTACAACGA TTGGCAAG | 5809 |
| 5114 | GGAAGAAA A UGAUGCAG | 4524 | CTGCATCA GGCTAGCTACAACGA TTTCTTCC | 5810 |
| 5117 | AGAAAAUG A UGCAGCUC | 4525 | GAGCTGCA GGCTAGCTACAACGA CATTTTCT | 5811 |
| 5119 | AAAAUGAU G CAGCUCUG | 4526 | CAGAGCTG GGCTAGCTACAACGA ATCATTTT | 5812 |
| 5122 | AUGAUGCA G CUCUGGCU | 4527 | AGCCAGAG GGCTAGCTACAACGA TGCATCAT | 5813 |
| 5128 | CAGCUCUG G CUCCUUGU | 4528 | ACAAGGAG GGCTAGCTACAACGA CAGAGCTG | 5814 |
| 5135 | GGCUCCUU G UCUCCCAG | 4529 | CTGGGAGA GGCTAGCTACAACGA AAGGAGCC | 5815 |
| 5144 | UCUCCCAG G CUGAUCCU | 4530 | AGGATCAG GGCTAGCTACAACGA CTGGGAGA | 5816 |
| 5148 | CCAGGCUG A UCCUUUAU | 4531 | ATAAAGGA GGCTAGCTACAACGA CAGCCTGG | 5817 |
| 5155 | GAUCCULTU A UUCAGAAU | 4532 | ATTCTGAA GGCTAGCTACAACGA AAAGGATC | 5818 |
| 5162 | UAWCAGA A UACCACAA | 4533 | TTGTGGTA GGCTAGCTACAACGA TCTGAATA | 5819 |
| 5164 | UUCAGAAU A CCACAAAG | 4534 | CTTTGTGG GGCTAGCTACAACGA ATTCTGAA | 5820 |
| 5167 | AGAAUACC A CAAAGAAA | 4535 | TTTCTTTG GGCTAGCTACAACGA GGTATTCT | 5821 |
| 5178 | AAGAAAGG A CAUUCAGC | 4536 | GCTGAATG GGCTAGCTACAACGA CCTTTCTT | 5822 |
| 5180 | GAAAGGAC A UUCAGCUC | 4537 | GAGCTGAA GGCTAGCTACAACGA GTCCTTTC | 5823 |
| 5185 | GACAUUCA G CUCAAGGC | 4538 | GCCTTGAG GGCTAGCTACAACGA TGAATGTC | 5824 |
| 5192 | AGCUCAAG G CUCCCUGC | 4539 | GCAGGGAG GGCTAGCTACAACGA CTTGAGCT | 5825 |
| 5199 | GGCUCCCU G CCGUGUUG | 4540 | CAACACGG GGCTAGCTACAACGA AGGGAGCC | 5826 |
| 5202 | UCCCUGCC G UGWGAAG | 4541 | CTTCAACA GGCTAGCTACAACGA GGCAGGGA | 5827 |
| 5204 | CCUGCCGU G UUGAAGAG | 4542 | CTCTTCAA GGCTAGCTACAACGA ACGGCAGG | 5828 |
| 5212 | GUUGAAGA G UUCUGACU | 4543 | AGTCAGAA GGCTAGCTACAACGA TCTTCAAC | 5829 |
| 5218 | GAGWCUG A CUGCACAA | 4544 | TTGTGCAG GGCTAGCTACAACGA CAGAACTC | 5830 |
| 5221 | UUCUGACU G CACAAACC | 4545 | GGTTTGTG GGCTAGCTACAACGA AGTCAGAA | 5831 |
| 5223 | CUGACUGC A CAAACCAG | 4546 | CTGGTTTG GGCTAGCTACAACGA GCAGTCAG | 5832 |
| 5227 | CUGCACAA A CCAGCWC | 4547 | GAAGCTGG GGCTAGCTACAACGA TTGTGCAG | 5833 |
| 5231 | ACAAACCA G CUUCUGGU | 4548 | ACCAGAAG GGCTAGCTACAACGA TGGTTTGT | 5834 |
| 5236 | AGCUUCUG G UUUCUUCU | 4549 | AGAAGAAA GGCTAGCTACAACGA CAGAAGCT | 5835 |
| 5250 | CUUCUGGA A UGAAUACC | 4550 | GGTATTCA GGCTAGCTACAACGA TCCAGAAG | 5836 |
| 5254 | UGGAAUGA A UACCCUCA | 4551 | TGAGGGTA GGCTAGCTACAACGA TCATTCCA | 5837 |
| 5256 | GAAUGAAU A CCCUCAUA | 4552 | TATGAGGG GGCTAGCTACAACGA ATTCATTC | 5838 |
| 5262 | AUACCCUC A UAUCUGUC | 4553 | GACAGATA GGCTAGCTACAACGA GAGGGTAT | 5839 |
| 5264 | ACCCUCAU A STCUGUCCU | 4554 | AGGACAGA GGCTAGCTACAACGA ATGAGGGT | 5840 |
| 5Z68 | UCAUAUCU G UCCUGAUG | 4555 | CATCAGGA GGCTAGCTACAACGA AGATATGA | 5841 |
| 5274 | CUGUCCUG A UGUGAUAU | 4556 | ATATCACA GGCTAGCTACAACGA CAGGACAG | 5842 |
| 5276 | GUCCUGAU G UGAUAUGU | 4557 | ACATATCA GGCTAGCTACAACGA ATCAGGAC | 5843 |
| 5279 | CUGAUGUG A UAUGUCUG | 4558 | CAGACATA GGCTAGCTACAACGA CACATCAG | 5844 |
| 5281 | GAUGUGAU A UGUCUGAG | 4559 | CTCAGACA GGCTAGCTACAACGA ATCACATC | 5845 |
| 5283 | UGUGAUAU G UCUGAGAC | 4560 | GTCTCAGA GGCTAGCTACAACGA ATATCACA | 5846 |
| 5290 | UGUCUGAG A CUGAAUGC | 4561 | GCATTCAG GGCTAGCTACAACGA CTCAGACA | 5847 |
| 5295 | GAGACUGA A UGCGGGAG | 4562 | CTCCCGCA GGCTAGCTACAACGA TCAGTCTC | 5848 |
| 5297 | GACUGAAU G CGGGAGGU | 4563 | ACCTCCCG GGCTAGCTACAACGA ATTCAGTC | 5849 |
| 5304 | UGCGGGAG G UUCAAUGU | 4564 | ACATTGAA GGCTAGCTACAACGA CTCCCGCA | 5850 |
| 5309 | GAGGWCA A UGUGAAGC | 4565 | GCTTCACA GGCTAGCTACAACGA TGAACCTC | 5851 |
| 5311 | GGWCAAU G UGAAGCUG | 4566 | CAGCTTCA GGCTAGCTACAACGA ATTGAACC | 5852 |
| 5316 | AAUGUGAA G CUGUGUGU | 4567 | ACACACAG GGCTAGCTACAACGA TTCACATT | 5853 |
| 5319 | GUGAAGCU G UGUGUGGU | 4568 | ACCACACA GGCTAGCTACAACGA AGCTTCAC | 5854 |
| 5321 | GAAGCUGU G UGUGGUGU | 4569 | ACACCACA GGCTAGCTACAACGA ACAGCTTC | 5855 |
| 5323 | AGCUGUGU G UGGUGUCA | 4570 | TGACACCA GGCTAGCTACAACGA ACACAGCT | 5856 |
| 5326 | UGUGUGUG G UGUCAAAG | 4571 | CTTTGACA GGCTAGCTACAACGA CACACACA | 5857 |
| 5328 | UGUGUGGU G UCAAAGUU | 4572 | AACTTTGA GGCTAGCTACAACGA ACCACACA | 5858 |
| 5334 | GUGUCAAA G UUUCAGGA | 4573 | TCCTGAAA GGCTAGCTACAACGA TTTGACAC | 5859 |
| 5346 | CAGGAAGG A UUUUACCC | 4574 | GGGTAAAA GGCTAGCTACAACGA CCTTCCTG | 5860 |
| 5351 | AGGAUUUU A CCCUUUUG | 4575 | CAAAAGGG GGCTAGCTACAACGA AAAATCCT | 5861 |
| 5359 | ACCCUUUU G UUCUUCCC | 4576 | GGGAAGAA GGCTAGCTACAACGA AAAAGGGT | 5862 |
| 5371 | UUCCCCCU G UCCCCAAC | 4577 | GTTGGGGA GGCTAGCTACAACGA AGGGGGAA | 5863 |
| 5378 | UGUCCCCA A CCCACUCU | 4578 | AGAGTGGG GGCTAGCTACAACGA TGGGGACA | 5864 |
| 5382 | CCCAACCC A CUCUCACC | 4579 | GGTGAGAG GGCTAGCTACAACGA GGGTTGGG | 5865 |
| 5388 | CCACUCUC A CCCCGCAA | 4580 | TTGCGGGG GGCTAGCTACAACGA GAGAGTGG | 5866 |
| 5393 | CUCACCCC G CAACCCAU | 4581 | ATGGGTTG GGCTAGCTACAACGA GGGGTGAG | 5867 |
| 5396 | ACCCCGCA A CCCAUCAG | 4582 | CTGATGGG GGCTAGCTACAACGA TGCGGGGT | 5868 |
| 5400 | CGCAACCC A UCAGUAUU | 4583 | AATACTGA GGCTAGCTACAACGA GGGTTGCG | 5869 |
| 5404 | ACCCAUCA G UAUUUUAG | 4584 | CTAAAATA GGCTAGCTACAACGA TGATGGGT | 5870 |
| 5406 | CCAUCAGU A UUUUAGUU | 4585 | AACTAAAA GGCTAGCTACAACGA ACTGATGG | 5871 |
| 5412 | GUAUUUUA G UUAUUUGG | 4586 | CCAAATAA GGCTAGCTACAACGA TAAAATAC | 5872 |
| 5415 | UUUUAGUU A UUUGGCCU | 4587 | AGGCCAAA GGCTAGCTACAACGA AACTAAAA | 5873 |
| 5420 | GUUAUUUG G CCUCUACU | 4588 | AGTAGAGG GGCTAGCTACAACGA CAAATAAC | 5874 |
| 5426 | UGGCCUCU A CUCCAGUA | 4589 | TACTGGAG GGCTAGCTACAACGA AGAGGCCA | 5875 |
| 5432 | CUACUCCA G UAAACCUG | 4590 | CAGGTTTA GGCTAGCTACAACGA TGGAGTAG | 5876 |
| 5436 | UCCAGUAA A CCUGAUUG | 4591 | CAATCAGG GGCTAGCTACAACGA TTACTGGA | 5877 |
| 5441 | UAAACCUG A UUGGGUUU | 4592 | AAACCCAA GGCTAGCTACAACGA CAGGTTTA | 5878 |
| 5446 | CUGAUUGG G UUUGUUCA | 4593 | TGAACAAA GGCTAGCTACAACGA CCAATCAG | 5879 |
| 5450 | UUGGGUUU G UUCACUCU | 4594 | AGAGTGAA GGCTAGCTACAACGA AAACCCAA | 5880 |
| 5454 | GUUUGUUC A CUCUCUGA | 4595 | TCAGAGAG GGCTAGCTACAACGA GAACAAAC | 5881 |
| 5463 | CUCUCUGA A UGAUUAUU | 4596 | AATAATCA GGCTAGCTACAACGA TCAGAGAG | 5882 |
| 5466 | UCUGAAUG A UUAUUAGC | 4597 | GCTAATAA GGCTAGCTACAACGA CATTCAGA | 5883 |
| 5469 | GAAUGAUU A UUAGCCAG | 4598 | CTGGCTAA GGCTAGCTACAACGA AATCATTC | 5884 |
| 5473 | GAUUAUUA G CCAGACUU | 4599 | AAGTCTGG GGCTAGCTACAACGA TAATAATC | 5865 |
| 5478 | UUAGCCAG A CUUCAAAA | 4600 | TTTTGAAG GGCTAGCTACAACGA CTGGCTAA | 5886 |
| 5486 | ACUUCAAA A UUAUUUUA | 4601 | TAAAATAA GGCTAGCTACAACGA TTTGAAGT | 5887 |
| 5489 | UCAAAAUU A UUUUAUAG | 4602 | CTATAAAA GGCTAGCTACAACGA AATTTTGA | 5888 |
| 5494 | AUUAUUUU A UAGCCCAA | 4603 | TTGGGCTA GGCTAGCTACAACGA AAAATAAT | 5889 |
| 5497 | AUUUUAUA G CCCAAAUU | 4604 | AATTTGGG GGCTAGCTACAACGA TATAAAAT | 5890 |
| 5503 | UAGCCCAA A UUAUAACA | 4605 | TGTTATAA GGCTAGCTACAACGA TTGGGCTA | 5891 |
| 5506 | CCCAAAUU A UAACAUCU | 4606 | AGATGTTA GGCTAGCTACAACGA AATTTGGG | 5892 |
| 5509 | AAAUUAUA A CAUCUAUU | 4607 | AATAGATG GCCTAGCTACAACGA TATAATTT | 5893 |
| 5511 | AUUAUAAC A UCUAUUGU | 4608 | ACAATAGA GGCTAGCTACAACGA GTTATAAT | 5894 |
| 5515 | UAACAUCU A UUGUAUUA | 4609 | TAATACAA GGCTAGCTACAACGA AGATGTTA | 5895 |
| 5518 | CAUCUAUU G UAUUAUUU | 4610 | AAATAATA GGCTAGCTACAACGA AATAGATG | 5896 |
| 5520 | UCUAUUGU A UUAUUUAG | 4611 | CTAAATAA GGCTAGCTACAACGA ACAATAGA | 5897 |
| 5523 | AUUGUAUU A UUUAGACU | 4612 | AGTCTAAA GGCTAGCTACAACGA AATACAAT | 5898 |
| 5529 | UUAUUUAG A CUUUUAAC | 4613 | GTTAAAAG GGCTAGCTACAACGA CTAAATAA | 5899 |
| 5536 | GACUUUUA A CAUAUAGA | 4614 | TCTATATG GGCTAGCTACAACGA TAAAAGTC | 5900 |
| 5538 | CUUUUAAC A UAUAGAGC | 4615 | GCTCTATA GGCTAGCTACAACGA GTTAAAAG | 5901 |
| 5540 | UUUAACAU A UAGAGCUA | 4616 | TAGCTCTA GGCTAGCTACAACGA ATGTTAAA | 5902 |
| 5545 | CAUAUAGA G CUAUUUCU | 4617 | AGAAATAG GGCTAGCTACAACGA TCTATATG | 5903 |
| 5548 | AUAGAGCU A UUUCUACU | 4618 | AGTAGAAA GGCTAGCTACAACGA AGCTCTAT | 5904 |
| 5554 | CUAUUUCU A CUGAUUUU | 4619 | AAAATCAG GGCTAGCTACAACGA AGAAATAG | 5905 |
| 5558 | UUCUACUG A UUUUUGCC | 4620 | GGCAAAAA GGCTAGCTACAACGA CAGTAGAA | 5906 |
| 5564 | UGAUUUUU G CCCUUGUU | 4621 | AACAAGGG GGCTAGCTACAACGA AAAAATCA | 5907 |
| 5570 | UUGGCCUU G UUCUGUCC | 4622 | GGACAGAA GGCTAGCTACAACGA AAGGGCAA | 5908 |
| 5575 | CUUGUUCU G UCCUUUUU | 4623 | AAAAAGGA GGCTAGCTACAACGA AGAACAAG | 5909 |
| 5597 | AAAAGAAA A UGUGUUUU | 4624 | AAAACACA GGCTAGCTACAACGA TTTCTTTT | 5910 |
| 5599 | AAGAAAAU G UGUUUUUU | 4625 | AAAAAACA GGCTAGCTACAACGA ATTTTCTT | 5911 |
| 5601 | GAAAAUGU G UUUUUUGU | 4626 | ACAAAAAA GGCTAGCTACAACGA ACATTTTC | 5912 |
| 5608 | UGUUUUUU G UUUGGUAC | 4627 | GTACCAAA GGCTAGCTACAACGA AAAAAACA | 5913 |
| 5613 | UUUGUUUG G UACCAUAG | 4628 | CTATGGTA GGCTAGCTACAACGA CAAACAAA | 5914 |
| 5615 | UGUUUGGU A CCAUAGUG | 4629 | CACTATGG GGCTAGCTACAACGA ACCAAACA | 5915 |
| 5618 | UUGGUACC A UAGUGUGA | 4630 | TCACACTA GGCTAGCTACAACGA GGTACCAA | 5916 |
| 5621 | GUACCAUA G UGUGAAAU | 4631 | ATTTCACA GGCTAGCTACAACGA TATGGTAC | 5917 |
| 5623 | ACCAUAGU G UGAAAUGC | 4632 | GCATTTCA GGCTAGCTACAACGA ACTATGGT | 5918 |
| 5628 | AGUGUGAA A UGCUGGGA | 4633 | TCCCAGCA GGCTAGCTACAACGA TTCACACT | 5919 |
| 5630 | UGUGAAAU G CUGGGAAC | 4634 | GTTCCCAG GGCTAGCTACAACGA ATTTCACA | 5920 |
| 5637 | UGCUGGGA A CAAUGACU | 4635 | AGTCATTG GGCTAGCTACAACGA TCCCAGCA | 5921 |
| 5640 | UGGGAACA A UGACUAUA | 4636 | TATAGTCA GGCTAGCTACAACGA TGTTCCCA | 5922 |
| 5643 | GAACAAUG A CUAUAAGA | 4637 | TCTTATAG GGCTAGCTACAACGA CATTGTTC | 5923 |
| 5646 | CAAUGACU A UAAGACAU | 4638 | ATGTCTTA GGCTAGCTACAACGA AGTCATTG | 5924 |
| 5651 | ACUAUAAG A CAUGCUAU | 4639 | ATAGCATG GGCTAGCTACAACGA CTTATAGT | 5925 |
| 5653 | UAUAAGAC A UGCUAUGG | 4640 | CCATAGCA GGCTAGCTACAACGA GTCTTATA | 5926 |
| 5655 | UAAGACAU G CUAUGGCA | 4641 | TGCCATAG GGCTAGCTACAACGA ATGTCTTA | 5927 |
| 5658 | GACAUGCU A UGGCACAU | 4642 | ATGTGCCA GGCTAGCTACAACGA AGGATGTC | 5928 |
| 5661 | AUGCUAUG G CACAUAUA | 4643 | TATATGTG GGGTAGCTACAACGA CATAGCAT | 5929 |
| 5663 | GCUAUGGC A CAUAUAUU | 4644 | AATATATG GGCTAGCTACAACGA GCCATAGC | 5930 |
| 5665 | UAUGGCAC A UAUAUUUA | 4645 | TAAATATA GGCTAGCTACAACGA GTGCCATA | 5931 |
| 5667 | UGGCACAU A UAUUUAUA | 4646 | TATAAATA GGCTAGCTACAACGA ATGTGCCA | 5932 |
| 5669 | GCACAUAU A UUUAUAGU | 4647 | ACTATAAA GGCTAGCTACAACGA ATATGTGC | 5933 |
| 5673 | AUAUAUUU A UAGUCUGU | 4648 | ACAGACTA GGCTAGCTACAACGA AAATATAT | 5934 |
| 5676 | UAUUUAUA G UCUGUUUA | 4649 | TAAACAGA GGCTAGCTACAACGA TATAAATA | 5935 |
| 5680 | UAUAGUCU G UUUAUGUA | 4650 | TACATAAA GGCTAGCTACAACGA AGACTATA | 5936 |
| 5684 | GUCUGUUU A UGUAGAAA | 4651 | TTTCTACA GGCTAGCTACAACGA AAACAGAC | 5937 |
| 5686 | CUGUUUAU G UAGAAACA | 4652 | TGTTTCTA GGCTAGCTACAACGA ATAAACAG | 5938 |
| 5692 | AUGUAGAA A CAAAUGUA | 4653 | TACATTTG GGCTAGCTACAACGA TTCTACAT | 5939 |
| 5696 | AGAAACAA A UGUAAUAU | 4654 | ATATTACA GGCTAGCTACAACGA TTGTTTCT | 5940 |
| 5698 | AAACAAAU G UAAUAUAU | 4655 | ATATATTA GGCTAGCTACAACGA ATTTGTTT | 5941 |
| 5701 | CAAAUGUA A UAUAUUAA | 4656 | TTAATATA GGCTAGCTACAACGA TACATTTG | 5942 |
| 5703 | AAUGUAAU A UAUUAAAG | 4657 | CTTTAATA GGCTAGCTACAACGA ATTACATT | 5943 |
| 5705 | UGUAAUAU A UUAAAGCC | 4658 | GGCTTTAA GGCTAGCTACAACGA ATATTACA | 5944 |
| 5711 | AUAUUAAA G CCUUAUAU | 4659 | ATATAAGG GGCTAGCTACAACGA TTTAATAT | 5945 |
| 5716 | AAAGCCUU A UAUAUAAU | 4660 | ATTATATA GGCTAGCTACAACGA AAGGCTTT | 5946 |
| 5718 | AGCCUUAU A UAUAAUGA | 4661 | TCATTATA GGCTAGCTACAACGA ATAAGGCT | 5947 |
| 5720 | CCUUAUAU A UAAUGAAC | 4662 | GTTCATTA GGCTAGCTACAACGA ATATAAGG | 5948 |
| 5723 | UAUAUAUA A UGAACUUU | 4663 | AAAGTTCA GGCTAGCTACAACGA TATATATA | 5949 |
| 5727 | UAUAAUGA A CUUUGUAC | 4664 | GTACAAAG GGCTAGCTACAACGA TCATTATA | 5950 |
| 5732 | UGAACUUU G UACUAUUC | 4665 | GAATAGTA GGCTAGCTACAACGA AAAGTTCA | 5951 |
| 5734 | AACUUUGU A CUAUUCAC | 4666 | GTGAATAG GGCTAGCTACAACGA ACAAAGTT | 5952 |
| 5737 | UUUGUACU A UUCACAUU | 4667 | AATGTGAA GGCTAGCTACAACGA AGTACAAA | 5953 |
| 5741 | UACUAUUC A CAUUUUGU | 4668 | ACAAAATG GGCTAGCTACAACGA GAATAGTA | 5954 |
| 5743 | CUAUUCAC A UUUUGUAU | 4669 | ATACAAAA GGCTAGCTACAACGA GTGAATAG | 5955 |
| 5748 | CACAUUUU G UAUCAGUA | 4670 | TACTGATA GGCTAGCTACAACGA AAAATGTG | 5956 |
| 5750 | CAUUUUGU A UCAGUAUU | 4671 | AATACTGA GGCTAGCTACAACGA ACAAAATG | 5957 |
| 5754 | UUGUAUCA G UAUUAUGU | 4672 | ACATAATA GGCTAGCTACAACGA TGATACAA | 5958 |
| 5756 | GUAUCAGU A UUAUGUAG | 4673 | CTACATAA GGCTAGCTACAACGA ACTGATAC | 5959 |
| 5759 | UCAGUAUU A UGUAGCAU | 4674 | ATGCTACA GGCTAGCTACAACGA AATACTGA | 5960 |
| 5761 | AGUAUUAU G UAGCAUAA | 4675 | TTATGCTA GGCTAGCTACAACGA ATAATACT | 5961 |
| 5764 | AUUAUGUA G CAUAACAA | 4676 | TTGTTATG GGCTAGCTACAACGA TACATAAT | 5962 |
| 5766 | UAUGUAGC A UAACAAAG | 4677 | CTTTGTTA GGCTAGCTACAACGA GCTACATA | 5963 |
| 5769 | GUAGCAUA A CAAAGGUC | 4678 | GACCTTTG GGCTAGCTACAACGA TATGCTAC | 5964 |
| 5775 | UAACAAAG G UCAUAAUG | 4679 | CATTATGA GGCTAGCTACAACGA CTTTGTTA | 5965 |
| 5778 | CAAAGGUC A UAAUGCUU | 4680 | AAGCATTA GGCTAGCTACAACGA GACCTTTG | 5966 |
| 5781 | AGGUCAUA A UGCUUUCA | 4681 | TGAAAGCA GGCTAGCTACAACGA TATGACCT | 5967 |
| 5783 | GUCAUAAU G CUUUCAGC | 4682 | GCTGAAAG GGCTAGCTACAACGA ATTATGAC | 5968 |
| 5790 | UGCUUUCA G CAAUUGAU | 4683 | ATCAATTG GGCTAGCTACAACGA TGAAAGCA | 5969 |
| 5793 | UUUCAGCA A UUGAUGUC | 4684 | GACATCAA GGCTAGCTACAACGA TGCTGAAA | 5970 |
| 5797 | AGCAAUUG A UGUCAUUU | 4685 | AAATGACA GGCTAGCTACAACGA CAATTGCT | 5971 |
| 5799 | CAAUUGAU G UCAUUUUA | 4686 | TAAAATGA GGCTAGCTACAACGA ATCAATTG | 5972 |
| 5802 | UUGAUGUC A UUUUAUUA | 4687 | TAATAAAA GGCTAGCTACAACGA GACATCAA | 5973 |
| 5807 | GUCAUUUU A UUAAAGAA | 4688 | TTCTTTAA GGCTAGCTACAACGA AAAATGAC | 5974 |
| 5815 | AUUAAAGA A CAUUGAAA | 4689 | TTTCAATG GGCTAGCTACAACGA TCTTTAAT | 5975 |
| 5817 | UAAAGAAC A UUGAAAAA | 4690 | TTTTTCAA GGCTAGCTACAACGA GTTCTTTA | 5976 |

| | | | | |
|---|---|---|---|---|
| Input Sequence = AF035121. Cut Site = R/Y Arm Length = 8. Core Sequence = GGCTAGCTACAACGA AF035121 (Homo sapiens KDR/flk-1 protein mRNA, complete eds.; Acc# AF035121; 5830 bp) | | | | |

### PREFERRED EMBODIMENTS OF THE INVENTION

1. A compound having Formula II: (SEQ ID NO: 5978)

   **5'-uₛaₛcₛ aₛau ucU GAu Gag gcg aaa gcc Gaa Aag aca aB-3'**

   wherein each a is2'-O-methyl adenosine nucleotide, each g is a 2'-O methyl guanosine nucleotide, each c is a 2'-O-methyl cytidine nucleotide, each **u** is a 2'-O-methyl uridine nucleotide, each **A** is adenosine, each **G** is guanosine, each s individually represents a phosphorothioate internucleotide linkage, **U** is 2'-deoxy-2'-C-allyl uridine, and **B** is an inverted deoxyabasic moiety.
2. A composition comprising the compound of embodiment 1 and a pharmaceutically acceptable carrier or diluent.
3. A method of administering to a cell the compound of embodiment 1 comprising contacting said cell with the compound under conditions suitable for said administration.
4. The method of embodiment 3, wherein said cell is a mammalian cell.
5. The method of embodiment 3, wherein said cell is a human cell.
6. The method of embodiment 3, wherein said administration is in the presence of a delivery reagent.
7. The method of embodiment 6, wherein said delivery reagent is a lipid.
8. The method of embodiment 7, wherein said lipid is a cationic lipid.
9. The method of embodiment 7, wherein said lipid is a phospholipid.
10. The method of embodiment 6, wherein said delivery reagent is a liposome.
11. A method of administering to a cell the compound of embodiment 1 in conjunction with one or more other drug comprising contacting said cell with the compound and the other drug (s) under conditions suitable for said administration.
12. A method of inhibiting ocular angiogenesis in a subject comprising the step of contacting said subject with the compound of embodiment 1 under conditions suitable for said inhibition.
13. The method of embodiment 12, wherein said angiogenesis is associated with diabetic retinopathy.
14. The method of embodiment 12, wherein said angiogenesis is associated with age related diabetic retinopathy.
15. A method of cleaving RNA comprising a sequence of KDR RNA comprising contacting the compound of embodiment 1 with said RNA under conditions suitable for the cleavage of said RNA.
16. The method of embodiment 15, wherein said cleavage is carried out in the presence of a divalent cation.
17. The method of embodiment 16, wherein said divalent cation is Mg2+.
18. A method of administering to a mammal the compound of embodiment 1 comprising contacting said mammal with the compound under conditions suitable for said administration.
19. The method of embodiment 18, wherein said mammal is a human.
20. The method of embodiment 18 wherein said administration is in the presence of a delivery reagent.
21. The method of embodiment 18, wherein said delivery reagent is a lipid.
22. The method of embodiment 21, wherein said lipid is a cationic lipid.
23. The method of embodiment 21, wherein said lipid is a phospholipid.
24. The method of embodiment 20, wherein said delivery reagent is a liposome.
25. A method for treating a subject having endometriosis, comprising contacting said subject with a nucleic acid molecule that modulates the expression of VEGF, VEGFR1, and/or VEGFR2, under conditions suitable for said treatment.
26. The method of embodiment 25, wherein said nucleic acid molecule is an enzymatic nucleic acid molecule.
27. The method of embodiment 25, wherein said nucleic acid molecule is an antisense nucleic acid molecule.
28. The method of embodiment 25, wherein said nucleic acid molecule is a dsRNA nucleic acid molecule.
29. The method of embodiment 25, wherein said nucleic acid molecule is a nucleic acid aptamer.
30. The method of embodiment 25, wherein said nucleic acid molecule comprises a sequence having SEQ ID NO: 5977.
31. The method of embodiment 26, wherein said enzymatic nucleic acid molecule has an endonuclease activity to cleave RNA encoded by an VEGFR1and/or VEGFR2 gene.
32. The method of embodiment 26, wherein said enzymatic nucleic acid molecule is in a hammerhead configuration.
33. The method of embodiment 26, wherein said enzymatic nucleic acid molecule is in an Inozyme configuration.
34. The method of embodiment 26, wherein said enzymatic nucleic acid molecule is in a Zinzyme configuration.
35. The method of embodiment 26, wherein said enzymatic nucleic acid molecule is in a DNAzyme configuration.
36. The method of embodiment 26, wherein said enzymatic nucleic acid molecule is in a G-cleaver configuration.
37. The method of embodiment 26, wherein said enzymatic nucleic acid molecule is in an Amberzyme configuration.
38. The method of embodiment 26, wherein said enzymatic nucleic acid molecule is an allozyme.
39. The method of embodiment 25, wherein said nucleic acid molecule is chemically synthesized.
40. The method of embodiment 25, wherein said nucleic acid molecule comprises at least one 2'-sugar modification.
41. The method of embodiment 25, wherein said nucleic acid molecule comprises at least one nucleic acid base modification.
42. The method of embodiment 25, wherein said nucleic acid molecule comprises at least one phosphate backbone modification.
43. The method of embodiment 25, wherein said subject is a human.
44. A method for treating a subject having endometriosis, comprising administering to the subject a nucleic acid molecule that modulates the expression of VEGF, VEGFR1, and/or VEGFR2, under conditions suitable for said treatment.
45. The method of embodiment 44 wherein said administration is in the presence of a delivery reagent.
46. The method of embodiment 45, wherein said delivery reagent is a lipid.
47. The method of embodiment 46, wherein said lipid is a cationic lipid.
48. The method of embodiment 46, wherein said lipid is a phospholipid.
49. The method of embodiment 45, wherein said delivery reagent is a liposome.
50. The method of embodiment 44, further comprising administering one or more other drug(s).
51. The method of embodiment 50, wherein said other drug(s) are chosen fromGnRH (gonadotropin releasing hormone) agonists, Lupron Depot (Leuprolide Acetate), Synarel (naferalin acetate), Zolodex (goserelin acetate), Suprefact (buserelin acetate), Danazol, and oral contraceptives.
52. A compound havingFormula I : (SEQ ID NO: 5977)

   **5'gₛaₛgₛuₛugcUGAuGagg ccgaaa ggccGaaAgucugB 3'**

   wherein each **a** is2'-O-methyl adenosine nucleotide, each g is a 2'-O methyl guanosine nucleotide, each **c** is a 2'-O-methyl cytidine nucleotide, each **u** is a 2'-O-methyl uridine nucleotide, each **A** is adenosine, each **G** is guanosine, each **s** individually represents a phosphorothioate intemucleotide linkage, **U** is 2'-deoxy-2'-C-allyl uridine, and B is an inverted deoxyabasic moiety.
53. A composition comprising a compound of embodiment 52 in a pharmaceutically acceptable carrier or diluent.
54. A method of administering to a cell the compound of embodiment 52 comprising contacting said cell with the compound under conditions suitable for said administration.
55. The method of embodiment 54, wherein said cell is a mammalian cell.
56. The method of embodiment 54, wherein said cell is a human cell.
57. The method of embodiment 54, wherein said administration is in the presence of a delivery reagent.
58. The method of embodiment 57, wherein said delivery reagent is a lipid.
59. The method of embodiment 58, wherein said lipid is a cationic lipid.
60. The method of embodiment 58, wherein said lipid is a phospholipid.
61. The method of embodiment 57, wherein said delivery reagent is a liposome.
62. A method of administering to a cell the compound of embodiment 52 in conjunction with a chemotherapeutic agent comprising contacting said cell with the compound and the chemotherapeutic agent under conditions suitable for said administration.
63. The method of embodiment 62, wherein said chemotherapeutic agent is 5-fluoro uridine.
64. The method of embodiment 62, wherein said chemotherapeutic agent is Leucovorin.
65. The method of embodiment 62, wherein said chemotherapeutic agent is chosen from Irinotecan, CAMPTOSAR®, CPT-11, Camptothecin-11, or Campto.
66. The method of embodiment 62, wherein said chemotherapeutic agent is Paclitaxel.
67. The method of embodiment 62, wherein said chemotherapeutic agent is Carboplatin.
68. A mammalian cell comprising the compound of embodiment 52..
69. The mammalian cell of embodiment 68, wherein said mammalian cell is a human cell.
70. A method of inhibiting angiogenesis in a subject, comprising the step of contacting said subject with the compound of embodiment 52, under conditions suitable for said inhibition.
71. The method of embodiment 70, wherein said angiogenesis is tumor angiogenesis.
72. A method of treatment of a subject having a condition associated with an increased level of VEGF receptor comprising contacting cells of said subject with the compound of embodiment 52, under conditions suitable for said treatment.
73. The method of embodiment 72 further comprising the use of one or more drug therapies under conditions suitable for said treatment.
74. A method of cleaving RNA comprising a sequence of VEGFR1 (flt-1), comprising contacting the compound of embodiment 52 with said RNA under conditions suitable for the cleavage of said RNA.
75. The method of embodiment 74, wherein said cleavage is carried out in the presence of a divalent cation.
76. The method of embodiment 75, wherein said divalent cation is Mg2+.
77. The method of embodiment 72, wherein said condition is cancer.
78. The method of embodiment 77, wherein said cancer is breast cancer.
79. The method of embodiment 77, wherein said cancer is lung cancer.
80. The method of embodiment 77, wherein said cancer is colorectal cancer.
81. The method of embodiment 77, wherein said cancer is renal cancer.
82. The method of embodiment 77, wherein said cancer is melanoma.
83. The method of embodiment 77, wherein said cancer is pancreatic cancer.
84. The method of embodiment 79, wherein said lung cancer is non-small cell lung carcinoma.
85. The method of embodiment 81, wherein said renal cancer is renal cell carcinoma.
86. The method of embodiment 73, wherein said other therapy is 5-fluoro uridine.
87. The method of embodiment 73, wherein said other therapy is Leucovorin.
88. The method of embodiment 73, wherein said other therapy is Irinotecan, CAMPTOSAR®, CPT-11, Camptothecin-11, or Campto.
89. The method of embodiment 73, wherein said other therapy is Paclitaxel.
90. The method of embodiment 73, wherein said other therapy is Carboplatin.
91. A method of administering to a mammal the compound of embodiment 52 comprising contacting said mammal with the compound under conditions suitable for said administration.
92. The method of embodiment 91, wherein said mammal is a human.
93. The method of embodiment 91, wherein said administration is in the presence of a delivery reagent.
94. The method of embodiment 93, wherein said delivery reagent is a lipid.
95. The method of embodiment 94, wherein said lipid is a cationic lipid.
96. The method of embodiment 94, wherein said lipid is a phospholipid.
97. The method of embodiment 93, wherein said delivery reagent is a liposome.
98. A method of administering to a mammal the compound of embodiment 52 in conjunction with a chemotherapeutic agent comprising contacting said mammal with the compound and the chemotherapeutic agent under conditions suitable for said administration.
99. The method of embodiment 98, wherein said chemotherapeutic agent is 5-fluoro uridine.
100. The method of embodiment 98, wherein said chemotherapeutic agent is Leucovorin.
101. The method of embodiment 98, wherein said chemotherapeutic agent is Irinotecan, CAMPTOSAR®, CPT-11, Camptothecin-11, or Campto.
102. The method of embodiment 98, wherein said chemotherapeutic agent is Paclitaxel.
103. The method of embodiment 98, wherein said chemotherapeutic agent is Carboplatin.

## Claims

1. A method of locally administering to a tissue or cell a synthetic double stranded RNA comprising nucleotide sequence that is complementary to nucleotide sequence of a Vascular Endothelial Growth Factor receptor (VEGF receptor) encoding RNA, comprising contacting said tissue or cell with said double stranded RNA under conditions suitable for local administration.

2. The method of claim 1, wherein said tissue is ocular tissue.

3. The method of claim 1, wherein said cell is an ocular cell.

4. The method of claim 2, wherein said ocular tissue is retinal tissue.

5. The method of claim 3, wherein said ocular cell is a retinal cell.

6. The method of claim 1, wherein said double stranded RNA is administered to said tissue or cell via injection.

7. The method of claim 6, wherein said injection comprises intraocular injection.

8. The method of claim 1, wherein said Vascular Endothelial Growth Factor receptor (VEGF receptor) is Vascular Endothelial Growth Factor receptor 1 (VEGFR1).

9. The method of claim 1, wherein said double stranded RNA is chemically synthesized.

10. The method of claim 1, wherein said double stranded RNA comprises at least one nucleic acid sugar modification.

11. The method of claim 10, wherein said sugar modification comprises a 2'-deoxy-2'-fluoro modification.

12. The method of claim 10, wherein said sugar modification comprises a 2'-deoxy modification.

13. The method of claim 10, wherein said sugar modification comprises a 2'-O-alkl modification.

14. The method of claim 13, wherein said 2'-O-alkyl modification is 2'-O-methyl.

15. The method of claim 13, wherein said 2'-O-alkyl modification is 2'-O-allyl.

16. The method of claim 1, wherein said double stranded RNA comprises at least one nucleic acid base modification.

17. The method of claim 1, wherein said double stranded RNA comprises at least one nucleic acid backbone modification.

18. The method of claim 17, wherein said backbone modification comprises a phosphorothioate internucleotide linkage.

19. The method of claim 1, wherein said double stranded RNA comprises at least one non-nucleotide.

20. The method of claim 19, wherein said non-nucleotide comprises an abasic moiety.

21. The method of claim 20, wherein said abasic moiety is present at the 3'-end, 5'-end, or both 3'- and 5'-ends of at least one strand of the double stranded RNA.

22. The method of claim 1, wherein said double stranded RNA comprises a cap structure at the 3'-end, 5'-end, or both 3'- and 5'-ends of at least one strand of the double stranded RNA.

23. The method of claim 22, wherein said cap structure is an inverted nucleotide.

24. The method of claim 22, wherein said cap structure is an inverted abasic moiety.

25. The method of claim 24, wherein said inverted abasic moiety is an inverted deoxyabasic moiety.
